# EUROPEAN PATENT APPLICATION

(11) **EP 3 995 142 A2**
(43) Date of publication of application: **11.05.2022**
(21) Application number: 21199232.6
(22) Date of filing: 29.04.2016
(51) Int. Cl.: A61K 35/17, A61K 39/395, C12N 15/00

(54) **MODIFIED HEMATOPOIETIC STEM/PROGENITOR AND NON-T EFFECTOR CELLS, AND USES THEREOF**

(30) Priority: 29.04.2015 US 201562154565 P
(62) Divisional of application: 16787279.5
(71) Applicant: Fred Hutchinson Cancer Research Center, Seattle, WA 98109 (US)
(72) Inventor: DELANEY, Colleen, Seattle, WA 98109 (US); RIDDELL, Stanley R., Seattle, WA 98109 (US)
(74) Representative: Forresters IP LLP

(57) **Abstract**

Hematopoeitic stem/progenitor cells (HSPC) and/or non-T effector cells are modified to express an extracellular component including a tag cassette. The tag cassette can be used to activate, promote proliferation of, detect, enrich, isolate, track, deplete and/or eliminate modified cells. The cells can also be modified to express a binding domain.

SEQUENCE LISTING

<110>  Fred Hutchinson Cancer Research Center

<120>  MODIFIED HEMATOPOIETIC STEM/PROGENITOR AND NON-T EFFECTOR CELLS,
       AND USES THEREOF

<130>  F053-0020USPCT

<150>  US 62/154,565
<151>  2015-04-29

<160>  157

<170>  PatentIn version 3.5

<210>  1
<211>  126
<212>  DNA
<213>  Homo sapiens

<400>  1
aaacggggca gaaagaaact cctgtatata ttcaaacaac catttatgag accagtacaa      60

actactcaag aggaagatgg ctgtagctgc cgatttccag aagaagaaga aggaggatgt     120

gaactg                                                                126


<210>  2
<211>  135
<212>  DNA
<213>  Homo sapiens

<400>  2
aaacggggca gaaagaaact cctgtatata ttcaaacaac catttatgag accagtacaa      60

actactcaag aggaagatgg ctgtagctgc cgatttccag aagaagaaga aggaggatgt     120

gaactgcggg tgaag                                                      135


<210>  3
<211>  22
<212>  PRT
<213>  Homo sapiens

<400>  3

Met Ala Leu Ile Val Leu Gly Gly Val Ala Gly Leu Leu Leu Phe Ile
1               5                   10                  15


Gly Leu Gly Ile Phe Phe
            20


<210>  4
<211>  45
<212>  PRT
<213>  Homo sapiens

<400>  4

Lys Arg Gly Arg Lys Lys Leu Leu Tyr Ile Phe Lys Gln Pro Phe Met
1               5                   10                  15

Arg Pro Val Gln Thr Thr Gln Glu Glu Asp Gly Cys Ser Cys Arg Phe
            20                  25                  30

Pro Glu Glu Glu Glu Gly Gly Cys Glu Leu Arg Val Lys
        35                  40                  45

<210>  5
<211>  210
<212>  DNA
<213>  Homo sapiens

<400>  5
atgttctggg tgctggtggt ggtgggcggg gtgctggcct gctacagcct gctggtgaca      60

gtggccttca tcatcttttg ggtgaaacgg ggcagaaaga aactcctgta tatattcaaa     120

caaccattta tgagaccagt acaaactact caagaggaag atggctgtag ctgccgattt     180

ccagaagaag aagaaggagg atgtgaactg                                      210

<210>  6
<211>  42
<212>  PRT
<213>  Homo sapiens

<400>  6

Lys Arg Gly Arg Lys Lys Leu Leu Tyr Ile Phe Lys Gln Pro Phe Met
1               5                   10                  15

Arg Pro Val Gln Thr Thr Gln Glu Glu Asp Gly Cys Ser Cys Arg Phe
            20                  25                  30

Pro Glu Glu Glu Glu Gly Gly Cys Glu Leu
        35                  40

<210>  7
<211>  556
<212>  PRT
<213>  Homo sapiens

<400>  7

<210>  8
<211>  21
<212>  DNA
<213>  Artificial Sequence

<220>
<223>  CD19Rop primer

<400>  8
aggaagatat cgccacctac t                                                21

<210>  9
<211>  245
<212>  PRT
<213>  Homo sapiens

<400>  9

Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Ser Lys Tyr
            20                  25                  30

Leu Asn Trp Tyr Gln Gln Lys Pro Asp Gly Thr Val Lys Leu Leu Ile
        35                  40                  45

Tyr His Thr Ser Arg Leu His Ser Gly Val Pro Ser Arg Phe Ser Gly
    50                  55                  60

Ser Gly Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Asn Thr Leu Pro Tyr
                85                  90                  95

Thr Phe Gly Gly Gly Thr Lys Leu Glu Ile Thr Gly Ser Thr Ser Gly
            100                 105                 110

Ser Gly Lys Pro Gly Ser Gly Glu Gly Ser Thr Lys Gly Glu Val Lys
        115                 120                 125

Leu Gln Glu Ser Gly Pro Gly Leu Val Ala Pro Ser Gln Ser Leu Ser
    130                 135                 140

Val Thr Cys Thr Val Ser Gly Val Ser Leu Pro Asp Tyr Gly Val Ser
145                 150                 155                 160

Trp Ile Arg Gln Pro Pro Arg Lys Gly Leu Glu Trp Leu Gly Val Ile
                165                 170                 175

Trp Gly Ser Glu Thr Thr Tyr Tyr Asn Ser Ala Leu Lys Ser Arg Leu
            180                 185                 190

Thr Ile Ile Lys Asp Asn Ser Lys Ser Gln Val Phe Leu Lys Met Asn
        195                 200                 205

Ser Leu Gln Thr Asp Asp Thr Ala Ile Tyr Tyr Cys Ala Lys His Tyr
    210                 215                 220

Tyr Tyr Gly Gly Ser Tyr Ala Met Asp Tyr Trp Gly Gln Gly Thr Ser
225                 230                 235                 240

Val Thr Val Ser Ser
                245

<210>  10
<211>  735
<212>  DNA
<213>  Homo sapiens

<400>  10
gacatccaga tgacccagac cacctccagc ctgagcgcca gcctgggcga ccgggtgacc      60

atcagctgcc gggccagcca ggacatcagc aagtacctga actggtatca gcagaagccc     120

gacggcaccg tcaagctgct gatctaccac accagccggc tgcacagcgg cgtgcccagc     180

cggtttagcg gcagcggctc cggcaccgac tacagcctga ccatctccaa cctggaacag     240

gaagatatcg ccacctactt ttgccagcag ggcaacacac tgccctacac ctttggcggc     300

ggaacaaagc tggaaatcac cggcagcacc tccggcagcg gcaagcctgg cagcggcgag     360

ggcagcacca agggcgaggt gaagctgcag gaaagcggcc ctggcctggt ggcccccagc     420

cagagcctga gcgtgacctg caccgtgagc ggcgtgagcc tgcccgacta cggcgtgagc     480

tggatccggc agccccccag gaagggcctg gaatggctgg gcgtgatctg gggcagcgag     540

accacctact acaacagcgc cctgaagagc cggctgacca tcatcaagga caacagcaag     600

agccaggtgt tcctgaagat gaacagcctg cagaccgacg acaccgccat ctactactgc     660

gccaagcact actactacgg cggcagctac gccatggact actggggcca gggcaccagc     720

gtgaccgtga gcagc                                                      735

<210>  11
<211>  297
<212>  PRT
<213>  Homo sapiens

<400>  11

Met Thr Thr Pro Arg Asn Ser Val Asn Gly Thr Phe Pro Ala Glu Pro
1               5                   10                  15

Met Lys Gly Pro Ile Ala Met Gln Ser Gly Pro Lys Pro Leu Phe Arg
            20                  25                  30

Arg Met Ser Ser Leu Val Gly Pro Thr Gln Ser Phe Phe Met Arg Glu
        35                  40                  45

Ser Lys Thr Leu Gly Ala Val Gln Ile Met Asn Gly Leu Phe His Ile
    50                  55                  60

Ala Leu Gly Gly Leu Leu Met Ile Pro Ala Gly Ile Tyr Ala Pro Ile
65                  70                  75                  80

Cys Val Thr Val Trp Tyr Pro Leu Trp Gly Gly Ile Met Tyr Ile Ile
                85                  90                  95

Ser Gly Ser Leu Leu Ala Ala Thr Glu Lys Asn Ser Arg Lys Cys Leu
            100                 105                 110

Val Lys Gly Lys Met Ile Met Asn Ser Leu Ser Leu Phe Ala Ala Ile
        115                 120                 125

Ser Gly Met Ile Leu Ser Ile Met Asp Ile Leu Asn Ile Lys Ile Ser
    130                 135                 140

His Phe Leu Lys Met Glu Ser Leu Asn Phe Ile Arg Ala His Thr Pro
145                 150                 155                 160

Tyr Ile Asn Ile Tyr Asn Cys Glu Pro Ala Asn Pro Ser Glu Lys Asn
                165                 170                 175

Ser Pro Ser Thr Gln Tyr Cys Tyr Ser Ile Gln Ser Leu Phe Leu Gly
            180                 185                 190

Ile Leu Ser Val Met Leu Ile Phe Ala Phe Phe Gln Glu Leu Val Ile
        195                 200                 205

Ala Gly Ile Val Glu Asn Glu Trp Lys Arg Thr Cys Ser Arg Pro Lys
    210                 215                 220

Ser Asn Ile Val Leu Leu Ser Ala Glu Glu Lys Lys Glu Gln Thr Ile
225                 230                 235                 240

Glu Ile Lys Glu Glu Val Val Gly Leu Thr Glu Thr Ser Ser Gln Pro

245                 250                 255

Lys Asn Glu Glu Asp Ile Glu Ile Ile Pro Ile Gln Glu Glu Glu Glu
            260                 265                 270

Glu Glu Thr Glu Thr Asn Phe Pro Glu Pro Pro Gln Asp Gln Glu Ser
        275                 280                 285

Ser Pro Ile Glu Asn Asp Ser Ser Pro
    290                 295

<210>  12
<211>  84
<212>  DNA
<213>  Homo sapiens

<400>  12
atgttctggg tgctggtggt ggtcggaggc gtgctggcct gctacagcct gctggtcacc      60

gtggccttca tcatcttttg ggtg                                              84

<210>  13
<211>  28
<212>  PRT
<213>  Homo sapiens

<400>  13

Met Phe Trp Val Leu Val Val Val Gly Gly Val Leu Ala Cys Tyr Ser
1               5                   10                  15

Leu Leu Val Thr Val Ala Phe Ile Ile Phe Trp Val
            20                  25

<210>  14
<211>  84
<212>  DNA
<213>  Homo sapiens

<400>  14
atgttctggg tgctggtggt ggtgggcggg gtgctggcct gctacagcct gctggtgaca      60

gtggccttca tcatcttttg ggtg                                              84

<210>  15
<211>  112
<212>  PRT
<213>  Homo sapiens

<400>  15

Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210>  16
<211>  336
<212>  DNA
<213>  Homo sapiens

<400>  16
cgggtgaagt tcagcagaag cgccgacgcc cctgcctacc agcagggcca gaatcagctg      60

tacaacgagc tgaacctggg cagaagggaa gagtacgacg tcctggataa gcggagaggc     120

cgggaccctg agatgggcgg caagcctcgg cggaagaacc cccaggaagg cctgtataac     180

gaactgcaga aagacaagat ggccgaggcc tacagcgaga tcggcatgaa gggcgagcgg     240

aggcggggca agggccacga cggcctgtat cagggcctgt ccaccgccac caaggatacc     300

tacgacgccc tgcacatgca ggccctgccc ccaagg                               336

<210>  17
<211>  109
<212>  PRT
<213>  Homo sapiens

<400>  17

Phe Ser Arg Ser Ala Asp Ala Pro Ala Tyr Gln Gln Gly Gln Asn Gln
1               5                   10                  15

Leu Tyr Asn Glu Leu Asn Leu Gly Arg Arg Glu Glu Tyr Asp Val Leu
            20                  25                  30

Asp Lys Arg Arg Gly Arg Asp Pro Glu Met Gly Gly Lys Pro Arg Arg
        35                  40                  45

Lys Asn Pro Gln Glu Gly Leu Tyr Asn Glu Leu Gln Lys Asp Lys Met

50                  55                  60

Ala Glu Ala Tyr Ser Glu Ile Gly Met Lys Gly Glu Arg Arg Arg Gly
65                  70                  75                  80

Lys Gly His Asp Gly Leu Tyr Gln Gly Leu Ser Thr Ala Thr Lys Asp
                85                  90                  95

Thr Tyr Asp Ala Leu His Met Gln Ala Leu Pro Pro Arg
            100                 105

<210>  18
<211>  327
<212>  DNA
<213>  Homo sapiens

<400>  18
ttcagcagaa gcgccgacgc ccctgcctac cagcagggcc agaatcagct gtacaacgag      60

ctgaacctgg gcagaaggga agagtacgac gtcctggata agcggagagg ccgggaccct     120

gagatgggcg gcaagcctcg gcggaagaac ccccaggaag gcctgtataa cgaactgcag     180

aaagacaaga tggccgaggc ctacagcgag atcggcatga agggcgagcg gaggcggggc     240

aagggccacg acggcctgta tcagggcctg tccaccgcca ccaaggatac ctacgacgcc     300

ctgcacatgc aggccctgcc cccaagg                                         327

<210>  19
<211>  110
<212>  PRT
<213>  Homo sapiens

<400>  19

Ala Pro Glu Phe Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser Gln Glu Asp Pro Glu Val Gln Phe Asn Trp Tyr
        35                  40                  45

Val Asp Gly Val Glu Val His Asn Ala Lys Thr Lys Pro Arg Glu Glu
    50                  55                  60

Gln Phe Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Gly Leu Pro Ser Ser Ile Glu Lys Thr Ile Ser Lys Ala Lys
            100                 105                 110

<210>  20
<211>  330
<212>  DNA
<213>  Homo sapiens

<400>  20
gcccccgagt tcctgggcgg acccagcgtg ttcctgttcc cccccaagcc caaggacacc      60

ctgatgatca gccggacccc cgaggtgacc tgcgtggtgg tggacgtgag ccaggaagat     120

cccgaggtcc agttcaattg gtacgtggac ggcgtggaag tgcacaacgc caagaccaag     180

cccagagagg aacagttcaa cagcacctac cgggtggtgt ctgtgctgac cgtgctgcac     240

caggactggc tgaacggcaa agaatacaag tgcaaggtgt ccaacaaggg cctgcccagc     300

agcatcgaaa agaccatcag caaggccaag                                      330

<210>  21
<211>  321
<212>  DNA
<213>  Homo sapiens

<400>  21
ggccagcctc gcgagcccca ggtgtacacc ctgcctccct cccaggaaga gatgaccaag      60

aaccaggtgt ccctgacctg cctggtgaag ggcttctacc ccagcgacat cgccgtggag     120

tgggagagca acggccagcc tgagaacaac tacaagacca cccctcccgt gctggacagc     180

gacggcagct tcttcctgta cagccggctg accgtggaca agagccggtg gcaggaaggc     240

aacgtcttta gctgcagcgt gatgcacgag gccctgcaca accactacac ccagaagagc     300

ctgagcctgt ccctgggcaa g                                               321

<210>  22
<211>  107
<212>  PRT
<213>  Homo sapiens

<400>  22

Gly Gln Pro Arg Glu Pro Gln Val Tyr Thr Leu Pro Pro Ser Gln Glu
1               5                   10                  15

Glu Met Thr Lys Asn Gln Val Ser Leu Thr Cys Leu Val Lys Gly Phe
            20                  25                  30

Tyr Pro Ser Asp Ile Ala Val Glu Trp Glu Ser Asn Gly Gln Pro Glu
        35                  40                  45

Asn Asn Tyr Lys Thr Thr Pro Pro Val Leu Asp Ser Asp Gly Ser Phe
    50                  55                  60

Phe Leu Tyr Ser Arg Leu Thr Val Asp Lys Ser Arg Trp Gln Glu Gly
65                  70                  75                  80

Asn Val Phe Ser Cys Ser Val Met His Glu Ala Leu His Asn His Tyr
                85                  90                  95

Thr Gln Lys Ser Leu Ser Leu Ser Leu Gly Lys
            100                 105

<210>  23
<211>  18
<212>  DNA
<213>  Artificial Sequence

<220>
<223>  CMV primer

<400>  23
tagcggtttg actcacgg                                                    18

<210>  24
<211>  18
<212>  DNA
<213>  Artificial Sequence

<220>
<223>  CoE1 ori primer

<400>  24
caggtatccg gtaagcgg                                                    18

<210>  25
<211>  26
<212>  DNA
<213>  Artificial Sequence

<220>
<223>  delU3 primer

<400>  25
ccgtaccttt aagaccaatg acttac                                           26

<210>  26
<211>  16
<212>  DNA
<213>  Artificial Sequence

<220>
<223>  EF1p primer

<400>  26
tcgcaacggg tttgcc                                                      16

<210>  27
<211>  1074
<212>  DNA
<213>  Homo sapiens

<400>  27
atgcttctcc tggtgacaag ccttctgctc tgtgagttac cacacccagc attcctcctg      60

atcccacgca aagtgtgtaa cggaataggt attggtgaat ttaaagactc actctccata     120

aatgctacga atattaaaca cttcaaaaac tgcacctcca tcagtggcga tctccacatc     180

ctgccggtgg catttagggg tgactccttc acacatactc ctcctctgga tccacaggaa     240

ctggatattc tgaaaaccgt aaaggaaatc acagggtttt tgctgattca ggcttggcct     300

gaaaacagga cggacctcca tgcctttgag aacctagaaa tcatacgcgg caggaccaag     360

caacatggtc agttttctct tgcagtcgtc agcctgaaca taacatcctt gggattacgc     420

tccctcaagg agataagtga tggagatgtg ataatttcag gaaacaaaaa tttgtgctat     480

gcaaatacaa taaactggaa aaaactgttt gggacctccg gtcagaaaac caaaattata     540

agcaacagag gtgaaaacag ctgcaaggcc acaggccagg tctgccatgc cttgtgctcc     600

cccgagggct gctggggccc ggagcccagg gactgcgtct cttgccggaa tgtcagccga     660

ggcagggaat gcgtggacaa gtgcaacctt ctggagggtg agccaaggga gtttgtggag     720

aactctgagt gcatacagtg ccacccagag tgcctgcctc aggccatgaa catcacctgc     780

acaggacggg gaccagacaa ctgtatccag tgtgcccact acattgacgg cccccactgc     840

gtcaagacct gcccggcagg agtcatggga gaaaacaaca ccctggtctg gaagtacgca     900

gacgccggcc atgtgtgcca cctgtgccat ccaaactgca cctacggatg cactgggcca     960

ggtcttgaag gctgtccaac gaatgggcct aagatcccgt ccatcgccac tgggatggtg    1020

ggggccctcc tcttgctgct ggtggtggcc ctggggatcg gcctcttcat gtga          1074

<210>  28
<211>  357
<212>  PRT
<213>  Homo sapiens

<400>  28

Met Leu Leu Leu Val Thr Ser Leu Leu Leu Cys Glu Leu Pro His Pro
1               5                   10                  15

Ala Phe Leu Leu Ile Pro Arg Lys Val Cys Asn Gly Ile Gly Ile Gly
            20                  25                  30

Glu Phe Lys Asp Ser Leu Ser Ile Asn Ala Thr Asn Ile Lys His Phe
        35                  40                  45

Lys Asn Cys Thr Ser Ile Ser Gly Asp Leu His Ile Leu Pro Val Ala
    50                  55                  60

Phe Arg Gly Asp Ser Phe Thr His Thr Pro Pro Leu Asp Pro Gln Glu
65                  70                  75                  80

Leu Asp Ile Leu Lys Thr Val Lys Glu Ile Thr Gly Phe Leu Leu Ile
                85                  90                  95

Gln Ala Trp Pro Glu Asn Arg Thr Asp Leu His Ala Phe Glu Asn Leu
            100                 105                 110

Glu Ile Ile Arg Gly Arg Thr Lys Gln His Gly Gln Phe Ser Leu Ala
        115                 120                 125

Val Val Ser Leu Asn Ile Thr Ser Leu Gly Leu Arg Ser Leu Lys Glu
    130                 135                 140

Ile Ser Asp Gly Asp Val Ile Ile Ser Gly Asn Lys Asn Leu Cys Tyr
145                 150                 155                 160

Ala Asn Thr Ile Asn Trp Lys Lys Leu Phe Gly Thr Ser Gly Gln Lys
                165                 170                 175

Thr Lys Ile Ile Ser Asn Arg Gly Glu Asn Ser Cys Lys Ala Thr Gly
            180                 185                 190

Gln Val Cys His Ala Leu Cys Ser Pro Glu Gly Cys Trp Gly Pro Glu
        195                 200                 205

Pro Arg Asp Cys Val Ser Cys Arg Asn Val Ser Arg Gly Arg Glu Cys
    210                 215                 220

Val Asp Lys Cys Asn Leu Leu Glu Gly Glu Pro Arg Glu Phe Val Glu
225                 230                 235                 240

Asn Ser Glu Cys Ile Gln Cys His Pro Glu Cys Leu Pro Gln Ala Met
                245                 250                 255

Asn Ile Thr Cys Thr Gly Arg Gly Pro Asp Asn Cys Ile Gln Cys Ala
            260                 265                 270

His Tyr Ile Asp Gly Pro His Cys Val Lys Thr Cys Pro Ala Gly Val
        275                 280                 285

Met Gly Glu Asn Asn Thr Leu Val Trp Lys Tyr Ala Asp Ala Gly His
    290                 295                 300

Val Cys His Leu Cys His Pro Asn Cys Thr Tyr Gly Cys Thr Gly Pro
305                 310                 315                 320

Gly Leu Glu Gly Cys Pro Thr Asn Gly Pro Lys Ile Pro Ser Ile Ala
                325                 330                 335

Thr Gly Met Val Gly Ala Leu Leu Leu Leu Leu Val Val Ala Leu Gly
            340                 345                 350

Ile Gly Leu Phe Met
        355

<210>  29
<211>  20
<212>  DNA
<213>  Artificial Sequence

<220>
<223>  EGFRt primer

<400>  29
atgcttctcc tggtgacaag                                                   20

<210>  30
<211>  18
<212>  PRT
<213>  Artificial Sequence

<220>
<223>  Flexible Linker

<400>  30

Gly Ser Thr Ser Gly Ser Gly Lys Pro Gly Ser Gly Glu Gly Ser Thr
1               5                   10                  15

Lys Gly

<210>  31
<211>  66
<212>  DNA
<213>  Homo sapiens

<400>  31
atgctgctgc tggtgaccag cctgctgctg tgcgagctgc cccaccccgc ctttctgctg      60

atcccc                                                                  66

<210>  32
<211>  22
<212>  PRT
<213>  Artificial Sequence

<220>
<223>  GMCSFRss

<400>  32

Met Leu Leu Leu Val Thr Ser Leu Leu Leu Cys Glu Leu Pro His Pro
1               5                   10                  15

Ala Phe Leu Leu Ile Pro
            20

<210>  33
<211>  2529
<212>  DNA
<213>  Artificial Sequence

<220>
<223>  GMCSFRss-CD19scFv-IgG4hinge-CD28tm-41BB-Zeta-T2A-EGFRt

<400>  33
atgctgctgc tggtgaccag cctgctgctg tgcgagctgc cccaccccgc ctttctgctg      60

atccccgaca tccagatgac ccagaccacc tccagcctga gcgccagcct gggcgaccgg     120

gtgaccatca gctgccgggc cagccaggac atcagcaagt acctgaactg gtatcagcag     180

aagcccgacg gcaccgtcaa gctgctgatc taccacacca gccggctgca cagcggcgtg     240

cccagccggt ttagcggcag cggctccggc accgactaca gcctgaccat ctccaacctg     300

gaacaggaag atatcgccac ctacttttgc cagcagggca acacactgcc ctacaccttt     360

ggcggcggaa caaagctgga aatcaccggc agcacctccg gcagcggcaa gcctggcagc     420

ggcgagggca gcaccaaggg cgaggtgaag ctgcaggaaa gcggccctgg cctggtggcc     480

cccagccaga gcctgagcgt gacctgcacc gtgagcggcg tgagcctgcc cgactacggc     540

gtgagctgga tccggcagcc ccccaggaag ggcctggaat ggctgggcgt gatctggggc     600

agcgagacca cctactacaa cagcgccctg aagagccggc tgaccatcat caaggacaac     660

agcaagagcc aggtgttcct gaagatgaac agcctgcaga ccgacgacac cgccatctac     720

tactgcgcca agcactacta ctacggcggc agctacgcca tggactactg gggccagggc     780

accagcgtga ccgtgagcag cgagagcaag tacggaccgc cctgcccccc ttgccctatg     840

ttctgggtgc tggtggtggt cggaggcgtg ctggcctgct acagcctgct ggtcaccgtg     900

gccttcatca tcttttgggt gaaacggggc agaaagaaac tcctgtatat attcaaacaa     960

ccatttatga gaccagtaca aactactcaa gaggaagatg gctgtagctg ccgatttcca    1020

gaagaagaag aaggaggatg tgaactgcgg gtgaagttca gcagaagcgc cgacgcccct    1080

gcctaccagc agggccagaa tcagctgtac aacgagctga acctgggcag aagggaagag    1140

tacgacgtcc tggataagcg gagaggccgg gaccctgaga tgggcggcaa gcctcggcgg    1200

aagaaccccc aggaaggcct gtataacgaa ctgcagaaag acaagatggc cgaggcctac    1260

agcgagatcg gcatgaaggg cgagcggagg cggggcaagg gccacgacgg cctgtatcag 1320

ggcctgtcca ccgccaccaa ggatacctac gacgccctgc acatgcaggc cctgccccca 1380

aggctcgagg gcggcggaga gggcagagga agtcttctaa catgcggtga cgtggaggag 1440

aatcccggcc ctaggatgct tctcctggtg acaagccttc tgctctgtga gttaccacac 1500

ccagcattcc tcctgatccc acgcaaagtg tgtaacggaa taggtattgg tgaatttaaa 1560

gactcactct ccataaatgc tacgaatatt aaacacttca aaaactgcac ctccatcagt 1620

ggcgatctcc acatcctgcc ggtggcattt aggggtgact ccttcacaca tactcctcct 1680

ctggatccac aggaactgga tattctgaaa accgtaaagg aaatcacagg gtttttgctg 1740

attcaggctt ggcctgaaaa caggacggac ctccatgcct ttgagaacct agaaatcata 1800

cgcggcagga ccaagcaaca tggtcagttt tctcttgcag tcgtcagcct gaacataaca 1860

tccttgggat tacgctccct caaggagata agtgatggag atgtgataat ttcaggaaac 1920

aaaaatttgt gctatgcaaa tacaataaac tggaaaaaac tgtttgggac ctccggtcag 1980

aaaaccaaaa ttataagcaa cagaggtgaa aacagctgca aggccacagg ccaggtctgc 2040

catgccttgt gctcccccga gggctgctgg ggcccggagc ccagggactg cgtctcttgc 2100

cggaatgtca gccgaggcag ggaatgcgtg gacaagtgca accttctgga gggtgagcca 2160

agggagtttg tggagaactc tgagtgcata cagtgccacc cagagtgcct gcctcaggcc 2220

atgaacatca cctgcacagg acggggacca gacaactgta tccagtgtgc ccactacatt 2280

gacggccccc actgcgtcaa gacctgcccg gcaggagtca tgggagaaaa caacaccctg 2340

gtctggaagt acgcagacgc cggccatgtg tgccacctgt gccatccaaa ctgcacctac 2400

ggatgcactg ggccaggtct tgaaggctgt ccaacgaatg ggcctaagat cccgtccatc 2460

gccactggga tggtgggggc cctcctcttg ctgctggtgg tggccctggg gatcggcctc 2520

ttcatgtga 2529

<210> 34
<211> 842
<212> PRT
<213> Artificial Sequence

<220>
<223> GMCSFRss-CD19scFv-IgG4hinge-CD28tm-41BB-Zeta-T2A-EGFRt

<400> 34

Met Leu Leu Leu Val Thr Ser Leu Leu Leu Cys Glu Leu Pro His Pro
1               5                   10                  15

Ala Phe Leu Leu Ile Pro Asp Ile Gln Met Thr Gln Thr Thr Ser Ser
            20                  25                  30

Leu Ser Ala Ser Leu Gly Asp Arg Val Thr Ile Ser Cys Arg Ala Ser
        35                  40                  45

Gln Asp Ile Ser Lys Tyr Leu Asn Trp Tyr Gln Gln Lys Pro Asp Gly
    50                  55                  60

Thr Val Lys Leu Leu Ile Tyr His Thr Ser Arg Leu His Ser Gly Val
65                  70                  75                  80

Pro Ser Arg Phe Ser Gly Ser Gly Ser Gly Thr Asp Tyr Ser Leu Thr
                85                  90                  95

Ile Ser Asn Leu Glu Gln Glu Asp Ile Ala Thr Tyr Phe Cys Gln Gln
            100                 105                 110

Gly Asn Thr Leu Pro Tyr Thr Phe Gly Gly Gly Thr Lys Leu Glu Ile
        115                 120                 125

Thr Gly Ser Thr Ser Gly Ser Gly Lys Pro Gly Ser Gly Glu Gly Ser
    130                 135                 140

Thr Lys Gly Glu Val Lys Leu Gln Glu Ser Gly Pro Gly Leu Val Ala
145                 150                 155                 160

Pro Ser Gln Ser Leu Ser Val Thr Cys Thr Val Ser Gly Val Ser Leu
                165                 170                 175

Pro Asp Tyr Gly Val Ser Trp Ile Arg Gln Pro Pro Arg Lys Gly Leu
            180                 185                 190

Glu Trp Leu Gly Val Ile Trp Gly Ser Glu Thr Thr Tyr Tyr Asn Ser
        195                 200                 205

Ala Leu Lys Ser Arg Leu Thr Ile Ile Lys Asp Asn Ser Lys Ser Gln
    210                 215                 220

Val Phe Leu Lys Met Asn Ser Leu Gln Thr Asp Asp Thr Ala Ile Tyr
225                 230                 235                 240

Tyr Cys Ala Lys His Tyr Tyr Tyr Gly Gly Ser Tyr Ala Met Asp Tyr
                245                 250                 255

Trp Gly Gln Gly Thr Ser Val Thr Val Ser Ser Glu Ser Lys Tyr Gly
            260                 265                 270

Pro Pro Cys Pro Pro Cys Pro Met Phe Trp Val Leu Val Val Val Gly
        275                 280                 285

Gly Val Leu Ala Cys Tyr Ser Leu Leu Val Thr Val Ala Phe Ile Ile
    290                 295                 300

Phe Trp Val Lys Arg Gly Arg Lys Lys Leu Leu Tyr Ile Phe Lys Gln
305                 310                 315                 320

Pro Phe Met Arg Pro Val Gln Thr Thr Gln Glu Glu Asp Gly Cys Ser
                325                 330                 335

Cys Arg Phe Pro Glu Glu Glu Glu Gly Gly Cys Glu Leu Arg Val Lys
            340                 345                 350

Phe Ser Arg Ser Ala Asp Ala Pro Ala Tyr Gln Gln Gly Gln Asn Gln
        355                 360                 365

Leu Tyr Asn Glu Leu Asn Leu Gly Arg Arg Glu Glu Tyr Asp Val Leu
    370                 375                 380

Asp Lys Arg Arg Gly Arg Asp Pro Glu Met Gly Gly Lys Pro Arg Arg
385                 390                 395                 400

Lys Asn Pro Gln Glu Gly Leu Tyr Asn Glu Leu Gln Lys Asp Lys Met
                405                 410                 415

Ala Glu Ala Tyr Ser Glu Ile Gly Met Lys Gly Glu Arg Arg Arg Gly
            420                 425                 430

Lys Gly His Asp Gly Leu Tyr Gln Gly Leu Ser Thr Ala Thr Lys Asp
        435                 440                 445

Thr Tyr Asp Ala Leu His Met Gln Ala Leu Pro Pro Arg Leu Glu Gly
    450                 455                 460

Gly Gly Glu Gly Arg Gly Ser Leu Leu Thr Cys Gly Asp Val Glu Glu
465                 470                 475                 480

Asn Pro Gly Pro Arg Met Leu Leu Leu Val Thr Ser Leu Leu Leu Cys
                485                 490                 495

Glu Leu Pro His Pro Ala Phe Leu Leu Ile Pro Arg Lys Val Cys Asn
            500                 505                 510

Gly Ile Gly Ile Gly Glu Phe Lys Asp Ser Leu Ser Ile Asn Ala Thr
        515                 520                 525

Asn Ile Lys His Phe Lys Asn Cys Thr Ser Ile Ser Gly Asp Leu His

530                 535                 540

Ile Leu Pro Val Ala Phe Arg Gly Asp Ser Phe Thr His Thr Pro Pro
545                 550                 555                 560

Leu Asp Pro Gln Glu Leu Asp Ile Leu Lys Thr Val Lys Glu Ile Thr
                565                 570                 575

Gly Phe Leu Leu Ile Gln Ala Trp Pro Glu Asn Arg Thr Asp Leu His
            580                 585                 590

Ala Phe Glu Asn Leu Glu Ile Ile Arg Gly Arg Thr Lys Gln His Gly
        595                 600                 605

Gln Phe Ser Leu Ala Val Val Ser Leu Asn Ile Thr Ser Leu Gly Leu
    610                 615                 620

Arg Ser Leu Lys Glu Ile Ser Asp Gly Asp Val Ile Ile Ser Gly Asn
625                 630                 635                 640

Lys Asn Leu Cys Tyr Ala Asn Thr Ile Asn Trp Lys Lys Leu Phe Gly
                645                 650                 655

Thr Ser Gly Gln Lys Thr Lys Ile Ile Ser Asn Arg Gly Glu Asn Ser
            660                 665                 670

Cys Lys Ala Thr Gly Gln Val Cys His Ala Leu Cys Ser Pro Glu Gly
        675                 680                 685

Cys Trp Gly Pro Glu Pro Arg Asp Cys Val Ser Cys Arg Asn Val Ser
    690                 695                 700

Arg Gly Arg Glu Cys Val Asp Lys Cys Asn Leu Leu Glu Gly Glu Pro
705                 710                 715                 720

Arg Glu Phe Val Glu Asn Ser Glu Cys Ile Gln Cys His Pro Glu Cys
                725                 730                 735

Leu Pro Gln Ala Met Asn Ile Thr Cys Thr Gly Arg Gly Pro Asp Asn
            740                 745                 750

Cys Ile Gln Cys Ala His Tyr Ile Asp Gly Pro His Cys Val Lys Thr
        755                 760                 765

Cys Pro Ala Gly Val Met Gly Glu Asn Asn Thr Leu Val Trp Lys Tyr
    770                 775                 780

Ala Asp Ala Gly His Val Cys His Leu Cys His Pro Asn Cys Thr Tyr
785                 790                 795                 800

Gly Cys Thr Gly Pro Gly Leu Glu Gly Cys Pro Thr Asn Gly Pro Lys
                805                 810                 815

Ile Pro Ser Ile Ala Thr Gly Met Val Gly Ala Leu Leu Leu Leu Leu
            820                 825                 830

Val Val Ala Leu Gly Ile Gly Leu Phe Met
        835                 840

<210>  35
<211>  66
<212>  DNA
<213>  Artificial Sequence

<220>
<223>  GMCSFss Leader

<400>  35
atgcttctcc tggtgacaag ccttctgctc tgtgagttac cacacccagc attcctcctg      60

atccca                                                                  66

<210>  36
<211>  2529
<212>  DNA
<213>  Artificial Sequence

<220>
<223>  GMCSFss-Her2scFv-IgG4hinge-CD28tm-41BB-Zeta-T2A-EGFRt

<400>  36
atgcttctcc tggtgacaag ccttctgctc tgtgagttac cacacccagc attcctcctg      60

atcccagata tccagatgac ccagtccccg agctccctgt ccgcctctgt gggcgatagg     120

gtcaccatca cctgccgtgc cagtcaggat gtgaatactg ctgtagcctg gtatcaacag     180

aaaccaggaa aagctccgaa actactgatt tactcggcat ccttcctcta ctctggagtc     240

ccttctcgct tctctggttc cagatctggg acggatttca ctctgaccat cagcagtctg     300

cagccggaag acttcgcaac ttattactgt cagcaacatt atactactcc tcccacgttc     360

ggacagggta ccaaggtgga gatcaaaggc agtactagcg gcggtggctc cgggggcgga     420

tccggtgggg gcggcagcag cgaggttcag ctggtggagt ctggcggtgg cctggtgcag     480

ccagggggct cactccgttt gtcctgtgca gcttctggct tcaacattaa agacacctat     540

atacactggg tgcgtcaggc cccgggtaag ggcctggaat gggttgcaag gatttatcct     600

acgaatggtt atactagata tgccgatagc gtcaagggcc gtttcactat aagcgcagac     660

acatccaaaa acacagccta cctgcagatg aacagcctgc gtgctgagga cactgccgtc     720

tattattgtt ctagatgggg aggggacggc ttctatgcta tggactactg gggtcaagga 780

accctggtca ccgtctcgag tgagagcaag tacggaccgc cctgcccccc ttgccctatg 840

ttctgggtgc tggtggtggt cggaggcgtg ctggcctgct acagcctgct ggtcaccgtg 900

gccttcatca tcttttgggt gaaacggggc agaaagaaac tcctgtatat attcaaacaa 960

ccatttatga gaccagtaca aactactcaa gaggaagatg gctgtagctg ccgatttcca 1020

gaagaagaag aaggaggatg tgaactgcgg gtgaagttca gcagaagcgc cgacgcccct 1080

gcctaccagc agggccagaa tcagctgtac aacgagctga acctgggcag aagggaagag 1140

tacgacgtcc tggataagcg gagaggccgg gaccctgaga tgggcggcaa gcctcggcgg 1200

aagaaccccc aggaaggcct gtataacgaa ctgcagaaag acaagatggc cgaggcctac 1260

agcgagatcg gcatgaaggg cgagcggagg cggggcaagg gccacgacgg cctgtatcag 1320

ggcctgtcca ccgccaccaa ggatacctac gacgccctgc acatgcaggc cctgccccca 1380

aggctcgagg gcggcggaga gggcagagga agtcttctaa catgcggtga cgtggaggag 1440

aatcccggcc ctaggatgct tctcctggtg acaagccttc tgctctgtga gttaccacac 1500

ccagcattcc tcctgatccc acgcaaagtg tgtaacggaa taggtattgg tgaatttaaa 1560

gactcactct ccataaatgc tacgaatatt aaacacttca aaaactgcac ctccatcagt 1620

ggcgatctcc acatcctgcc ggtggcattt aggggtgact ccttcacaca tactcctcct 1680

ctggatccac aggaactgga tattctgaaa accgtaaagg aaatcacagg gtttttgctg 1740

attcaggctt ggcctgaaaa caggacggac ctccatgcct ttgagaacct agaaatcata 1800

cgcggcagga ccaagcaaca tggtcagttt tctcttgcag tcgtcagcct gaacataaca 1860

tccttgggat tacgctccct caaggagata agtgatggag atgtgataat ttcaggaaac 1920

aaaaatttgt gctatgcaaa tacaataaac tggaaaaaac tgtttgggac ctccggtcag 1980

aaaaccaaaa ttataagcaa cagaggtgaa aacagctgca aggccacagg ccaggtctgc 2040

catgccttgt gctcccccga gggctgctgg ggcccggagc ccagggactg cgtctcttgc 2100

cggaatgtca gccgaggcag ggaatgcgtg gacaagtgca accttctgga gggtgagcca 2160

agggagtttg tggagaactc tgagtgcata cagtgccacc cagagtgcct gcctcaggcc 2220

atgaacatca cctgcacagg acggggacca gacaactgta tccagtgtgc ccactacatt 2280

gacggccccc actgcgtcaa gacctgcccg gcaggagtca tgggagaaaa caacaccctg 2340

gtctggaagt acgcagacgc cggccatgtg tgccacctgt gccatccaaa ctgcacctac 2400

ggatgcactg ggccaggtct tgaaggctgt ccaacgaatg ggcctaagat cccgtccatc 2460

gccactggga tggtgggggc cctcctcttg ctgctggtgg tggccctggg gatcggcctc 2520

ttcatgtga 2529

<210> 37
<211> 2850
<212> DNA
<213> Artificial Sequence

<220>
<223> Her 2 construct-intermediate spacer

<400> 37
atgcttctcc tggtgacaag ccttctgctc tgtgagttac cacacccagc attcctcctg 60

atcccagata tccagatgac ccagtccccg agctccctgt ccgcctctgt gggcgatagg 120

gtcaccatca cctgccgtgc cagtcaggat gtgaatactg ctgtagcctg gtatcaacag 180

aaaccaggaa aagctccgaa actactgatt tactcggcat ccttcctcta ctctggagtc 240

ccttctcgct tctctggttc cagatctggg acggatttca ctctgaccat cagcagtctg 300

cagccggaag acttcgcaac ttattactgt cagcaacatt atactactcc tcccacgttc 360

ggacagggta ccaaggtgga gatcaaaggc agtactagcg gcggtggctc cgggggcgga 420

tccggtgggg gcggcagcag cgaggttcag ctggtggagt ctggcggtgg cctggtgcag 480

ccagggggct cactccgttt gtcctgtgca gcttctggct tcaacattaa agacacctat 540

atacactggg tgcgtcaggc cccgggtaag ggcctggaat gggttgcaag gatttatcct 600

acgaatggtt atactagata tgccgatagc gtcaagggcc gtttcactat aagcgcagac 660

acatccaaaa acacagccta cctgcagatg aacagcctgc gtgctgagga cactgccgtc 720

tattattgtt ctagatgggg aggggacggc ttctatgcta tggactactg gggtcaagga 780

accctggtca ccgtctcgag tgagagcaag tacggaccgc cctgcccccc ttgccctggc 840

cagcctagag aaccccaggt gtacaccctg cctcccagcc aggaagagat gaccaagaac 900

caggtgtccc tgacctgcct ggtcaaaggc ttctacccca gcgatatcgc cgtggaatgg 960

gagagcaacg gccagcccga gaacaactac aagaccaccc cccctgtgct ggacagcgac 1020

ggcagcttct tcctgtactc ccggctgacc gtggacaaga gccggtggca ggaaggcaac 1080

gtcttcagct gcagcgtgat gcacgaggcc ctgcacaacc actacaccca gaagtccctg 1140

agcctgagcc tgggcaagat gttctgggtg ctggtggtgg tcggaggcgt gctggcctgc 1200

tacagcctgc tggtcaccgt ggccttcatc atcttttggg tgaaacgggg cagaaagaaa 1260

ctcctgtata tattcaaaca accatttatg agaccagtac aaactactca agaggaagat 1320

ggctgtagct gccgatttcc agaagaagaa gaaggaggat gtgaactgcg ggtgaagttc 1380

agcagaagcg ccgacgcccc tgcctaccag cagggccaga atcagctgta caacgagctg 1440

aacctgggca gaagggaaga gtacgacgtc ctggataagc ggagaggccg ggaccctgag 1500

atgggcggca agcctcggcg gaagaacccc caggaaggcc tgtataacga actgcagaaa 1560

gacaagatgg ccgaggccta cagcgagatc ggcatgaagg gcgagcggag gcggggcaag 1620

ggccacgacg gcctgtatca gggcctgtcc accgccacca aggataccta cgacgccctg 1680

cacatgcagg ccctgccccc aaggctcgag ggcggcggag agggcagagg aagtcttcta 1740

acatgcggtg acgtggagga gaatcccggc cctaggatgc ttctcctggt gacaagcctt 1800

ctgctctgtg agttaccaca cccagcattc ctcctgatcc cacgcaaagt gtgtaacgga 1860

ataggtattg gtgaatttaa agactcactc tccataaatg ctacgaatat taaacacttc 1920

aaaaactgca cctccatcag tggcgatctc cacatcctgc cggtggcatt taggggtgac 1980

tccttcacac atactcctcc tctggatcca caggaactgg atattctgaa aaccgtaaag 2040

gaaatcacag ggtttttgct gattcaggct tggcctgaaa acaggacgga cctccatgcc 2100

tttgagaacc tagaaatcat acgcggcagg accaagcaac atggtcagtt ttctcttgca 2160

gtcgtcagcc tgaacataac atccttggga ttacgctccc tcaaggagat aagtgatgga 2220

gatgtgataa tttcaggaaa caaaaatttg tgctatgcaa atacaataaa ctggaaaaaa 2280

ctgtttggga cctccggtca gaaaaccaaa attataagca acagaggtga aaacagctgc 2340

aaggccacag gccaggtctg ccatgccttg tgctcccccg agggctgctg gggcccggag 2400

cccagggact gcgtctcttg ccggaatgtc agccgaggca gggaatgcgt ggacaagtgc 2460

aaccttctgg agggtgagcc aagggagttt gtggagaact ctgagtgcat acagtgccac 2520

ccagagtgcc tgcctcaggc catgaacatc acctgcacag gacggggacc agacaactgt 2580

atccagtgtg cccactacat tgacggcccc cactgcgtca agacctgccc ggcaggagtc 2640

atgggagaaa acaacaccct ggtctggaag tacgcagacg ccggccatgt gtgccacctg 2700

tgccatccaa actgcaccta cggatgcact gggccaggtc ttgaaggctg tccaacgaat 2760

gggcctaaga tcccgtccat cgccactggg atggtggggg ccctcctctt gctgctggtg 2820

gtggccctgg ggatcggcct cttcatgtga 2850

<210> 38
<211> 3180
<212> DNA
<213> Artificial Sequence

<220>
<223> Her 2 construct-long spacer

<400> 38
atgcttctcc tggtgacaag ccttctgctc tgtgagttac cacacccagc attcctcctg 60

atcccagata tccagatgac ccagtccccg agctccctgt ccgcctctgt gggcgatagg 120

gtcaccatca cctgccgtgc cagtcaggat gtgaatactg ctgtagcctg gtatcaacag 180

aaaccaggaa aagctccgaa actactgatt tactcggcat ccttcctcta ctctggagtc 240

ccttctcgct tctctggttc cagatctggg acggatttca ctctgaccat cagcagtctg 300

cagccggaag acttcgcaac ttattactgt cagcaacatt atactactcc tcccacgttc 360

ggacagggta ccaaggtgga gatcaaaggc agtactagcg gcggtggctc cgggggcgga 420
tccggtgggg gcggcagcag cgaggttcag ctggtggagt ctggcggtgg cctggtgcag 480
ccagggggct cactccgttt gtcctgtgca gcttctggct tcaacattaa agacacctat 540
atacactggg tgcgtcaggc cccgggtaag ggcctggaat gggttgcaag gatttatcct 600
acgaatggtt atactagata tgccgatagc gtcaagggcc gtttcactat aagcgcagac 660
acatccaaaa acacagccta cctgcagatg aacagcctgc gtgctgagga cactgccgtc 720
tattattgtt ctagatgggg aggggacggc ttctatgcta tggactactg gggtcaagga 780
accctggtca ccgtctcgag tgagagcaag tacggaccgc cctgcccccc ttgccctgcc 840
cccgagttcc tgggcggacc cagcgtgttc ctgttccccc ccaagcccaa ggacaccctg 900
atgatcagcc ggacccccga ggtgacctgc gtggtggtgg acgtgagcca ggaagatccc 960
gaggtccagt tcaattggta cgtggacggc gtggaagtgc acaacgccaa gaccaagccc 1020
agagaggaac agttcaacag cacctaccgg gtggtgtctg tgctgaccgt gctgcaccag 1080
gactggctga acggcaaaga atacaagtgc aaggtgtcca acaagggcct gcccagcagc 1140
atcgaaaaga ccatcagcaa ggccaagggc cagcctcgcg agccccaggt gtacaccctg 1200
cctccctccc aggaagagat gaccaagaac caggtgtccc tgacctgcct ggtgaagggc 1260
ttctacccca gcgacatcgc cgtggagtgg gagagcaacg gccagcctga gaacaactac 1320
aagaccaccc ctcccgtgct ggacagcgac ggcagcttct tcctgtacag ccggctgacc 1380
gtggacaaga gccggtggca ggaaggcaac gtctttagct gcagcgtgat gcacgaggcc 1440
ctgcacaacc actacaccca gaagagcctg agcctgtccc tgggcaagat gttctgggtg 1500
ctggtggtgg tgggcggggt gctggcctgc tacagcctgc tggtgacagt ggccttcatc 1560
atcttttggg tgaaacgggg cagaaagaaa ctcctgtata tattcaaaca accatttatg 1620
agaccagtac aaactactca agaggaagat ggctgtagct gccgatttcc agaagaagaa 1680
gaaggaggat gtgaactgcg ggtgaagttc agcagaagcg ccgacgcccc tgcctaccag 1740
cagggccaga atcagctgta caacgagctg aacctgggca gaagggaaga gtacgacgtc 1800
ctggataagc ggagaggccg ggaccctgag atgggcggca agcctcggcg gaagaacccc 1860
caggaaggcc tgtataacga actgcagaaa gacaagatgg ccgaggccta cagcgagatc 1920
ggcatgaagg gcgagcggag gcggggcaag ggccacgacg gcctgtatca gggcctgtcc 1980
accgccacca aggataccta cgacgccctg cacatgcagg ccctgccccc aaggctcgag 2040
ggcggcggag agggcagagg aagtcttcta acatgcggtg acgtggagga gaatcccggc 2100
cctaggatgc ttctcctggt gacaagcctt ctgctctgtg agttaccaca cccagcattc 2160
ctcctgatcc cacgcaaagt gtgtaacgga ataggtattg gtgaatttaa agactcactc 2220

tccataaatg ctacgaatat taaacacttc aaaaactgca cctccatcag tggcgatctc 2280

cacatcctgc cggtggcatt taggggtgac tccttcacac atactcctcc tctggatcca 2340

caggaactgg atattctgaa aaccgtaaag gaaatcacag ggtttttgct gattcaggct 2400

tggcctgaaa acaggacgga cctccatgcc tttgagaacc tagaaatcat acgcggcagg 2460

accaagcaac atggtcagtt ttctcttgca gtcgtcagcc tgaacataac atccttggga 2520

ttacgctccc tcaaggagat aagtgatgga gatgtgataa tttcaggaaa caaaaatttg 2580

tgctatgcaa atacaataaa ctggaaaaaa ctgtttggga cctccggtca gaaaaccaaa 2640

attataagca acagaggtga aaacagctgc aaggccacag gccaggtctg ccatgccttg 2700

tgctcccccg agggctgctg gggcccggag cccagggact gcgtctcttg ccggaatgtc 2760

agccgaggca gggaatgcgt ggacaagtgc aaccttctgg agggtgagcc aagggagttt 2820

gtggagaact ctgagtgcat acagtgccac ccagagtgcc tgcctcaggc catgaacatc 2880

acctgcacag gacggggacc agacaactgt atccagtgtg cccactacat tgacggcccc 2940

cactgcgtca agacctgccc ggcaggagtc atgggagaaa acaacaccct ggtctggaag 3000

tacgcagacg ccggccatgt gtgccacctg tgccatccaa actgcaccta cggatgcact 3060

gggccaggtc ttgaaggctg tccaacgaat gggcctaaga tcccgtccat cgccactggg 3120

atggtggggg ccctcctctt gctgctggtg gtggccctgg ggatcggcct cttcatgtga 3180

<210> 39
<211> 735
<212> DNA
<213> Homo sapiens

<400> 39
gatatccaga tgacccagtc cccgagctcc ctgtccgcct ctgtgggcga tagggtcacc 60

atcacctgcc gtgccagtca ggatgtgaat actgctgtag cctggtatca acagaaacca 120

ggaaaagctc cgaaactact gatttactcg gcatccttcc tctactctgg agtcccttct 180

cgcttctctg gttccagatc tgggacggat ttcactctga ccatcagcag tctgcagccg 240

gaagacttcg caacttatta ctgtcagcaa cattatacta ctcctcccac gttcggacag 300

ggtaccaagg tggagatcaa aggcagtact agcggcggtg gctccggggg cggatccggt 360

gggggcggca gcagcgaggt tcagctggtg gagtctggcg gtggcctggt gcagccaggg 420

ggctcactcc gtttgtcctg tgcagcttct ggcttcaaca ttaaagacac ctatatacac 480

tgggtgcgtc aggccccggg taagggcctg gaatgggttg caaggattta tcctacgaat 540

ggttatacta gatatgccga tagcgtcaag ggccgtttca ctataagcgc agacacatcc 600

aaaaacacag cctacctgca gatgaacagc ctgcgtgctg aggacactgc cgtctattat 660

tgttctagat ggggagggga cggcttctat gctatggact actggggtca aggaaccctg 720

gtcaccgtct cgagt                                                    735

<210>  40
<211>  753
<212>  DNA
<213>  Homo sapiens

<400>  40
gcattcctcc tgatcccaga tatccagatg acccagtccc cgagctccct gtccgcctct      60

gtgggcgata gggtcaccat cacctgccgt gccagtcagg atgtgaatac tgctgtagcc     120

tggtatcaac agaaaccagg aaaagctccg aaactactga tttactcggc atccttcctc     180

tactctggag tcccttctcg cttctctggt tccagatctg ggacggattt cactctgacc     240

atcagcagtc tgcagccgga agacttcgca acttattact gtcagcaaca ttatactact     300

cctcccacgt tcggacaggg taccaaggtg gagatcaaag gcagtactag cggcggtggc     360

tccgggggcg gatccggtgg gggcggcagc agcgaggttc agctggtgga gtctggcggt     420

ggcctggtgc agccaggggg ctcactccgt ttgtcctgtg cagcttctgg cttcaacatt     480

aaagacacct atatacactg ggtgcgtcag gccccgggta agggcctgga atgggttgca     540

aggatttatc ctacgaatgg ttatactaga tatgccgata gcgtcaaggg ccgtttcact     600

ataagcgcag acacatccaa aaacacagcc tacctgcaga tgaacagcct gcgtgctgag     660

gacactgccg tctattattg ttctagatgg ggaggggacg gcttctatgc tatggactac     720

tggggtcaag gaaccctggt caccgtctcg agt                                  753

<210>  41
<211>  357
<212>  DNA
<213>  Artificial Sequence

<220>
<223>  Hinge Spacer

<400>  41
gagagcaagt acggaccgcc ctgcccccct tgccctggcc agcctagaga accccaggtg      60

tacaccctgc ctcccagcca ggaagagatg accaagaacc aggtgtccct gacctgcctg     120

gtcaaaggct tctaccccag cgatatcgcc gtggaatggg agagcaacgg ccagcccgag     180

aacaactaca agaccacccc ccctgtgctg gacagcgacg gcagcttctt cctgtactcc     240

cggctgaccg tggacaagag ccggtggcag gaaggcaacg tcttcagctg cagcgtgatg     300

cacgaggccc tgcacaacca ctacacccag aagtccctga gcctgagcct gggcaag        357

<210>  42
<211>  356
<212>  DNA
<213>  Artificial Sequence

<220>
<223>  Hinge/Spacer

<400>  42
taggaccgcc ctgcccccct tgccctgccc ccgagttcct gggcggaccc agcgtgttcc      60

tgttcccccc caagcccaag gacaccctga tgatcagccg gacccccgag gtgacctgcg     120

tggtggtgga cgtgagccag gaagatcccg aggtccagtt caattggtac gtggacggcg     180

tggaagtgca caacgccaag accaagccca gagaggaaca gttcaacagc acctaccggg     240

tggtgtctgt gctgaccgtg ctgcaccagg actggctgaa cggcaaagaa tacaagtgca     300

aggtgtccaa caagggcctg cccagcagca tcgaaaagac catcagcaag gccaag         356


<210>  43
<211>  348
<212>  DNA
<213>  Artificial Sequence

<220>
<223>  Hinge/Spacer

<400>  43
tacggaccgc cctgcccccc ttgccctggc cagcctcgcg agccccaggt gtacaccctg      60

cctccctccc aggaagagat gaccaagaac caggtgtccc tgacctgcct ggtgaagggc     120

ttctacccca gcgacatcgc cgtggagtgg gagagcaacg gccagcctga gaacaactac     180

aagaccaccc ctcccgtgct ggacagcgac ggcagcttct tcctgtacag ccggctgacc     240

gtggacaaga gccggtggca ggaaggcaac gtctttagct gcagcgtgat gcacgaggcc     300

ctgcacaacc actacaccca gaagagcctg agcctgtccc tgggcaag                  348


<210>  44
<211>  15
<212>  PRT
<213>  Homo sapiens

<400>  44

Glu Pro Lys Ser Cys Asp Lys Thr His Thr Cys Pro Pro Cys Pro
1               5                   10                  15


<210>  45
<211>  16
<212>  PRT
<213>  Homo sapiens

<400>  45

Glu Leu Lys Thr Pro Leu Gly Asp Thr His Thr Cys Pro Arg Cys Pro
1               5                   10                  15


<210>  46
<211>  15

<212>  PRT
<213>  Homo sapiens

<400>  46

Glu Pro Lys Ser Cys Asp Thr Pro Pro Pro Cys Pro Arg Cys Pro
1               5                   10                  15


<210>  47
<211>  12
<212>  PRT
<213>  Homo sapiens

<400>  47

Glu Ser Lys Tyr Gly Pro Pro Cys Pro Ser Cys Pro
1               5                   10


<210>  48
<211>  12
<212>  PRT
<213>  Homo sapiens

<400>  48

Glu Arg Lys Cys Cys Val Glu Cys Pro Pro Cys Pro
1               5                   10


<210>  49
<211>  27
<212>  DNA
<213>  Homo sapiens

<400>  49
tacggaccgc cctgcccccc ttgccct                                          27


<210>  50
<211>  36
<212>  DNA
<213>  Homo sapiens

<400>  50
gaatctaagt acggaccgcc ctgcccccct tgccct                                36


<210>  51
<211>  36
<212>  DNA
<213>  Homo sapiens

<400>  51
gagagcaagt acggaccgcc ctgcccccct tgccct                                36


<210>  52
<211>  119
<212>  PRT
<213>  Artificial Sequence

<220>
<223>  Intermediate Spacer

<400>  52

Glu Ser Lys Tyr Gly Pro Pro Cys Pro Pro Cys Pro Gly Gln Pro Arg
1               5                   10                  15

Glu Pro Gln Val Tyr Thr Leu Pro Pro Ser Gln Glu Glu Met Thr Lys
            20                  25                  30

Asn Gln Val Ser Leu Thr Cys Leu Val Lys Gly Phe Tyr Pro Ser Asp
        35                  40                  45

Ile Ala Val Glu Trp Glu Ser Asn Gly Gln Pro Glu Asn Asn Tyr Lys
    50                  55                  60

Thr Thr Pro Pro Val Leu Asp Ser Asp Gly Ser Phe Phe Leu Tyr Ser
65                  70                  75                  80

Arg Leu Thr Val Asp Lys Ser Arg Trp Gln Glu Gly Asn Val Phe Ser
                85                  90                  95

Cys Ser Val Met His Glu Ala Leu His Asn His Tyr Thr Gln Lys Ser
            100                 105                 110

Leu Ser Leu Ser Leu Gly Lys
        115


<210>  53
<211>  838
<212>  PRT
<213>  Artificial Sequence

<220>
<223>  Leader _R11- Hinge- CD28tm/41BB-Z-T2A-tEGFR

<400>  53

Met Leu Leu Leu Val Thr Ser Leu Leu Leu Cys Glu Leu Pro His Pro
1               5                   10                  15

Ala Phe Leu Leu Ile Pro Gln Ser Val Lys Glu Ser Glu Gly Asp Leu
            20                  25                  30

Val Thr Pro Ala Gly Asn Leu Thr Leu Thr Cys Thr Ala Ser Gly Ser
        35                  40                  45

Asp Ile Asn Asp Tyr Pro Ile Ser Trp Val Arg Gln Ala Pro Gly Lys
    50                  55                  60

Gly Leu Glu Trp Ile Gly Phe Ile Asn Ser Gly Gly Ser Thr Trp Tyr
65                  70                  75                  80

Ala Ser Trp Val Lys Gly Arg Phe Thr Ile Ser Arg Thr Ser Thr Thr
                85                  90                  95

Val Asp Leu Lys Met Thr Ser Leu Thr Thr Asp Asp Thr Ala Thr Tyr
            100                 105                 110

Phe Cys Ala Arg Gly Tyr Ser Thr Tyr Tyr Gly Asp Phe Asn Ile Trp
        115                 120                 125

Gly Pro Gly Thr Leu Val Thr Ile Ser Ser Gly Gly Gly Gly Ser Gly
    130                 135                 140

Gly Gly Gly Ser Gly Gly Gly Gly Ser Glu Leu Val Met Thr Gln Thr
145                 150                 155                 160

Pro Ser Ser Thr Ser Gly Ala Val Gly Gly Thr Val Thr Ile Asn Cys
                165                 170                 175

Gln Ala Ser Gln Ser Ile Asp Ser Asn Leu Ala Trp Phe Gln Gln Lys
            180                 185                 190

Pro Gly Gln Pro Pro Thr Leu Leu Ile Tyr Arg Ala Ser Asn Leu Ala
        195                 200                 205

Ser Gly Val Pro Ser Arg Phe Ser Gly Ser Arg Ser Gly Thr Glu Tyr
    210                 215                 220

Thr Leu Thr Ile Ser Gly Val Gln Arg Glu Asp Ala Ala Thr Tyr Tyr
225                 230                 235                 240

Cys Leu Gly Gly Val Gly Asn Val Ser Tyr Arg Thr Ser Phe Gly Gly
                245                 250                 255

Gly Thr Glu Val Val Val Lys Glu Ser Lys Tyr Gly Pro Pro Cys Pro
            260                 265                 270

Pro Cys Pro Met Phe Trp Val Leu Val Val Val Gly Gly Val Leu Ala
        275                 280                 285

Cys Tyr Ser Leu Leu Val Thr Val Ala Phe Ile Ile Phe Trp Val Lys
    290                 295                 300

Arg Gly Arg Lys Lys Leu Leu Tyr Ile Phe Lys Gln Pro Phe Met Arg
305                 310                 315                 320

Pro Val Gln Thr Thr Gln Glu Glu Asp Gly Cys Ser Cys Arg Phe Pro
                325                 330                 335

Glu Glu Glu Glu Gly Gly Cys Glu Leu Arg Val Lys Phe Ser Arg Ser
            340                 345                 350

Ala Asp Ala Pro Ala Tyr Gln Gln Gly Gln Asn Gln Leu Tyr Asn Glu
        355                 360                 365

Leu Asn Leu Gly Arg Arg Glu Glu Tyr Asp Val Leu Asp Lys Arg Arg
    370                 375                 380

Gly Arg Asp Pro Glu Met Gly Gly Lys Pro Arg Arg Lys Asn Pro Gln
385                 390                 395                 400

Glu Gly Leu Tyr Asn Glu Leu Gln Lys Asp Lys Met Ala Glu Ala Tyr
                405                 410                 415

Ser Glu Ile Gly Met Lys Gly Glu Arg Arg Arg Gly Lys Gly His Asp
            420                 425                 430

Gly Leu Tyr Gln Gly Leu Ser Thr Ala Thr Lys Asp Thr Tyr Asp Ala
        435                 440                 445

Leu His Met Gln Ala Leu Pro Pro Arg Leu Glu Gly Gly Gly Glu Gly
    450                 455                 460

Arg Gly Ser Leu Leu Thr Cys Gly Asp Val Glu Glu Asn Pro Gly Pro
465                 470                 475                 480

Arg Met Leu Leu Leu Val Thr Ser Leu Leu Leu Cys Glu Leu Pro His
                485                 490                 495

Pro Ala Phe Leu Leu Ile Pro Arg Lys Val Cys Asn Gly Ile Gly Ile
            500                 505                 510

Gly Glu Phe Lys Asp Ser Leu Ser Ile Asn Ala Thr Asn Ile Lys His
        515                 520                 525

Phe Lys Asn Cys Thr Ser Ile Ser Gly Asp Leu His Ile Leu Pro Val
    530                 535                 540

Ala Phe Arg Gly Asp Ser Phe Thr His Thr Pro Pro Leu Asp Pro Gln
545                 550                 555                 560

Glu Leu Asp Ile Leu Lys Thr Val Lys Glu Ile Thr Gly Phe Leu Leu
                565                 570                 575

Ile Gln Ala Trp Pro Glu Asn Arg Thr Asp Leu His Ala Phe Glu Asn
            580                 585                 590

Leu Glu Ile Ile Arg Gly Arg Thr Lys Gln His Gly Gln Phe Ser Leu
        595                 600                 605

Ala Val Val Ser Leu Asn Ile Thr Ser Leu Gly Leu Arg Ser Leu Lys
    610                 615                 620

Glu Ile Ser Asp Gly Asp Val Ile Ile Ser Gly Asn Lys Asn Leu Cys
625                 630                 635                 640

Tyr Ala Asn Thr Ile Asn Trp Lys Lys Leu Phe Gly Thr Ser Gly Gln
                645                 650                 655

Lys Thr Lys Ile Ile Ser Asn Arg Gly Glu Asn Ser Cys Lys Ala Thr
            660                 665                 670

Gly Gln Val Cys His Ala Leu Cys Ser Pro Glu Gly Cys Trp Gly Pro
        675                 680                 685

Glu Pro Arg Asp Cys Val Ser Cys Arg Asn Val Ser Arg Gly Arg Glu
    690                 695                 700

Cys Val Asp Lys Cys Asn Leu Leu Glu Gly Glu Pro Arg Glu Phe Val
705                 710                 715                 720

Glu Asn Ser Glu Cys Ile Gln Cys His Pro Glu Cys Leu Pro Gln Ala
                725                 730                 735

Met Asn Ile Thr Cys Thr Gly Arg Gly Pro Asp Asn Cys Ile Gln Cys
            740                 745                 750

Ala His Tyr Ile Asp Gly Pro His Cys Val Lys Thr Cys Pro Ala Gly
        755                 760                 765

Val Met Gly Glu Asn Asn Thr Leu Val Trp Lys Tyr Ala Asp Ala Gly
    770                 775                 780

His Val Cys His Leu Cys His Pro Asn Cys Thr Tyr Gly Cys Thr Gly
785                 790                 795                 800

Pro Gly Leu Glu Gly Cys Pro Thr Asn Gly Pro Lys Ile Pro Ser Ile
                805                 810                 815

Ala Thr Gly Met Val Gly Ala Leu Leu Leu Leu Leu Val Val Ala Leu

            820                 825                 830

Gly Ile Gly Leu Phe Met
        835

<210> 54
<211> 1055
<212> PRT
<213> Artificial Sequence

<220>
<223> Leader _R11- Hinge-CH2-CH3- CD28tm/41BB-Z-T2A-tEGFR

<400> 54

<210>  55
<211>  945
<212>  PRT
<213>  Artificial Sequence

<220>
<223>  Leader _R11- Hinge-CH3- CD28tm/41BB-Z-T2A-tEGFR

<400>  55
   Met Leu Leu Leu Val Thr Ser Leu Leu Leu Cys Glu Leu Pro His Pro
   1               5                   10                  15
   Ala Phe Leu Leu Ile Pro Gln Ser Val Lys Glu Ser Glu Gly Asp Leu
               20                  25                  30
   Val Thr Pro Ala Gly Asn Leu Thr Leu Thr Cys Thr Ala Ser Gly Ser
           35                  40                  45
   Asp Ile Asn Asp Tyr Pro Ile Ser Trp Val Arg Gln Ala Pro Gly Lys
       50                  55                  60
   Gly Leu Glu Trp Ile Gly Phe Ile Asn Ser Gly Gly Ser Thr Trp Tyr
   65                  70                  75                  80
   Ala Ser Trp Val Lys Gly Arg Phe Thr Ile Ser Arg Thr Ser Thr Thr
   85 90 95
   Val Asp Leu Lys Met Thr Ser Leu Thr Thr Asp Asp Thr Ala Thr Tyr
   100 105 110
   Phe Cys Ala Arg Gly Tyr Ser Thr Tyr Tyr Gly Asp Phe Asn Ile Trp
   115 120 125
   Gly Pro Gly Thr Leu Val Thr Ile Ser Ser Gly Gly Gly Gly Ser Gly
   130 135 140
   Gly Gly Gly Ser Gly Gly Gly Gly Ser Glu Leu Val Met Thr Gln Thr
   145 150 155 160
   Pro Ser Ser Thr Ser Gly Ala Val Gly Gly Thr Val Thr Ile Asn Cys
   165 170 175
   Gln Ala Ser Gln Ser Ile Asp Ser Asn Leu Ala Trp Phe Gln Gln Lys
   180 185 190
   Pro Gly Gln Pro Pro Thr Leu Leu Ile Tyr Arg Ala Ser Asn Leu Ala
   195 200 205
   Ser Gly Val Pro Ser Arg Phe Ser Gly Ser Arg Ser Gly Thr Glu Tyr
   210 215 220
   Thr Leu Thr Ile Ser Gly Val Gln Arg Glu Asp Ala Ala Thr Tyr Tyr
   225 230 235 240
   Cys Leu Gly Gly Val Gly Asn Val Ser Tyr Arg Thr Ser Phe Gly Gly
   245 250 255
   Gly Thr Glu Val Val Val Lys Glu Ser Lys Tyr Gly Pro Pro Cys Pro
   260 265 270
   Pro Cys Pro Gly Gln Pro Arg Glu Pro Gln Val Tyr Thr Leu Pro Pro
   275 280 285
   Ser Gln Glu Glu Met Thr Lys Asn Gln Val Ser Leu Thr Cys Leu Val
   290 295 300
   Lys Gly Phe Tyr Pro Ser Asp Ile Ala Val Glu Trp Glu Ser Asn Gly
   305 310 315 320
   Gln Pro Glu Asn Asn Tyr Lys Thr Thr Pro Pro Val Leu Asp Ser Asp
   325 330 335
   Gly Ser Phe Phe Leu Tyr Ser Arg Leu Thr Val Asp Lys Ser Arg Trp
               340                 345                 350
   Gln Glu Gly Asn Val Phe Ser Cys Ser Val Met His Glu Ala Leu His
           355                 360                 365
   Asn His Tyr Thr Gln Lys Ser Leu Ser Leu Ser Leu Gly Lys Met Phe
       370                 375                 380
   Trp Val Leu Val Val Val Gly Gly Val Leu Ala Cys Tyr Ser Leu Leu
   385                 390                 395                 400
   Val Thr Val Ala Phe Ile Ile Phe Trp Val Lys Arg Gly Arg Lys Lys
                   405                 410                 415
   Leu Leu Tyr Ile Phe Lys Gln Pro Phe Met Arg Pro Val Gln Thr Thr
               420                 425                 430
   Gln Glu Glu Asp Gly Cys Ser Cys Arg Phe Pro Glu Glu Glu Glu Gly
           435                 440                 445
   Gly Cys Glu Leu Arg Val Lys Phe Ser Arg Ser Ala Asp Ala Pro Ala
       450                 455                 460
   Tyr Gln Gln Gly Gln Asn Gln Leu Tyr Asn Glu Leu Asn Leu Gly Arg
   465                 470                 475                 480
   Arg Glu Glu Tyr Asp Val Leu Asp Lys Arg Arg Gly Arg Asp Pro Glu
                   485                 490                 495
   Met Gly Gly Lys Pro Arg Arg Lys Asn Pro Gln Glu Gly Leu Tyr Asn
               500                 505                 510
   Glu Leu Gln Lys Asp Lys Met Ala Glu Ala Tyr Ser Glu Ile Gly Met
           515                 520                 525
   Lys Gly Glu Arg Arg Arg Gly Lys Gly His Asp Gly Leu Tyr Gln Gly
       530                 535                 540
   Leu Ser Thr Ala Thr Lys Asp Thr Tyr Asp Ala Leu His Met Gln Ala
   545                 550                 555                 560
   Leu Pro Pro Arg Leu Glu Gly Gly Gly Glu Gly Arg Gly Ser Leu Leu
                   565                 570                 575
   Thr Cys Gly Asp Val Glu Glu Asn Pro Gly Pro Arg Met Leu Leu Leu
               580                 585                 590
   Val Thr Ser Leu Leu Leu Cys Glu Leu Pro His Pro Ala Phe Leu Leu
           595                 600                 605
   Ile Pro Arg Lys Val Cys Asn Gly Ile Gly Ile Gly Glu Phe Lys Asp
       610                 615                 620
   Ser Leu Ser Ile Asn Ala Thr Asn Ile Lys His Phe Lys Asn Cys Thr
   625                 630                 635                 640
   Ser Ile Ser Gly Asp Leu His Ile Leu Pro Val Ala Phe Arg Gly Asp
                   645                 650                 655
   Ser Phe Thr His Thr Pro Pro Leu Asp Pro Gln Glu Leu Asp Ile Leu
               660                 665                 670
   Lys Thr Val Lys Glu Ile Thr Gly Phe Leu Leu Ile Gln Ala Trp Pro
           675                 680                 685
   Glu Asn Arg Thr Asp Leu His Ala Phe Glu Asn Leu Glu Ile Ile Arg
       690                 695                 700
   Gly Arg Thr Lys Gln His Gly Gln Phe Ser Leu Ala Val Val Ser Leu
   705                 710                 715                 720
   Asn Ile Thr Ser Leu Gly Leu Arg Ser Leu Lys Glu Ile Ser Asp Gly
                   725                 730                 735
   Asp Val Ile Ile Ser Gly Asn Lys Asn Leu Cys Tyr Ala Asn Thr Ile
               740                 745                 750
   Asn Trp Lys Lys Leu Phe Gly Thr Ser Gly Gln Lys Thr Lys Ile Ile
           755                 760                 765
   Ser Asn Arg Gly Glu Asn Ser Cys Lys Ala Thr Gly Gln Val Cys His
       770                 775                 780
   Ala Leu Cys Ser Pro Glu Gly Cys Trp Gly Pro Glu Pro Arg Asp Cys
   785                 790                 795                 800
   Val Ser Cys Arg Asn Val Ser Arg Gly Arg Glu Cys Val Asp Lys Cys
                   805                 810                 815
   Asn Leu Leu Glu Gly Glu Pro Arg Glu Phe Val Glu Asn Ser Glu Cys
               820                 825                 830
   Ile Gln Cys His Pro Glu Cys Leu Pro Gln Ala Met Asn Ile Thr Cys
   835                 840                 845
   Thr Gly Arg Gly Pro Asp Asn Cys Ile Gln Cys Ala His Tyr Ile Asp
       850                 855                 860
   Gly Pro His Cys Val Lys Thr Cys Pro Ala Gly Val Met Gly Glu Asn
   865                 870                 875                 880
   Asn Thr Leu Val Trp Lys Tyr Ala Asp Ala Gly His Val Cys His Leu
                   885                 890                 895
   Cys His Pro Asn Cys Thr Tyr Gly Cys Thr Gly Pro Gly Leu Glu Gly
               900                 905                 910
   Cys Pro Thr Asn Gly Pro Lys Ile Pro Ser Ile Ala Thr Gly Met Val
           915                 920                 925
   Gly Ala Leu Leu Leu Leu Leu Val Val Ala Leu Gly Ile Gly Leu Phe
       930                 935                 940
   Met
   945

<210>  56
<211>  845
<212>  PRT
<213>  Artificial Sequence

<220>
<223>  Leader _R12 - CD28tm/41BB-Z-T2A-tEGFR

<400>  56

Met Leu Leu Leu Val Thr Ser Leu Leu Leu Cys Glu Leu Pro His Pro
1               5                   10                  15

Ala Phe Leu Leu Ile Pro Gln Glu Gln Leu Val Glu Ser Gly Gly Arg
            20                  25                  30

Leu Val Thr Pro Gly Gly Ser Leu Thr Leu Ser Cys Lys Ala Ser Gly
        35                  40                  45

Phe Asp Phe Ser Ala Tyr Tyr Met Ser Trp Val Arg Gln Ala Pro Gly
    50                  55                  60

Lys Gly Leu Glu Trp Ile Ala Thr Ile Tyr Pro Ser Ser Gly Lys Thr
65                  70                  75                  80

Tyr Tyr Ala Thr Trp Val Asn Gly Arg Phe Thr Ile Ser Ser Asp Asn
                85                  90                  95

Ala Gln Asn Thr Val Asp Leu Gln Met Asn Ser Leu Thr Ala Ala Asp
            100                 105                 110

Arg Ala Thr Tyr Phe Cys Ala Arg Asp Ser Tyr Ala Asp Asp Gly Ala
        115                 120                 125

Leu Phe Asn Ile Trp Gly Pro Gly Thr Leu Val Thr Ile Ser Ser Gly
    130                 135                 140

Gly Gly Gly Ser Gly Gly Gly Gly Ser Gly Gly Gly Gly Ser Glu Leu
145                 150                 155                 160

Val Leu Thr Gln Ser Pro Ser Val Ser Ala Ala Leu Gly Ser Pro Ala
                165                 170                 175

Lys Ile Thr Cys Thr Leu Ser Ser Ala His Lys Thr Asp Thr Ile Asp
            180                 185                 190

Trp Tyr Gln Gln Leu Gln Gly Glu Ala Pro Arg Tyr Leu Met Gln Val
        195                 200                 205

Gln Ser Asp Gly Ser Tyr Thr Lys Arg Pro Gly Val Pro Asp Arg Phe
    210                 215                 220

Ser Gly Ser Ser Ser Gly Ala Asp Arg Tyr Leu Ile Ile Pro Ser Val
225                 230                 235                 240

Gln Ala Asp Asp Glu Ala Asp Tyr Tyr Cys Gly Ala Asp Tyr Ile Gly
                245                 250                 255

Gly Tyr Val Phe Gly Gly Gly Thr Gln Leu Thr Val Thr Gly Glu Ser
            260                 265                 270

Lys Tyr Gly Pro Pro Cys Pro Pro Cys Pro Met Phe Trp Val Leu Val
        275                 280                 285

Val Val Gly Gly Val Leu Ala Cys Tyr Ser Leu Leu Val Thr Val Ala
    290                 295                 300

Phe Ile Ile Phe Trp Val Lys Arg Gly Arg Lys Lys Leu Leu Tyr Ile
305                 310                 315                 320

Phe Lys Gln Pro Phe Met Arg Pro Val Gln Thr Thr Gln Glu Glu Asp
                325                 330                 335

Gly Cys Ser Cys Arg Phe Pro Glu Glu Glu Glu Gly Gly Cys Glu Leu

340                 345                 350

Arg Val Lys Phe Ser Arg Ser Ala Asp Ala Pro Ala Tyr Gln Gln Gly
        355                 360                 365

Gln Asn Gln Leu Tyr Asn Glu Leu Asn Leu Gly Arg Arg Glu Glu Tyr
    370                 375                 380

Asp Val Leu Asp Lys Arg Arg Gly Arg Asp Pro Glu Met Gly Gly Lys
385                 390                 395                 400

Pro Arg Arg Lys Asn Pro Gln Glu Gly Leu Tyr Asn Glu Leu Gln Lys
                405                 410                 415

Asp Lys Met Ala Glu Ala Tyr Ser Glu Ile Gly Met Lys Gly Glu Arg
            420                 425                 430

Arg Arg Gly Lys Gly His Asp Gly Leu Tyr Gln Gly Leu Ser Thr Ala
        435                 440                 445

Thr Lys Asp Thr Tyr Asp Ala Leu His Met Gln Ala Leu Pro Pro Arg
    450                 455                 460

Leu Glu Gly Gly Gly Glu Gly Arg Gly Ser Leu Leu Thr Cys Gly Asp
465                 470                 475                 480

Val Glu Glu Asn Pro Gly Pro Arg Met Leu Leu Leu Val Thr Ser Leu
                485                 490                 495

Leu Leu Cys Glu Leu Pro His Pro Ala Phe Leu Leu Ile Pro Arg Lys
            500                 505                 510

Val Cys Asn Gly Ile Gly Ile Gly Glu Phe Lys Asp Ser Leu Ser Ile
        515                 520                 525

Asn Ala Thr Asn Ile Lys His Phe Lys Asn Cys Thr Ser Ile Ser Gly
    530                 535                 540

Asp Leu His Ile Leu Pro Val Ala Phe Arg Gly Asp Ser Phe Thr His
545                 550                 555                 560

Thr Pro Pro Leu Asp Pro Gln Glu Leu Asp Ile Leu Lys Thr Val Lys
                565                 570                 575

Glu Ile Thr Gly Phe Leu Leu Ile Gln Ala Trp Pro Glu Asn Arg Thr
            580                 585                 590

Asp Leu His Ala Phe Glu Asn Leu Glu Ile Ile Arg Gly Arg Thr Lys
        595                 600                 605

Gln His Gly Gln Phe Ser Leu Ala Val Val Ser Leu Asn Ile Thr Ser
    610                 615                 620

Leu Gly Leu Arg Ser Leu Lys Glu Ile Ser Asp Gly Asp Val Ile Ile
625                 630                 635                 640

Ser Gly Asn Lys Asn Leu Cys Tyr Ala Asn Thr Ile Asn Trp Lys Lys
                645                 650                 655

Leu Phe Gly Thr Ser Gly Gln Lys Thr Lys Ile Ile Ser Asn Arg Gly
            660                 665                 670

Glu Asn Ser Cys Lys Ala Thr Gly Gln Val Cys His Ala Leu Cys Ser
        675                 680                 685

Pro Glu Gly Cys Trp Gly Pro Glu Pro Arg Asp Cys Val Ser Cys Arg
    690                 695                 700

Asn Val Ser Arg Gly Arg Glu Cys Val Asp Lys Cys Asn Leu Leu Glu
705                 710                 715                 720

Gly Glu Pro Arg Glu Phe Val Glu Asn Ser Glu Cys Ile Gln Cys His
                725                 730                 735

Pro Glu Cys Leu Pro Gln Ala Met Asn Ile Thr Cys Thr Gly Arg Gly
            740                 745                 750

Pro Asp Asn Cys Ile Gln Cys Ala His Tyr Ile Asp Gly Pro His Cys
        755                 760                 765

Val Lys Thr Cys Pro Ala Gly Val Met Gly Glu Asn Asn Thr Leu Val
    770                 775                 780

Trp Lys Tyr Ala Asp Ala Gly His Val Cys His Leu Cys His Pro Asn
785                 790                 795                 800

Cys Thr Tyr Gly Cys Thr Gly Pro Gly Leu Glu Gly Cys Pro Thr Asn
                805                 810                 815

Gly Pro Lys Ile Pro Ser Ile Ala Thr Gly Met Val Gly Ala Leu Leu
            820                 825                 830

Leu Leu Leu Val Val Ala Leu Gly Ile Gly Leu Phe Met
        835                 840                 845

<210>  57
<211>  952
<212>  PRT
<213>  Artificial Sequence

<220>
<223>  Leader _R12- Hinge- CH3- CD28tm/41BB-Z-T2A-tEGFR

<400>  57

Met Leu Leu Leu Val Thr Ser Leu Leu Leu Cys Glu Leu Pro His Pro
1               5                   10                  15

Ala Phe Leu Leu Ile Pro Gln Glu Gln Leu Val Glu Ser Gly Gly Arg
            20                  25                  30

Leu Val Thr Pro Gly Gly Ser Leu Thr Leu Ser Cys Lys Ala Ser Gly
        35                  40                  45

Phe Asp Phe Ser Ala Tyr Tyr Met Ser Trp Val Arg Gln Ala Pro Gly
    50                  55                  60

Lys Gly Leu Glu Trp Ile Ala Thr Ile Tyr Pro Ser Ser Gly Lys Thr
65                  70                  75                  80

Tyr Tyr Ala Thr Trp Val Asn Gly Arg Phe Thr Ile Ser Ser Asp Asn
                85                  90                  95

Ala Gln Asn Thr Val Asp Leu Gln Met Asn Ser Leu Thr Ala Ala Asp
            100                 105                 110

Arg Ala Thr Tyr Phe Cys Ala Arg Asp Ser Tyr Ala Asp Asp Gly Ala
        115                 120                 125

Leu Phe Asn Ile Trp Gly Pro Gly Thr Leu Val Thr Ile Ser Ser Gly
    130                 135                 140

Gly Gly Gly Ser Gly Gly Gly Gly Ser Gly Gly Gly Gly Ser Glu Leu
145                 150                 155                 160

Val Leu Thr Gln Ser Pro Ser Val Ser Ala Ala Leu Gly Ser Pro Ala
                165                 170                 175

Lys Ile Thr Cys Thr Leu Ser Ser Ala His Lys Thr Asp Thr Ile Asp
            180                 185                 190

Trp Tyr Gln Gln Leu Gln Gly Glu Ala Pro Arg Tyr Leu Met Gln Val
        195                 200                 205

Gln Ser Asp Gly Ser Tyr Thr Lys Arg Pro Gly Val Pro Asp Arg Phe
    210                 215                 220

Ser Gly Ser Ser Ser Gly Ala Asp Arg Tyr Leu Ile Ile Pro Ser Val
225                 230                 235                 240

Gln Ala Asp Asp Glu Ala Asp Tyr Tyr Cys Gly Ala Asp Tyr Ile Gly
                245                 250                 255

Gly Tyr Val Phe Gly Gly Gly Thr Gln Leu Thr Val Thr Gly Glu Ser
            260                 265                 270

Lys Tyr Gly Pro Pro Cys Pro Pro Cys Pro Gly Gln Pro Arg Glu Pro
        275                 280                 285

Gln Val Tyr Thr Leu Pro Pro Ser Gln Glu Glu Met Thr Lys Asn Gln
    290                 295                 300

Val Ser Leu Thr Cys Leu Val Lys Gly Phe Tyr Pro Ser Asp Ile Ala
305                 310                 315                 320

Val Glu Trp Glu Ser Asn Gly Gln Pro Glu Asn Asn Tyr Lys Thr Thr
                325                 330                 335

Pro Pro Val Leu Asp Ser Asp Gly Ser Phe Phe Leu Tyr Ser Arg Leu
            340                 345                 350

Thr Val Asp Lys Ser Arg Trp Gln Glu Gly Asn Val Phe Ser Cys Ser
        355                 360                 365

Val Met His Glu Ala Leu His Asn His Tyr Thr Gln Lys Ser Leu Ser
    370                 375                 380

Leu Ser Leu Gly Lys Met Phe Trp Val Leu Val Val Val Gly Gly Val
385                 390                 395                 400

Leu Ala Cys Tyr Ser Leu Leu Val Thr Val Ala Phe Ile Ile Phe Trp
                405                 410                 415

Val Lys Arg Gly Arg Lys Lys Leu Leu Tyr Ile Phe Lys Gln Pro Phe
            420                 425                 430

Met Arg Pro Val Gln Thr Thr Gln Glu Glu Asp Gly Cys Ser Cys Arg
        435                 440                 445

Phe Pro Glu Glu Glu Glu Gly Gly Cys Glu Leu Arg Val Lys Phe Ser
    450                 455                 460

Arg Ser Ala Asp Ala Pro Ala Tyr Gln Gln Gly Gln Asn Gln Leu Tyr
465                 470                 475                 480

Asn Glu Leu Asn Leu Gly Arg Arg Glu Glu Tyr Asp Val Leu Asp Lys
                485                 490                 495

Arg Arg Gly Arg Asp Pro Glu Met Gly Gly Lys Pro Arg Arg Lys Asn
            500                 505                 510

Pro Gln Glu Gly Leu Tyr Asn Glu Leu Gln Lys Asp Lys Met Ala Glu
        515                 520                 525

Ala Tyr Ser Glu Ile Gly Met Lys Gly Glu Arg Arg Arg Gly Lys Gly
    530                 535                 540

His Asp Gly Leu Tyr Gln Gly Leu Ser Thr Ala Thr Lys Asp Thr Tyr
545                 550                 555                 560

Asp Ala Leu His Met Gln Ala Leu Pro Pro Arg Leu Glu Gly Gly Gly
                565                 570                 575

Glu Gly Arg Gly Ser Leu Leu Thr Cys Gly Asp Val Glu Glu Asn Pro
            580                 585                 590

Gly Pro Arg Met Leu Leu Leu Val Thr Ser Leu Leu Leu Cys Glu Leu
        595                 600                 605

Pro His Pro Ala Phe Leu Leu Ile Pro Arg Lys Val Cys Asn Gly Ile
    610                 615                 620

Gly Ile Gly Glu Phe Lys Asp Ser Leu Ser Ile Asn Ala Thr Asn Ile
625                 630                 635                 640

Lys His Phe Lys Asn Cys Thr Ser Ile Ser Gly Asp Leu His Ile Leu
                645                 650                 655

Pro Val Ala Phe Arg Gly Asp Ser Phe Thr His Thr Pro Pro Leu Asp
            660                 665                 670

Pro Gln Glu Leu Asp Ile Leu Lys Thr Val Lys Glu Ile Thr Gly Phe
        675                 680                 685

Leu Leu Ile Gln Ala Trp Pro Glu Asn Arg Thr Asp Leu His Ala Phe
    690                 695                 700

Glu Asn Leu Glu Ile Ile Arg Gly Arg Thr Lys Gln His Gly Gln Phe
705                 710                 715                 720

Ser Leu Ala Val Val Ser Leu Asn Ile Thr Ser Leu Gly Leu Arg Ser
                725                 730                 735

Leu Lys Glu Ile Ser Asp Gly Asp Val Ile Ile Ser Gly Asn Lys Asn
            740                 745                 750

Leu Cys Tyr Ala Asn Thr Ile Asn Trp Lys Lys Leu Phe Gly Thr Ser
        755                 760                 765

Gly Gln Lys Thr Lys Ile Ile Ser Asn Arg Gly Glu Asn Ser Cys Lys
    770                 775                 780

Ala Thr Gly Gln Val Cys His Ala Leu Cys Ser Pro Glu Gly Cys Trp
785                 790                 795                 800

Gly Pro Glu Pro Arg Asp Cys Val Ser Cys Arg Asn Val Ser Arg Gly
                805                 810                 815

Arg Glu Cys Val Asp Lys Cys Asn Leu Leu Glu Gly Glu Pro Arg Glu
            820                 825                 830

Phe Val Glu Asn Ser Glu Cys Ile Gln Cys His Pro Glu Cys Leu Pro
        835                 840                 845

Gln Ala Met Asn Ile Thr Cys Thr Gly Arg Gly Pro Asp Asn Cys Ile
    850                 855                 860

Gln Cys Ala His Tyr Ile Asp Gly Pro His Cys Val Lys Thr Cys Pro
865                 870                 875                 880

Ala Gly Val Met Gly Glu Asn Asn Thr Leu Val Trp Lys Tyr Ala Asp
                885                 890                 895

Ala Gly His Val Cys His Leu Cys His Pro Asn Cys Thr Tyr Gly Cys
            900                 905                 910

Thr Gly Pro Gly Leu Glu Gly Cys Pro Thr Asn Gly Pro Lys Ile Pro
        915                 920                 925

Ser Ile Ala Thr Gly Met Val Gly Ala Leu Leu Leu Leu Leu Val Val
    930                 935                 940

Ala Leu Gly Ile Gly Leu Phe Met
945                 950

<210>  58

<211>  1062
<212>  PRT
<213>  Artificial Sequence

<220>
<223>  Leader _R12- Hinge-CH2-CH3- CD28tm/41BB-Z-T2A-tEGFR

<400>  58

Met Leu Leu Leu Val Thr Ser Leu Leu Leu Cys Glu Leu Pro His Pro
1               5                   10                  15

Ala Phe Leu Leu Ile Pro Gln Glu Gln Leu Val Glu Ser Gly Gly Arg
            20                  25                  30

Leu Val Thr Pro Gly Gly Ser Leu Thr Leu Ser Cys Lys Ala Ser Gly
        35                  40                  45

Phe Asp Phe Ser Ala Tyr Tyr Met Ser Trp Val Arg Gln Ala Pro Gly
    50                  55                  60

Lys Gly Leu Glu Trp Ile Ala Thr Ile Tyr Pro Ser Ser Gly Lys Thr
65                  70                  75                  80

Tyr Tyr Ala Thr Trp Val Asn Gly Arg Phe Thr Ile Ser Ser Asp Asn
                85                  90                  95

Ala Gln Asn Thr Val Asp Leu Gln Met Asn Ser Leu Thr Ala Ala Asp
            100                 105                 110

Arg Ala Thr Tyr Phe Cys Ala Arg Asp Ser Tyr Ala Asp Asp Gly Ala
        115                 120                 125

Leu Phe Asn Ile Trp Gly Pro Gly Thr Leu Val Thr Ile Ser Ser Gly
    130                 135                 140

Gly Gly Gly Ser Gly Gly Gly Gly Ser Gly Gly Gly Gly Ser Glu Leu
145                 150                 155                 160

Val Leu Thr Gln Ser Pro Ser Val Ser Ala Ala Leu Gly Ser Pro Ala
                165                 170                 175

Lys Ile Thr Cys Thr Leu Ser Ser Ala His Lys Thr Asp Thr Ile Asp
            180                 185                 190

Trp Tyr Gln Gln Leu Gln Gly Glu Ala Pro Arg Tyr Leu Met Gln Val
        195                 200                 205

Gln Ser Asp Gly Ser Tyr Thr Lys Arg Pro Gly Val Pro Asp Arg Phe
    210                 215                 220

Ser Gly Ser Ser Ser Gly Ala Asp Arg Tyr Leu Ile Ile Pro Ser Val
225                 230                 235                 240

Gln Ala Asp Asp Glu Ala Asp Tyr Tyr Cys Gly Ala Asp Tyr Ile Gly
                245                 250                 255

Gly Tyr Val Phe Gly Gly Gly Thr Gln Leu Thr Val Thr Gly Glu Ser
            260                 265                 270

Lys Tyr Gly Pro Pro Cys Pro Pro Cys Pro Ala Pro Glu Phe Leu Gly
        275                 280                 285

Gly Pro Ser Val Phe Leu Phe Pro Pro Lys Pro Lys Asp Thr Leu Met
    290                 295                 300

Ile Ser Arg Thr Pro Glu Val Thr Cys Val Val Val Asp Val Ser Gln
305                 310                 315                 320

Glu Asp Pro Glu Val Gln Phe Asn Trp Tyr Val Asp Gly Val Glu Val
                325                 330                 335

His Asn Ala Lys Thr Lys Pro Arg Glu Glu Gln Phe Asn Ser Thr Tyr
            340                 345                 350

Arg Val Val Ser Val Leu Thr Val Leu His Gln Asp Trp Leu Asn Gly
        355                 360                 365

Lys Glu Tyr Lys Cys Lys Val Ser Asn Lys Gly Leu Pro Ser Ser Ile
    370                 375                 380

Glu Lys Thr Ile Ser Lys Ala Lys Gly Gln Pro Arg Glu Pro Gln Val
385                 390                 395                 400

Tyr Thr Leu Pro Pro Ser Gln Glu Glu Met Thr Lys Asn Gln Val Ser
                405                 410                 415

Leu Thr Cys Leu Val Lys Gly Phe Tyr Pro Ser Asp Ile Ala Val Glu
            420                 425                 430

Trp Glu Ser Asn Gly Gln Pro Glu Asn Asn Tyr Lys Thr Thr Pro Pro
        435                 440                 445

Val Leu Asp Ser Asp Gly Ser Phe Phe Leu Tyr Ser Arg Leu Thr Val
    450                 455                 460

Asp Lys Ser Arg Trp Gln Glu Gly Asn Val Phe Ser Cys Ser Val Met

465                 470                 475                 480

His Glu Ala Leu His Asn His Tyr Thr Gln Lys Ser Leu Ser Leu Ser
                485                 490                 495

Leu Gly Lys Met Phe Trp Val Leu Val Val Val Gly Gly Val Leu Ala
            500                 505                 510

Cys Tyr Ser Leu Leu Val Thr Val Ala Phe Ile Ile Phe Trp Val Lys
        515                 520                 525

Arg Gly Arg Lys Lys Leu Leu Tyr Ile Phe Lys Gln Pro Phe Met Arg
    530                 535                 540

Pro Val Gln Thr Thr Gln Glu Glu Asp Gly Cys Ser Cys Arg Phe Pro
545                 550                 555                 560

Glu Glu Glu Glu Gly Gly Cys Glu Leu Arg Val Lys Phe Ser Arg Ser
                565                 570                 575

Ala Asp Ala Pro Ala Tyr Gln Gln Gly Gln Asn Gln Leu Tyr Asn Glu
            580                 585                 590

Leu Asn Leu Gly Arg Arg Glu Glu Tyr Asp Val Leu Asp Lys Arg Arg
        595                 600                 605

Gly Arg Asp Pro Glu Met Gly Gly Lys Pro Arg Arg Lys Asn Pro Gln
    610                 615                 620

Glu Gly Leu Tyr Asn Glu Leu Gln Lys Asp Lys Met Ala Glu Ala Tyr
625                 630                 635                 640

Ser Glu Ile Gly Met Lys Gly Glu Arg Arg Arg Gly Lys Gly His Asp
                645                 650                 655

Gly Leu Tyr Gln Gly Leu Ser Thr Ala Thr Lys Asp Thr Tyr Asp Ala
            660                 665                 670

Leu His Met Gln Ala Leu Pro Pro Arg Leu Glu Gly Gly Gly Glu Gly
        675                 680                 685

Arg Gly Ser Leu Leu Thr Cys Gly Asp Val Glu Glu Asn Pro Gly Pro
    690                 695                 700

Arg Met Leu Leu Leu Val Thr Ser Leu Leu Leu Cys Glu Leu Pro His
705                 710                 715                 720

Pro Ala Phe Leu Leu Ile Pro Arg Lys Val Cys Asn Gly Ile Gly Ile
                725                 730                 735

Gly Glu Phe Lys Asp Ser Leu Ser Ile Asn Ala Thr Asn Ile Lys His
            740                 745                 750

Phe Lys Asn Cys Thr Ser Ile Ser Gly Asp Leu His Ile Leu Pro Val
        755                 760                 765

Ala Phe Arg Gly Asp Ser Phe Thr His Thr Pro Pro Leu Asp Pro Gln
    770                 775                 780

Glu Leu Asp Ile Leu Lys Thr Val Lys Glu Ile Thr Gly Phe Leu Leu
785                 790                 795                 800

Ile Gln Ala Trp Pro Glu Asn Arg Thr Asp Leu His Ala Phe Glu Asn
                805                 810                 815

Leu Glu Ile Ile Arg Gly Arg Thr Lys Gln His Gly Gln Phe Ser Leu
            820                 825                 830

Ala Val Val Ser Leu Asn Ile Thr Ser Leu Gly Leu Arg Ser Leu Lys
        835                 840                 845

Glu Ile Ser Asp Gly Asp Val Ile Ile Ser Gly Asn Lys Asn Leu Cys
    850                 855                 860

Tyr Ala Asn Thr Ile Asn Trp Lys Lys Leu Phe Gly Thr Ser Gly Gln
865                 870                 875                 880

Lys Thr Lys Ile Ile Ser Asn Arg Gly Glu Asn Ser Cys Lys Ala Thr
                885                 890                 895

Gly Gln Val Cys His Ala Leu Cys Ser Pro Glu Gly Cys Trp Gly Pro
            900                 905                 910

Glu Pro Arg Asp Cys Val Ser Cys Arg Asn Val Ser Arg Gly Arg Glu
        915                 920                 925

Cys Val Asp Lys Cys Asn Leu Leu Glu Gly Glu Pro Arg Glu Phe Val
    930                 935                 940

Glu Asn Ser Glu Cys Ile Gln Cys His Pro Glu Cys Leu Pro Gln Ala
945                 950                 955                 960

Met Asn Ile Thr Cys Thr Gly Arg Gly Pro Asp Asn Cys Ile Gln Cys
                965                 970                 975

Ala His Tyr Ile Asp Gly Pro His Cys Val Lys Thr Cys Pro Ala Gly
            980                 985                 990

Val Met Gly Glu Asn Asn Thr Leu Val Trp Lys Tyr Ala Asp Ala Gly
        995                 1000                1005

His Val Cys His Leu Cys His Pro Asn Cys Thr Tyr Gly Cys Thr
    1010                1015                1020

Gly Pro Gly Leu Glu Gly Cys Pro Thr Asn Gly Pro Lys Ile Pro
    1025                1030                1035

Ser Ile Ala Thr Gly Met Val Gly Ala Leu Leu Leu Leu Leu Val
    1040                1045                1050

Val Ala Leu Gly Ile Gly Leu Phe Met
    1055                1060

<210> 59
<211> 48
<212> DNA
<213> Artificial Sequence

<220>
<223> Leader Sequence

<400> 59
atgcttctcc tggtgacaag ccttctgctc tgtgagttac cacaccca                48

<210> 60
<211> 15
<212> PRT
<213> Artificial Sequence

<220>
<223> Linker Peptide

<400> 60

Gly Gly Gly Gly Ser Gly Gly Gly Gly Ser Gly Gly Gly Gly Ser
1               5                   10                  15

<210> 61
<211> 229
<212> PRT
<213> Artificial Sequence

<220>
<223> Long Spacer

<400> 61

Glu Ser Lys Tyr Gly Pro Pro Cys Pro Pro Cys Pro Ala Pro Glu Phe
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys
225

<210> 62
<211> 687
<212> DNA
<213> Artificial Sequence

<220>
<223>  Long Spacer

<400>  62
gagagcaagt acggaccgcc ctgcccccct tgccctgccc ccgagttcct gggcggaccc      60

agcgtgttcc tgttcccccc caagcccaag gacaccctga tgatcagccg gacccccgag     120

gtgacctgcg tggtggtgga cgtgagccag gaagatcccg aggtccagtt caattggtac     180

gtggacggcg tggaagtgca caacgccaag accaagccca gagaggaaca gttcaacagc     240

acctaccggg tggtgtctgt gctgaccgtg ctgcaccagg actggctgaa cggcaaagaa     300

tacaagtgca aggtgtccaa caagggcctg cccagcagca tcgaaaagac catcagcaag     360

gccaagggcc agcctcgcga gccccaggtg tacaccctgc ctccctccca ggaagagatg     420

accaagaacc aggtgtccct gacctgcctg gtgaagggct tctaccccag cgacatcgcc     480

gtggagtggg agagcaacgg ccagcctgag aacaactaca agaccacccc tcccgtgctg     540

gacagcgacg gcagcttctt cctgtacagc cggctgaccg tggacaagag ccggtggcag     600

gaaggcaacg tctttagctg cagcgtgatg cacgaggccc tgcacaacca ctacacccag     660

aagagcctga gcctgtccct gggcaag                                         687

<210>  63
<211>  622
<212>  PRT
<213>  Homo sapiens

<400>  63

<210>  64
<211>  18
<212>  DNA
<213>  Artificial Sequence

<220>
<223>  mid-Ampr primer

<400>  64
ttgagagttt tcgccccg                                                    18

<210>  65
<211>  13
<212>  PRT
<213>  Artificial Sequence

<220>
<223>  Modified Human IgG4

<400>  65

Glu Val Val Lys Tyr Gly Pro Pro Cys Pro Pro Cys Pro
1               5                   10

<210>  66
<211>  10
<212>  PRT
<213>  Artificial Sequence

<220>
<223>  Modified Human IgG4

<400>  66

Lys Tyr Gly Pro Pro Cys Pro Pro Cys Pro
1               5                   10

<210>  67
<211>  9
<212>  PRT
<213>  Artificial Sequence

<220>
<223>  Modified Human IgG4

<400>  67

Tyr Gly Pro Pro Cys Pro Pro Cys Pro
1               5

<210>  68
<211>  12
<212>  PRT
<213>  Artificial Sequence

<220>

<223>  Modified Human IgG4

<400>  68

Glu Ser Lys Tyr Gly Pro Pro Cys Pro Pro Cys Pro
1               5                   10


<210>  69
<211>  750
<212>  PRT
<213>  Homo sapiens

<400>  69

Met Trp Asn Leu Leu His Glu Thr Asp Ser Ala Val Ala Thr Ala Arg
1               5                   10                  15

Arg Pro Arg Trp Leu Cys Ala Gly Ala Leu Val Leu Ala Gly Gly Phe
            20                  25                  30

Phe Leu Leu Gly Phe Leu Phe Gly Trp Phe Ile Lys Ser Ser Asn Glu
        35                  40                  45

Ala Thr Asn Ile Thr Pro Lys His Asn Met Lys Ala Phe Leu Asp Glu
    50                  55                  60

Leu Lys Ala Glu Asn Ile Lys Lys Phe Leu Tyr Asn Phe Thr Gln Ile
65                  70                  75                  80

Pro His Leu Ala Gly Thr Glu Gln Asn Phe Gln Leu Ala Lys Gln Ile
                85                  90                  95

Gln Ser Gln Trp Lys Glu Phe Gly Leu Asp Ser Val Glu Leu Ala His
            100                 105                 110

Tyr Asp Val Leu Leu Ser Tyr Pro Asn Lys Thr His Pro Asn Tyr Ile
        115                 120                 125

Ser Ile Ile Asn Glu Asp Gly Asn Glu Ile Phe Asn Thr Ser Leu Phe
    130                 135                 140

Glu Pro Pro Pro Pro Gly Tyr Glu Asn Val Ser Asp Ile Val Pro Pro
145                 150                 155                 160

Phe Ser Ala Phe Ser Pro Gln Gly Met Pro Glu Gly Asp Leu Val Tyr
                165                 170                 175

Val Asn Tyr Ala Arg Thr Glu Asp Phe Phe Lys Leu Glu Arg Asp Met
            180                 185                 190

Lys Ile Asn Cys Ser Gly Lys Ile Val Ile Ala Arg Tyr Gly Lys Val
        195                 200                 205

Phe Arg Gly Asn Lys Val Lys Asn Ala Gln Leu Ala Gly Ala Lys Gly
    210                 215                 220

Val Ile Leu Tyr Ser Asp Pro Ala Asp Tyr Phe Ala Pro Gly Val Lys
225                 230                 235                 240

Ser Tyr Pro Asp Gly Trp Asn Leu Pro Gly Gly Gly Val Gln Arg Gly
            245                 250                 255

Asn Ile Leu Asn Leu Asn Gly Ala Gly Asp Pro Leu Thr Pro Gly Tyr
        260                 265                 270

Pro Ala Asn Glu Tyr Ala Tyr Arg Arg Gly Ile Ala Glu Ala Val Gly
        275                 280                 285

Leu Pro Ser Ile Pro Val His Pro Ile Gly Tyr Tyr Asp Ala Gln Lys
    290                 295                 300

Leu Leu Glu Lys Met Gly Gly Ser Ala Pro Pro Asp Ser Ser Trp Arg
305                 310                 315                 320

Gly Ser Leu Lys Val Pro Tyr Asn Val Gly Pro Gly Phe Thr Gly Asn
            325                 330                 335

Phe Ser Thr Gln Lys Val Lys Met His Ile His Ser Thr Asn Glu Val
        340                 345                 350

Thr Arg Ile Tyr Asn Val Ile Gly Thr Leu Arg Gly Ala Val Glu Pro
        355                 360                 365

Asp Arg Tyr Val Ile Leu Gly Gly His Arg Asp Ser Trp Val Phe Gly
    370                 375                 380

Gly Ile Asp Pro Gln Ser Gly Ala Ala Val Val His Glu Ile Val Arg
385                 390                 395                 400

Ser Phe Gly Thr Leu Lys Lys Glu Gly Trp Arg Pro Arg Arg Thr Ile
            405                 410                 415

Leu Phe Ala Ser Trp Asp Ala Glu Glu Phe Gly Leu Leu Gly Ser Thr
        420                 425                 430

Glu Trp Ala Glu Glu Asn Ser Arg Leu Leu Gln Glu Arg Gly Val Ala
        435                 440                 445

Tyr Ile Asn Ala Asp Ser Ser Ile Glu Gly Asn Tyr Thr Leu Arg Val
    450                 455                 460

Asp Cys Thr Pro Leu Met Tyr Ser Leu Val His Asn Leu Thr Lys Glu
465                 470                 475                 480

Leu Lys Ser Pro Asp Glu Gly Phe Glu Gly Lys Ser Leu Tyr Glu Ser
                485                 490                 495

Trp Thr Lys Lys Ser Pro Ser Pro Glu Phe Ser Gly Met Pro Arg Ile
            500                 505                 510

Ser Lys Leu Gly Ser Gly Asn Asp Phe Glu Val Phe Phe Gln Arg Leu
        515                 520                 525

Gly Ile Ala Ser Gly Arg Ala Arg Tyr Thr Lys Asn Trp Glu Thr Asn
    530                 535                 540

Lys Phe Ser Gly Tyr Pro Leu Tyr His Ser Val Tyr Glu Thr Tyr Glu
545                 550                 555                 560

Leu Val Glu Lys Phe Tyr Asp Pro Met Phe Lys Tyr His Leu Thr Val
                565                 570                 575

Ala Gln Val Arg Gly Gly Met Val Phe Glu Leu Ala Asn Ser Ile Val
            580                 585                 590

Leu Pro Phe Asp Cys Arg Asp Tyr Ala Val Val Leu Arg Lys Tyr Ala
        595                 600                 605

Asp Lys Ile Tyr Ser Ile Ser Met Lys His Pro Gln Glu Met Lys Thr
    610                 615                 620

Tyr Ser Val Ser Phe Asp Ser Leu Phe Ser Ala Val Lys Asn Phe Thr
625                 630                 635                 640

Glu Ile Ala Ser Lys Phe Ser Glu Arg Leu Gln Asp Phe Asp Lys Ser
                645                 650                 655

Asn Pro Ile Val Leu Arg Met Met Asn Asp Gln Leu Met Phe Leu Glu
            660                 665                 670

Arg Ala Phe Ile Asp Pro Leu Gly Leu Pro Asp Arg Pro Phe Tyr Arg
        675                 680                 685

His Val Ile Tyr Ala Pro Ser Ser His Asn Lys Tyr Ala Gly Glu Ser
    690                 695                 700

Phe Pro Gly Ile Tyr Asp Ala Leu Phe Asp Ile Glu Ser Lys Val Asp
705                 710                 715                 720

Pro Ser Lys Ala Trp Gly Glu Val Lys Arg Gln Ile Tyr Val Ala Ala
                725                 730                 735

Phe Thr Val Gln Ala Ala Ala Glu Thr Leu Ser Glu Val Ala
            740                 745                 750

<210>  70
<211>  24
<212>  DNA
<213>  Artificial Sequence

<220>
<223>  post-Ampr primer

<400>  70
aatagacaga tcgctgagat aggt                                              24

<210>  71
<211>  26
<212>  DNA
<213>  Artificial Sequence

<220>
<223>  pre-U5 primer

<400>  71
atcaaaagaa tagaccgaga tagggt                                            26

<210>  72
<211>  123
<212>  PRT
<213>  Homo sapiens

<400>  72

Met Lys Ala Val Leu Leu Ala Leu Leu Met Ala Gly Leu Ala Leu Gln
1               5                   10                  15

Pro Gly Thr Ala Leu Leu Cys Tyr Ser Cys Lys Ala Gln Val Ser Asn
            20                  25                  30

Glu Asp Cys Leu Gln Val Glu Asn Cys Thr Gln Leu Gly Glu Gln Cys
        35                  40                  45

Trp Thr Ala Arg Ile Arg Ala Val Gly Leu Leu Thr Val Ile Ser Lys
    50                  55                  60

Gly Cys Ser Leu Asn Cys Val Asp Asp Ser Gln Asp Tyr Tyr Val Gly
65                  70                  75                  80

Lys Lys Asn Ile Thr Cys Cys Asp Thr Asp Leu Cys Asn Ala Ser Gly
                85                  90                  95

Ala His Ala Leu Gln Pro Ala Ala Ala Ile Leu Ala Leu Leu Pro Ala
            100                 105                 110

Leu Gly Leu Leu Leu Trp Gly Pro Gly Gln Leu
        115                 120

<210>  73
<211>  20
<212>  DNA
<213>  Artificial Sequence

<220>
<223>  psi primer

<400>  73
gcagggagct agaacgattc                                                   20

<210>  74
<211>  10014
<212>  DNA
<213>  Artificial Sequence

<220>
<223>  R11 intermediate spacer CAR: PJ_R11-CH3-41BB-Z-T2A-tEGFR

<400>  74
gttagaccag atctgagcct gggagctctc tggctaacta gggaacccac tgcttaagcc      60

tcaataaagc ttgccttgag tgcttcaagt agtgtgtgcc cgtctgttgt gtgactctgg     120

taactagaga tccctcagac ccttttagtc agtgtggaaa atctctagca gtggcgcccg     180

aacagggact tgaaagcgaa agggaaacca gaggagctct ctcgacgcag gactcggctt     240

gctgaagcgc gcacggcaag aggcgagggg cggcgactgg tgagtacgcc aaaaattttg     300

actagcggag gctagaagga gagagatggg tgcgagagcg tcagtattaa gcgggggaga     360

attagatcga tgggaaaaaa ttcggttaag gccaggggga aagaaaaaat ataaattaaa     420

acatatagta tgggcaagca gggagctaga acgattcgca gttaatcctg gcctgttaga     480

aacatcagaa ggctgtagac aaatactggg acagctacaa ccatcccttc agacaggatc     540

agaagaactt agatcattat ataatacagt agcaaccctc tattgtgtgc atcaaaggat     600

agagataaaa gacaccaagg aagctttaga caagatagag gaagagcaaa acaaaagtaa     660

gaaaaaagca cagcaagcag cagctgacac aggacacagc aatcaggtca gccaaaatta     720

ccctatagtg cagaacatcc aggggcaaat ggtacatcag gccatatcac ctagaacttt     780

aaatgcatgg gtaaaagtag tagaagagaa ggctttcagc ccagaagtga tacccatgtt     840

ttcagcatta tcagaaggag ccaccccaca agatttaaac accatgctaa acacagtggg 900

gggacatcaa gcagccatgc aaatgttaaa agagaccatc aatgaggaag ctgcaggcaa 960

agagaagagt ggtgcagaga gaaaaaagag cagtgggaat aggagctttg ttccttgggt 1020

tcttgggagc agcaggaagc actatgggcg cagcgtcaat gacgctgacg gtacaggcca 1080

gacaattatt gtctggtata gtgcagcagc agaacaattt gctgagggct attgaggcgc 1140

aacagcatct gttgcaactc acagtctggg gcatcaagca gctccaggca agaatcctgg 1200

ctgtggaaag atacctaaag gatcaacagc tcctggggat ttggggttgc tctggaaaac 1260

tcatttgcac cactgctgtg ccttggatct acaaatggca gtattcatcc acaattttaa 1320

aagaaaaggg gggattgggg ggtacagtgc aggggaaaga atagtagaca taatagcaac 1380

agacatacaa actaaagaat tacaaaaaca aattacaaaa attcaaaatt ttcgggttta 1440

ttacagggac agcagagatc cagtttgggg atcaattgca tgaagaatct gcttagggtt 1500

aggcgttttg cgctgcttcg cgaggatctg cgatcgctcc ggtgcccgtc agtgggcaga 1560

gcgcacatcg cccacagtcc ccgagaagtt ggggggaggg gtcggcaatt gaaccggtgc 1620

ctagagaagg tggcgcgggg taaactggga aagtgatgtc gtgtactggc tccgcctttt 1680

tcccgagggt gggggagaac cgtatataag tgcagtagtc gccgtgaacg ttctttttcg 1740

caacgggttt gccgccagaa cacagctgaa gcttcgaggg gctcgcatct ctccttcacg 1800

cgcccgccgc cctacctgag gccgccatcc acgccggttg agtcgcgttc tgccgcctcc 1860

cgcctgtggt gcctcctgaa ctgcgtccgc cgtctaggta agtttaaagc tcaggtcgag 1920

accgggcctt tgtccggcgc tcccttggag cctacctaga ctcagccggc tctccacgct 1980

ttgcctgacc ctgcttgctc aactctacgt ctttgtttcg ttttctgttc tgcgccgtta 2040

cagatccaag ctgtgaccgg cgcctacggc tagcgaattc gccaccatgc tgctgctggt 2100

gacaagcctg ctgctgtgcg agctgcccca ccccgccttt ctgctgatcc cccagagcgt 2160

gaaagagtcc gagggcgacc tggtcacacc agccggcaac ctgaccctga cctgtaccgc 2220

cagcggcagc gacatcaacg actaccccat ctcttgggtc cgccaggctc ctggcaaggg 2280

actggaatgg atcggcttca tcaacagcgg cggcagcact tggtacgcca gctgggtcaa 2340

aggccggttc accatcagcc ggaccagcac caccgtggac ctgaagatga caagcctgac 2400

caccgacgac accgccacct acttttgcgc cagaggctac agcacctact acggcgactt 2460

caacatctgg ggccctggca ccctggtcac aatctctagc ggcggaggcg gcagcggagg 2520

tggaggaagt ggcggcggag gatccgagct ggtcatgacc cagaccccca gcagcacatc 2580

tggcgccgtg ggcggcaccg tgaccatcaa ttgccaggcc agccagagca tcgacagcaa 2640

cctggcctgg ttccagcaga agcccggcca gccccccacc ctgctgatct acagagcctc 2700

caacctggcc agcggcgtgc caagcagatt cagcggcagc agatctggca ccgagtacac 2760

cctgaccatc tccggcgtgc agagagagga cgccgctacc tattactgcc tgggcggcgt 2820

gggcaacgtg tcctacagaa ccagcttcgg cggaggtact gaggtggtcg tcaaatagga 2880

ccgccctgcc ccccttgccc tgcccccgag ttcctgggcg gacccagcgt gttcctgttc 2940

ccccccaagc ccaaggacac cctgatgatc agccggaccc ccgaggtgac ctgcgtggtg 3000

gtggacgtga gccaggaaga tcccgaggtc cagttcaatt ggtacgtgga cggcgtggaa 3060

gtgcacaacg ccaagaccaa gcccagagag gaacagttca acagcaccta ccgggtggtg 3120

tctgtgctga ccgtgctgca ccaggactgg ctgaacggca aagaatacaa gtgcaaggtg 3180

tccaacaagg gcctgcccag cagcatcgaa aagaccatca gcaaggccaa gggccagcct 3240

cgcgagcccc aggtgtacac cctgcctccc tcccaggaag agatgaccaa gaaccaggtg 3300

tccctgacct gcctggtgaa gggcttctac cccagcgaca tcgccgtgga gtgggagagc 3360

aacggccagc ctgagaacaa ctacaagacc acccctcccg tgctggacag cgacggcagc 3420

ttcttcctgt acagccggct gaccgtggac aagagccggt ggcaggaagg caacgtcttt 3480

agctgcagcg tgatgcacga ggccctgcac aaccactaca cccagaagag cctgagcctg 3540

tccctgggca agatgttctg ggtgctggtg gtggtgggcg gggtgctggc ctgctacagc 3600

ctgctggtga cagtggcctt catcatcttt tgggtgaaac ggggcagaaa gaaactcctg 3660

tatatattca aacaaccatt tatgagacca gtacaaacta ctcaagagga agatggctgt 3720

agctgccgat ttccagaaga agaagaagga ggatgtgaac tgcgggtgaa gttcagcaga 3780

agcgccgacg cccctgccta ccagcagggc cagaatcagc tgtacaacga gctgaacctg 3840

ggcagaaggg aagagtacga cgtcctggat aagcggagag gccgggaccc tgagatgggc 3900

ggcaagcctc ggcggaagaa cccccaggaa ggcctgtata acgaactgca gaaagacaag 3960

atggccgagg cctacagcga gatcggcatg aagggcgagc ggaggcgggg caagggccac 4020

gacggcctgt atcagggcct gtccaccgcc accaaggata cctacgacgc cctgcacatg 4080

caggccctgc ccccaaggct cgagggcggc ggagagggca gaggaagtct tctaacatgc 4140

ggtgacgtgg aggagaatcc cggccctagg atgcttctcc tggtgacaag ccttctgctc 4200

tgtgagttac cacacccagc attcctcctg atcccacgca aagtgtgtaa cggaataggt 4260

attggtgaat ttaaagactc actctccata aatgctacga atattaaaca cttcaaaaac 4320

tgcacctcca tcagtggcga tctccacatc ctgccggtgg catttagggg tgactccttc 4380

acacatactc ctcctctgga tccacaggaa ctggatattc tgaaaaccgt aaaggaaatc 4440

acagggtttt tgctgattca ggcttggcct gaaaacagga cggacctcca tgcctttgag 4500

aacctagaaa tcatacgcgg caggaccaag caacatggtc agttttctct tgcagtcgtc 4560

agcctgaaca taacatcctt gggattacgc tccctcaagg agataagtga tggagatgtg 4620

ataatttcag gaaacaaaaa tttgtgctat gcaaatacaa taaactggaa aaaactgttt 4680

gggacctccg gtcagaaaac caaaattata agcaacagag gtgaaaacag ctgcaaggcc 4740

acaggccagg tctgccatgc cttgtgctcc cccgagggct gctggggccc ggagcccagg 4800

gactgcgtct cttgccggaa tgtcagccga ggcagggaat gcgtggacaa gtgcaacctt 4860

ctggagggtg agccaaggga gtttgtggag aactctgagt gcatacagtg ccacccagag 4920

tgcctgcctc aggccatgaa catcacctgc acaggacggg gaccagacaa ctgtatccag 4980

tgtgcccact acattgacgg cccccactgc gtcaagacct gcccggcagg agtcatggga 5040

gaaaacaaca ccctggtctg gaagtacgca gacgccggcc atgtgtgcca cctgtgccat 5100

ccaaactgca cctacggatg cactgggcca ggtcttgaag gctgtccaac gaatgggcct 5160

aagatcccgt ccatcgccac tgggatggtg ggggccctcc tcttgctgct ggtggtggcc 5220

ctggggatcg gcctcttcat gtgagcggcc gctctagacc cgggctgcag gaattcgata 5280

tcaagcttat cgataatcaa cctctggatt acaaaatttg tgaaagattg actggtattc 5340

ttaactatgt tgctcctttt acgctatgtg gatacgctgc tttaatgcct ttgtatcatg 5400

ctattgcttc ccgtatggct ttcattttct cctccttgta taaatcctgg ttgctgtctc 5460

tttatgagga gttgtggccc gttgtcaggc aacgtggcgt ggtgtgcact gtgtttgctg 5520

acgcaacccc cactggttgg ggcattgcca ccacctgtca gctcctttcc gggactttcg 5580

ctttccccct ccctattgcc acggcggaac tcatcgccgc ctgccttgcc cgctgctgga 5640

caggggctcg gctgttgggc actgacaatt ccgtggtgtt gtcggggaaa tcatcgtcct 5700

ttccttggct gctcgcctgt gttgccacct ggattctgcg cgggacgtcc ttctgctacg 5760

tcccttcggc cctcaatcca gcggaccttc cttcccgcgg cctgctgccg gctctgcggc 5820

ctcttccgcg tcttcgcctt cgccctcaga cgagtcggat ctccctttgg gccgcctccc 5880

cgcatcgata ccgtcgacta gccgtacctt taagaccaat gacttacaag gcagctgtag 5940

atcttagcca ctttttaaaa gaaaaggggg gactggaagg gctaattcac tcccaaagaa 6000

gacaagatct gctttttgcc tgtactgggt ctctctggtt agaccagatc tgagcctggg 6060

agctctctgg ctaactaggg aacccactgc ttaagcctca ataaagcttg ccttgagtgc 6120

ttcaagtagt gtgtgcccgt ctgttgtgtg actctggtaa ctagagatcc ctcagaccct 6180

tttagtcagt gtggaaaatc tctagcagaa ttcgatatca agcttatcga taccgtcgac 6240

ctcgaggggg ggcccggtac ccaattcgcc ctatagtgag tcgtattaca attcactggc 6300

cgtcgtttta caacgtcgtg actgggaaaa ccctggcgtt acccaactta atcgccttgc 6360

agcacatccc cctttcgcca gctggcgtaa tagcgaagag gcccgcaccg atcgcccttc 6420

ccaacagttg cgcagcctga atggcgaatg gaaattgtaa gcgttaatat tttgttaaaa 6480

ttcgcgttaa atttttgtta aatcagctca ttttttaacc aataggccga aatcggcaaa 6540

atcccttata aatcaaaaga atagaccgag atagggttga gtgttgttcc agtttggaac 6600

aagagtccac tattaaagaa cgtggactcc aacgtcaaag ggcgaaaaac cgtctatcag 6660

ggcgatggcc cactacgtga accatcaccc taatcaagtt ttttggggtc gaggtgccgt 6720

aaagcactaa atcggaaccc taaagggagc ccccgattta gagcttgacg gggaaagccg 6780

gcgaacgtgg cgagaaagga agggaagaaa gcgaaaggag cgggcgctag ggcgctggca 6840

agtgtagcgg tcacgctgcg cgtaaccacc acacccgccg cgcttaatgc gccgctacag 6900

ggcgcgtcag gtggcacttt tcggggaaat gtgcgcggaa cccctatttg tttatttttc 6960

taaatacatt caaatatgta tccgctcatg agacaataac cctgataaat gcttcaataa 7020

tattgaaaaa ggaagagtat gagtattcaa catttccgtg tcgcccttat tccctttttt 7080

gcggcatttt gccttcctgt ttttgctcac ccagaaacgc tggtgaaagt aaaagatgct 7140

gaagatcagt tgggtgcacg agtgggttac atcgaactgg atctcaacag cggtaagatc 7200

cttgagagtt ttcgccccga agaacgtttt ccaatgatga gcacttttaa agttctgcta 7260

tgtggcgcgg tattatcccg tattgacgcc gggcaagagc aactcggtcg ccgcatacac 7320

tattctcaga atgacttggt tgagtactca ccagtcacag aaaagcatct tacggatggc 7380

atgacagtaa gagaattatg cagtgctgcc ataaccatga gtgataacac tgcggccaac 7440

ttacttctga caacgatcgg aggaccgaag gagctaaccg cttttttgca caacatgggg 7500

gatcatgtaa ctcgccttga tcgttgggaa ccggagctga atgaagccat accaaacgac 7560

gagcgtgaca ccacgatgcc tgtagcaatg gcaacaacgt tgcgcaaact attaactggc 7620

gaactactta ctctagcttc ccggcaacaa ttaatagact ggatggaggc ggataaagtt 7680

gcaggaccac ttctgcgctc ggcccttccg gctggctggt ttattgctga taaatctgga 7740

gccggtgagc gtgggtctcg cggtatcatt gcagcactgg ggccagatgg taagccctcc 7800

cgtatcgtag ttatctacac gacggggagt caggcaacta tggatgaacg aaatagacag 7860

atcgctgaga taggtgcctc actgattaag cattggtaac tgtcagacca agtttactca 7920

tatatacttt agattgattt aaaacttcat ttttaattta aaaggatcta ggtgaagatc 7980

ctttttgata atctcatgac caaaatccct taacgtgagt tttcgttcca ctgagcgtca 8040

gaccccgtag aaaagatcaa aggatcttct tgagatcctt tttttctgcg cgtaatctgc 8100

tgcttgcaaa caaaaaaacc accgctacca gcggtggttt gtttgccgga tcaagagcta 8160

ccaactcttt ttccgaaggt aactggcttc agcagagcgc agataccaaa tactgttctt 8220

ctagtgtagc cgtagttagg ccaccacttc aagaactctg tagcaccgcc tacatacctc 8280

gctctgctaa tcctgttacc agtggctgct gccagtggcg ataagtcgtg tcttaccggg 8340

ttggactcaa gacgatagtt accggataag gcgcagcggt cgggctgaac ggggggttcg 8400

tgcacacagc ccagcttgga gcgaacgacc tacaccgaac tgagatacct acagcgtgag 8460

ctatgagaaa gcgccacgct tcccgaaggg agaaaggcgg acaggtatcc ggtaagcggc 8520

agggtcggaa caggagagcg cacgagggag cttccagggg gaaacgcctg gtatctttat 8580

agtcctgtcg ggtttcgcca cctctgactt gagcgtcgat ttttgtgatg ctcgtcaggg 8640

gggcggagcc tatggaaaaa cgccagcaac gcggcctttt tacggttcct ggccttttgc 8700

tggccttttg ctcacatgtt ctttcctgcg ttatcccctg attctgtgga taaccgtatt 8760

accgcctttg agtgagctga taccgctcgc cgcagccgaa cgaccgagcg cagcgagtca 8820

gtgagcgagg aagcggaaga gcgcccaata cgcaaaccgc ctctccccgc gcgttggccg 8880

attcattaat gcagctggca cgacaggttt cccgactgga aagcgggcag tgagcgcaac 8940

gcaattaatg tgagttagct cactcattag gcaccccagg ctttacactt tatgcttccg 9000

gctcgtatgt tgtgtggaat tgtgagcgga taacaatttc acacaggaaa cagctatgac 9060

catgattacg ccaagctcga aattaaccct cactaaaggg aacaaaagct ggagctccac 9120

cgcggtggcg gcctcgaggt cgagatccgg tcgaccagca accatagtcc cgcccctaac 9180

tccgcccatc ccgcccctaa ctccgcccag ttccgcccat tctccgcccc atggctgact 9240

aatttttttt atttatgcag aggccgaggc cgcctcggcc tctgagctat tccagaagta 9300

gtgaggaggc ttttttggag gcctaggctt ttgcaaaaag cttcgacggt atcgattggc 9360

tcatgtccaa cattaccgcc atgttgacat tgattattga ctagttatta atagtaatca 9420

attacggggt cattagttca tagcccatat atggagttcc gcgttacata acttacggta 9480

aatggcccgc ctggctgacc gcccaacgac ccccgcccat tgacgtcaat aatgacgtat 9540

gttcccatag taacgccaat agggactttc cattgacgtc aatgggtgga gtatttacgg 9600

taaactgccc acttggcagt acatcaagtg tatcatatgc caagtacgcc ccctattgac 9660

gtcaatgacg gtaaatggcc cgcctggcat tatgcccagt acatgacctt atgggacttt 9720

cctacttggc agtacatcta cgtattagtc atcgctatta ccatggtgat gcggttttgg 9780

cagtacatca atgggcgtgg atagcggttt gactcacggg gatttccaag tctccacccc 9840

attgacgtca atgggagttt gttttggcac caaaatcaac gggactttcc aaaatgtcgt 9900

aacaactccg ccccattgac gcaaatgggc ggtaggcgtg tacggaattc ggagtggcga 9960

gccctcagat cctgcatata agcagctgct ttttgcctgt actgggtctc tctg 10014

<210> 75
<211> 10015
<212> DNA
<213> Artificial Sequence

<220>
<223> R11 long spacer CAR: PJ_R11-CH2-CH3-41BB-Z-T2A-tEGFR

<400>  75
gttagaccag atctgagcct gggagctctc tggctaacta gggaacccac tgcttaagcc      60

tcaataaagc ttgccttgag tgcttcaagt agtgtgtgcc cgtctgttgt gtgactctgg     120

taactagaga tccctcagac ccttttagtc agtgtggaaa atctctagca gtggcgcccg     180

aacagggact tgaaagcgaa agggaaacca gaggagctct ctcgacgcag gactcggctt     240

gctgaagcgc gcacggcaag aggcgagggg cggcgactgg tgagtacgcc aaaaattttg     300

actagcggag gctagaagga gagagatggg tgcgagagcg tcagtattaa gcgggggaga     360

attagatcga tgggaaaaaa ttcggttaag gccaggggga aagaaaaaat ataaattaaa     420

acatatagta tgggcaagca gggagctaga acgattcgca gttaatcctg gcctgttaga     480

aacatcagaa ggctgtagac aaatactggg acagctacaa ccatcccttc agacaggatc     540

agaagaactt agatcattat ataatacagt agcaaccctc tattgtgtgc atcaaaggat     600

agagataaaa gacaccaagg aagctttaga caagatagag gaagagcaaa acaaaagtaa     660

gaaaaaagca cagcaagcag cagctgacac aggacacagc aatcaggtca gccaaaatta     720

ccctatagtg cagaacatcc aggggcaaat ggtacatcag gccatatcac ctagaacttt     780

aaatgcatgg gtaaaagtag tagaagagaa ggctttcagc ccagaagtga tacccatgtt     840

ttcagcatta tcagaaggag ccaccccaca agatttaaac accatgctaa acacagtggg     900

gggacatcaa gcagccatgc aaatgttaaa agagaccatc aatgaggaag ctgcaggcaa     960

agagaagagt ggtgcagaga gaaaaaagag cagtgggaat aggagctttg ttccttgggt    1020

tcttgggagc agcaggaagc actatgggcg cagcgtcaat gacgctgacg gtacaggcca    1080

gacaattatt gtctggtata gtgcagcagc agaacaattt gctgagggct attgaggcgc    1140

aacagcatct gttgcaactc acagtctggg gcatcaagca gctccaggca agaatcctgg    1200

ctgtggaaag atacctaaag gatcaacagc tcctggggat ttggggttgc tctggaaaac    1260

tcatttgcac cactgctgtg ccttggatct acaaatggca gtattcatcc acaattttaa    1320

aagaaaaggg gggattgggg ggtacagtgc aggggaaaga atagtagaca taatagcaac    1380

agacatacaa actaaagaat tacaaaaaca aattacaaaa attcaaaatt ttcgggttta    1440

ttacagggac agcagagatc cagtttgggg atcaattgca tgaagaatct gcttagggtt    1500

aggcgttttg cgctgcttcg cgaggatctg cgatcgctcc ggtgcccgtc agtgggcaga    1560

gcgcacatcg cccacagtcc ccgagaagtt ggggggaggg gtcggcaatt gaaccggtgc    1620

ctagagaagg tggcgcgggg taaactggga aagtgatgtc gtgtactggc tccgcctttt    1680

tcccgagggt gggggagaac cgtatataag tgcagtagtc gccgtgaacg ttctttttcg    1740

caacgggttt gccgccagaa cacagctgaa gcttcgaggg gctcgcatct ctccttcacg    1800

cgcccgccgc cctacctgag gccgccatcc acgccggttg agtcgcgttc tgccgcctcc    1860

cgcctgtggt gcctcctgaa ctgcgtccgc cgtctaggta agtttaaagc tcaggtcgag 1920

accgggcctt tgtccggcgc tcccttggag cctacctaga ctcagccggc tctccacgct 1980

ttgcctgacc ctgcttgctc aactctacgt ctttgtttcg ttttctgttc tgcgccgtta 2040

cagatccaag ctgtgaccgg cgcctacggc tagcgaattc gccaccatgc tgctgctggt 2100

gacaagcctg ctgctgtgcg agctgcccca ccccgccttt ctgctgatcc cccagagcgt 2160

gaaagagtcc gagggcgacc tggtcacacc agccggcaac ctgaccctga cctgtaccgc 2220

cagcggcagc gacatcaacg actaccccat ctcttgggtc cgccaggctc ctggcaaggg 2280

actggaatgg atcggcttca tcaacagcgg cggcagcact tggtacgcca gctgggtcaa 2340

aggccggttc accatcagcc ggaccagcac caccgtggac ctgaagatga caagcctgac 2400

caccgacgac accgccacct acttttgcgc cagaggctac agcacctact acggcgactt 2460

caacatctgg ggccctggca ccctggtcac aatctctagc ggcggaggcg gcagcggagg 2520

tggaggaagt ggcggcggag gatccgagct ggtcatgacc cagaccccca gcagcacatc 2580

tggcgccgtg ggcggcaccg tgaccatcaa ttgccaggcc agccagagca tcgacagcaa 2640

cctggcctgg ttccagcaga agcccggcca gccccccacc ctgctgatct acagagcctc 2700

caacctggcc agcggcgtgc caagcagatt cagcggcagc agatctggca ccgagtacac 2760

cctgaccatc tccggcgtgc agagagagga cgccgctacc tattactgcc tgggcggcgt 2820

gggcaacgtg tcctacagaa ccagcttcgg cggaggtact gaggtggtcg tcaaatacgg 2880

accgccctgc cccccttgcc ctgcccccga gttcctgggc ggacccagcg tgttcctgtt 2940

cccccccaag cccaaggaca ccctgatgat cagccggacc cccgaggtga cctgcgtggt 3000

ggtggacgtg agccaggaag atcccgaggt ccagttcaat tggtacgtgg acggcgtgga 3060

agtgcacaac gccaagacca agcccagaga ggaacagttc aacagcacct accgggtggt 3120

gtctgtgctg accgtgctgc accaggactg gctgaacggc aaagaataca agtgcaaggt 3180

gtccaacaag ggcctgccca gcagcatcga aaagaccatc agcaaggcca agggccagcc 3240

tcgcgagccc caggtgtaca ccctgcctcc ctcccaggaa gagatgacca agaaccaggt 3300

gtccctgacc tgcctggtga agggcttcta ccccagcgac atcgccgtgg agtgggagag 3360

caacggccag cctgagaaca actacaagac cacccctccc gtgctggaca gcgacggcag 3420

cttcttcctg tacagccggc tgaccgtgga caagagccgg tggcaggaag gcaacgtctt 3480

tagctgcagc gtgatgcacg aggccctgca caaccactac acccagaaga gcctgagcct 3540

gtccctgggc aagatgttct gggtgctggt ggtggtgggc ggggtgctgg cctgctacag 3600

cctgctggtg acagtggcct tcatcatctt ttgggtgaaa cggggcagaa agaaactcct 3660

gtatatattc aaacaaccat ttatgagacc agtacaaact actcaagagg aagatggctg 3720

tagctgccga tttccagaag aagaagaagg aggatgtgaa ctgcgggtga agttcagcag 3780

aagcgccgac gcccctgcct accagcaggg ccagaatcag ctgtacaacg agctgaacct 3840

gggcagaagg gaagagtacg acgtcctgga taagcggaga ggccgggacc ctgagatggg 3900

cggcaagcct cggcggaaga acccccagga aggcctgtat aacgaactgc agaaagacaa 3960

gatggccgag gcctacagcg agatcggcat gaagggcgag cggaggcggg gcaagggcca 4020

cgacggcctg tatcagggcc tgtccaccgc caccaaggat acctacgacg ccctgcacat 4080

gcaggccctg cccccaaggc tcgagggcgg cggagagggc agaggaagtc ttctaacatg 4140

cggtgacgtg gaggagaatc ccggccctag gatgcttctc ctggtgacaa gccttctgct 4200

ctgtgagtta ccacacccag cattcctcct gatcccacgc aaagtgtgta acggaatagg 4260

tattggtgaa tttaaagact cactctccat aaatgctacg aatattaaac acttcaaaaa 4320

ctgcacctcc atcagtggcg atctccacat cctgccggtg gcatttaggg gtgactcctt 4380

cacacatact cctcctctgg atccacagga actggatatt ctgaaaaccg taaaggaaat 4440

cacagggttt ttgctgattc aggcttggcc tgaaaacagg acggacctcc atgcctttga 4500

gaacctagaa atcatacgcg gcaggaccaa gcaacatggt cagttttctc ttgcagtcgt 4560

cagcctgaac ataacatcct tgggattacg ctccctcaag gagataagtg atggagatgt 4620

gataatttca ggaaacaaaa atttgtgcta tgcaaataca ataaactgga aaaaactgtt 4680

tgggacctcc ggtcagaaaa ccaaaattat aagcaacaga ggtgaaaaca gctgcaaggc 4740

cacaggccag gtctgccatg ccttgtgctc ccccgagggc tgctggggcc cggagcccag 4800

ggactgcgtc tcttgccgga atgtcagccg aggcagggaa tgcgtggaca agtgcaacct 4860

tctggagggt gagccaaggg agtttgtgga gaactctgag tgcatacagt gccacccaga 4920

gtgcctgcct caggccatga acatcacctg cacaggacgg ggaccagaca actgtatcca 4980

gtgtgcccac tacattgacg gcccccactg cgtcaagacc tgcccggcag gagtcatggg 5040

agaaaacaac accctggtct ggaagtacgc agacgccggc catgtgtgcc acctgtgcca 5100

tccaaactgc acctacggat gcactgggcc aggtcttgaa ggctgtccaa cgaatgggcc 5160

taagatcccg tccatcgcca ctgggatggt gggggccctc ctcttgctgc tggtggtggc 5220

cctggggatc ggcctcttca tgtgagcggc cgctctagac ccgggctgca ggaattcgat 5280

atcaagctta tcgataatca acctctggat tacaaaattt gtgaaagatt gactggtatt 5340

cttaactatg ttgctccttt tacgctatgt ggatacgctg ctttaatgcc tttgtatcat 5400

gctattgctt cccgtatggc tttcattttc tcctccttgt ataaatcctg gttgctgtct 5460

ctttatgagg agttgtggcc cgttgtcagg caacgtggcg tggtgtgcac tgtgtttgct 5520

gacgcaaccc ccactggttg gggcattgcc accacctgtc agctcctttc cgggactttc 5580

gctttccccc tccctattgc cacggcggaa ctcatcgccg cctgccttgc ccgctgctgg 5640

acaggggctc ggctgttggg cactgacaat tccgtggtgt tgtcggggaa atcatcgtcc 5700

tttccttggc tgctcgcctg tgttgccacc tggattctgc gcgggacgtc cttctgctac 5760

gtcccttcgg ccctcaatcc agcggacctt ccttcccgcg gcctgctgcc ggctctgcgg 5820

cctcttccgc gtcttcgcct tcgccctcag acgagtcgga tctccctttg ggccgcctcc 5880

ccgcatcgat accgtcgact agccgtacct ttaagaccaa tgacttacaa ggcagctgta 5940

gatcttagcc actttttaaa agaaaagggg ggactggaag ggctaattca ctcccaaaga 6000

agacaagatc tgctttttgc ctgtactggg tctctctggt tagaccagat ctgagcctgg 6060

gagctctctg gctaactagg gaacccactg cttaagcctc aataaagctt gccttgagtg 6120

cttcaagtag tgtgtgcccg tctgttgtgt gactctggta actagagatc cctcagaccc 6180

ttttagtcag tgtggaaaat ctctagcaga attcgatatc aagcttatcg ataccgtcga 6240

cctcgagggg gggcccggta cccaattcgc cctatagtga gtcgtattac aattcactgg 6300

ccgtcgtttt acaacgtcgt gactgggaaa accctggcgt tacccaactt aatcgccttg 6360

cagcacatcc ccctttcgcc agctggcgta atagcgaaga ggcccgcacc gatcgccctt 6420

cccaacagtt gcgcagcctg aatggcgaat ggaaattgta agcgttaata ttttgttaaa 6480

attcgcgtta aatttttgtt aaatcagctc attttttaac caataggccg aaatcggcaa 6540

aatcccttat aaatcaaaag aatagaccga gatagggttg agtgttgttc cagtttggaa 6600

caagagtcca ctattaaaga acgtggactc caacgtcaaa gggcgaaaaa ccgtctatca 6660

gggcgatggc ccactacgtg aaccatcacc ctaatcaagt tttttggggt cgaggtgccg 6720

taaagcacta aatcggaacc ctaaagggag cccccgattt agagcttgac ggggaaagcc 6780

ggcgaacgtg gcgagaaagg aagggaagaa agcgaaagga gcgggcgcta gggcgctggc 6840

aagtgtagcg gtcacgctgc gcgtaaccac cacacccgcc gcgcttaatg cgccgctaca 6900

gggcgcgtca ggtggcactt ttcggggaaa tgtgcgcgga acccctattt gtttattttt 6960

ctaaatacat tcaaatatgt atccgctcat gagacaataa ccctgataaa tgcttcaata 7020

atattgaaaa aggaagagta tgagtattca acatttccgt gtcgccctta ttcccttttt 7080

tgcggcattt tgccttcctg tttttgctca cccagaaacg ctggtgaaag taaaagatgc 7140

tgaagatcag ttgggtgcac gagtgggtta catcgaactg gatctcaaca gcggtaagat 7200

ccttgagagt tttcgccccg aagaacgttt tccaatgatg agcactttta aagttctgct 7260

atgtggcgcg gtattatccc gtattgacgc cgggcaagag caactcggtc gccgcataca 7320

ctattctcag aatgacttgg ttgagtactc accagtcaca gaaaagcatc ttacggatgg 7380

catgacagta agagaattat gcagtgctgc cataaccatg agtgataaca ctgcggccaa 7440

cttacttctg acaacgatcg gaggaccgaa ggagctaacc gcttttttgc acaacatggg 7500

ggatcatgta actcgccttg atcgttggga accggagctg aatgaagcca taccaaacga 7560

cgagcgtgac accacgatgc ctgtagcaat ggcaacaacg ttgcgcaaac tattaactgg 7620

cgaactactt actctagctt cccggcaaca attaatagac tggatggagg cggataaagt 7680

tgcaggacca cttctgcgct cggcccttcc ggctggctgg tttattgctg ataaatctgg 7740

agccggtgag cgtgggtctc gcggtatcat tgcagcactg gggccagatg gtaagccctc 7800

ccgtatcgta gttatctaca cgacggggag tcaggcaact atggatgaac gaaatagaca 7860

gatcgctgag ataggtgcct cactgattaa gcattggtaa ctgtcagacc aagtttactc 7920

atatatactt tagattgatt taaaacttca tttttaattt aaaaggatct aggtgaagat 7980

cctttttgat aatctcatga ccaaaatccc ttaacgtgag ttttcgttcc actgagcgtc 8040

agaccccgta gaaaagatca aaggatcttc ttgagatcct ttttttctgc gcgtaatctg 8100

ctgcttgcaa acaaaaaaac caccgctacc agcggtggtt tgtttgccgg atcaagagct 8160

accaactctt tttccgaagg taactggctt cagcagagcg cagataccaa atactgttct 8220

tctagtgtag ccgtagttag gccaccactt caagaactct gtagcaccgc ctacatacct 8280

cgctctgcta atcctgttac cagtggctgc tgccagtggc gataagtcgt gtcttaccgg 8340

gttggactca agacgatagt taccggataa ggcgcagcgg tcgggctgaa cggggggttc 8400

gtgcacacag cccagcttgg agcgaacgac ctacaccgaa ctgagatacc tacagcgtga 8460

gctatgagaa agcgccacgc ttcccgaagg gagaaaggcg gacaggtatc cggtaagcgg 8520

cagggtcgga acaggagagc gcacgaggga gcttccaggg ggaaacgcct ggtatcttta 8580

tagtcctgtc gggtttcgcc acctctgact tgagcgtcga tttttgtgat gctcgtcagg 8640

ggggcggagc ctatggaaaa acgccagcaa cgcggccttt ttacggttcc tggccttttg 8700

ctggcctttt gctcacatgt tctttcctgc gttatcccct gattctgtgg ataaccgtat 8760

taccgccttt gagtgagctg ataccgctcg ccgcagccga acgaccgagc gcagcgagtc 8820

agtgagcgag gaagcggaag agcgcccaat acgcaaaccg cctctccccg cgcgttggcc 8880

gattcattaa tgcagctggc acgacaggtt tcccgactgg aaagcgggca gtgagcgcaa 8940

cgcaattaat gtgagttagc tcactcatta ggcaccccag gctttacact ttatgcttcc 9000

ggctcgtatg ttgtgtggaa ttgtgagcgg ataacaattt cacacaggaa acagctatga 9060

ccatgattac gccaagctcg aaattaaccc tcactaaagg gaacaaaagc tggagctcca 9120

ccgcggtggc ggcctcgagg tcgagatccg gtcgaccagc aaccatagtc ccgcccctaa 9180

ctccgcccat cccgccccta actccgccca gttccgccca ttctccgccc catggctgac 9240

taattttttt tatttatgca gaggccgagg ccgcctcggc ctctgagcta ttccagaagt 9300

agtgaggagg cttttttgga ggcctaggct tttgcaaaaa gcttcgacgg tatcgattgg 9360

ctcatgtcca acattaccgc catgttgaca ttgattattg actagttatt aatagtaatc 9420

aattacgggg tcattagttc atagcccata tatggagttc cgcgttacat aacttacggt 9480

aaatggcccg cctggctgac cgcccaacga cccccgccca ttgacgtcaa taatgacgta 9540

tgttcccata gtaacgccaa tagggacttt ccattgacgt caatgggtgg agtatttacg 9600

gtaaactgcc cacttggcag tacatcaagt gtatcatatg ccaagtacgc cccctattga 9660

cgtcaatgac ggtaaatggc ccgcctggca ttatgcccag tacatgacct tatgggactt 9720

tcctacttgg cagtacatct acgtattagt catcgctatt accatggtga tgcggttttg 9780

gcagtacatc aatgggcgtg gatagcggtt tgactcacgg ggatttccaa gtctccaccc 9840

cattgacgtc aatgggagtt tgttttggca ccaaaatcaa cgggactttc caaaatgtcg 9900

taacaactcc gccccattga cgcaaatggg cggtaggcgt gtacggaatt cggagtggcg 9960

agccctcaga tcctgcatat aagcagctgc tttttgcctg tactgggtct ctctg 10015

<210> 76
<211> 241
<212> PRT
<213> Homo sapiens

<400> 76

Gln Ser Val Lys Glu Ser Glu Gly Asp Leu Val Thr Pro Ala Gly Asn
1               5                   10                  15

Leu Thr Leu Thr Cys Thr Ala Ser Gly Ser Asp Ile Asn Asp Tyr Pro
            20                  25                  30

Ile Ser Trp Val Arg Gln Ala Pro Gly Lys Gly Leu Glu Trp Ile Gly
        35                  40                  45

Phe Ile Asn Ser Gly Gly Ser Thr Trp Tyr Ala Ser Trp Val Lys Gly
    50                  55                  60

Arg Phe Thr Ile Ser Arg Thr Ser Thr Thr Val Asp Leu Lys Met Thr
65                  70                  75                  80

Ser Leu Thr Thr Asp Asp Thr Ala Thr Tyr Phe Cys Ala Arg Gly Tyr
                85                  90                  95

Ser Thr Tyr Tyr Gly Asp Phe Asn Ile Trp Gly Pro Gly Thr Leu Val
            100                 105                 110

Thr Ile Ser Ser Gly Gly Gly Gly Ser Gly Gly Gly Gly Ser Gly Gly
        115                 120                 125

Gly Gly Ser Glu Leu Val Met Thr Gln Thr Pro Ser Ser Thr Ser Gly
    130                 135                 140

Ala Val Gly Gly Thr Val Thr Ile Asn Cys Gln Ala Ser Gln Ser Ile
145                 150                 155                 160

Asp Ser Asn Leu Ala Trp Phe Gln Gln Lys Pro Gly Gln Pro Pro Thr
                165                 170                 175

Leu Leu Ile Tyr Arg Ala Ser Asn Leu Ala Ser Gly Val Pro Ser Arg
            180                 185                 190

Phe Ser Gly Ser Arg Ser Gly Thr Glu Tyr Thr Leu Thr Ile Ser Gly
        195                 200                 205

Val Gln Arg Glu Asp Ala Ala Thr Tyr Tyr Cys Leu Gly Gly Val Gly
    210                 215                 220

Asn Val Ser Tyr Arg Thr Ser Phe Gly Gly Gly Thr Glu Val Val Val
225                 230                 235                 240

Lys

<210>  77
<211>  801
<212>  DNA
<213>  Homo sapiens

<400>  77
gaattcgcca ccatgctgct gctggtgaca agcctgctgc tgtgcgagct gccccacccc      60

gcctttctgc tgatccccca gagcgtgaaa gagtccgagg gcgacctggt cacaccagcc     120

ggcaacctga ccctgacctg taccgccagc ggcagcgaca tcaacgacta ccccatctct     180

tgggtccgcc aggctcctgg caagggactg gaatggatcg gcttcatcaa cagcggcggc     240

agcacttggt acgccagctg ggtcaaaggc cggttcacca tcagccggac cagcaccacc     300

gtggacctga agatgacaag cctgaccacc gacgacaccg ccacctactt ttgcgccaga     360

ggctacagca cctactacgg cgacttcaac atctggggcc ctggcaccct ggtcacaatc     420

tctagcggcg gaggcggcag cggaggtgga ggaagtggcg gcggaggatc cgagctggtc     480

atgacccaga cccccagcag cacatctggc gccgtgggcg gcaccgtgac catcaattgc     540

caggccagcc agagcatcga cagcaacctg gcctggttcc agcagaagcc cggccagccc     600

cccaccctgc tgatctacag agcctccaac ctggccagcg gcgtgccaag cagattcagc     660

ggcagcagat ctggcaccga gtacaccctg accatctccg gcgtgcagag agaggacgcc     720

gctacctatt actgcctggg cggcgtgggc aacgtgtcct acagaaccag cttcggcgga     780

ggtactgagg tggtcgtcaa a                                               801

<210> 78
<211> 9685
<212> DNA
<213> Artificial Sequence

<220>
<223> R11 short spacer CAR: PJ_R11- 41BB-Z-T2A-tEGFR

<400> 78
gttagaccag atctgagcct gggagctctc tggctaacta gggaacccac tgcttaagcc 60

tcaataaagc ttgccttgag tgcttcaagt agtgtgtgcc cgtctgttgt gtgactctgg 120

taactagaga tccctcagac ccttttagtc agtgtggaaa atctctagca gtggcgcccg 180

aacagggact tgaaagcgaa agggaaacca gaggagctct ctcgacgcag gactcggctt 240

gctgaagcgc gcacggcaag aggcgagggg cggcgactgg tgagtacgcc aaaaattttg 300

actagcggag gctagaagga gagagatggg tgcgagagcg tcagtattaa gcgggggaga 360

attagatcga tgggaaaaaa ttcggttaag gccaggggga aagaaaaaat ataaattaaa 420

acatatagta tgggcaagca gggagctaga acgattcgca gttaatcctg gcctgttaga 480

aacatcagaa ggctgtagac aaatactggg acagctacaa ccatcccttc agacaggatc 540

agaagaactt agatcattat ataatacagt agcaaccctc tattgtgtgc atcaaaggat 600

agagataaaa gacaccaagg aagctttaga caagatagag gaagagcaaa acaaaagtaa 660

gaaaaaagca cagcaagcag cagctgacac aggacacagc aatcaggtca gccaaaatta 720

ccctatagtg cagaacatcc aggggcaaat ggtacatcag gccatatcac ctagaacttt 780

aaatgcatgg gtaaaagtag tagaagagaa ggctttcagc ccagaagtga tacccatgtt 840

ttcagcatta tcagaaggag ccaccccaca agatttaaac accatgctaa acacagtggg 900

gggacatcaa gcagccatgc aaatgttaaa agagaccatc aatgaggaag ctgcaggcaa 960

agagaagagt ggtgcagaga gaaaaaagag cagtgggaat aggagctttg ttccttgggt 1020

tcttgggagc agcaggaagc actatgggcg cagcgtcaat gacgctgacg gtacaggcca 1080

gacaattatt gtctggtata gtgcagcagc agaacaattt gctgagggct attgaggcgc 1140

aacagcatct gttgcaactc acagtctggg gcatcaagca gctccaggca agaatcctgg 1200

ctgtggaaag atacctaaag gatcaacagc tcctggggat ttggggttgc tctggaaaac 1260

tcatttgcac cactgctgtg ccttggatct acaaatggca gtattcatcc acaattttaa 1320

aagaaaaggg gggattgggg ggtacagtgc aggggaaaga atagtagaca taatagcaac 1380

agacatacaa actaaagaat tacaaaaaca aattacaaaa attcaaaatt ttcgggttta 1440

ttacagggac agcagagatc cagtttgggg atcaattgca tgaagaatct gcttagggtt 1500

aggcgttttg cgctgcttcg cgaggatctg cgatcgctcc ggtgcccgtc agtgggcaga 1560

gcgcacatcg cccacagtcc ccgagaagtt ggggggaggg gtcggcaatt gaaccggtgc 1620

ctagagaagg tggcgcgggg taaactggga aagtgatgtc gtgtactggc tccgcctttt 1680

tcccgagggt gggggagaac cgtatataag tgcagtagtc gccgtgaacg ttctttttcg 1740

caacgggttt gccgccagaa cacagctgaa gcttcgaggg gctcgcatct ctccttcacg 1800

cgcccgccgc cctacctgag gccgccatcc acgccggttg agtcgcgttc tgccgcctcc 1860

cgcctgtggt gcctcctgaa ctgcgtccgc cgtctaggta agtttaaagc tcaggtcgag 1920

accgggcctt tgtccggcgc tcccttggag cctacctaga ctcagccggc tctccacgct 1980

ttgcctgacc ctgcttgctc aactctacgt ctttgtttcg ttttctgttc tgcgccgtta 2040

cagatccaag ctgtgaccgg cgcctacggc tagcgaattc gccaccatgc tgctgctggt 2100

gacaagcctg ctgctgtgcg agctgcccca ccccgccttt ctgctgatcc cccagagcgt 2160

gaaagagtcc gagggcgacc tggtcacacc agccggcaac ctgaccctga cctgtaccgc 2220

cagcggcagc gacatcaacg actaccccat ctcttgggtc cgccaggctc ctggcaaggg 2280

actggaatgg atcggcttca tcaacagcgg cggcagcact tggtacgcca gctgggtcaa 2340

aggccggttc accatcagcc ggaccagcac caccgtggac ctgaagatga caagcctgac 2400

caccgacgac accgccacct acttttgcgc cagaggctac agcacctact acggcgactt 2460

caacatctgg ggccctggca ccctggtcac aatctctagc ggcggaggcg gcagcggagg 2520

tggaggaagt ggcggcggag gatccgagct ggtcatgacc cagaccccca gcagcacatc 2580

tggcgccgtg ggcggcaccg tgaccatcaa ttgccaggcc agccagagca tcgacagcaa 2640

cctggcctgg ttccagcaga agcccggcca gccccccacc ctgctgatct acagagcctc 2700

caacctggcc agcggcgtgc caagcagatt cagcggcagc agatctggca ccgagtacac 2760

cctgaccatc tccggcgtgc agagagagga cgccgctacc tattactgcc tgggcggcgt 2820

gggcaacgtg tcctacagaa ccagcttcgg cggaggtact gaggtggtcg tcaaatacgg 2880

accgccctgc cccccttgcc ctggccagcc tcgcgagccc caggtgtaca ccctgcctcc 2940

ctcccaggaa gagatgacca agaaccaggt gtccctgacc tgcctggtga agggcttcta 3000

ccccagcgac atcgccgtgg agtgggagag caacggccag cctgagaaca actacaagac 3060

cacccctccc gtgctggaca gcgacggcag cttcttcctg tacagccggc tgaccgtgga 3120

caagagccgg tggcaggaag gcaacgtctt tagctgcagc gtgatgcacg aggccctgca 3180

caaccactac acccagaaga gcctgagcct gtccctgggc aagatgttct gggtgctggt 3240

ggtggtgggc ggggtgctgg cctgctacag cctgctggtg acagtggcct tcatcatctt 3300

ttgggtgaaa cggggcagaa agaaactcct gtatatattc aaacaaccat ttatgagacc 3360

agtacaaact actcaagagg aagatggctg tagctgccga tttccagaag aagaagaagg 3420

aggatgtgaa ctgcgggtga agttcagcag aagcgccgac gcccctgcct accagcaggg 3480

ccagaatcag ctgtacaacg agctgaacct gggcagaagg gaagagtacg acgtcctgga 3540

taagcggaga ggccgggacc ctgagatggg cggcaagcct cggcggaaga acccccagga 3600

aggcctgtat aacgaactgc agaaagacaa gatggccgag gcctacagcg agatcggcat 3660

gaagggcgag cggaggcggg gcaagggcca cgacggcctg tatcagggcc tgtccaccgc 3720

caccaaggat acctacgacg ccctgcacat gcaggccctg cccccaaggc tcgagggcgg 3780

cggagagggc agaggaagtc ttctaacatg cggtgacgtg gaggagaatc ccggccctag 3840

gatgcttctc ctggtgacaa gccttctgct ctgtgagtta ccacacccag cattcctcct 3900

gatcccacgc aaagtgtgta acggaatagg tattggtgaa tttaaagact cactctccat 3960

aaatgctacg aatattaaac acttcaaaaa ctgcacctcc atcagtggcg atctccacat 4020

cctgccggtg gcatttaggg gtgactcctt cacacatact cctcctctgg atccacagga 4080

actggatatt ctgaaaaccg taaaggaaat cacagggttt ttgctgattc aggcttggcc 4140

tgaaaacagg acggacctcc atgcctttga gaacctagaa atcatacgcg gcaggaccaa 4200

gcaacatggt cagttttctc ttgcagtcgt cagcctgaac ataacatcct tgggattacg 4260

ctccctcaag gagataagtg atggagatgt gataatttca ggaaacaaaa atttgtgcta 4320

tgcaaataca ataaactgga aaaaactgtt tgggacctcc ggtcagaaaa ccaaaattat 4380

aagcaacaga ggtgaaaaca gctgcaaggc cacaggccag gtctgccatg ccttgtgctc 4440

ccccgagggc tgctggggcc cggagcccag ggactgcgtc tcttgccgga atgtcagccg 4500

aggcagggaa tgcgtggaca agtgcaacct tctggagggt gagccaaggg agtttgtgga 4560

gaactctgag tgcatacagt gccacccaga gtgcctgcct caggccatga acatcacctg 4620

cacaggacgg ggaccagaca actgtatcca gtgtgcccac tacattgacg gcccccactg 4680

cgtcaagacc tgcccggcag gagtcatggg agaaaacaac accctggtct ggaagtacgc 4740

agacgccggc catgtgtgcc acctgtgcca tccaaactgc acctacggat gcactgggcc 4800

aggtcttgaa ggctgtccaa cgaatggccc taagatcccg tccatcgcca ctgggatggt 4860

gggggccctc ctcttgctgc tggtggtggc cctggggatc ggcctcttca tgtgagcggc 4920

cgctctagac ccgggctgca ggaattcgat atcaagctta tcgataatca acctctggat 4980

tacaaaattt gtgaaagatt gactggtatt cttaactatg ttgctccttt tacgctatgt 5040

ggatacgctg ctttaatgcc tttgtatcat gctattgctt cccgtatggc tttcattttc 5100

tcctccttgt ataaatcctg gttgctgtct ctttatgagg agttgtggcc cgttgtcagg 5160

caacgtggcg tggtgtgcac tgtgtttgct gacgcaaccc ccactggttg gggcattgcc 5220

accacctgtc agctcctttc cgggactttc gctttccccc tccctattgc cacggcggaa 5280

ctcatcgccg cctgccttgc ccgctgctgg acaggggctc ggctgttggg cactgacaat 5340

tccgtggtgt tgtcggggaa atcatcgtcc tttccttggc tgctcgcctg tgttgccacc 5400

tggattctgc gcgggacgtc cttctgctac gtcccttcgg ccctcaatcc agcggacctt 5460

ccttcccgcg gcctgctgcc ggctctgcgg cctcttccgc gtcttcgcct tcgccctcag 5520

acgagtcgga tctccctttg ggccgcctcc ccgcatcgat accgtcgact agccgtacct 5580

ttaagaccaa tgacttacaa ggcagctgta gatcttagcc actttttaaa agaaaagggg 5640

ggactggaag ggctaattca ctcccaaaga agacaagatc tgctttttgc ctgtactggg 5700

tctctctggt tagaccagat ctgagcctgg gagctctctg gctaactagg gaacccactg 5760

cttaagcctc aataaagctt gccttgagtg cttcaagtag tgtgtgcccg tctgttgtgt 5820

gactctggta actagagatc cctcagaccc ttttagtcag tgtggaaaat ctctagcaga 5880

attcgatatc aagcttatcg ataccgtcga cctcgagggg gggcccggta cccaattcgc 5940

cctatagtga gtcgtattac aattcactgg ccgtcgtttt acaacgtcgt gactgggaaa 6000

accctggcgt tacccaactt aatcgccttg cagcacatcc ccctttcgcc agctggcgta 6060

atagcgaaga ggcccgcacc gatcgccctt cccaacagtt gcgcagcctg aatggcgaat 6120

ggaaattgta agcgttaata ttttgttaaa attcgcgtta aatttttgtt aaatcagctc 6180

attttttaac caataggccg aaatcggcaa aatcccttat aaatcaaaag aatagaccga 6240

gatagggttg agtgttgttc cagtttggaa caagagtcca ctattaaaga acgtggactc 6300

caacgtcaaa gggcgaaaaa ccgtctatca gggcgatggc ccactacgtg aaccatcacc 6360

ctaatcaagt tttttggggt cgaggtgccg taaagcacta aatcggaacc ctaaagggag 6420

cccccgattt agagcttgac ggggaaagcc ggcgaacgtg gcgagaaagg aagggaagaa 6480

agcgaaagga gcgggcgcta gggcgctggc aagtgtagcg gtcacgctgc gcgtaaccac 6540

cacacccgcc gcgcttaatg cgccgctaca gggcgcgtca ggtggcactt ttcggggaaa 6600

tgtgcgcgga acccctattt gtttattttt ctaaatacat tcaaatatgt atccgctcat 6660

gagacaataa ccctgataaa tgcttcaata atattgaaaa aggaagagta tgagtattca 6720

acatttccgt gtcgccctta ttcccttttt tgcggcattt tgccttcctg tttttgctca 6780

cccagaaacg ctggtgaaag taaaagatgc tgaagatcag ttgggtgcac gagtgggtta 6840

catcgaactg gatctcaaca gcggtaagat ccttgagagt tttcgccccg aagaacgttt 6900

tccaatgatg agcactttta aagttctgct atgtggcgcg gtattatccc gtattgacgc 6960

cgggcaagag caactcggtc gccgcataca ctattctcag aatgacttgg ttgagtactc 7020

accagtcaca gaaaagcatc ttacggatgg catgacagta agagaattat gcagtgctgc 7080

cataaccatg agtgataaca ctgcggccaa cttacttctg acaacgatcg gaggaccgaa 7140

ggagctaacc gcttttttgc acaacatggg ggatcatgta actcgccttg atcgttggga 7200

accggagctg aatgaagcca taccaaacga cgagcgtgac accacgatgc ctgtagcaat 7260

ggcaacaacg ttgcgcaaac tattaactgg cgaactactt actctagctt cccggcaaca 7320

attaatagac tggatggagg cggataaagt tgcaggacca cttctgcgct cggcccttcc 7380

ggctggctgg tttattgctg ataaatctgg agccggtgag cgtgggtctc gcggtatcat 7440

tgcagcactg gggccagatg gtaagccctc ccgtatcgta gttatctaca cgacggggag 7500

tcaggcaact atggatgaac gaaatagaca gatcgctgag ataggtgcct cactgattaa 7560

gcattggtaa ctgtcagacc aagtttactc atatatactt tagattgatt taaaacttca 7620

tttttaattt aaaaggatct aggtgaagat cctttttgat aatctcatga ccaaaatccc 7680

ttaacgtgag ttttcgttcc actgagcgtc agaccccgta gaaaagatca aaggatcttc 7740

ttgagatcct ttttttctgc gcgtaatctg ctgcttgcaa acaaaaaaac caccgctacc 7800

agcggtggtt tgtttgccgg atcaagagct accaactctt tttccgaagg taactggctt 7860

cagcagagcg cagataccaa atactgttct tctagtgtag ccgtagttag gccaccactt 7920

caagaactct gtagcaccgc ctacatacct cgctctgcta atcctgttac cagtggctgc 7980

tgccagtggc gataagtcgt gtcttaccgg gttggactca agacgatagt taccggataa 8040

ggcgcagcgg tcgggctgaa cggggggttc gtgcacacag cccagcttgg agcgaacgac 8100

ctacaccgaa ctgagatacc tacagcgtga gctatgagaa agcgccacgc ttcccgaagg 8160

gagaaaggcg gacaggtatc cggtaagcgg cagggtcgga acaggagagc gcacgaggga 8220

gcttccaggg ggaaacgcct ggtatcttta tagtcctgtc gggtttcgcc acctctgact 8280

tgagcgtcga tttttgtgat gctcgtcagg ggggcggagc ctatggaaaa acgccagcaa 8340

cgcggccttt ttacggttcc tggccttttg ctggcctttt gctcacatgt tctttcctgc 8400

gttatcccct gattctgtgg ataaccgtat taccgccttt gagtgagctg ataccgctcg 8460

ccgcagccga acgaccgagc gcagcgagtc agtgagcgag gaagcggaag agcgcccaat 8520

acgcaaaccg cctctccccg cgcgttggcc gattcattaa tgcagctggc acgacaggtt 8580

tcccgactgg aaagcgggca gtgagcgcaa cgcaattaat gtgagttagc tcactcatta 8640

ggcaccccag gctttacact ttatgcttcc ggctcgtatg ttgtgtggaa ttgtgagcgg 8700

ataacaattt cacacaggaa acagctatga ccatgattac gccaagctcg aaattaaccc 8760

tcactaaagg gaacaaaagc tggagctcca ccgcggtggc ggcctcgagg tcgagatccg 8820

gtcgaccagc aaccatagtc ccgcccctaa ctccgcccat cccgccccta actccgccca 8880

gttccgccca ttctccgccc catggctgac taattttttt tatttatgca gaggccgagg 8940

ccgcctcggc ctctgagcta ttccagaagt agtgaggagg cttttttgga ggcctaggct 9000

tttgcaaaaa gcttcgacgg tatcgattgg ctcatgtcca acattaccgc catgttgaca 9060

ttgattattg actagttatt aatagtaatc aattacgggg tcattagttc atagcccata 9120

tatggagttc cgcgttacat aacttacggt aaatggcccg cctggctgac cgcccaacga 9180

cccccgccca ttgacgtcaa taatgacgta tgttcccata gtaacgccaa tagggacttt 9240

ccattgacgt caatgggtgg agtatttacg gtaaactgcc cacttggcag tacatcaagt 9300

gtatcatatg ccaagtacgc cccctattga cgtcaatgac ggtaaatggc ccgcctggca 9360

ttatgcccag tacatgacct tatgggactt tcctacttgg cagtacatct acgtattagt 9420

catcgctatt accatggtga tgcggttttg gcagtacatc aatgggcgtg gatagcggtt 9480

tgactcacgg ggatttccaa gtctccaccc cattgacgtc aatgggagtt tgttttggca 9540

ccaaaatcaa cgggactttc caaaatgtcg taacaactcc gccccattga cgcaaatggg 9600

cggtaggcgt gtacggaatt cggagtggcg agccctcaga tcctgcatat aagcagctgc 9660

tttttgcctg tactgggtct ctctg 9685

<210> 79
<211> 9721
<212> DNA
<213> Artificial Sequence

<220>
<223> R12 intermediate spacer CAR: PJ_R12-CH3-41BB-Z-T2A-tEGFR

<400> 79
gttagaccag atctgagcct gggagctctc tggctaacta gggaacccac tgcttaagcc 60

tcaataaagc ttgccttgag tgcttcaagt agtgtgtgcc cgtctgttgt gtgactctgg 120

taactagaga tccctcagac ccttttagtc agtgtggaaa atctctagca gtggcgcccg 180

aacagggact tgaaagcgaa agggaaacca gaggagctct ctcgacgcag gactcggctt 240

gctgaagcgc gcacggcaag aggcgagggg cggcgactgg tgagtacgcc aaaaattttg 300

actagcggag gctagaagga gagagatggg tgcgagagcg tcagtattaa gcgggggaga 360

attagatcga tgggaaaaaa ttcggttaag gccaggggga aagaaaaaat ataaattaaa 420

acatatagta tgggcaagca gggagctaga acgattcgca gttaatcctg gcctgttaga 480

aacatcagaa ggctgtagac aaatactggg acagctacaa ccatcccttc agacaggatc 540

agaagaactt agatcattat ataatacagt agcaaccctc tattgtgtgc atcaaaggat 600

agagataaaa gacaccaagg aagctttaga caagatagag gaagagcaaa acaaaagtaa 660

gaaaaaagca cagcaagcag cagctgacac aggacacagc aatcaggtca gccaaaatta 720

ccctatagtg cagaacatcc aggggcaaat ggtacatcag gccatatcac ctagaacttt 780

aaatgcatgg gtaaaagtag tagaagagaa ggctttcagc ccagaagtga tacccatgtt 840

ttcagcatta tcagaaggag ccaccccaca agatttaaac accatgctaa acacagtggg 900

gggacatcaa gcagccatgc aaatgttaaa agagaccatc aatgaggaag ctgcaggcaa 960

agagaagagt ggtgcagaga gaaaaaagag cagtgggaat aggagctttg ttccttgggt 1020

tcttgggagc agcaggaagc actatgggcg cagcgtcaat gacgctgacg gtacaggcca 1080

gacaattatt gtctggtata gtgcagcagc agaacaattt gctgagggct attgaggcgc 1140

aacagcatct gttgcaactc acagtctggg gcatcaagca gctccaggca agaatcctgg 1200

ctgtggaaag atacctaaag gatcaacagc tcctggggat ttggggttgc tctggaaaac 1260

tcatttgcac cactgctgtg ccttggatct acaaatggca gtattcatcc acaattttaa 1320

aagaaaaggg gggattgggg ggtacagtgc aggggaaaga atagtagaca taatagcaac 1380

agacatacaa actaaagaat tacaaaaaca aattacaaaa attcaaaatt ttcgggttta 1440

ttacagggac agcagagatc cagtttgggg atcaattgca tgaagaatct gcttagggtt 1500

aggcgttttg cgctgcttcg cgaggatctg cgatcgctcc ggtgcccgtc agtgggcaga 1560

gcgcacatcg cccacagtcc ccgagaagtt ggggggaggg gtcggcaatt gaaccggtgc 1620

ctagagaagg tggcgcgggg taaactggga aagtgatgtc gtgtactggc tccgcctttt 1680

tcccgagggt gggggagaac cgtatataag tgcagtagtc gccgtgaacg ttctttttcg 1740

caacgggttt gccgccagaa cacagctgaa gcttcgaggg gctcgcatct ctccttcacg 1800

cgcccgccgc cctacctgag gccgccatcc acgccggttg agtcgcgttc tgccgcctcc 1860

cgcctgtggt gcctcctgaa ctgcgtccgc cgtctaggta agtttaaagc tcaggtcgag 1920

accgggcctt tgtccggcgc tcccttggag cctacctaga ctcagccggc tctccacgct 1980

ttgcctgacc ctgcttgctc aactctacgt ctttgtttcg ttttctgttc tgcgccgtta 2040

cagatccaag ctgtgaccgg cgcctacggc tagcgaattc ctcgaggcca ccatgctgct 2100

gctggtgaca agcctgctgc tgtgcgagct gccccacccc gcctttctgc tgatccccca 2160

ggaacagctc gtcgaaagcg gcggcagact ggtgacacct ggcggcagcc tgaccctgag 2220

ctgcaaggcc agcggcttcg acttcagcgc ctactacatg agctgggtcc gccaggcccc 2280

tggcaaggga ctggaatgga tcgccaccat ctaccccagc agcggcaaga cctactacgc 2340

cacctgggtg aacggacggt tcaccatctc cagcgacaac gcccagaaca ccgtggacct 2400

gcagatgaac agcctgacag ccgccgaccg ggccacctac ttttgcgcca gagacagcta 2460

cgccgacgac ggcgccctgt tcaacatctg gggccctggc accctggtga caatctctag 2520

cggcggaggc ggatctggtg gcggaggaag tggcggcgga ggatctgagc tggtgctgac 2580

ccagagcccc tctgtgtctg ctgccctggg aagccctgcc aagatcacct gtaccctgag 2640

cagcgcccac aagaccgaca ccatcgactg gtatcagcag ctgcagggcg aggcccccag 2700

atacctgatg caggtgcaga gcgacggcag ctacaccaag aggccaggcg tgcccgaccg 2760

gttcagcgga tctagctctg gcgccgaccg ctacctgatc atccccagcg tgcaggccga 2820

tgacgaggcc gattactact gtggcgccga ctacatcggc ggctacgtgt tcggcggagg 2880

cacccagctg accgtgaccg gcgagtctaa gtacggaccg ccctgccccc cttgccctgg 2940

ccagcctcgc gagccccagg tgtacaccct gcctccctcc caggaagaga tgaccaagaa 3000

ccaggtgtcc ctgacctgcc tggtgaaggg cttctacccc agcgacatcg ccgtggagtg 3060

ggagagcaac ggccagcctg agaacaacta caagaccacc cctcccgtgc tggacagcga 3120

cggcagcttc ttcctgtaca gccggctgac cgtggacaag agccggtggc aggaaggcaa 3180

cgtctttagc tgcagcgtga tgcacgaggc cctgcacaac cactacaccc agaagagcct 3240

gagcctgtcc ctgggcaaga tgttctgggt gctggtggtg gtgggcgggg tgctggcctg 3300

ctacagcctg ctggtgacag tggccttcat catcttttgg gtgaaacggg gcagaaagaa 3360

actcctgtat atattcaaac aaccatttat gagaccagta caaactactc aagaggaaga 3420

tggctgtagc tgccgatttc cagaagaaga agaaggagga tgtgaactgc gggtgaagtt 3480

cagcagaagc gccgacgccc ctgcctacca gcagggccag aatcagctgt acaacgagct 3540

gaacctgggc agaagggaag agtacgacgt cctggataag cggagaggcc gggaccctga 3600

gatgggcggc aagcctcggc ggaagaaccc ccaggaaggc ctgtataacg aactgcagaa 3660

agacaagatg gccgaggcct acagcgagat cggcatgaag ggcgagcgga ggcggggcaa 3720

gggccacgac ggcctgtatc agggcctgtc caccgccacc aaggatacct acgacgccct 3780

gcacatgcag gccctgcccc caaggctcga gggcggcgga gagggcagag gaagtcttct 3840

aacatgcggt gacgtggagg agaatcccgg ccctaggatg cttctcctgg tgacaagcct 3900

tctgctctgt gagttaccac acccagcatt cctcctgatc ccacgcaaag tgtgtaacgg 3960

aataggtatt ggtgaattta aagactcact ctccataaat gctacgaata ttaaacactt 4020

caaaaactgc acctccatca gtggcgatct ccacatcctg ccggtggcat ttaggggtga 4080

ctccttcaca catactcctc ctctggatcc acaggaactg gatattctga aaaccgtaaa 4140

ggaaatcaca gggtttttgc tgattcaggc ttggcctgaa aacaggacgg acctccatgc 4200

ctttgagaac ctagaaatca tacgcggcag gaccaagcaa catggtcagt tttctcttgc 4260

agtcgtcagc ctgaacataa catccttggg attacgctcc ctcaaggaga taagtgatgg 4320

agatgtgata atttcaggaa acaaaaattt gtgctatgca aatacaataa actggaaaaa 4380

actgtttggg acctccggtc agaaaaccaa aattataagc aacagaggtg aaaacagctg 4440

caaggccaca ggccaggtct gccatgcctt gtgctccccc gagggctgct ggggcccgga 4500

gcccagggac tgcgtctctt gccggaatgt cagccgaggc agggaatgcg tggacaagtg 4560

caaccttctg gagggtgagc caagggagtt tgtggagaac tctgagtgca tacagtgcca 4620

cccagagtgc ctgcctcagg ccatgaacat cacctgcaca ggacggggac cagacaactg 4680

tatccagtgt gcccactaca ttgacggccc ccactgcgtc aagacctgcc cggcaggagt 4740

catgggagaa aacaacaccc tggtctggaa gtacgcagac gccggccatg tgtgccacct 4800

gtgccatcca aactgcacct acggatgcac tgggccaggt cttgaaggct gtccaacgaa 4860

tgggcctaag atcccgtcca tcgccactgg gatggtgggg gccctcctct tgctgctggt 4920

ggtggccctg gggatcggcc tcttcatgtg agcggccgct ctagacccgg gctgcaggaa 4980

ttcgatatca agcttatcga taatcaacct ctggattaca aaatttgtga aagattgact 5040

ggtattctta actatgttgc tccttttacg ctatgtggat acgctgcttt aatgcctttg 5100

tatcatgcta ttgcttcccg tatggctttc attttctcct ccttgtataa atcctggttg 5160

ctgtctcttt atgaggagtt gtggcccgtt gtcaggcaac gtggcgtggt gtgcactgtg 5220

tttgctgacg caacccccac tggttggggc attgccacca cctgtcagct cctttccggg 5280

actttcgctt tccccctccc tattgccacg gcggaactca tcgccgcctg ccttgcccgc 5340

tgctggacag gggctcggct gttgggcact gacaattccg tggtgttgtc ggggaaatca 5400

tcgtcctttc cttggctgct cgcctgtgtt gccacctgga ttctgcgcgg gacgtccttc 5460

tgctacgtcc cttcggccct caatccagcg gaccttcctt cccgcggcct gctgccggct 5520

ctgcggcctc ttccgcgtct tcgccttcgc cctcagacga gtcggatctc cctttgggcc 5580

gcctccccgc atcgataccg tcgactagcc gtacctttaa gaccaatgac ttacaaggca 5640

gctgtagatc ttagccactt tttaaaagaa aaggggggac tggaagggct aattcactcc 5700

caaagaagac aagatctgct ttttgcctgt actgggtctc tctggttaga ccagatctga 5760

gcctgggagc tctctggcta actagggaac ccactgctta agcctcaata aagcttgcct 5820

tgagtgcttc aagtagtgtg tgcccgtctg ttgtgtgact ctggtaacta gagatccctc 5880

agaccctttt agtcagtgtg gaaaatctct agcagaattc gatatcaagc ttatcgatac 5940

cgtcgacctc gagggggggc ccggtaccca attcgcccta tagtgagtcg tattacaatt 6000

cactggccgt cgttttacaa cgtcgtgact gggaaaaccc tggcgttacc caacttaatc 6060

gccttgcagc acatccccct ttcgccagct ggcgtaatag cgaagaggcc cgcaccgatc 6120

gcccttccca acagttgcgc agcctgaatg gcgaatggaa attgtaagcg ttaatatttt 6180

gttaaaattc gcgttaaatt tttgttaaat cagctcattt tttaaccaat aggccgaaat 6240

cggcaaaatc ccttataaat caaaagaata gaccgagata gggttgagtg ttgttccagt 6300

ttggaacaag agtccactat taaagaacgt ggactccaac gtcaaagggc gaaaaaccgt 6360

ctatcagggc gatggcccac tacgtgaacc atcaccctaa tcaagttttt tggggtcgag 6420

gtgccgtaaa gcactaaatc ggaaccctaa agggagcccc cgatttagag cttgacgggg 6480

aaagccggcg aacgtggcga gaaaggaagg gaagaaagcg aaaggagcgg gcgctagggc 6540

gctggcaagt gtagcggtca cgctgcgcgt aaccaccaca cccgccgcgc ttaatgcgcc 6600

gctacagggc gcgtcaggtg gcacttttcg gggaaatgtg cgcggaaccc ctatttgttt 6660

atttttctaa atacattcaa atatgtatcc gctcatgaga caataaccct gataaatgct 6720

tcaataatat tgaaaaagga agagtatgag tattcaacat ttccgtgtcg cccttattcc 6780

cttttttgcg gcattttgcc ttcctgtttt tgctcaccca gaaacgctgg tgaaagtaaa 6840

agatgctgaa gatcagttgg gtgcacgagt gggttacatc gaactggatc tcaacagcgg 6900

taagatcctt gagagttttc gccccgaaga acgttttcca atgatgagca cttttaaagt 6960

tctgctatgt ggcgcggtat tatcccgtat tgacgccggg caagagcaac tcggtcgccg 7020

catacactat tctcagaatg acttggttga gtactcacca gtcacagaaa agcatcttac 7080

ggatggcatg acagtaagag aattatgcag tgctgccata accatgagtg ataacactgc 7140

ggccaactta cttctgacaa cgatcggagg accgaaggag ctaaccgctt ttttgcacaa 7200

catgggggat catgtaactc gccttgatcg ttgggaaccg gagctgaatg aagccatacc 7260

aaacgacgag cgtgacacca cgatgcctgt agcaatggca acaacgttgc gcaaactatt 7320

aactggcgaa ctacttactc tagcttcccg gcaacaatta atagactgga tggaggcgga 7380

taaagttgca ggaccacttc tgcgctcggc ccttccggct ggctggttta ttgctgataa 7440

atctggagcc ggtgagcgtg ggtctcgcgg tatcattgca gcactggggc cagatggtaa 7500

gccctcccgt atcgtagtta tctacacgac ggggagtcag gcaactatgg atgaacgaaa 7560

tagacagatc gctgagatag gtgcctcact gattaagcat tggtaactgt cagaccaagt 7620

ttactcatat atactttaga ttgatttaaa acttcatttt taatttaaaa ggatctaggt 7680

gaagatcctt tttgataatc tcatgaccaa aatcccttaa cgtgagtttt cgttccactg 7740

agcgtcagac cccgtagaaa agatcaaagg atcttcttga gatccttttt ttctgcgcgt 7800

aatctgctgc ttgcaaacaa aaaaaccacc gctaccagcg gtggtttgtt tgccggatca 7860

agagctacca actctttttc cgaaggtaac tggcttcagc agagcgcaga taccaaatac 7920

tgttcttcta gtgtagccgt agttaggcca ccacttcaag aactctgtag caccgcctac 7980

atacctcgct ctgctaatcc tgttaccagt ggctgctgcc agtggcgata agtcgtgtct 8040

taccgggttg gactcaagac gatagttacc ggataaggcg cagcggtcgg gctgaacggg 8100

gggttcgtgc acacagccca gcttggagcg aacgacctac accgaactga gatacctaca 8160

gcgtgagcta tgagaaagcg ccacgcttcc cgaagggaga aaggcggaca ggtatccggt 8220

aagcggcagg gtcggaacag gagagcgcac gagggagctt ccagggggaa acgcctggta 8280

tctttatagt cctgtcgggt ttcgccacct ctgacttgag cgtcgatttt tgtgatgctc 8340

gtcagggggg cggagcctat ggaaaaacgc cagcaacgcg gcctttttac ggttcctggc 8400

cttttgctgg ccttttgctc acatgttctt tcctgcgtta tcccctgatt ctgtggataa 8460

ccgtattacc gcctttgagt gagctgatac cgctcgccgc agccgaacga ccgagcgcag 8520

cgagtcagtg agcgaggaag cggaagagcg cccaatacgc aaaccgcctc tccccgcgcg 8580

ttggccgatt cattaatgca gctggcacga caggtttccc gactggaaag cgggcagtga 8640

gcgcaacgca attaatgtga gttagctcac tcattaggca ccccaggctt tacactttat 8700

gcttccggct cgtatgttgt gtggaattgt gagcggataa caatttcaca caggaaacag 8760

ctatgaccat gattacgcca agctcgaaat taaccctcac taaagggaac aaaagctgga 8820

gctccaccgc ggtggcggcc tcgaggtcga gatccggtcg accagcaacc atagtcccgc 8880

ccctaactcc gcccatcccg cccctaactc cgcccagttc cgcccattct ccgccccatg 8940

gctgactaat tttttttatt tatgcagagg ccgaggccgc ctcggcctct gagctattcc 9000

agaagtagtg aggaggcttt tttggaggcc taggcttttg caaaaagctt cgacggtatc 9060

gattggctca tgtccaacat taccgccatg ttgacattga ttattgacta gttattaata 9120

gtaatcaatt acggggtcat tagttcatag cccatatatg gagttccgcg ttacataact 9180

tacggtaaat ggcccgcctg gctgaccgcc caacgacccc cgcccattga cgtcaataat 9240

gacgtatgtt cccatagtaa cgccaatagg gactttccat tgacgtcaat gggtggagta 9300

tttacggtaa actgcccact tggcagtaca tcaagtgtat catatgccaa gtacgccccc 9360

tattgacgtc aatgacggta aatggcccgc ctggcattat gcccagtaca tgaccttatg 9420

ggactttcct acttggcagt acatctacgt attagtcatc gctattacca tggtgatgcg 9480

gttttggcag tacatcaatg ggcgtggata gcggtttgac tcacggggat ttccaagtct 9540

ccaccccatt gacgtcaatg ggagtttgtt ttggcaccaa aatcaacggg actttccaaa 9600

atgtcgtaac aactccgccc cattgacgca aatgggcggt aggcgtgtac ggaattcgga 9660

gtggcgagcc ctcagatcct gcatataagc agctgctttt tgcctgtact gggtctctct 9720

g 9721

<210> 80
<211> 10051
<212> DNA
<213> Artificial Sequence

<220>
<223> R12 long spacer CAR: PJ_R12-CH2-CH3-41BB-Z-T2A-tEGFR

<400> 80
gttagaccag atctgagcct gggagctctc tggctaacta gggaacccac tgcttaagcc 60

tcaataaagc ttgccttgag tgcttcaagt agtgtgtgcc cgtctgttgt gtgactctgg 120

taactagaga tccctcagac ccttttagtc agtgtggaaa atctctagca gtggcgcccg 180

aacagggact tgaaagcgaa agggaaacca gaggagctct ctcgacgcag gactcggctt 240

gctgaagcgc gcacggcaag aggcgagggg cggcgactgg tgagtacgcc aaaaattttg 300

actagcggag gctagaagga gagagatggg tgcgagagcg tcagtattaa gcgggggaga 360

attagatcga tgggaaaaaa ttcggttaag gccaggggga aagaaaaaat ataaattaaa 420

acatatagta tgggcaagca gggagctaga acgattcgca gttaatcctg gcctgttaga 480

aacatcagaa ggctgtagac aaatactggg acagctacaa ccatcccttc agacaggatc 540

agaagaactt agatcattat ataatacagt agcaaccctc tattgtgtgc atcaaaggat 600

agagataaaa gacaccaagg aagctttaga caagatagag gaagagcaaa acaaaagtaa 660

gaaaaaagca cagcaagcag cagctgacac aggacacagc aatcaggtca gccaaaatta 720

ccctatagtg cagaacatcc aggggcaaat ggtacatcag gccatatcac ctagaacttt 780

aaatgcatgg gtaaaagtag tagaagagaa ggctttcagc ccagaagtga tacccatgtt 840

ttcagcatta tcagaaggag ccaccccaca agatttaaac accatgctaa acacagtggg 900

gggacatcaa gcagccatgc aaatgttaaa agagaccatc aatgaggaag ctgcaggcaa 960

agagaagagt ggtgcagaga gaaaaaagag cagtgggaat aggagctttg ttccttgggt 1020

tcttgggagc agcaggaagc actatgggcg cagcgtcaat gacgctgacg gtacaggcca 1080

gacaattatt gtctggtata gtgcagcagc agaacaattt gctgagggct attgaggcgc 1140

aacagcatct gttgcaactc acagtctggg gcatcaagca gctccaggca agaatcctgg 1200

ctgtggaaag atacctaaag gatcaacagc tcctggggat ttggggttgc tctggaaaac 1260

tcatttgcac cactgctgtg ccttggatct acaaatggca gtattcatcc acaattttaa 1320

aagaaaaggg gggattgggg ggtacagtgc aggggaaaga atagtagaca taatagcaac 1380

agacatacaa actaaagaat tacaaaaaca aattacaaaa attcaaaatt ttcgggttta 1440

ttacagggac agcagagatc cagtttgggg atcaattgca tgaagaatct gcttagggtt 1500

aggcgttttg cgctgcttcg cgaggatctg cgatcgctcc ggtgcccgtc agtgggcaga 1560

gcgcacatcg cccacagtcc ccgagaagtt ggggggaggg gtcggcaatt gaaccggtgc 1620

ctagagaagg tggcgcgggg taaactggga aagtgatgtc gtgtactggc tccgcctttt 1680

tcccgagggt gggggagaac cgtatataag tgcagtagtc gccgtgaacg ttctttttcg 1740

caacgggttt gccgccagaa cacagctgaa gcttcgaggg gctcgcatct ctccttcacg 1800

cgcccgccgc cctacctgag gccgccatcc acgccggttg agtcgcgttc tgccgcctcc 1860

cgcctgtggt gcctcctgaa ctgcgtccgc cgtctaggta agtttaaagc tcaggtcgag 1920

accgggcctt tgtccggcgc tcccttggag cctacctaga ctcagccggc tctccacgct 1980

ttgcctgacc ctgcttgctc aactctacgt ctttgtttcg ttttctgttc tgcgccgtta 2040

cagatccaag ctgtgaccgg cgcctacggc tagcgaattc ctcgaggcca ccatgctgct 2100

gctggtgaca agcctgctgc tgtgcgagct gccccacccc gcctttctgc tgatccccca 2160

ggaacagctc gtcgaaagcg gcggcagact ggtgacacct ggcggcagcc tgaccctgag 2220

ctgcaaggcc agcggcttcg acttcagcgc ctactacatg agctgggtcc gccaggcccc 2280

tggcaaggga ctggaatgga tcgccaccat ctaccccagc agcggcaaga cctactacgc 2340

cacctgggtg aacggacggt tcaccatctc cagcgacaac gcccagaaca ccgtggacct 2400

gcagatgaac agcctgacag ccgccgaccg ggccacctac ttttgcgcca gagacagcta 2460

cgccgacgac ggcgccctgt tcaacatctg gggccctggc accctggtga caatctctag 2520

cggcggaggc ggatctggtg gcggaggaag tggcggcgga ggatctgagc tggtgctgac 2580

ccagagcccc tctgtgtctg ctgccctggg aagccctgcc aagatcacct gtaccctgag 2640

cagcgcccac aagaccgaca ccatcgactg gtatcagcag ctgcagggcg aggcccccag 2700

atacctgatg caggtgcaga gcgacggcag ctacaccaag aggccaggcg tgcccgaccg 2760

gttcagcgga tctagctctg gcgccgaccg ctacctgatc atccccagcg tgcaggccga 2820

tgacgaggcc gattactact gtggcgccga ctacatcggc ggctacgtgt tcggcggagg 2880

cacccagctg accgtgaccg gcgagtctaa gtacggaccg ccctgccccc cttgccctgc 2940

ccccgagttc ctgggcggac ccagcgtgtt cctgttcccc cccaagccca aggacaccct 3000

gatgatcagc cggacccccg aggtgacctg cgtggtggtg gacgtgagcc aggaagatcc 3060

cgaggtccag ttcaattggt acgtggacgg cgtggaagtg cacaacgcca agaccaagcc 3120

cagagaggaa cagttcaaca gcacctaccg ggtggtgtct gtgctgaccg tgctgcacca 3180

ggactggctg aacggcaaag aatacaagtg caaggtgtcc aacaagggcc tgcccagcag 3240

catcgaaaag accatcagca aggccaaggg ccagcctcgc gagccccagg tgtacaccct 3300

gcctccctcc caggaagaga tgaccaagaa ccaggtgtcc ctgacctgcc tggtgaaggg 3360

cttctacccc agcgacatcg ccgtggagtg ggagagcaac ggccagcctg agaacaacta 3420

caagaccacc cctcccgtgc tggacagcga cggcagcttc ttcctgtaca gccggctgac 3480

cgtggacaag agccggtggc aggaaggcaa cgtctttagc tgcagcgtga tgcacgaggc 3540

cctgcacaac cactacaccc agaagagcct gagcctgtcc ctgggcaaga tgttctgggt 3600

gctggtggtg gtgggcgggg tgctggcctg ctacagcctg ctggtgacag tggccttcat 3660

catcttttgg gtgaaacggg gcagaaagaa actcctgtat atattcaaac aaccatttat 3720

gagaccagta caaactactc aagaggaaga tggctgtagc tgccgatttc cagaagaaga 3780

agaaggagga tgtgaactgc gggtgaagtt cagcagaagc gccgacgccc ctgcctacca 3840

gcagggccag aatcagctgt acaacgagct gaacctgggc agaagggaag agtacgacgt 3900

cctggataag cggagaggcc gggaccctga gatgggcggc aagcctcggc ggaagaaccc 3960

ccaggaaggc ctgtataacg aactgcagaa agacaagatg gccgaggcct acagcgagat 4020

cggcatgaag ggcgagcgga ggcggggcaa gggccacgac ggcctgtatc agggcctgtc 4080

caccgccacc aaggatacct acgacgccct gcacatgcag gccctgcccc caaggctcga 4140

gggcggcgga gagggcagag gaagtcttct aacatgcggt gacgtggagg agaatcccgg 4200

ccctaggatg cttctcctgg tgacaagcct tctgctctgt gagttaccac acccagcatt 4260

cctcctgatc ccacgcaaag tgtgtaacgg aataggtatt ggtgaattta aagactcact 4320

ctccataaat gctacgaata ttaaacactt caaaaactgc acctccatca gtggcgatct 4380

ccacatcctg ccggtggcat ttaggggtga ctccttcaca catactcctc ctctggatcc 4440

acaggaactg gatattctga aaaccgtaaa ggaaatcaca gggtttttgc tgattcaggc 4500

ttggcctgaa aacaggacgg acctccatgc ctttgagaac ctagaaatca tacgcggcag 4560

gaccaagcaa catggtcagt tttctcttgc agtcgtcagc ctgaacataa catccttggg 4620

attacgctcc ctcaaggaga taagtgatgg agatgtgata atttcaggaa acaaaaattt 4680

gtgctatgca aatacaataa actggaaaaa actgtttggg acctccggtc agaaaaccaa 4740

aattataagc aacagaggtg aaaacagctg caaggccaca ggccaggtct gccatgcctt 4800

gtgctccccc gagggctgct ggggcccgga gcccagggac tgcgtctctt gccggaatgt 4860

cagccgaggc agggaatgcg tggacaagtg caaccttctg gagggtgagc caagggagtt 4920

tgtggagaac tctgagtgca tacagtgcca cccagagtgc ctgcctcagg ccatgaacat 4980

cacctgcaca ggacggggac cagacaactg tatccagtgt gcccactaca ttgacggccc 5040

ccactgcgtc aagacctgcc cggcaggagt catgggagaa aacaacaccc tggtctggaa 5100

gtacgcagac gccggccatg tgtgccacct gtgccatcca aactgcacct acggatgcac 5160

tgggccaggt cttgaaggct gtccaacgaa tgggcctaag atcccgtcca tcgccactgg 5220

gatggtgggg gccctcctct tgctgctggt ggtggccctg gggatcggcc tcttcatgtg 5280

agcggccgct ctagacccgg gctgcaggaa ttcgatatca agcttatcga taatcaacct 5340

ctggattaca aaatttgtga aagattgact ggtattctta actatgttgc tccttttacg 5400

ctatgtggat acgctgcttt aatgcctttg tatcatgcta ttgcttcccg tatggctttc 5460

attttctcct ccttgtataa atcctggttg ctgtctcttt atgaggagtt gtggcccgtt 5520

gtcaggcaac gtggcgtggt gtgcactgtg tttgctgacg caacccccac tggttggggc 5580

attgccacca cctgtcagct cctttccggg actttcgctt tccccctccc tattgccacg 5640

gcggaactca tcgccgcctg ccttgcccgc tgctggacag gggctcggct gttgggcact 5700

gacaattccg tggtgttgtc ggggaaatca tcgtcctttc cttggctgct cgcctgtgtt 5760

gccacctgga ttctgcgcgg gacgtccttc tgctacgtcc cttcggccct caatccagcg 5820

gaccttcctt cccgcggcct gctgccggct ctgcggcctc ttccgcgtct tcgccttcgc 5880

cctcagacga gtcggatctc cctttgggcc gcctccccgc atcgataccg tcgactagcc 5940

gtacctttaa gaccaatgac ttacaaggca gctgtagatc ttagccactt tttaaaagaa 6000

aaggggggac tggaagggct aattcactcc caaagaagac aagatctgct ttttgcctgt 6060

actgggtctc tctggttaga ccagatctga gcctgggagc tctctggcta actagggaac 6120

ccactgctta agcctcaata aagcttgcct tgagtgcttc aagtagtgtg tgcccgtctg 6180

ttgtgtgact ctggtaacta gagatccctc agaccctttt agtcagtgtg gaaaatctct 6240

agcagaattc gatatcaagc ttatcgatac cgtcgacctc gagggggggc ccggtaccca 6300

attcgcccta tagtgagtcg tattacaatt cactggccgt cgttttacaa cgtcgtgact 6360

gggaaaaccc tggcgttacc caacttaatc gccttgcagc acatccccct ttcgccagct 6420

ggcgtaatag cgaagaggcc cgcaccgatc gcccttccca acagttgcgc agcctgaatg 6480

gcgaatggaa attgtaagcg ttaatatttt gttaaaattc gcgttaaatt tttgttaaat 6540

cagctcattt tttaaccaat aggccgaaat cggcaaaatc ccttataaat caaaagaata 6600

gaccgagata gggttgagtg ttgttccagt ttggaacaag agtccactat taaagaacgt 6660

ggactccaac gtcaaagggc gaaaaaccgt ctatcagggc gatggcccac tacgtgaacc 6720

atcaccctaa tcaagttttt tggggtcgag gtgccgtaaa gcactaaatc ggaaccctaa 6780

agggagcccc cgatttagag cttgacgggg aaagccggcg aacgtggcga gaaaggaagg 6840

gaagaaagcg aaaggagcgg gcgctagggc gctggcaagt gtagcggtca cgctgcgcgt 6900

aaccaccaca cccgccgcgc ttaatgcgcc gctacagggc gcgtcaggtg gcacttttcg 6960

gggaaatgtg cgcggaaccc ctatttgttt atttttctaa atacattcaa atatgtatcc 7020

gctcatgaga caataaccct gataaatgct tcaataatat tgaaaaagga agagtatgag 7080

tattcaacat ttccgtgtcg cccttattcc cttttttgcg gcattttgcc ttcctgtttt 7140

tgctcaccca gaaacgctgg tgaaagtaaa agatgctgaa gatcagttgg gtgcacgagt 7200

gggttacatc gaactggatc tcaacagcgg taagatcctt gagagttttc gccccgaaga 7260

acgttttcca atgatgagca cttttaaagt tctgctatgt ggcgcggtat tatcccgtat 7320

tgacgccggg caagagcaac tcggtcgccg catacactat tctcagaatg acttggttga 7380

gtactcacca gtcacagaaa agcatcttac ggatggcatg acagtaagag aattatgcag 7440

tgctgccata accatgagtg ataacactgc ggccaactta cttctgacaa cgatcggagg 7500

accgaaggag ctaaccgctt ttttgcacaa catgggggat catgtaactc gccttgatcg 7560

ttgggaaccg gagctgaatg aagccatacc aaacgacgag cgtgacacca cgatgcctgt 7620

agcaatggca acaacgttgc gcaaactatt aactggcgaa ctacttactc tagcttcccg 7680

gcaacaatta atagactgga tggaggcgga taaagttgca ggaccacttc tgcgctcggc 7740

ccttccggct ggctggttta ttgctgataa atctggagcc ggtgagcgtg ggtctcgcgg 7800

tatcattgca gcactggggc cagatggtaa gccctcccgt atcgtagtta tctacacgac 7860

ggggagtcag gcaactatgg atgaacgaaa tagacagatc gctgagatag gtgcctcact 7920

gattaagcat tggtaactgt cagaccaagt ttactcatat atactttaga ttgatttaaa 7980

acttcatttt taatttaaaa ggatctaggt gaagatcctt tttgataatc tcatgaccaa 8040

aatcccttaa cgtgagtttt cgttccactg agcgtcagac cccgtagaaa agatcaaagg 8100

atcttcttga gatccttttt ttctgcgcgt aatctgctgc ttgcaaacaa aaaaaccacc 8160

gctaccagcg gtggtttgtt tgccggatca agagctacca actctttttc cgaaggtaac 8220

tggcttcagc agagcgcaga taccaaatac tgttcttcta gtgtagccgt agttaggcca 8280

ccacttcaag aactctgtag caccgcctac atacctcgct ctgctaatcc tgttaccagt 8340

ggctgctgcc agtggcgata agtcgtgtct taccgggttg gactcaagac gatagttacc 8400

ggataaggcg cagcggtcgg gctgaacggg gggttcgtgc acacagccca gcttggagcg 8460

aacgacctac accgaactga gatacctaca gcgtgagcta tgagaaagcg ccacgcttcc 8520

cgaagggaga aaggcggaca ggtatccggt aagcggcagg gtcggaacag gagagcgcac 8580

gagggagctt ccagggggaa acgcctggta tctttatagt cctgtcgggt ttcgccacct 8640

ctgacttgag cgtcgatttt tgtgatgctc gtcagggggg cggagcctat ggaaaaacgc 8700

cagcaacgcg gcctttttac ggttcctggc cttttgctgg ccttttgctc acatgttctt 8760

tcctgcgtta tcccctgatt ctgtggataa ccgtattacc gcctttgagt gagctgatac 8820

cgctcgccgc agccgaacga ccgagcgcag cgagtcagtg agcgaggaag cggaagagcg 8880

cccaatacgc aaaccgcctc tccccgcgcg ttggccgatt cattaatgca gctggcacga 8940

caggtttccc gactggaaag cgggcagtga gcgcaacgca attaatgtga gttagctcac 9000

tcattaggca ccccaggctt tacactttat gcttccggct cgtatgttgt gtggaattgt 9060

gagcggataa caatttcaca caggaaacag ctatgaccat gattacgcca agctcgaaat 9120

taaccctcac taaagggaac aaaagctgga gctccaccgc ggtggcggcc tcgaggtcga 9180

gatccggtcg accagcaacc atagtcccgc ccctaactcc gcccatcccg cccctaactc 9240

cgcccagttc cgcccattct ccgccccatg gctgactaat tttttttatt tatgcagagg 9300

ccgaggccgc ctcggcctct gagctattcc agaagtagtg aggaggcttt tttggaggcc 9360

taggcttttg caaaaagctt cgacggtatc gattggctca tgtccaacat taccgccatg 9420

ttgacattga ttattgacta gttattaata gtaatcaatt acggggtcat tagttcatag 9480

cccatatatg gagttccgcg ttacataact tacggtaaat ggcccgcctg gctgaccgcc 9540

caacgacccc cgcccattga cgtcaataat gacgtatgtt cccatagtaa cgccaatagg 9600

gactttccat tgacgtcaat gggtggagta tttacggtaa actgcccact tggcagtaca 9660

tcaagtgtat catatgccaa gtacgccccc tattgacgtc aatgacggta aatggcccgc 9720

ctggcattat gcccagtaca tgaccttatg ggactttcct acttggcagt acatctacgt 9780

attagtcatc gctattacca tggtgatgcg gttttggcag tacatcaatg ggcgtggata 9840

gcggtttgac tcacggggat ttccaagtct ccaccccatt gacgtcaatg ggagtttgtt 9900

ttggcaccaa aatcaacggg actttccaaa atgtcgtaac aactccgccc cattgacgca 9960

aatgggcggt aggcgtgtac ggaattcgga gtggcgagcc ctcagatcct gcatataagc 10020

agctgctttt tgcctgtact gggtctctct g 10051

<210> 81
<211> 822
<212> DNA
<213> Homo sapiens

<400> 81
accatgctgc tgctggtgac aagcctgctg ctgtgcgagc tgccccaccc cgcctttctg 60

ctgatccccc aggaacagct cgtcgaaagc ggcggcagac tggtgacacc tggcggcagc 120

ctgaccctga gctgcaaggc cagcggcttc gacttcagcg cctactacat gagctgggtc 180

cgccaggccc ctggcaaggg actggaatgg atcgccacca tctaccccag cagcggcaag 240

acctactacg ccacctgggt gaacggacgg ttcaccatct ccagcgacaa cgcccagaac 300

accgtggacc tgcagatgaa cagcctgaca gccgccgacc gggccaccta cttttgcgcc 360

agagacagct acgccgacga cggcgccctg ttcaacatct ggggccctgg caccctggtg 420

acaatctcta gcggcggagg cggatctggt ggcggaggaa gtggcggcgg aggatctgag 480

ctggtgctga cccagagccc ctctgtgtct gctgccctgg gaagccctgc caagatcacc 540

tgtaccctga gcagcgccca caagaccgac accatcgact ggtatcagca gctgcagggc 600

gaggccccca gatacctgat gcaggtgcag agcgacggca gctacaccaa gaggccaggc 660

gtgcccgacc ggttcagcgg atctagctct ggcgccgacc gctacctgat catccccagc 720

gtgcaggccg atgacgaggc cgattactac tgtggcgccg actacatcgg cggctacgtg 780

ttcggcggag gcacccagct gaccgtgacc ggcgagtcta ag 822

<210> 82
<211> 248
<212> PRT
<213> Homo sapiens

<400> 82

Gln Glu Gln Leu Val Glu Ser Gly Gly Arg Leu Val Thr Pro Gly Gly
1               5                   10                  15

Ser Leu Thr Leu Ser Cys Lys Ala Ser Gly Phe Asp Phe Ser Ala Tyr
            20                  25                  30

Tyr Met Ser Trp Val Arg Gln Ala Pro Gly Lys Gly Leu Glu Trp Ile
        35                  40                  45

Ala Thr Ile Tyr Pro Ser Ser Gly Lys Thr Tyr Tyr Ala Thr Trp Val
    50                  55                  60

Asn Gly Arg Phe Thr Ile Ser Ser Asp Asn Ala Gln Asn Thr Val Asp
65                  70                  75                  80

Leu Gln Met Asn Ser Leu Thr Ala Ala Asp Arg Ala Thr Tyr Phe Cys
                85                  90                  95

Ala Arg Asp Ser Tyr Ala Asp Asp Gly Ala Leu Phe Asn Ile Trp Gly
            100                 105                 110

Pro Gly Thr Leu Val Thr Ile Ser Ser Gly Gly Gly Gly Ser Gly Gly
        115                 120                 125

Gly Gly Ser Gly Gly Gly Gly Ser Glu Leu Val Leu Thr Gln Ser Pro
    130                 135                 140

Ser Val Ser Ala Ala Leu Gly Ser Pro Ala Lys Ile Thr Cys Thr Leu
145                 150                 155                 160

Ser Ser Ala His Lys Thr Asp Thr Ile Asp Trp Tyr Gln Gln Leu Gln
                165                 170                 175

Gly Glu Ala Pro Arg Tyr Leu Met Gln Val Gln Ser Asp Gly Ser Tyr
            180                 185                 190

Thr Lys Arg Pro Gly Val Pro Asp Arg Phe Ser Gly Ser Ser Ser Gly
        195                 200                 205

Ala Asp Arg Tyr Leu Ile Ile Pro Ser Val Gln Ala Asp Asp Glu Ala
    210                 215                 220

Asp Tyr Tyr Cys Gly Ala Asp Tyr Ile Gly Gly Tyr Val Phe Gly Gly
225                 230                 235                 240

Gly Thr Gln Leu Thr Val Thr Gly
                245

<210>  83
<211>  9384
<212>  DNA
<213>  Artificial Sequence

<220>
<223>  R12 short spacer CAR: PJ_R12-Hinge-41BB-Z-T2A-tEGFR

<400>  83
gttagaccag atctgagcct gggagctctc tggctaacta gggaacccac tgcttaagcc        60

tcaataaagc ttgccttgag tgcttcaagt agtgtgtgcc cgtctgttgt gtgactctgg 120

taactagaga tccctcagac ccttttagtc agtgtggaaa atctctagca gtggcgcccg 180

aacagggact tgaaagcgaa agggaaacca gaggagctct ctcgacgcag gactcggctt 240

gctgaagcgc gcacggcaag aggcgagggg cggcgactgg tgagtacgcc aaaaattttg 300

actagcggag gctagaagga gagagatggg tgcgagagcg tcagtattaa gcgggggaga 360

attagatcga tgggaaaaaa ttcggttaag gccaggggga aagaaaaaat ataaattaaa 420

acatatagta tgggcaagca gggagctaga acgattcgca gttaatcctg gcctgttaga 480

aacatcagaa ggctgtagac aaatactggg acagctacaa ccatcccttc agacaggatc 540

agaagaactt agatcattat ataatacagt agcaaccctc tattgtgtgc atcaaaggat 600

agagataaaa gacaccaagg aagctttaga caagatagag gaagagcaaa acaaaagtaa 660

gaaaaaagca cagcaagcag cagctgacac aggacacagc aatcaggtca gccaaaatta 720

ccctatagtg cagaacatcc aggggcaaat ggtacatcag gccatatcac ctagaacttt 780

aaatgcatgg gtaaaagtag tagaagagaa ggctttcagc ccagaagtga tacccatgtt 840

ttcagcatta tcagaaggag ccaccccaca agatttaaac accatgctaa acacagtggg 900

gggacatcaa gcagccatgc aaatgttaaa agagaccatc aatgaggaag ctgcaggcaa 960

agagaagagt ggtgcagaga gaaaaaagag cagtgggaat aggagctttg ttccttgggt 1020

tcttgggagc agcaggaagc actatgggcg cagcgtcaat gacgctgacg gtacaggcca 1080

gacaattatt gtctggtata gtgcagcagc agaacaattt gctgagggct attgaggcgc 1140

aacagcatct gttgcaactc acagtctggg gcatcaagca gctccaggca agaatcctgg 1200

ctgtggaaag atacctaaag gatcaacagc tcctggggat ttggggttgc tctggaaaac 1260

tcatttgcac cactgctgtg ccttggatct acaaatggca gtattcatcc acaattttaa 1320

aagaaaaggg gggattgggg ggtacagtgc aggggaaaga atagtagaca taatagcaac 1380

agacatacaa actaaagaat tacaaaaaca aattacaaaa attcaaaatt ttcgggttta 1440

ttacagggac agcagagatc cagtttgggg atcaattgca tgaagaatct gcttagggtt 1500

aggcgttttg cgctgcttcg cgaggatctg cgatcgctcc ggtgcccgtc agtgggcaga 1560

gcgcacatcg cccacagtcc ccgagaagtt ggggggaggg gtcggcaatt gaaccggtgc 1620

ctagagaagg tggcgcgggg taaactggga aagtgatgtc gtgtactggc tccgcctttt 1680

tcccgagggt gggggagaac cgtatataag tgcagtagtc gccgtgaacg ttctttttcg 1740

caacgggttt gccgccagaa cacagctgaa gcttcgaggg gctcgcatct ctccttcacg 1800

cgcccgccgc cctacctgag gccgccatcc acgccggttg agtcgcgttc tgccgcctcc 1860

cgcctgtggt gcctcctgaa ctgcgtccgc cgtctaggta agtttaaagc tcaggtcgag 1920

accgggcctt tgtccggcgc tcccttggag cctacctaga ctcagccggc tctccacgct 1980

ttgcctgacc ctgcttgctc aactctacgt ctttgtttcg ttttctgttc tgcgccgtta 2040

cagatccaag ctgtgaccgg cgcctacggc tagaccatgc tgctgctggt gacaagcctg 2100

ctgctgtgcg agctgcccca ccccgccttt ctgctgatcc cccaggaaca gctcgtcgaa 2160

agcggcggca gactggtgac acctggcggc agcctgaccc tgagctgcaa ggccagcggc 2220

ttcgacttca gcgcctacta catgagctgg gtccgccagg cccctggcaa gggactggaa 2280

tggatcgcca ccatctaccc cagcagcggc aagacctact acgccacctg ggtgaacgga 2340

cggttcacca tctccagcga caacgcccag aacaccgtgg acctgcagat gaacagcctg 2400

acagccgccg accgggccac ctacttttgc gccagagaca gctacgccga cgacggcgcc 2460

ctgttcaaca tctggggccc tggcaccctg gtgacaatct ctagcggcgg aggcggatct 2520

ggtggcggag gaagtggcgg cggaggatct gagctggtgc tgacccagag cccctctgtg 2580

tctgctgccc tgggaagccc tgccaagatc acctgtaccc tgagcagcgc ccacaagacc 2640

gacaccatcg actggtatca gcagctgcag ggcgaggccc ccagatacct gatgcaggtg 2700

cagagcgacg gcagctacac caagaggcca ggcgtgcccg accggttcag cggatctagc 2760

tctggcgccg accgctacct gatcatcccc agcgtgcagg ccgatgacga ggccgattac 2820

tactgtggcg ccgactacat cggcggctac gtgttcggcg gaggcaccca gctgaccgtg 2880

accggcgagt ctaagtacgg accgccctgc cccccttgcc ctatgttctg ggtgctggtg 2940

gtggtgggcg gggtgctggc ctgctacagc ctgctggtga cagtggcctt catcatcttt 3000

tgggtgaaac ggggcagaaa gaaactcctg tatatattca aacaaccatt tatgagacca 3060

gtacaaacta ctcaagagga agatggctgt agctgccgat ttccagaaga agaagaagga 3120

ggatgtgaac tgcgggtgaa gttcagcaga agcgccgacg cccctgccta ccagcagggc 3180

cagaatcagc tgtacaacga gctgaacctg ggcagaaggg aagagtacga cgtcctggat 3240

aagcggagag gccgggaccc tgagatgggc ggcaagcctc ggcggaagaa cccccaggaa 3300

ggcctgtata acgaactgca gaaagacaag atggccgagg cctacagcga gatcggcatg 3360

aagggcgagc ggaggcgggg caagggccac gacggcctgt atcagggcct gtccaccgcc 3420

accaaggata cctacgacgc cctgcacatg caggccctgc ccccaaggct cgagggcggc 3480

ggagagggca gaggaagtct tctaacatgc ggtgacgtgg aggagaatcc cggccctagg 3540

atgcttctcc tggtgacaag ccttctgctc tgtgagttac cacacccagc attcctcctg 3600

atcccacgca aagtgtgtaa cggaataggt attggtgaat ttaaagactc actctccata 3660

aatgctacga atattaaaca cttcaaaaac tgcacctcca tcagtggcga tctccacatc 3720

ctgccggtgg catttagggg tgactccttc acacatactc ctcctctgga tccacaggaa 3780

ctggatattc tgaaaaccgt aaaggaaatc acagggtttt tgctgattca ggcttggcct 3840

gaaaacagga cggacctcca tgcctttgag aacctagaaa tcatacgcgg caggaccaag 3900

caacatggtc agttttctct tgcagtcgtc agcctgaaca taacatcctt gggattacgc 3960

tccctcaagg agataagtga tggagatgtg ataatttcag gaaacaaaaa tttgtgctat 4020

gcaaatacaa taaactggaa aaaactgttt gggacctccg gtcagaaaac caaaattata 4080

agcaacagag gtgaaaacag ctgcaaggcc acaggccagg tctgccatgc cttgtgctcc 4140

cccgagggct gctggggccc ggagcccagg gactgcgtct cttgccggaa tgtcagccga 4200

ggcagggaat gcgtggacaa gtgcaacctt ctggagggtg agccaaggga gtttgtggag 4260

aactctgagt gcatacagtg ccacccagag tgcctgcctc aggccatgaa catcacctgc 4320

acaggacggg gaccagacaa ctgtatccag tgtgcccact acattgacgg cccccactgc 4380

gtcaagacct gcccggcagg agtcatggga gaaaacaaca ccctggtctg gaagtacgca 4440

gacgccggcc atgtgtgcca cctgtgccat ccaaactgca cctacggatg cactgggcca 4500

ggtcttgaag gctgtccaac gaatgggcct aagatcccgt ccatcgccac tgggatggtg 4560

ggggccctcc tcttgctgct ggtggtggcc ctggggatcg gcctcttcat gtgagcggcc 4620

gctctagacc cgggctgcag gaattcgata tcaagcttat cgataatcaa cctctggatt 4680

acaaaatttg tgaaagattg actggtattc ttaactatgt tgctcctttt acgctatgtg 4740

gatacgctgc tttaatgcct ttgtatcatg ctattgcttc ccgtatggct ttcattttct 4800

cctccttgta taaatcctgg ttgctgtctc tttatgagga gttgtggccc gttgtcaggc 4860

aacgtggcgt ggtgtgcact gtgtttgctg acgcaacccc cactggttgg ggcattgcca 4920

ccacctgtca gctcctttcc gggactttcg ctttccccct ccctattgcc acggcggaac 4980

tcatcgccgc ctgccttgcc cgctgctgga caggggctcg gctgttgggc actgacaatt 5040

ccgtggtgtt gtcggggaaa tcatcgtcct ttccttggct gctcgcctgt gttgccacct 5100

ggattctgcg cgggacgtcc ttctgctacg tcccttcggc cctcaatcca gcggaccttc 5160

cttcccgcgg cctgctgccg gctctgcggc ctcttccgcg tcttcgcctt cgccctcaga 5220

cgagtcggat ctccctttgg gccgcctccc cgcatcgata ccgtcgacta gccgtacctt 5280

taagaccaat gacttacaag gcagctgtag atcttagcca ctttttaaaa gaaaaggggg 5340

gactggaagg gctaattcac tcccaaagaa gacaagatct gctttttgcc tgtactgggt 5400

ctctctggtt agaccagatc tgagcctggg agctctctgg ctaactaggg aacccactgc 5460

ttaagcctca ataaagcttg ccttgagtgc ttcaagtagt gtgtgcccgt ctgttgtgtg 5520

actctggtaa ctagagatcc ctcagaccct tttagtcagt gtggaaaatc tctagcagaa 5580

ttcgatatca agcttatcga taccgtcgac ctcgaggggg ggcccggtac ccaattcgcc 5640

ctatagtgag tcgtattaca attcactggc cgtcgtttta caacgtcgtg actgggaaaa 5700

ccctggcgtt acccaactta atcgccttgc agcacatccc cctttcgcca gctggcgtaa 5760

tagcgaagag gcccgcaccg atcgcccttc ccaacagttg cgcagcctga atggcgaatg 5820

gaaattgtaa gcgttaatat tttgttaaaa ttcgcgttaa atttttgtta aatcagctca 5880

ttttttaacc aataggccga aatcggcaaa atcccttata aatcaaaaga atagaccgag 5940

atagggttga gtgttgttcc agtttggaac aagagtccac tattaaagaa cgtggactcc 6000

aacgtcaaag ggcgaaaaac cgtctatcag ggcgatggcc cactacgtga accatcaccc 6060

taatcaagtt ttttggggtc gaggtgccgt aaagcactaa atcggaaccc taaagggagc 6120

ccccgattta gagcttgacg gggaaagccg gcgaacgtgg cgagaaagga agggaagaaa 6180

gcgaaaggag cgggcgctag ggcgctggca agtgtagcgg tcacgctgcg cgtaaccacc 6240

acacccgccg cgcttaatgc gccgctacag ggcgcgtcag gtggcacttt tcggggaaat 6300

gtgcgcggaa cccctatttg tttatttttc taaatacatt caaatatgta tccgctcatg 6360

agacaataac cctgataaat gcttcaataa tattgaaaaa ggaagagtat gagtattcaa 6420

catttccgtg tcgcccttat tccctttttt gcggcatttt gccttcctgt ttttgctcac 6480

ccagaaacgc tggtgaaagt aaaagatgct gaagatcagt tgggtgcacg agtgggttac 6540

atcgaactgg atctcaacag cggtaagatc cttgagagtt ttcgccccga agaacgtttt 6600

ccaatgatga gcacttttaa agttctgcta tgtggcgcgg tattatcccg tattgacgcc 6660

gggcaagagc aactcggtcg ccgcatacac tattctcaga atgacttggt tgagtactca 6720

ccagtcacag aaaagcatct tacggatggc atgacagtaa gagaattatg cagtgctgcc 6780

ataaccatga gtgataacac tgcggccaac ttacttctga caacgatcgg aggaccgaag 6840

gagctaaccg cttttttgca caacatgggg gatcatgtaa ctcgccttga tcgttgggaa 6900

ccggagctga atgaagccat accaaacgac gagcgtgaca ccacgatgcc tgtagcaatg 6960

gcaacaacgt tgcgcaaact attaactggc gaactactta ctctagcttc ccggcaacaa 7020

ttaatagact ggatggaggc ggataaagtt gcaggaccac ttctgcgctc ggcccttccg 7080

gctggctggt ttattgctga taaatctgga gccggtgagc gtgggtctcg cggtatcatt 7140

gcagcactgg ggccagatgg taagccctcc cgtatcgtag ttatctacac gacggggagt 7200

caggcaacta tggatgaacg aaatagacag atcgctgaga taggtgcctc actgattaag 7260

cattggtaac tgtcagacca agtttactca tatatacttt agattgattt aaaacttcat 7320

ttttaattta aaaggatcta ggtgaagatc ctttttgata atctcatgac caaaatccct 7380

taacgtgagt tttcgttcca ctgagcgtca gaccccgtag aaaagatcaa aggatcttct 7440

tgagatcctt tttttctgcg cgtaatctgc tgcttgcaaa caaaaaaacc accgctacca 7500

gcggtggttt gtttgccgga tcaagagcta ccaactcttt ttccgaaggt aactggcttc 7560

agcagagcgc agataccaaa tactgttctt ctagtgtagc cgtagttagg ccaccacttc 7620

aagaactctg tagcaccgcc tacatacctc gctctgctaa tcctgttacc agtggctgct 7680

gccagtggcg ataagtcgtg tcttaccggg ttggactcaa gacgatagtt accggataag 7740

gcgcagcggt cgggctgaac ggggggttcg tgcacacagc ccagcttgga gcgaacgacc 7800

tacaccgaac tgagatacct acagcgtgag ctatgagaaa gcgccacgct tcccgaaggg 7860

agaaaggcgg acaggtatcc ggtaagcggc agggtcggaa caggagagcg cacgagggag 7920

cttccagggg gaaacgcctg gtatctttat agtcctgtcg ggtttcgcca cctctgactt 7980

gagcgtcgat ttttgtgatg ctcgtcaggg gggcggagcc tatggaaaaa cgccagcaac 8040

gcggcctttt tacggttcct ggccttttgc tggccttttg ctcacatgtt ctttcctgcg 8100

ttatcccctg attctgtgga taaccgtatt accgcctttg agtgagctga taccgctcgc 8160

cgcagccgaa cgaccgagcg cagcgagtca gtgagcgagg aagcggaaga gcgcccaata 8220

cgcaaaccgc ctctccccgc gcgttggccg attcattaat gcagctggca cgacaggttt 8280

cccgactgga aagcgggcag tgagcgcaac gcaattaatg tgagttagct cactcattag 8340

gcaccccagg ctttacactt tatgcttccg gctcgtatgt tgtgtggaat tgtgagcgga 8400

taacaatttc acacaggaaa cagctatgac catgattacg ccaagctcga aattaaccct 8460

cactaaaggg aacaaaagct ggagctccac cgcggtggcg gcctcgaggt cgagatccgg 8520

tcgaccagca accatagtcc cgcccctaac tccgcccatc ccgcccctaa ctccgcccag 8580

ttccgcccat tctccgcccc atggctgact aatttttttt atttatgcag aggccgaggc 8640

cgcctcggcc tctgagctat tccagaagta gtgaggaggc ttttttggag gcctaggctt 8700

ttgcaaaaag cttcgacggt atcgattggc tcatgtccaa cattaccgcc atgttgacat 8760

tgattattga ctagttatta atagtaatca attacggggt cattagttca tagcccatat 8820

atggagttcc gcgttacata acttacggta aatggcccgc ctggctgacc gcccaacgac 8880

ccccgcccat tgacgtcaat aatgacgtat gttcccatag taacgccaat agggactttc 8940

cattgacgtc aatgggtgga gtatttacgg taaactgccc acttggcagt acatcaagtg 9000

tatcatatgc caagtacgcc ccctattgac gtcaatgacg gtaaatggcc cgcctggcat 9060

tatgcccagt acatgacctt atgggacttt cctacttggc agtacatcta cgtattagtc 9120

atcgctatta ccatggtgat gcggttttgg cagtacatca atgggcgtgg atagcggttt 9180

gactcacggg gatttccaag tctccacccc attgacgtca atgggagttt gttttggcac 9240

caaaatcaac gggactttcc aaaatgtcgt aacaactccg ccccattgac gcaaatgggc 9300

ggtaggcgtg tacggaattc ggagtggcga gccctcagat cctgcatata agcagctgct 9360

ttttgcctgt actgggtctc tctg 9384

<210> 84
<211> 937

<212>  PRT
<213>  Homo sapiens

<400>  84

Met His Arg Pro Arg Arg Arg Gly Thr Arg Pro Pro Leu Leu Ala Leu
1               5                   10                  15

Leu Ala Ala Leu Leu Leu Ala Ala Arg Gly Ala Ala Ala Gln Glu Thr
            20                  25                  30

Glu Leu Ser Val Ser Ala Glu Leu Val Pro Thr Ser Ser Trp Asn Ile
        35                  40                  45

Ser Ser Glu Leu Asn Lys Asp Ser Tyr Leu Thr Leu Asp Glu Pro Met
    50                  55                  60

Asn Asn Ile Thr Thr Ser Leu Gly Gln Thr Ala Glu Leu His Cys Lys
65                  70                  75                  80

Val Ser Gly Asn Pro Pro Pro Thr Ile Arg Trp Phe Lys Asn Asp Ala
                85                  90                  95

Pro Val Val Gln Glu Pro Arg Arg Leu Ser Phe Arg Ser Thr Ile Tyr
            100                 105                 110

Gly Ser Arg Leu Arg Ile Arg Asn Leu Asp Thr Thr Asp Thr Gly Tyr
        115                 120                 125

Phe Gln Cys Val Ala Thr Asn Gly Lys Glu Val Val Ser Ser Thr Gly
    130                 135                 140

Val Leu Phe Val Lys Phe Gly Pro Pro Pro Thr Ala Ser Pro Gly Tyr
145                 150                 155                 160

Ser Asp Glu Tyr Glu Glu Asp Gly Phe Cys Gln Pro Tyr Arg Gly Ile
                165                 170                 175

Ala Cys Ala Arg Phe Ile Gly Asn Arg Thr Val Tyr Met Glu Ser Leu
            180                 185                 190

His Met Gln Gly Glu Ile Glu Asn Gln Ile Thr Ala Ala Phe Thr Met
        195                 200                 205

Ile Gly Thr Ser Ser His Leu Ser Asp Lys Cys Ser Gln Phe Ala Ile
    210                 215                 220

Pro Ser Leu Cys His Tyr Ala Phe Pro Tyr Cys Asp Glu Thr Ser Ser
225                 230                 235                 240

Val Pro Lys Pro Arg Asp Leu Cys Arg Asp Glu Cys Glu Ile Leu Glu
                245                 250                 255

Asn Val Leu Cys Gln Thr Glu Tyr Ile Phe Ala Arg Ser Asn Pro Met
            260                 265                 270

Ile Leu Met Arg Leu Lys Leu Pro Asn Cys Glu Asp Leu Pro Gln Pro
        275                 280                 285

Glu Ser Pro Glu Ala Ala Asn Cys Ile Arg Ile Gly Ile Pro Met Ala
    290                 295                 300

Asp Pro Ile Asn Lys Asn His Lys Cys Tyr Asn Ser Thr Gly Val Asp
305                 310                 315                 320

Tyr Arg Gly Thr Val Ser Val Thr Lys Ser Gly Arg Gln Cys Gln Pro
                325                 330                 335

Trp Asn Ser Gln Tyr Pro His Thr His Thr Phe Thr Ala Leu Arg Phe
            340                 345                 350

Pro Glu Leu Asn Gly Gly His Ser Tyr Cys Arg Asn Pro Gly Asn Gln
        355                 360                 365

Lys Glu Ala Pro Trp Cys Phe Thr Leu Asp Glu Asn Phe Lys Ser Asp
    370                 375                 380

Leu Cys Asp Ile Pro Ala Cys Asp Ser Lys Asp Ser Lys Glu Lys Asn
385                 390                 395                 400

Lys Met Glu Ile Leu Tyr Ile Leu Val Pro Ser Val Ala Ile Pro Leu
                405                 410                 415

Ala Ile Ala Leu Leu Phe Phe Phe Ile Cys Val Cys Arg Asn Asn Gln
            420                 425                 430

Lys Ser Ser Ser Ala Pro Val Gln Arg Gln Pro Lys His Val Arg Gly
        435                 440                 445

Gln Asn Val Glu Met Ser Met Leu Asn Ala Tyr Lys Pro Lys Ser Lys
    450                 455                 460

Ala Lys Glu Leu Pro Leu Ser Ala Val Arg Phe Met Glu Glu Leu Gly
465                 470                 475                 480

Glu Cys Ala Phe Gly Lys Ile Tyr Lys Gly His Leu Tyr Leu Pro Gly

                485                 490                 495

Met Asp His Ala Gln Leu Val Ala Ile Lys Thr Leu Lys Asp Tyr Asn
            500                 505                 510

Asn Pro Gln Gln Trp Thr Glu Phe Gln Gln Glu Ala Ser Leu Met Ala
        515                 520                 525

Glu Leu His His Pro Asn Ile Val Cys Leu Leu Gly Ala Val Thr Gln
    530                 535                 540

Glu Gln Pro Val Cys Met Leu Phe Glu Tyr Ile Asn Gln Gly Asp Leu
545                 550                 555                 560

His Glu Phe Leu Ile Met Arg Ser Pro His Ser Asp Val Gly Cys Ser
                565                 570                 575

Ser Asp Glu Asp Gly Thr Val Lys Ser Ser Leu Asp His Gly Asp Phe
            580                 585                 590

Leu His Ile Ala Ile Gln Ile Ala Ala Gly Met Glu Tyr Leu Ser Ser
        595                 600                 605

His Phe Phe Val His Lys Asp Leu Ala Ala Arg Asn Ile Leu Ile Gly
    610                 615                 620

Glu Gln Leu His Val Lys Ile Ser Asp Leu Gly Leu Ser Arg Glu Ile
625                 630                 635                 640

Tyr Ser Ala Asp Tyr Tyr Arg Val Gln Ser Lys Ser Leu Leu Pro Ile
                645                 650                 655

Arg Trp Met Pro Pro Glu Ala Ile Met Tyr Gly Lys Phe Ser Ser Asp
            660                 665                 670

Ser Asp Ile Trp Ser Phe Gly Val Val Leu Trp Glu Ile Phe Ser Phe
        675                 680                 685

Gly Leu Gln Pro Tyr Tyr Gly Phe Ser Asn Gln Glu Val Ile Glu Met
    690                 695                 700

Val Arg Lys Arg Gln Leu Leu Pro Cys Ser Glu Asp Cys Pro Pro Arg
705                 710                 715                 720

Met Tyr Ser Leu Met Thr Glu Cys Trp Asn Glu Ile Pro Ser Arg Arg
                725                 730                 735

Pro Arg Phe Lys Asp Ile His Val Arg Leu Arg Ser Trp Glu Gly Leu
            740                 745                 750

Ser Ser His Thr Ser Ser Thr Thr Pro Ser Gly Gly Asn Ala Thr Thr
        755                 760                 765

Gln Thr Thr Ser Leu Ser Ala Ser Pro Val Ser Asn Leu Ser Asn Pro
    770                 775                 780

Arg Tyr Pro Asn Tyr Met Phe Pro Ser Gln Gly Ile Thr Pro Gln Gly
785                 790                 795                 800

Gln Ile Ala Gly Phe Ile Gly Pro Pro Ile Pro Gln Asn Gln Arg Phe
                805                 810                 815

Ile Pro Ile Asn Gly Tyr Pro Ile Pro Pro Gly Tyr Ala Ala Phe Pro
            820                 825                 830

Ala Ala His Tyr Gln Pro Thr Gly Pro Pro Arg Val Ile Gln His Cys
        835                 840                 845

Pro Pro Pro Lys Ser Arg Ser Pro Ser Ser Ala Ser Gly Ser Thr Ser
    850                 855                 860

Thr Gly His Val Thr Ser Leu Pro Ser Ser Gly Ser Asn Gln Glu Ala
865                 870                 875                 880

Asn Ile Pro Leu Leu Pro His Met Ser Ile Pro Asn His Pro Gly Gly
                885                 890                 895

Met Gly Ile Thr Val Phe Gly Asn Lys Ser Gln Lys Pro Tyr Lys Ile
            900                 905                 910

Asp Ser Lys Gln Ala Ser Leu Leu Gly Asp Ala Asn Ile His Gly His
        915                 920                 925

Thr Glu Ser Met Ile Ser Ala Glu Leu
    930                 935

<210>  85
<211>  22
<212>  DNA
<213>  Artificial Sequence

<220>
<223>  RRE primer

<400>  85
attgtctggt atagtgcagc ag                                              22

<210>  86
<211>  801
<212>  DNA
<213>  Homo sapiens

<400>  86
gaattcgcca ccatgctgct gctggtgaca agcctgctgc tgtgcgagct gccccacccc      60

gcctttctgc tgatccccca gagcgtgaaa gagtccgagg gcgacctggt cacaccagcc     120

ggcaacctga ccctgacctg taccgccagc ggcagcgaca tcaacgacta ccccatctct     180

tgggtccgcc aggctcctgg caagggactg gaatggatcg gcttcatcaa cagcggcggc     240

agcacttggt acgccagctg ggtcaaaggc cggttcacca tcagccggac cagcaccacc     300

gtggacctga agatgacaag cctgaccacc gacgacaccg ccacctactt ttgcgccaga     360

ggctacagca cctactacgg cgacttcaac atctggggcc ctggcaccct ggtcacaatc     420

tctagcggcg gaggcggcag cggaggtgga ggaagtggcg gcggaggatc cgagctggtc     480

atgacccaga cccccagcag cacatctggc gccgtgggcg gcaccgtgac catcaattgc     540

caggccagcc agagcatcga cagcaacctg gcctggttcc agcagaagcc cggccagccc     600

cccaccctgc tgatctacag agcctccaac ctggccagcg gcgtgccaag cagattcagc     660

ggcagcagat ctggcaccga gtacaccctg accatctccg gcgtgcagag agaggacgcc     720

gctacctatt actgcctggg cggcgtgggc aacgtgtcct acagaaccag cttcggcgga     780

ggtactgagg tggtcgtcaa a                                               801


<210>  87
<211>  19
<212>  DNA
<213>  Artificial Sequence

<220>
<223>  SV40 primer

<400>  87
cgaccagcaa ccatagtcc                                                   19


<210>  88
<211>  72
<212>  DNA
<213>  Homo sapiens

<400>  88
ctcgagggcg gcggagaggg cagaggaagt cttctaacat gcggtgacgt ggaggagaat      60

cccggcccta gg                                                          72


<210>  89
<211>  24
<212>  PRT
<213>  Homo sapiens

<400>  89

Leu Glu Gly Gly Gly Glu Gly Arg Gly Ser Leu Leu Thr Cys Gly Asp
1               5                   10                  15

Val Glu Glu Asn Pro Gly Pro Arg
            20

<210>  90
<211>  5844
<212>  DNA
<213>  Homo sapiens

<400>  90
atgcttctcc tggtgacaag ccttctgctc tgtgagttac cacacccagc attcctcctg      60

atcccacgca aagtgtgtaa cggaataggt attggtgaat ttaaagactc actctccata     120

aatgctacga atattaaaca cttcaaaaac tgcacctcca tcagtggcga tctccacatc     180

ctgccggtgg catttagggg tgactccttc acacatactc ctcctctgga tccacaggaa     240

ctggatattc tgaaaaccgt aaaggaaatc acagggtttt tgctgattca ggcttggcct     300

gaaaacagga cggacctcca tgcctttgag aacctagaaa tcatacgcgg caggaccaag     360

caacatggtc agttttctct tgcagtcgtc agcctgaaca taacatcctt gggattacgc     420

tccctcaagg agataagtga tggagatgtg ataatttcag gaaacaaaaa tttgtgctat     480

gcaaatacaa taaactggaa aaaactgttt gggacctccg gtcagaaaac caaaattata     540

agcaacagag gtgaaaacag ctgcaaggcc acaggccagg tctgccatgc cttgtgctcc     600

cccgagggct gctggggccc ggagcccagg gactgcgtct cttgccggaa tgtcagccga     660

ggcagggaat gcgtggacaa gtgcaacctt ctggagggtg agccaaggga gtttgtggag     720

aactctgagt gcatacagtg ccacccagag tgcctgcctc aggccatgaa catcacctgc     780

acaggacggg gaccagacaa ctgtatccag tgtgcccact acattgacgg cccccactgc     840

gtcaagacct gcccggcagg agtcatggga gaaaacaaca ccctggtctg gaagtacgca     900

gacgccggcc atgtgtgcca cctgtgccat ccaaactgca cctacggatg cactgggcca     960

ggtcttgaag gctgtccaac gaatgggcct aagatcccgt ccatcgccac tgggatggtg    1020

ggggccctcc tcttgctgct ggtggtggcc ctggggatcg gcctcttcat gtgagcggcc    1080

gctctagacc cgggctgcag gaattcgata tcaagcttat cgataatcaa cctctggatt    1140

acaaaatttg tgaaagattg actggtattc ttaactatgt tgctcctttt acgctatgtg    1200

gatacgctgc tttaatgcct ttgtatcatg ctattgcttc ccgtatggct ttcattttct    1260

cctccttgta taaatcctgg ttgctgtctc tttatgagga gttgtggccc gttgtcaggc    1320

aacgtggcgt ggtgtgcact gtgtttgctg acgcaacccc cactggttgg ggcattgcca    1380

ccacctgtca gctcctttcc gggactttcg ctttccccct ccctattgcc acggcggaac 1440

tcatcgccgc ctgccttgcc cgctgctgga caggggctcg gctgttgggc actgacaatt 1500

ccgtggtgtt gtcggggaaa tcatcgtcct ttccttggct gctcgcctgt gttgccacct 1560

ggattctgcg cgggacgtcc ttctgctacg tcccttcggc cctcaatcca gcggaccttc 1620

cttcccgcgg cctgctgccg gctctgcggc ctcttccgcg tcttcgcctt cgccctcaga 1680

cgagtcggat ctccctttgg gccgcctccc cgcatcgata ccgtcgacta gccgtacctt 1740

taagaccaat gacttacaag gcagctgtag atcttagcca ctttttaaaa gaaaaggggg 1800

gactggaagg gctaattcac tcccaaagaa gacaagatct gctttttgcc tgtactgggt 1860

ctctctggtt agaccagatc tgagcctggg agctctctgg ctaactaggg aacccactgc 1920

ttaagcctca ataaagcttg ccttgagtgc ttcaagtagt gtgtgcccgt ctgttgtgtg 1980

actctggtaa ctagagatcc ctcagaccct tttagtcagt gtggaaaatc tctagcagaa 2040

ttcgatatca agcttatcga taccgtcgac ctcgaggggg ggcccggtac ccaattcgcc 2100

ctatagtgag tcgtattaca attcactggc cgtcgtttta caacgtcgtg actgggaaaa 2160

ccctggcgtt acccaactta atcgccttgc agcacatccc cctttcgcca gctggcgtaa 2220

tagcgaagag gcccgcaccg atcgcccttc ccaacagttg cgcagcctga atggcgaatg 2280

gaaattgtaa gcgttaatat tttgttaaaa ttcgcgttaa atttttgtta aatcagctca 2340

ttttttaacc aataggccga aatcggcaaa atcccttata aatcaaaaga atagaccgag 2400

atagggttga gtgttgttcc agtttggaac aagagtccac tattaaagaa cgtggactcc 2460

aacgtcaaag ggcgaaaaac cgtctatcag ggcgatggcc cactacgtga accatcaccc 2520

taatcaagtt ttttggggtc gaggtgccgt aaagcactaa atcggaaccc taaagggagc 2580

ccccgattta gagcttgacg gggaaagccg gcgaacgtgg cgagaaagga agggaagaaa 2640

gcgaaaggag cgggcgctag ggcgctggca agtgtagcgg tcacgctgcg cgtaaccacc 2700

acacccgccg cgcttaatgc gccgctacag ggcgcgtcag gtggcacttt tcggggaaat 2760

gtgcgcggaa cccctatttg tttatttttc taaatacatt caaatatgta tccgctcatg 2820

agacaataac cctgataaat gcttcaataa tattgaaaaa ggaagagtat gagtattcaa 2880

catttccgtg tcgcccttat tccctttttt gcggcatttt gccttcctgt ttttgctcac 2940

ccagaaacgc tggtgaaagt aaaagatgct gaagatcagt tgggtgcacg agtgggttac 3000

atcgaactgg atctcaacag cggtaagatc cttgagagtt ttcgccccga agaacgtttt 3060

ccaatgatga gcacttttaa agttctgcta tgtggcgcgg tattatcccg tattgacgcc 3120

gggcaagagc aactcggtcg ccgcatacac tattctcaga atgacttggt tgagtactca 3180

ccagtcacag aaaagcatct tacggatggc atgacagtaa gagaattatg cagtgctgcc 3240

ataaccatga gtgataacac tgcggccaac ttacttctga caacgatcgg aggaccgaag 3300

gagctaaccg cttttttgca caacatgggg gatcatgtaa ctcgccttga tcgttgggaa 3360

ccggagctga atgaagccat accaaacgac gagcgtgaca ccacgatgcc tgtagcaatg 3420

gcaacaacgt tgcgcaaact attaactggc gaactactta ctctagcttc ccggcaacaa 3480

ttaatagact ggatggaggc ggataaagtt gcaggaccac ttctgcgctc ggcccttccg 3540

gctggctggt ttattgctga taaatctgga gccggtgagc gtgggtctcg cggtatcatt 3600

gcagcactgg ggccagatgg taagccctcc cgtatcgtag ttatctacac gacggggagt 3660

caggcaacta tggatgaacg aaatagacag atcgctgaga taggtgcctc actgattaag 3720

cattggtaac tgtcagacca agtttactca tatatacttt agattgattt aaaacttcat 3780

ttttaattta aaaggatcta ggtgaagatc ctttttgata atctcatgac caaaatccct 3840

taacgtgagt tttcgttcca ctgagcgtca gaccccgtag aaaagatcaa aggatcttct 3900

tgagatcctt tttttctgcg cgtaatctgc tgcttgcaaa caaaaaaacc accgctacca 3960

gcggtggttt gtttgccgga tcaagagcta ccaactcttt ttccgaaggt aactggcttc 4020

agcagagcgc agataccaaa tactgttctt ctagtgtagc cgtagttagg ccaccacttc 4080

aagaactctg tagcaccgcc tacatacctc gctctgctaa tcctgttacc agtggctgct 4140

gccagtggcg ataagtcgtg tcttaccggg ttggactcaa gacgatagtt accggataag 4200

gcgcagcggt cgggctgaac ggggggttcg tgcacacagc ccagcttgga gcgaacgacc 4260

tacaccgaac tgagatacct acagcgtgag ctatgagaaa gcgccacgct tcccgaaggg 4320

agaaaggcgg acaggtatcc ggtaagcggc agggtcggaa caggagagcg cacgagggag 4380

cttccagggg gaaacgcctg gtatctttat agtcctgtcg ggtttcgcca cctctgactt 4440

gagcgtcgat ttttgtgatg ctcgtcaggg gggcggagcc tatggaaaaa cgccagcaac 4500

gcggcctttt tacggttcct ggccttttgc tggccttttg ctcacatgtt ctttcctgcg 4560

ttatcccctg attctgtgga taaccgtatt accgcctttg agtgagctga taccgctcgc 4620

cgcagccgaa cgaccgagcg cagcgagtca gtgagcgagg aagcggaaga gcgcccaata 4680

cgcaaaccgc ctctccccgc gcgttggccg attcattaat gcagctggca cgacaggttt 4740

cccgactgga aagcgggcag tgagcgcaac gcaattaatg tgagttagct cactcattag 4800

gcaccccagg ctttacactt tatgcttccg gctcgtatgt tgtgtggaat tgtgagcgga 4860

taacaatttc acacaggaaa cagctatgac catgattacg ccaagctcga aattaaccct 4920

cactaaaggg aacaaaagct ggagctccac cgcggtggcg gcctcgaggt cgagatccgg 4980

tcgaccagca accatagtcc cgcccctaac tccgcccatc ccgcccctaa ctccgcccag 5040

ttccgcccat tctccgcccc atggctgact aatttttttt atttatgcag aggccgaggc 5100

cgcctcggcc tctgagctat tccagaagta gtgaggaggc ttttttggag gcctaggctt 5160

ttgcaaaaag cttcgacggt atcgattggc tcatgtccaa cattaccgcc atgttgacat 5220

tgattattga ctagttatta atagtaatca attacggggt cattagttca tagcccatat 5280

atggagttcc gcgttacata acttacggta aatggcccgc ctggctgacc gcccaacgac 5340

ccccgcccat tgacgtcaat aatgacgtat gttcccatag taacgccaat agggactttc 5400

cattgacgtc aatgggtgga gtatttacgg taaactgccc acttggcagt acatcaagtg 5460

tatcatatgc caagtacgcc ccctattgac gtcaatgacg gtaaatggcc cgcctggcat 5520

tatgcccagt acatgacctt atgggacttt cctacttggc agtacatcta cgtattagtc 5580

atcgctatta ccatggtgat gcggttttgg cagtacatca atgggcgtgg atagcggttt 5640

gactcacggg gatttccaag tctccacccc attgacgtca atgggagttt gttttggcac 5700

caaaatcaac gggactttcc aaaatgtcgt aacaactccg ccccattgac gcaaatgggc 5760

ggtaggcgtg tacggaattc ggagtggcga gccctcagat cctgcatata agcagctgct 5820

ttttgcctgt actgggtctc tctg 5844

<210> 91
<211> 356
<212> PRT
<213> Homo sapiens

<400> 91

Leu Leu Leu Val Thr Ser Leu Leu Leu Cys Glu Leu Pro His Pro Ala
1               5                   10                  15

Phe Leu Leu Ile Pro Arg Lys Val Cys Asn Gly Ile Gly Ile Gly Glu
            20                  25                  30

Phe Lys Asp Ser Leu Ser Ile Asn Ala Thr Asn Ile Lys His Phe Lys
        35                  40                  45

Asn Cys Thr Ser Ile Ser Gly Asp Leu His Ile Leu Pro Val Ala Phe
    50                  55                  60

Arg Gly Asp Ser Phe Thr His Thr Pro Pro Leu Asp Pro Gln Glu Leu
65                  70                  75                  80

Asp Ile Leu Lys Thr Val Lys Glu Ile Thr Gly Phe Leu Leu Ile Gln
                85                  90                  95

Ala Trp Pro Glu Asn Arg Thr Asp Leu His Ala Phe Glu Asn Leu Glu
            100                 105                 110

Ile Ile Arg Gly Arg Thr Lys Gln His Gly Gln Phe Ser Leu Ala Val
        115                 120                 125

Val Ser Leu Asn Ile Thr Ser Leu Gly Leu Arg Ser Leu Lys Glu Ile
    130                 135                 140

Ser Asp Gly Asp Val Ile Ile Ser Gly Asn Lys Asn Leu Cys Tyr Ala
145                 150                 155                 160

Asn Thr Ile Asn Trp Lys Lys Leu Phe Gly Thr Ser Gly Gln Lys Thr
                165                 170                 175

Lys Ile Ile Ser Asn Arg Gly Glu Asn Ser Cys Lys Ala Thr Gly Gln
            180                 185                 190

Val Cys His Ala Leu Cys Ser Pro Glu Gly Cys Trp Gly Pro Glu Pro
        195                 200                 205

Arg Asp Cys Val Ser Cys Arg Asn Val Ser Arg Gly Arg Glu Cys Val
    210                 215                 220

Asp Lys Cys Asn Leu Leu Glu Gly Glu Pro Arg Glu Phe Val Glu Asn
225                 230                 235                 240

Ser Glu Cys Ile Gln Cys His Pro Glu Cys Leu Pro Gln Ala Met Asn
                245                 250                 255

Ile Thr Cys Thr Gly Arg Gly Pro Asp Asn Cys Ile Gln Cys Ala His
            260                 265                 270

Tyr Ile Asp Gly Pro His Cys Val Lys Thr Cys Pro Ala Gly Val Met
        275                 280                 285

Gly Glu Asn Asn Thr Leu Val Trp Lys Tyr Ala Asp Ala Gly His Val
    290                 295                 300

Cys His Leu Cys His Pro Asn Cys Thr Tyr Gly Cys Thr Gly Pro Gly
305                 310                 315                 320

Leu Glu Gly Cys Pro Thr Asn Gly Pro Lys Ile Pro Ser Ile Ala Thr
                325                 330                 335

Gly Met Val Gly Ala Leu Leu Leu Leu Leu Val Val Ala Leu Gly Ile
            340                 345                 350

Gly Leu Phe Met
        355

<210>  92
<211>  327

<212>  PRT
<213>  Homo sapiens

<400>  92

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210>  93
<211>  220
<212>  PRT
<213>  Homo sapiens

<400>  93

Met Leu Arg Leu Leu Leu Ala Leu Asn Leu Phe Pro Ser Ile Gln Val
1               5                   10                  15

Thr Gly Asn Lys Ile Leu Val Lys Gln Ser Pro Met Leu Val Ala Tyr
            20                  25                  30

Asp Asn Ala Val Asn Leu Ser Cys Lys Tyr Ser Tyr Asn Leu Phe Ser
        35                  40                  45

Arg Glu Phe Arg Ala Ser Leu His Lys Gly Leu Asp Ser Ala Val Glu
    50                  55                  60

Val Cys Val Val Tyr Gly Asn Tyr Ser Gln Gln Leu Gln Val Tyr Ser
65                  70                  75                  80

Lys Thr Gly Phe Asn Cys Asp Gly Lys Leu Gly Asn Glu Ser Val Thr
                85                  90                  95

Phe Tyr Leu Gln Asn Leu Tyr Val Asn Gln Thr Asp Ile Tyr Phe Cys
            100                 105                 110

Lys Ile Glu Val Met Tyr Pro Pro Pro Tyr Leu Asp Asn Glu Lys Ser
        115                 120                 125

Asn Gly Thr Ile Ile His Val Lys Gly Lys His Leu Cys Pro Ser Pro
    130                 135                 140

Leu Phe Pro Gly Pro Ser Lys Pro Phe Trp Val Leu Val Val Val Gly
145                 150                 155                 160

Gly Val Leu Ala Cys Tyr Ser Leu Leu Val Thr Val Ala Phe Ile Ile
                165                 170                 175

Phe Trp Val Arg Ser Lys Arg Ser Arg Leu Leu His Ser Asp Tyr Met
            180                 185                 190

Asn Met Thr Pro Arg Arg Pro Gly Pro Thr Arg Lys His Tyr Gln Pro
        195                 200                 205

Tyr Ala Pro Pro Arg Asp Phe Ala Ala Tyr Arg Ser
    210                 215                 220

<210>  94
<211>  164
<212>  PRT
<213>  Homo sapiens

<400>  94

Met Lys Trp Lys Ala Leu Phe Thr Ala Ala Ile Leu Gln Ala Gln Leu
1               5                   10                  15

Pro Ile Thr Glu Ala Gln Ser Phe Gly Leu Leu Asp Pro Lys Leu Cys
            20                  25                  30

Tyr Leu Leu Asp Gly Ile Leu Phe Ile Tyr Gly Val Ile Leu Thr Ala
        35                  40                  45

Leu Phe Leu Arg Val Lys Phe Ser Arg Ser Ala Asp Ala Pro Ala Tyr
    50                  55                  60

Gln Gln Gly Gln Asn Gln Leu Tyr Asn Glu Leu Asn Leu Gly Arg Arg
65                  70                  75                  80

Glu Glu Tyr Asp Val Leu Asp Lys Arg Arg Gly Arg Asp Pro Glu Met
                85                  90                  95

Gly Gly Lys Pro Gln Arg Arg Lys Asn Pro Gln Glu Gly Leu Tyr Asn
            100                 105                 110

Glu Leu Gln Lys Asp Lys Met Ala Glu Ala Tyr Ser Glu Ile Gly Met
        115                 120                 125

Lys Gly Glu Arg Arg Arg Gly Lys Gly His Asp Gly Leu Tyr Gln Gly
    130                 135                 140

Leu Ser Thr Ala Thr Lys Asp Thr Tyr Asp Ala Leu His Met Gln Ala
145                 150                 155                 160

Leu Pro Pro Arg

<210>  95
<211>  240
<212>  PRT
<213>  Homo sapiens

<400>  95

Met Gly Asn Ser Cys Tyr Asn Ile Val Ala Thr Leu Leu Leu Val Leu
1               5                   10                  15

Asn Phe Glu Arg Thr Arg Ser Leu Gln Asp Pro Cys Ser Asn Cys Pro
            20                  25                  30

Ala Gly Thr Phe Cys Asp Asn Asn Arg Asn Gln Ile Cys Ser Pro Cys
        35                  40                  45

Pro Pro Asn Ser Phe Ser Ser Ala Gly Gly Gln Arg Thr Cys Asp Ile
    50                  55                  60

Cys Arg Gln Cys Lys Gly Val Phe Arg Thr Arg Lys Glu Cys Ser Ser
65                  70                  75                  80

Thr Ser Asn Ala Glu Cys Asp Cys Thr Pro Gly Phe His Cys Leu Gly
                85                  90                  95

Ala Gly Cys Ser Met Cys Glu Gln Asp Cys Lys Gln Gly Gln Glu Leu
            100                 105                 110

Thr Lys Lys Gly Cys Lys Asp Cys Cys Phe Gly Thr Phe Asn Asp Gln
        115                 120                 125

Lys Arg Gly Ile Cys Arg Pro Trp Thr Asn Cys Ser Leu Asp Gly Lys
    130                 135                 140

Ser Val Leu Val Asn Gly Thr Lys Glu Arg Asp Val Val Cys Gly Pro
145                 150                 155                 160

Ser Pro Ala Asp Leu Ser Pro Gly Ala Ser Ser Val Thr Pro Pro Ala
                165                 170                 175

Pro Ala Arg Glu Pro Gly His Ser Pro Gln Ile Ile Ser Phe Phe Leu
            180                 185                 190

Ala Leu Thr Ser Thr Ala Leu Leu Phe Leu Leu Phe Phe Leu Thr Leu
        195                 200                 205

Arg Phe Ser Val Val Lys Arg Gly Arg Lys Lys Leu Leu Tyr Ile Phe
    210                 215                 220

Lys Gln Pro Phe Met Arg Pro Val Gln Thr Thr Gln Glu Glu Asp Gly
225                 230                 235                 240

<210>  96
<211>  20
<212>  DNA
<213>  Artificial Sequence

<220>
<223>  WPRE primer

<400>  96
actgtgtttg ctgacgcaac                                                   20

<210>  97
<211>  168
<212>  PRT
<213>  Homo sapiens

<400>  97

Met Gly His His His His His His His His His His Ser Ser Gly His
1               5                   10                  15

Ile Glu Gly Arg His Met Arg Arg Val Pro Gly Val Ala Pro Thr Leu
            20                  25                  30

Val Arg Ser Ala Ser Glu Thr Ser Glu Lys Arg Pro Phe Met Cys Ala
        35                  40                  45

Tyr Pro Gly Cys Asn Lys Arg Tyr Phe Lys Leu Ser His Leu Gln Met
    50                  55                  60

His Ser Arg Lys His Thr Gly Glu Lys Pro Tyr Gln Cys Asp Phe Lys
65                  70                  75                  80

Asp Cys Glu Arg Arg Phe Phe Arg Ser Asp Gln Leu Lys Arg His Gln
                85                  90                  95

Arg Arg His Thr Gly Val Lys Pro Phe Gln Cys Lys Thr Cys Gln Arg
            100                 105                 110

Lys Phe Ser Arg Ser Asp His Leu Lys Thr His Thr Arg Thr His Thr
        115                 120                 125

Gly Glu Lys Pro Phe Ser Cys Arg Trp Pro Ser Cys Gln Lys Lys Phe
    130                 135                 140

Ala Arg Ser Asp Glu Leu Val Arg His His Asn Met His Gln Arg Asn
145                 150                 155                 160

Met Thr Lys Leu Gln Leu Ala Leu
                165

<210>  98
<211>  5
<212>  PRT
<213>  Artificial Sequence

<220>
<223>  Spacer Region

<220>
<221>  VARIANT
<222>  (1)..(1)
<223>  Xaa is cysteine, glycine, or arginine

<220>
<221>  VARIANT
<222>  (4)..(4)
<223>  Xaa is cysteine or threonine

<400>  98

Xaa Pro Pro Xaa Pro
1               5

<210>  99
<211>  20
<212>  DNA
<213>  Artificial Sequence

<220>
<223>  Zeta primer

<400>  99
cgggtgaagt tcagcagaag                                                   20

<210>  100
<211>  9
<212>  PRT
<213>  Homo sapiens

<400>  100

Ala Asp Arg Ala Thr Tyr Phe Cys Ala

1                 5


<210>  101
<211>  11
<212>  PRT
<213>  Homo sapiens

<400>  101

Ala Ser Gly Phe Asp Phe Ser Ala Tyr Tyr Met
1               5                   10


<210>  102
<211>  6
<212>  PRT
<213>  Homo sapiens

<400>  102

Asp Thr Ile Asp Trp Tyr
1               5


<210>  103
<211>  5
<212>  PRT
<213>  Homo sapiens

<400>  103

Asp Tyr Gly Val Ser
1               5


<210>  104
<211>  9
<212>  PRT
<213>  Homo sapiens

<400>  104

Gly Asn Thr Leu Pro Tyr Thr Phe Gly
1               5


<210>  105
<211>  7
<212>  PRT
<213>  Homo sapiens

<400>  105

Ile Asn Ser Gly Gly Ser Thr
1               5


<210>  106
<211>  8
<212>  PRT
<213>  Homo sapiens

<400>  106

Asn Val Ser Tyr Arg Thr Ser Phe
1               5


<210>  107
<211>  16
<212>  PRT
<213>  Homo sapiens

<400>  107

Arg Ala Ser Asn Leu Ala Ser Gly Val Pro Ser Arg Phe Ser Gly Ser
1               5                   10                  15


<210>  108
<211>  11
<212>  PRT
<213>  Homo sapiens

<400>  108

Arg Ala Ser Gln Asp Ile Ser Lys Tyr Leu Asn
1               5                   10


<210>  109
<211>  11
<212>  PRT
<213>  Homo sapiens

<400>  109

Ser Gly Ser Asp Ile Asn Asp Tyr Pro Ile Ser
1               5                   10


<210>  110
<211>  5
<212>  PRT
<213>  Homo sapiens

<400>  110

Ser Asn Leu Ala Trp
1               5


<210>  111
<211>  7
<212>  PRT
<213>  Homo sapiens

<400>  111

Ser Arg Leu His Ser Gly Val
1               5


<210>  112
<211>  7

<212>  PRT
<213>  Homo sapiens

<400>  112

Thr Ile Tyr Pro Ser Ser Gly
1               5


<210>  113
<211>  16
<212>  PRT
<213>  Homo sapiens

<400>  113

Val Gln Ser Asp Gly Ser Tyr Thr Lys Arg Pro Gly Val Pro Asp Arg
1               5                   10                  15


<210>  114
<211>  16
<212>  PRT
<213>  Homo sapiens

<400>  114

Val Thr Trp Gly Ser Glu Thr Thr Tyr Tyr Asn Ser Ala Leu Lys Ser
1               5                   10                  15


<210>  115
<211>  7
<212>  PRT
<213>  Homo sapiens

<400>  115

Tyr Ala Met Asp Tyr Trp Gly
1               5


<210>  116
<211>  8
<212>  PRT
<213>  Homo sapiens

<400>  116

Tyr Phe Cys Ala Arg Gly Tyr Ser
1               5


<210>  117
<211>  8
<212>  PRT
<213>  Homo sapiens

<400>  117

Tyr Ile Gly Gly Tyr Val Phe Gly
1               5

<210>  118
<211>  8
<212>  PRT
<213>  Artificial Sequence

<220>
<223>  STREP-TAG II

<400>  118

Trp Ser His Pro Gln Phe Glu Lys
1               5


<210>  119
<211>  10
<212>  PRT
<213>  Artificial Sequence

<220>
<223>  Myc tag

<400>  119

Glu Gln Lys Leu Ile Ser Glu Glu Asp Leu
1               5                   10


<210>  120
<211>  14
<212>  PRT
<213>  Artificial Sequence

<220>
<223>  V5 tag

<400>  120

Gly Lys Pro Ile Pro Asn Pro Leu Leu Gly Leu Asp Ser Thr
1               5                   10


<210>  121
<211>  8
<212>  PRT
<213>  Artificial Sequence

<220>
<223>  FLAG tag

<400>  121

Asp Tyr Lys Asp Asp Asp Asp Lys
1               5


<210>  122
<211>  10
<212>  PRT
<213>  Artificial Sequence

<220>

<223>  Linker

<400>  122

Gly Gly Gly Gly Ser Gly Gly Gly Gly Ser
1               5                   10


<210>  123
<211>  8
<212>  PRT
<213>  Artificial Sequence

<220>
<223>  Linker

<400>  123

Gly Gly Gly Ser Gly Gly Gly Ser
1               5


<210>  124
<211>  11
<212>  PRT
<213>  Artificial Sequence

<220>
<223>  Linker

<400>  124

Gly Gly Gly Ser Gly Gly Gly Ser Gly Gly Ser
1               5                   10


<210>  125
<211>  5
<212>  PRT
<213>  Artificial Sequence

<220>
<223>  Core Hinge Region

<400>  125

Cys Pro Pro Cys Pro
1               5


<210>  126
<211>  66
<212>  DNA
<213>  Artificial Sequence

<220>
<223>  Secretory Signal Peptide Coding Sequence

<400>  126
atgctgctgc tggtgaccag cctgctgctg tgcgagctgc cccaccccgc ctttctgctg      60

atcccc                                                                  66

<210>  127
<211>  24
<212>  DNA
<213>  Artificial Sequence

<220>
<223>  Strep-tag II Coding Sequence

<400>  127
tggagccacc cgcagttcga aaaa                                              24


<210>  128
<211>  824
<212>  DNA
<213>  Artificial Sequence

<220>
<223>  Secretory Signal Peptide-[anti-CD19 scFv (Tag-VH-VL)] Coding
       Sequence

<400>  128
atgctgctgc tggtgaccag cctgctgctg tgcgagctgc cccaccccgc ctttctgctg      60

atccccaatt ggagccaccc gcagttcgaa aaaggaggtg gaggttcagg tggtggaggc     120

tctgacatcc agatgaccca gaccacctcc agcctgagcg ccagcctggg cgaccgggtg     180

accatcagct gccgggccag ccaggacatc agcaagtacc tgaactggta tcagcagaag     240

cccgacggca ccgtcaagct gctgatctac cacaccagcc ggctgcacag cggcgtgccc     300

agccggttta gcggcagcgg ctccggcacc gactacagcc tgaccatctc caacctggaa     360

caggaagata tcgccaccta cttttgccag cagggcaaca cactgcccta cacctttggc     420

ggcggaacaa agctggaaat caccggcagc acctccggca gcggcaagcc tggcagcggc     480

gagggcagca ccaagggcga ggtgaagctg caggaaagcg gccctggcct ggtggccccc     540

agccagagcc tgagcgtgac ctgcaccgtg agcggcgtga gcctgcccga ctacggcgtg     600

agctggatca ggcagccccc caggaagggc ctggaatggc tgggcgtgat ctggggcagc     660

gagaccacct actacaacag cgccctgaag agccggctga ccatcatcaa ggacaacagc     720

aagagccagg tgttcctgaa gatgaacagc ctgcagaccg acgacaccgc catctactac     780

tgcgccaagc actactacta cggcggcagc tacgccatgg actactgggg ccagggcacc     840

agcgtgaccg tgagcagc                                                   858

<210>  129
<211>  6
<212>  PRT
<213>  Artificial Sequence

<220>
<223>  Linker

<400>  129

Gly Gly Ser Gly Ser Gly

1               5


<210>  130
<211>  249
<212>  PRT
<213>  Artificial Sequence

<220>
<223>  Anti-CD19 scFv (VH-Tag-VL)

<400>  130

Asp Ile Gln Met Thr Gln Thr Thr Ser Ser Leu Ser Ala Ser Leu Gly
1               5                   10                  15


Asp Arg Val Thr Ile Ser Cys Arg Ala Ser Gln Asp Ile Ser Lys Tyr
            20                  25                  30


Leu Asn Trp Tyr Gln Gln Lys Pro Asp Gly Thr Val Lys Leu Leu Ile
        35                  40                  45


Tyr His Thr Ser Arg Leu His Ser Gly Val Pro Ser Arg Phe Ser Gly
    50                  55                  60


Ser Gly Ser Gly Thr Asp Tyr Ser Leu Thr Ile Ser Asn Leu Glu Gln
65                  70                  75                  80


Glu Asp Ile Ala Thr Tyr Phe Cys Gln Gln Gly Asn Thr Leu Pro Tyr
                85                  90                  95


Thr Phe Gly Gly Gly Thr Lys Leu Glu Ile Thr Gly Gly Ser Gly Ser
            100                 105                 110


Gly Asn Trp Ser His Pro Gln Phe Glu Lys Gly Ser Gly Ser Gly Glu
        115                 120                 125


Val Lys Leu Gln Glu Ser Gly Pro Gly Leu Val Ala Pro Ser Gln Ser
    130                 135                 140


Leu Ser Val Thr Cys Thr His Arg Val Ser Gly Val Ser Leu Pro Asp
145                 150                 155                 160


Tyr Gly Val Ser Trp Ile Arg Gln Pro Pro Arg Lys Gly Leu Glu Trp
                165                 170                 175


Leu Gly Val Ile Trp Gly Ser Glu Thr Thr Tyr Tyr Asn Ser Ala Leu
            180                 185                 190


Lys Ser Arg Leu Thr Ile Ile Lys Asp Asn Ser Lys Ser Gln Val Phe
        195                 200                 205

Leu Lys Met Asn Ser Leu Gln Thr Asp Asp Thr Ala Ile Tyr Tyr Cys
    210                 215                 220

Ala Lys His Tyr Tyr Tyr Gly Gly Ser Tyr Ala Met Asp Tyr Trp Gly
225                 230                 235                 240

Gln Gly Thr Ser Val Thr Val Ser Ser
                245

<210>  131
<211>  8
<212>  PRT
<213>  Artificial Sequence

<220>
<223>  Xpress tag

<400>  131

Asp Leu Tyr Asp Asp Asp Asp Lys
1               5

<210>  132
<211>  15
<212>  PRT
<213>  Artificial Sequence

<220>
<223>  Avi Tag

<400>  132

Gly Leu Asn Asp Ile Phe Glu Ala Gln Lys Ile Glu Trp His Glu
1               5                   10                  15

<210>  133
<211>  26
<212>  PRT
<213>  Artificial Sequence

<220>
<223>  Calmodulin Tag

<400>  133

Lys Arg Arg Trp Lys Lys Asn Phe Ile Ala Val Ser Ala Ala Asn Arg
1               5                   10                  15

Phe Lys Lys Ile Ser Ser Ser Gly Ala Leu
            20                  25

<210>  134
<211>  9
<212>  PRT

<213>  Artificial Sequence

<220>
<223>  HA Tag

<400>  134

Tyr Pro Tyr Asp Val Pro Asp Tyr Ala
1               5


<210>  135
<211>  13
<212>  PRT
<213>  Artificial Sequence

<220>
<223>  Soft Tag 1

<400>  135

Ser Leu Ala Glu Leu Leu Asn Ala Gly Leu Gly Gly Ser
1               5                   10


<210>  136
<211>  8
<212>  PRT
<213>  Artificial Sequence

<220>
<223>  Softag 3

<400>  136

Thr Gln Asp Pro Ser Arg Val Gly
1               5


<210>  137
<211>  8
<212>  PRT
<213>  Artificial Sequence

<220>
<223>  Strep-Tag

<400>  137

Trp Arg His Pro Gln Phe Gly Gly
1               5


<210>  138
<211>  6
<212>  PRT
<213>  Artificial Sequence

<220>
<223>  Engineered Tag of a Minimal Chelation Site

<400>  138

His Gly Gly His His Gly
1               5

<210>  139
<211>  18
<212>  PRT
<213>  Artificial Sequence

<220>
<223>  Linker + Tag

<400>  139

Gly Gly Gly Gly Ser Gly Gly Gly Gly Ser Trp Ser His Pro Gln Phe
1               5                   10                  15

Glu Lys

<210>  140
<211>  18
<212>  PRT
<213>  Artificial Sequence

<220>
<223>  Linker + Tag

<400>  140

Trp Ser His Pro Gln Phe Glu Lys Gly Gly Gly Gly Ser Gly Gly Gly
1               5                   10                  15

Gly Ser

<210>  141
<211>  37
<212>  PRT
<213>  Artificial Sequence

<220>
<223>  Linker + Tag

<400>  141

Gly Gly Gly Gly Ser Gly Gly Gly Gly Ser Trp Ser His Pro Gln Phe
1               5                   10                  15

Glu Lys Gly Gly Gly Ser Gly Gly Gly Ser Gly Gly Ser Trp Ser His
            20                  25                  30

Pro Gln Phe Glu Lys
        35

<210>  142

<211>  37
<212>  PRT
<213>  Artificial Sequence

<220>
<223>  Linker + Tag

<400>  142

Trp Ser His Pro Gln Phe Glu Lys Gly Gly Gly Ser Gly Gly Gly Ser
1               5                   10                  15

Gly Gly Ser Trp Ser His Pro Gln Phe Glu Lys Gly Gly Gly Gly Ser
            20                  25                  30

Gly Gly Gly Gly Ser
        35

<210>  143
<211>  55
<212>  PRT
<213>  Artificial Sequence

<220>
<223>  Linker + Tag

<400>  143

Gly Gly Gly Gly Ser Gly Gly Gly Gly Ser Trp Ser His Pro Gln Phe
1               5                   10                  15

Glu Lys Gly Gly Gly Ser Gly Gly Gly Ser Gly Gly Ser Trp Ser His
            20                  25                  30

Pro Gln Phe Glu Lys Gly Gly Gly Gly Ser Gly Gly Gly Gly Ser Trp
        35                  40                  45

Ser His Pro Gln Phe Glu Lys
    50                  55

<210>  144
<211>  55
<212>  PRT
<213>  Artificial Sequence

<220>
<223>  Linker + Tag

<400>  144

Trp Ser His Pro Gln Phe Glu Lys Gly Gly Gly Gly Ser Gly Gly Gly
1               5                   10                  15

Gly Ser Trp Ser His Pro Gln Phe Glu Lys Gly Gly Gly Ser Gly Gly
            20                  25                  30

Gly Ser Gly Gly Ser Trp Ser His Pro Gln Phe Glu Lys Gly Gly Gly
        35                  40                  45

Gly Ser Gly Gly Gly Gly Ser
    50                  55

<210>  145
<211>  5
<212>  PRT
<213>  Artificial Sequence

<220>
<223>  Linker

<400>  145

Gly Gly Gly Gly Ser
1               5

<210>  146
<211>  4
<212>  PRT
<213>  Artificial Sequence

<220>
<223>  Linker

<400>  146

Gly Gly Gly Ser
1

<210>  147
<211>  9
<212>  PRT
<213>  Artificial Sequence

<220>
<223>  Linker

<400>  147

Gly Gly Gly Ser Gly Gly Gly Gly Ser
1               5

<210>  148
<211>  7
<212>  PRT
<213>  Artificial Sequence

<220>
<223>  Linker

<400>  148

Gly Gly Gly Ser Gly Gly Ser

1               5


<210>  149
<211>  5
<212>  PRT
<213>  Artificial Sequence

<220>
<223>  Linker

<400>  149

Gly Ser Gly Ser Gly
1               5


<210>  150
<211>  10
<212>  PRT
<213>  Artificial Sequence

<220>
<223>  Linker

<400>  150

Gly Gly Gly Gly Ser Gly Gly Gly Gly Ser
1               5                   10


<210>  151
<211>  8
<212>  PRT
<213>  Artificial Sequence

<220>
<223>  Linker

<400>  151

Gly Gly Gly Ser Gly Gly Gly Ser
1               5


<210>  152
<211>  11
<212>  PRT
<213>  Artificial Sequence

<220>
<223>  Linker

<400>  152

Gly Gly Gly Ser Gly Gly Gly Ser Gly Gly Ser
1               5                   10


<210>  153
<211>  247
<212>  PRT
<213>  Artificial Sequence

<220>
<223>  Anti-ROR1 scFv (VH-VL) from R12

<400>  153

Gln Glu Gln Leu Val Glu Ser Gly Gly Arg Leu Val Thr Pro Gly Gly
1               5                   10                  15

Ser Leu Thr Leu Ser Cys Lys Ala Ser Gly Phe Asp Phe Ser Ala Tyr
            20                  25                  30

Tyr Met Ser Trp Val Arg Gln Ala Pro Gly Lys Gly Leu Glu Trp Ile
        35                  40                  45

Ala Thr Ile Tyr Pro Ser Ser Gly Lys Thr Tyr Tyr Ala Thr Trp Val
    50                  55                  60

Asn Gly Arg Phe Thr Ile Ser Ser Asp Asn Ala Gln Asn Thr Val Asp
65                  70                  75                  80

Leu Gln Met Asn Ser Leu Thr Ala Ala Asp Arg Ala Thr Tyr Phe Cys
                85                  90                  95

Ala Arg Asp Ser Tyr Ala Asp Asp Gly Ala Leu Phe Asn Ile Trp Gly
            100                 105                 110

Pro Gly Thr Leu Val Thr Ile Ser Ser Gly Gly Gly Gly Ser Gly Gly
        115                 120                 125

Gly Gly Ser Gly Gly Gly Gly Ser Glu Leu Val Leu Thr Gln Ser Pro
    130                 135                 140

Ser Val Ser Ala Ala Leu Gly Ser Pro Ala Lys Ile Thr Cys Thr Leu
145                 150                 155                 160

Ser Ser Ala His Lys Thr Asp Thr Ile Asp Trp Tyr Gln Gln Leu Gln
                165                 170                 175

Gly Glu Ala Pro Arg Tyr Leu Met Gln Val Gln Ser Asp Gly Ser Tyr
            180                 185                 190

Thr Lys Arg Pro Gly Val Pro Asp Arg Phe Ser Gly Ser Ser Ser Gly
        195                 200                 205

Ala Asp Arg Tyr Leu Ile Ile Pro Ser Val Gln Ala Asp Asp Glu Ala
    210                 215                 220

Asp Tyr Tyr Cys Gly Ala Asp Tyr Ile Gly Gly Tyr Val Phe Gly Gly

225                 230                 235                 240

Gly Thr Gln Leu Thr Val Thr
                245

<210>  154
<211>  42
<212>  PRT
<213>  Artificial Sequence

<220>
<223>  4-1BB Portion

<400>  154

Lys Arg Gly Arg Lys Lys Leu Leu Tyr Ile Phe Lys Gln Pro Phe Met
1               5                   10                  15

Arg Pro Val Gln Thr Thr Gln Glu Glu Asp Gly Cys Ser Cys Arg Phe
            20                  25                  30

Pro Glu Glu Glu Glu Gly Gly Cys Glu Leu
        35                  40

<210>  155
<211>  20
<212>  PRT
<213>  Artificial Sequence

<220>
<223>  Variable Domain Linker + Imbedded Tag

<220>
<221>  misc_feature
<222>  (7)..(7)
<223>  Xaa can be any naturally occurring amino acid

<400>  155

Gly Gly Ser Gly Ser Gly Xaa Trp Ser His Pro Gln Phe Glu Lys Gly
1               5                   10                  15

Ser Gly Ser Gly
            20

<210>  156
<211>  11
<212>  PRT
<213>  Artificial Sequence

<220>
<223>  Linker

<400>  156

Gly Gly Gly Ser Gly Gly Gly Ser Gly Gly Ser
1               5                   10

<210>  157
<211>  10
<212>  PRT
<213>  Artificial Sequence

<220>
<223>  Linker

<400>  157

Gly Gly Gly Gly Ser Gly Gly Gly Gly Ser
1               5                   10

## Description

### CROSS REFERENCE TO RELATED APPLICATION

This application claims priority to U.S. Provisional Patent Application No. 62/154,565, filed on April 29, 2015, the entire contents of which are incorporated herein.

### FIELD OF THE DISCLOSURE

Hematopoeitic stem/progenitor cells (HSPC) and/or non-T effector cells are modified to express an extracellular component including a tag cassette. The tag cassette can be used to activate, promote proliferation of, detect, enrich, isolate, track, deplete and/or eliminate modified cells. The cells can also express a binding domain.

### BACKGROUND OF THE DISCLOSURE

Significant progress has been made in genetically engineering T cells of the immune system to target and kill unwanted cell types, such as cancer cells. For example, T cells have been genetically engineered to express molecules having extracellular components that bind particular target antigens and intracellular components that direct actions of the T cell when the extracellular component has bound the target antigen. As an example, the extracellular component can be designed to bind target antigens found on cancer cells and, when bound, the intracellular component directs the T cell to destroy the bound cancer cell. Examples of such molecules include genetically engineered T cell receptors (TCR) and chimeric antigen receptors (CAR).

While genetically engineered T cells provide a significant advance in the ability to target and destroy unwanted cell types, they require immunological matching with each particular subject before they can be used in a treatment setting. Once a donor match is found (or T cells are obtained from a subject needing treatment), the cells must be modified and expanded before they can be used in the subject. This time-intensive and expensive process can cause, in some instances, lethal delays in treatment.

### SUMMARY OF THE DISCLOSURE

**The** current disclosure provides genetically modified stem cells that can be administered to subjects without the need for immunological matching. Thus, these modified stem cells may be provided as "off-the-shelf treatments removing delays and expense in treatment associated with donor identification and subsequent cell modification and expansion. The modified stem cells can be administered alone or in combination with various other treatments to obtain numerous treatment objectives. In particular embodiments, the modified stem cells are differentiated into modified non-T effector cells before administration.

More particularly, hematopoietic stem/progenitor cells (HSPC) are genetically modified to express an extracellular component including a tag cassette. Tag cassettes can be used to activate, promote proliferation of, detect, enrich for, isolate, track, deplete and/or eliminate genetically modified cells *in vitro*, *in vivo* and/or *ex vivo.* Such modified cells can be identified and isolated at higher yields as compared to HSPC that do not express a tag cassette. In particular embodiments, modified HSPC can be differentiated into non-T effector cells before administration.

In additional embodiments, the modified cells additionally express a ligand binding domain that binds particular cellular markers preferentially found on unwanted cell types as part of the extracellular component. These embodiments additionally express an intracellular component that directs actions of the genetically modified cell when the extracellular component has bound the cellular marker. As an example, the extracellular component can be designed to bind cellular markers preferentially found on cancer cells and, when bound, the intracellular component directs the genetically modified cell to destroy the bound cancer cell. Examples of such molecules include genetically engineered T cell receptors (TCR), chimeric antigen receptors (CAR), and other molecules disclosed herein.

**The** genetically modified stem cells that express an extracellular component including a tag cassette also provide important research tools.

### BRIEF DESCRIPTION OF THE FIGURES

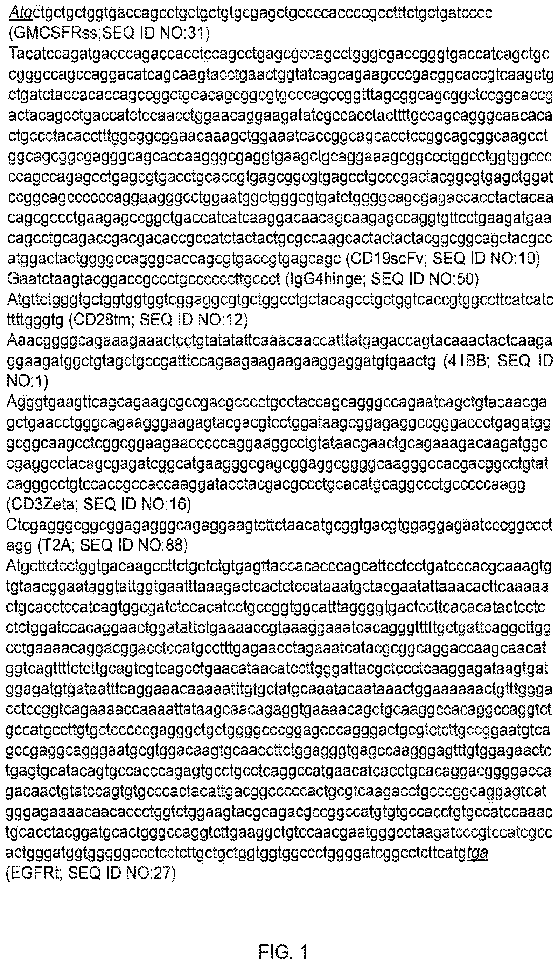

FIG. 1. Nucleotide sequence of anti-CD19 short spacer chimeric receptor, GMCSFRss-CD19scFv-lgG4hinge-CD28tm-41BB-Zeta-T2A-EGFRt. EGFRt can be replaced or supplemented with a tag cassette binding an exogenous cognate binding molecule (ExoCBM), such as STREP TAG^{®} ∥ (SEQ ID NO:118), Myc tag (SEQ ID NO:119), V5 tag (SEQ ID NO:120), FLAG^{®} tag (Sigma-Aldrich, Co., LLC, St. Louis, MO) (SEQ ID NO:121), His tag, or other peptides or molecules as disclosed herein.

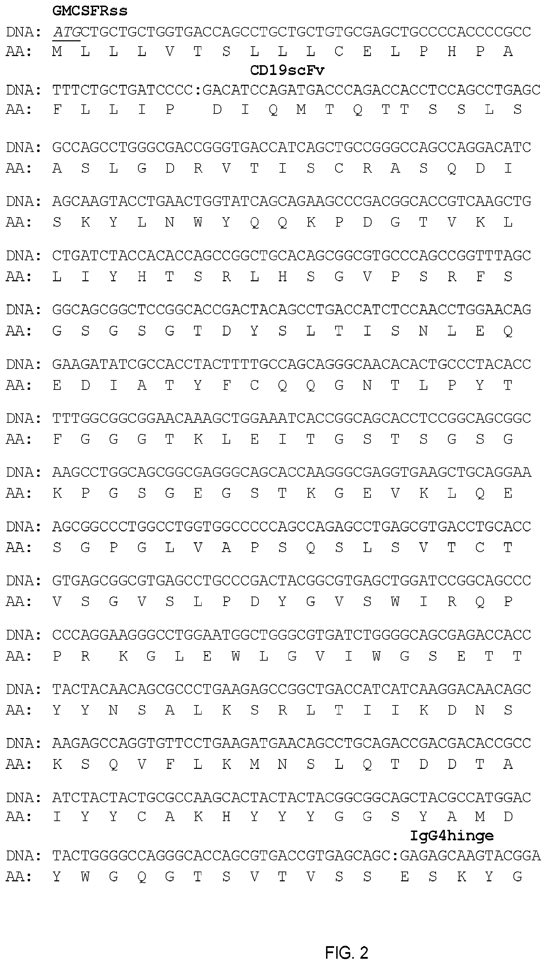

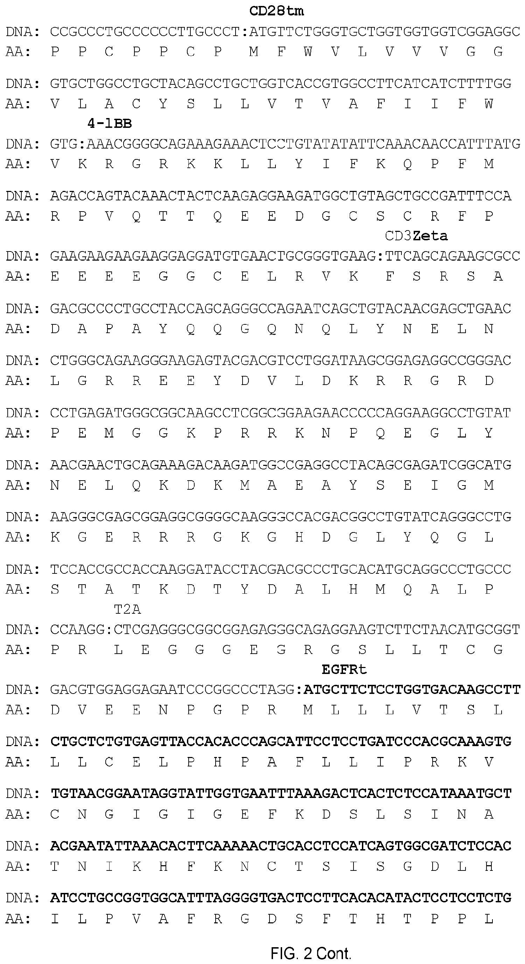

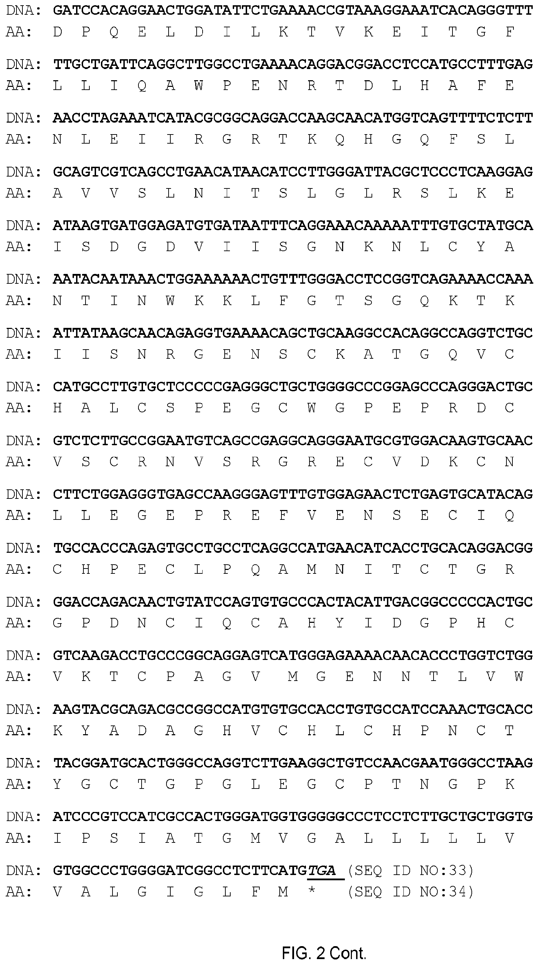

FIG 2. Amino acid sequence of GMCSFRss-CD19scFv-lgG4hinge-CD28tm-41BB-Zeta-T2A-EGFRt. EGFRt can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

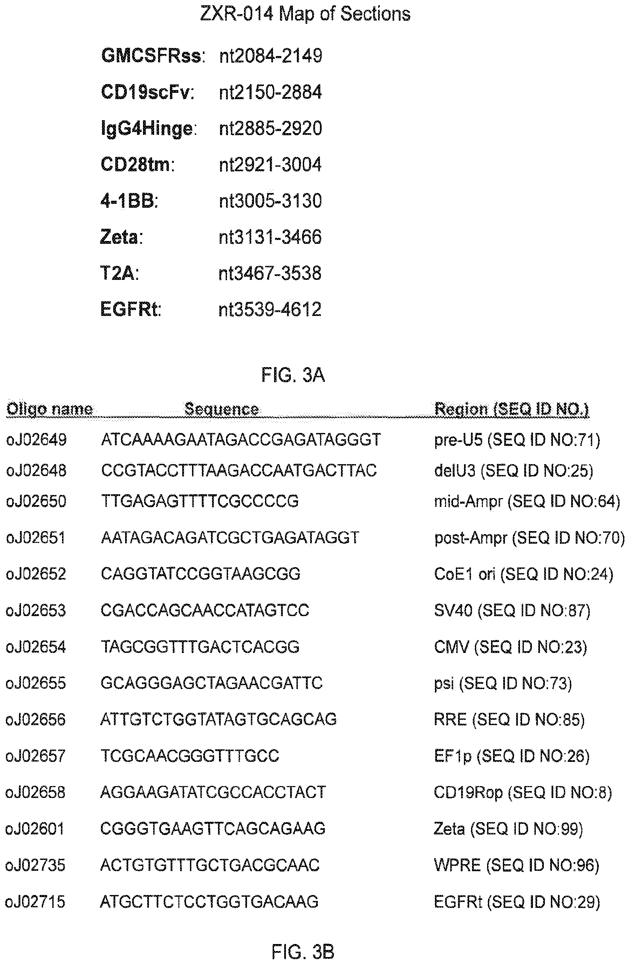

FIG. 3A and 3B. FIG. 3A shows a map of the sections of ZXR-014 nucleotide and amino acid sequences. FIG. 3B shows exemplary primer sequences.

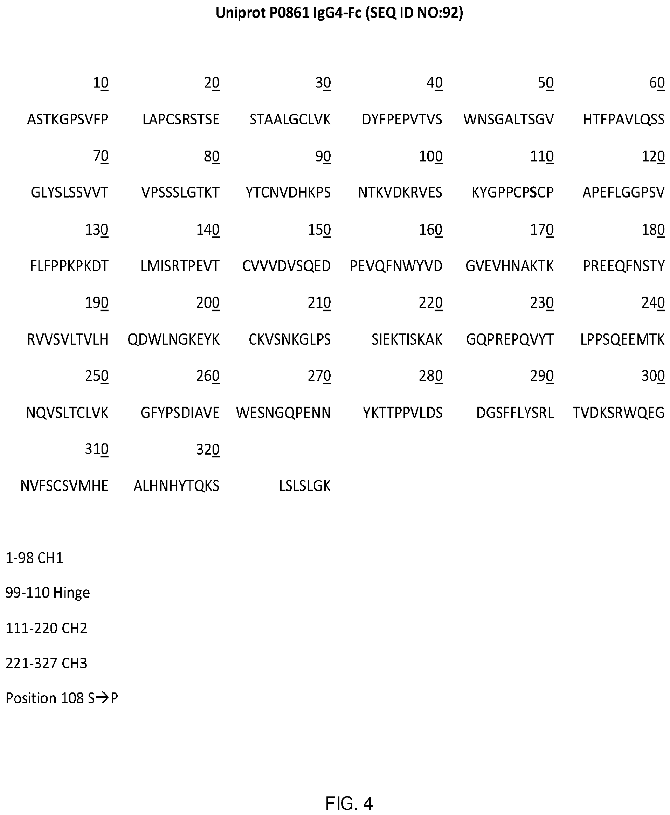

FIG. 4. Amino acid sequence and map of sections of Uniprot P0861 IgG4-Fc.

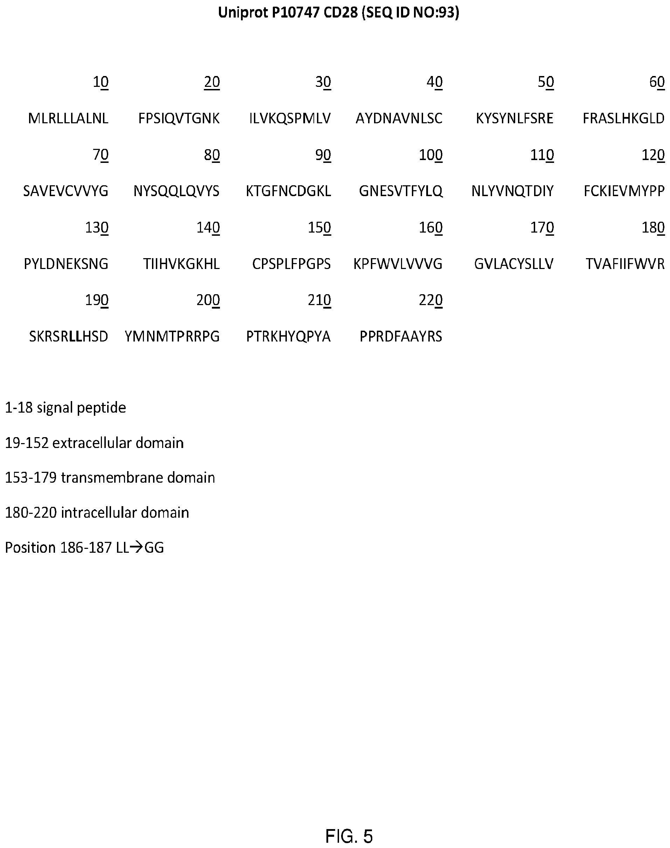

FIG. 5. Amino acid sequence and map of sections of Uniprot P10747 CD28.

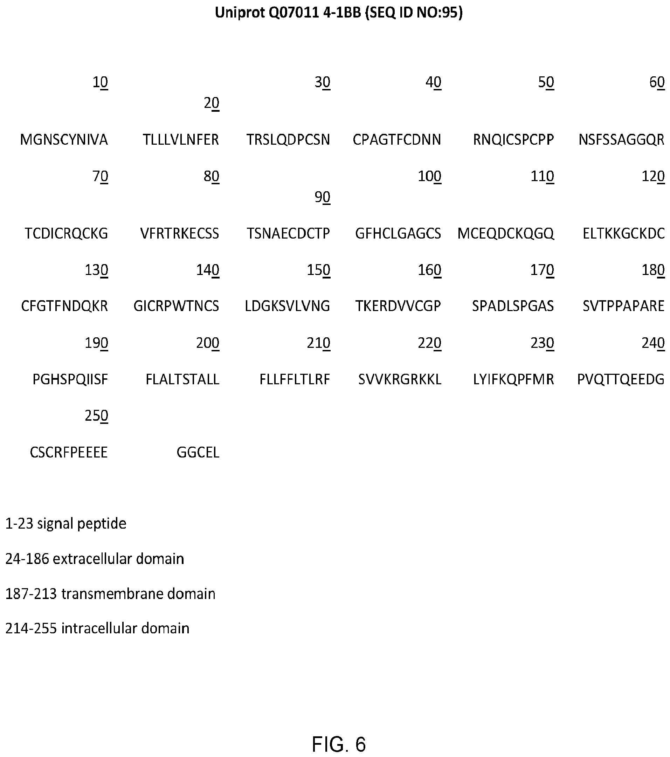

FIG. 6. Amino acid sequence and map of sections of Uniprot Q07011 4-1 BB.

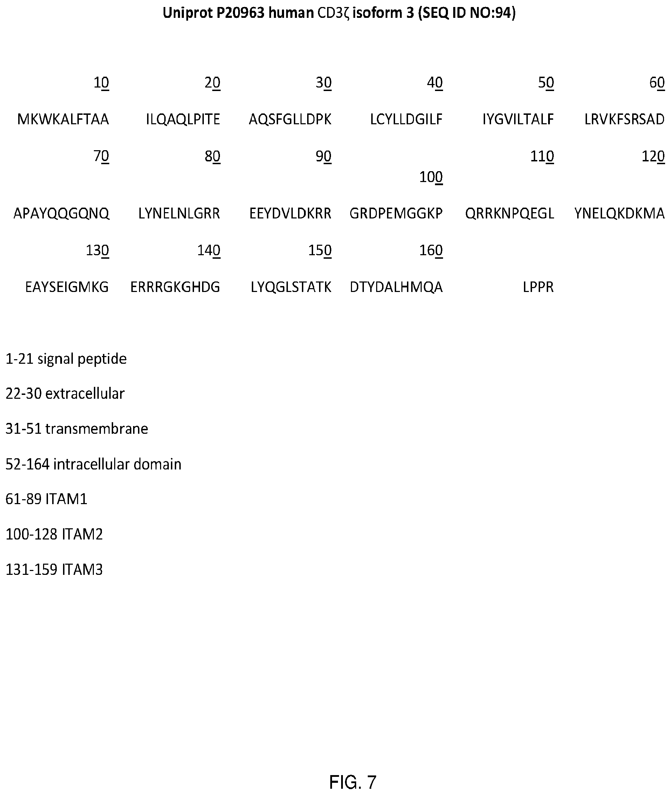

FIG. 7. Amino acid sequence and map of sections of Uniprot P20963 human CD3ζ isoform 3.

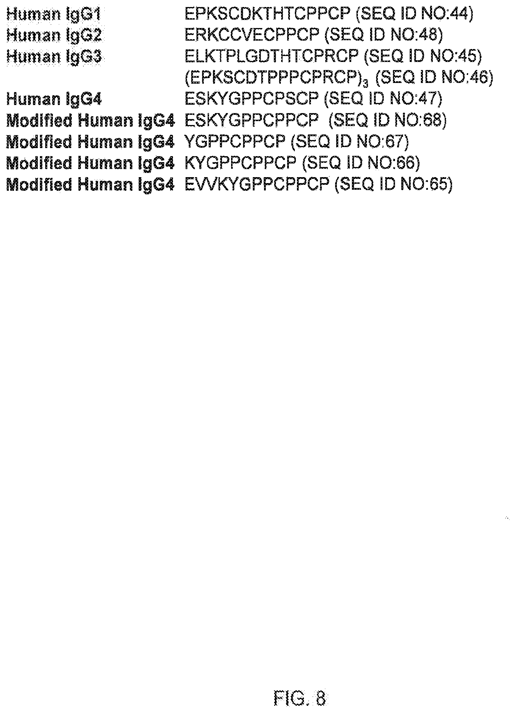

FIG. 8. Exemplary hinge region sequences.

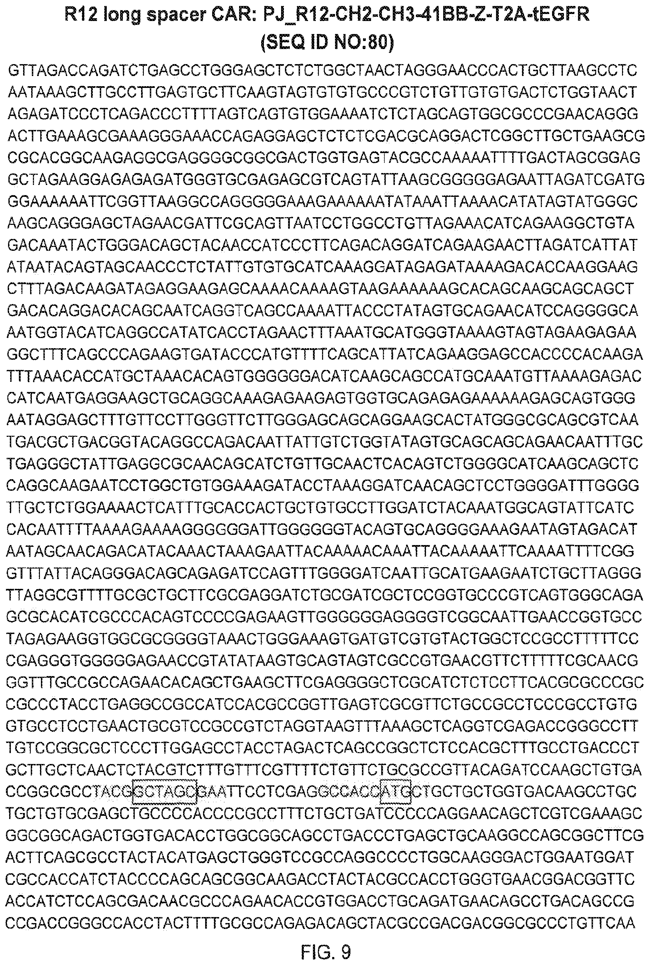

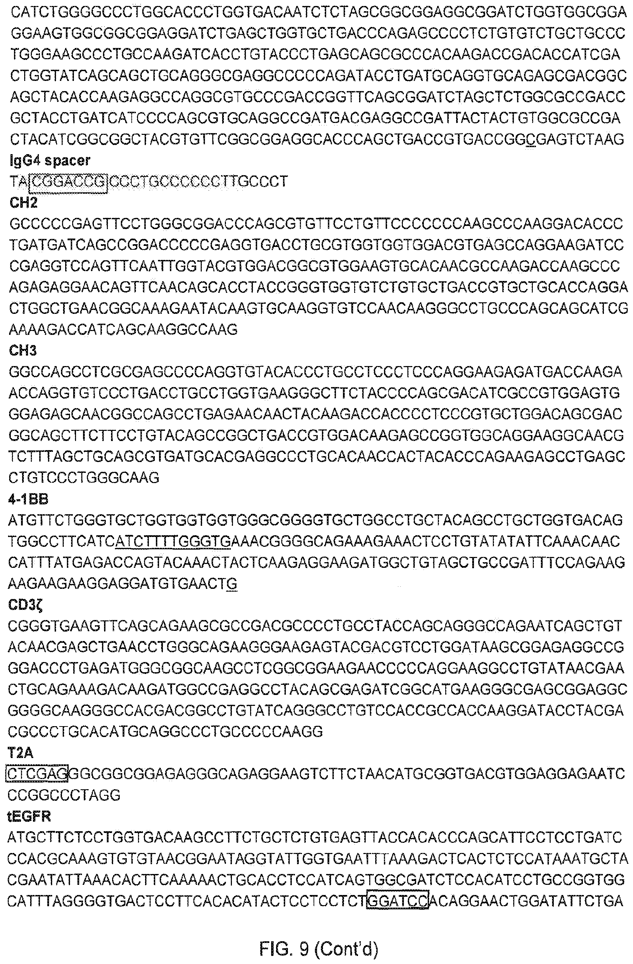

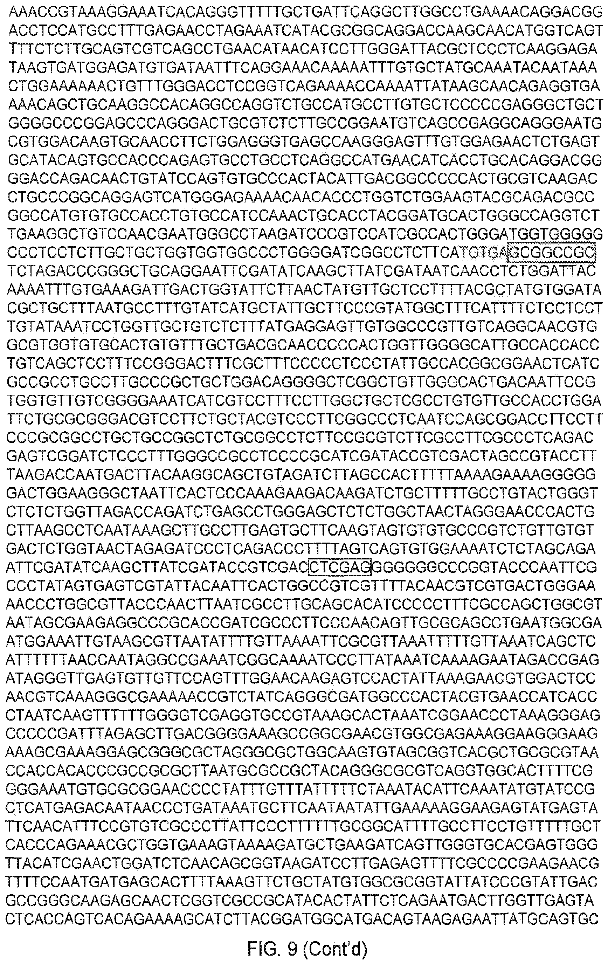

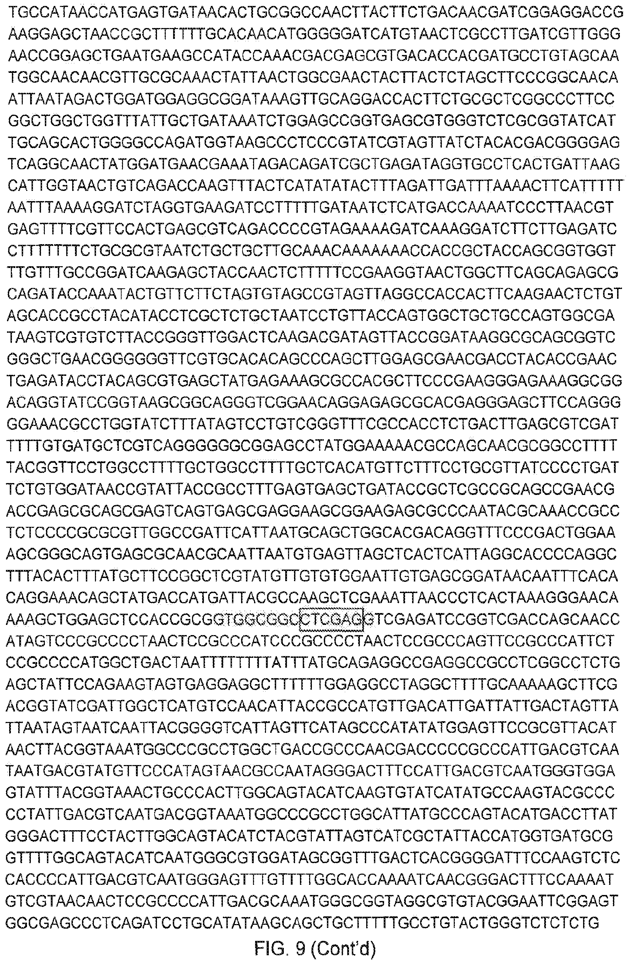

FIG. 9. Sequence of R12 long spacer CAR: PJ_R12-CH2-CH3-41BB-Z-T2A-tEGFR. tEGFR can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

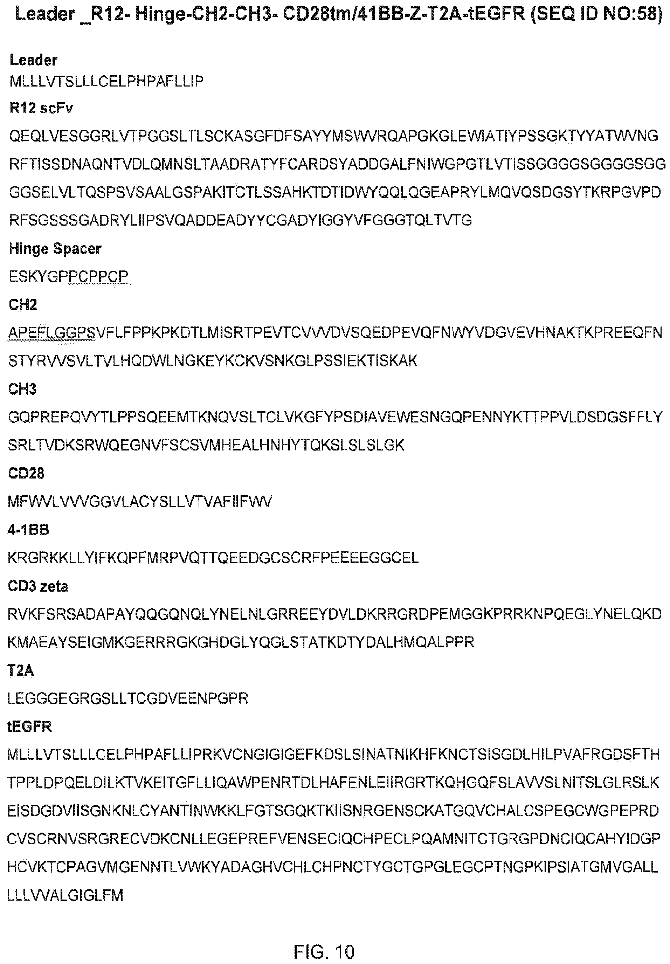

FIG. 10. Sequence of Leader_R12-Hinge-CH2-CH3-CD28tm/41 BB-Z-T2A-tEGFR. tEGFR can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

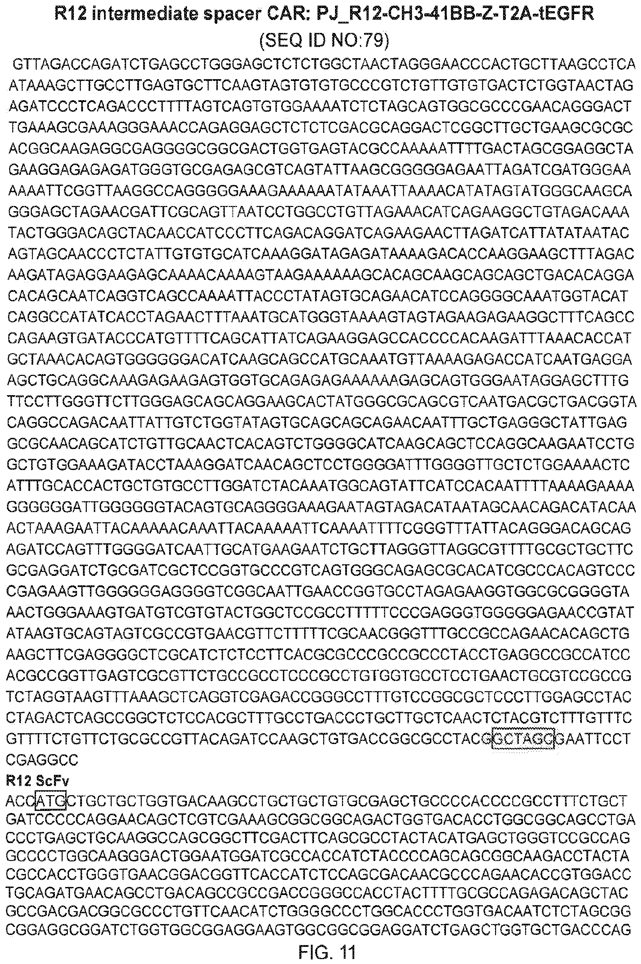

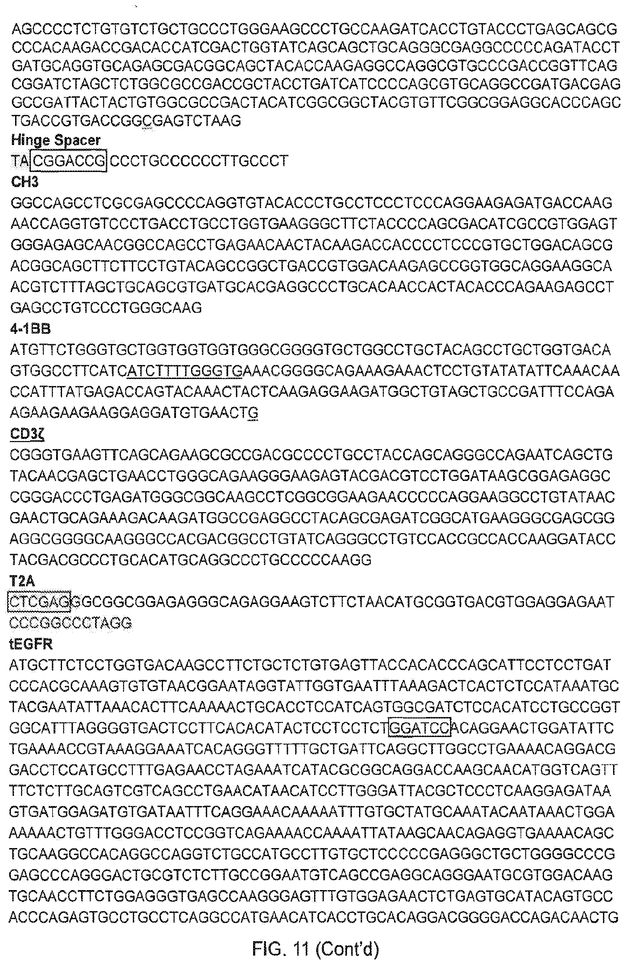

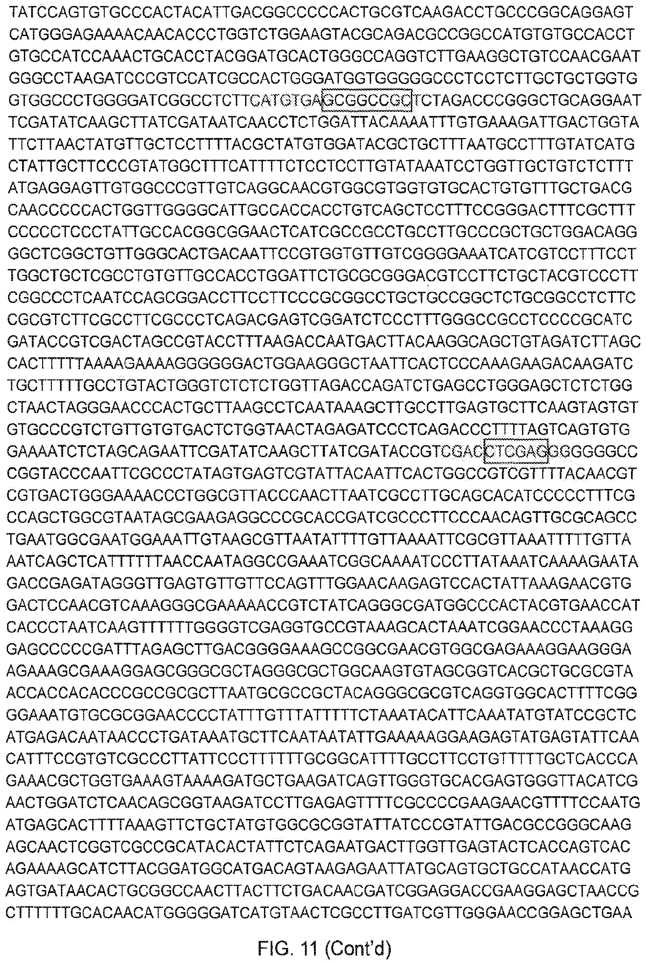

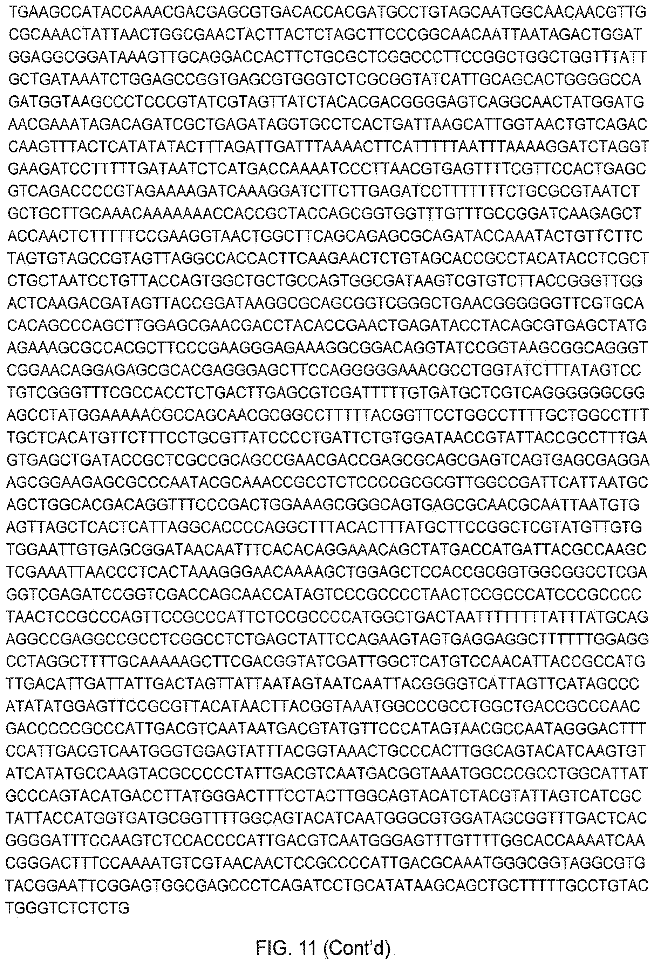

FIG. 11. Sequence of R12 intermediate spacer CAR: PJ_R12-CH3-41 BB-Z-T2A-tEGFR. tEGFR can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

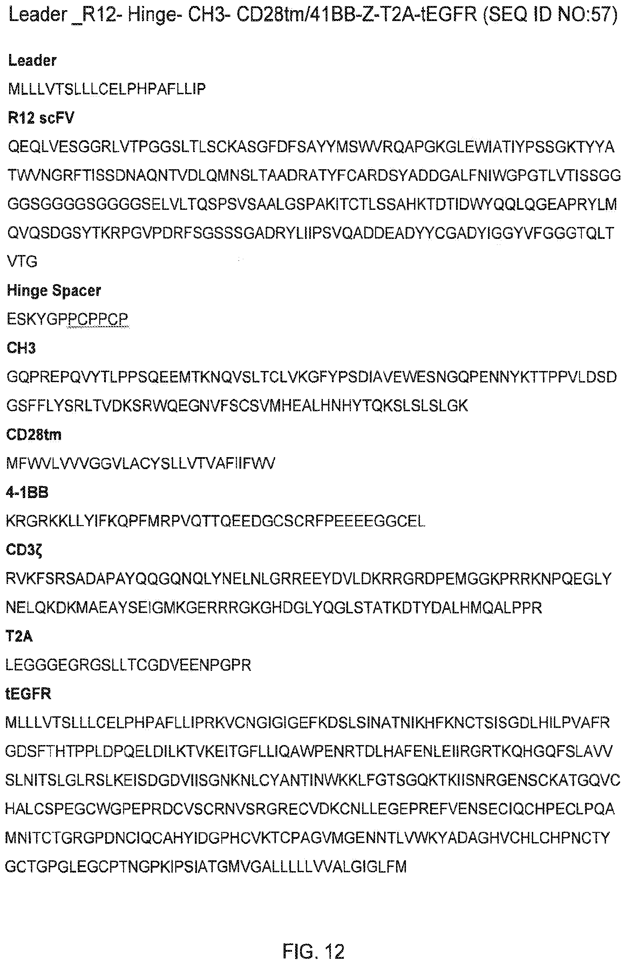

FIG. 12. Sequence of Leader_R12-Hinge-CH3-CD28tm/41 BB-Z-T2A-tEGFR. tEGFR can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

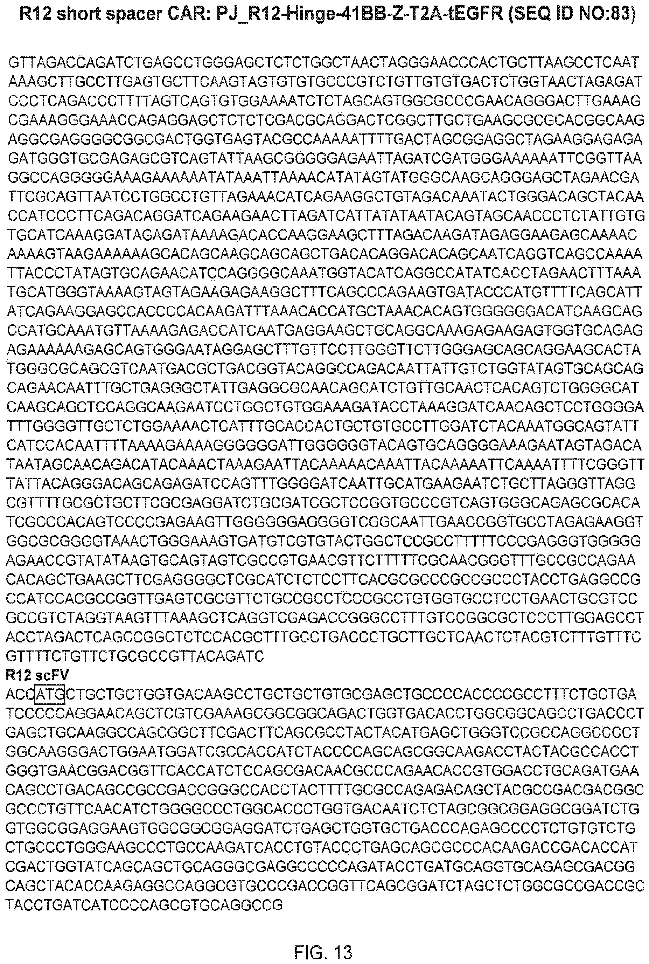

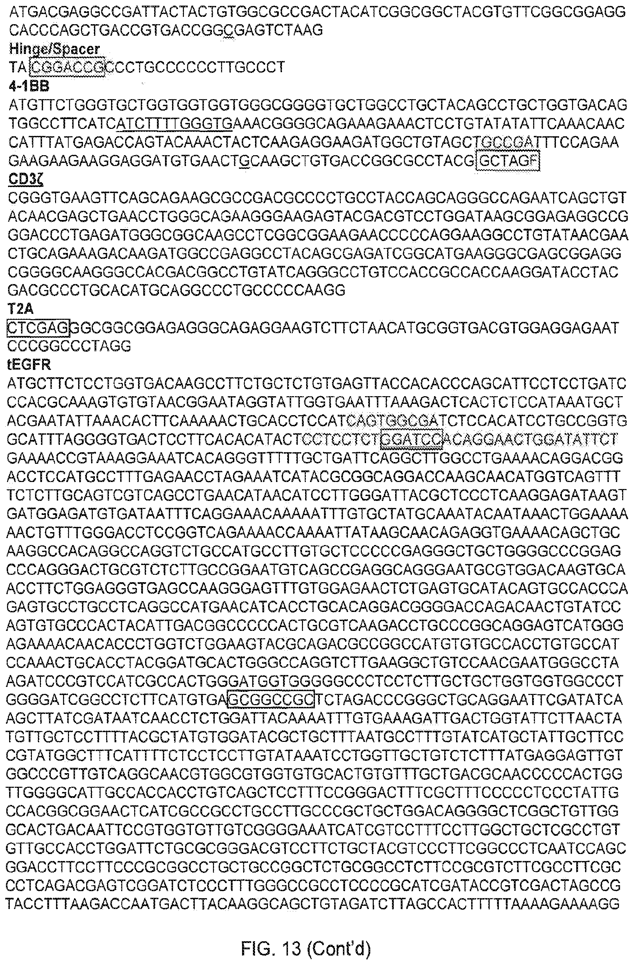

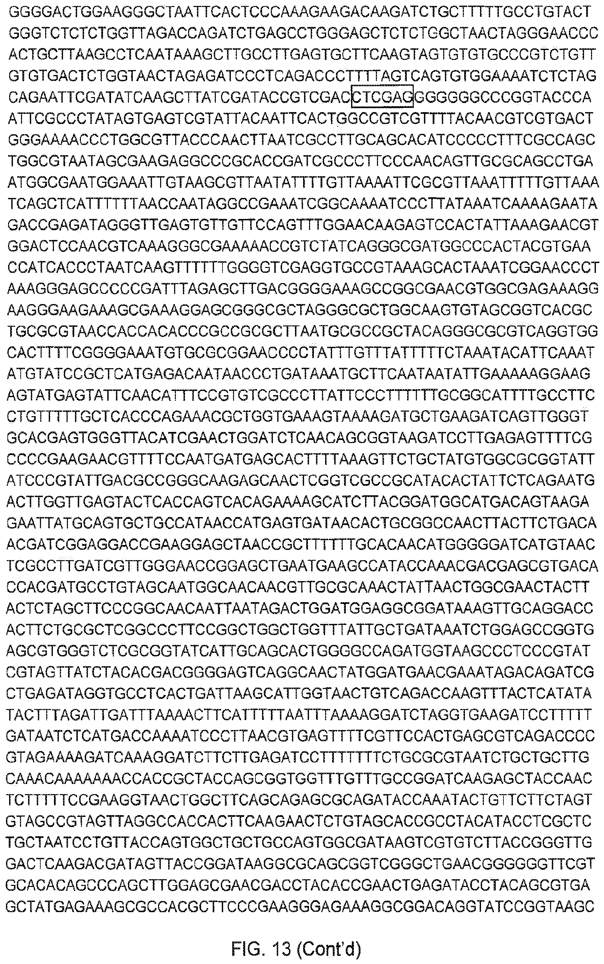

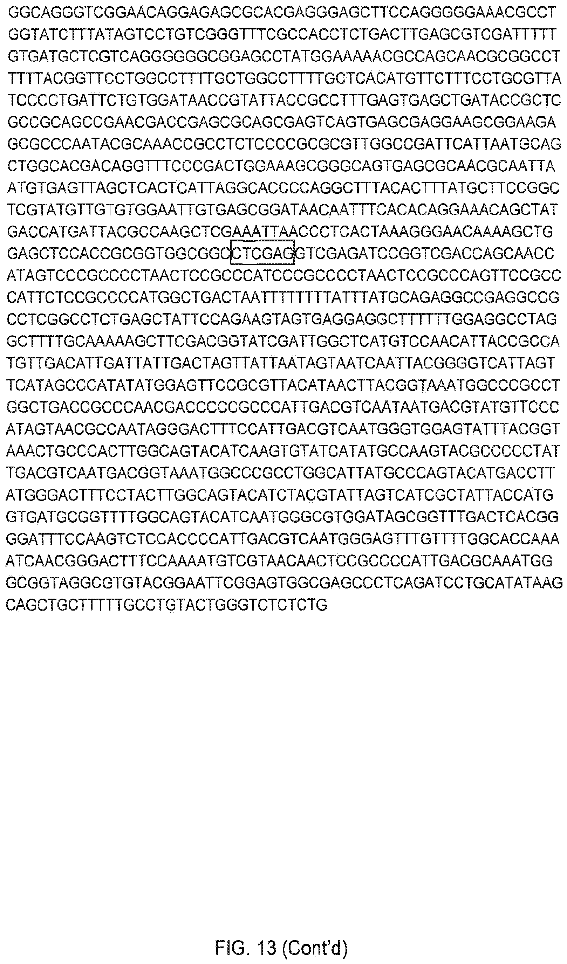

FIG. 13. Sequence of R12 short spacer CAR: PJ_R12-Hinge-41 BB-Z-T2A-tEGFR. tEGFR can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

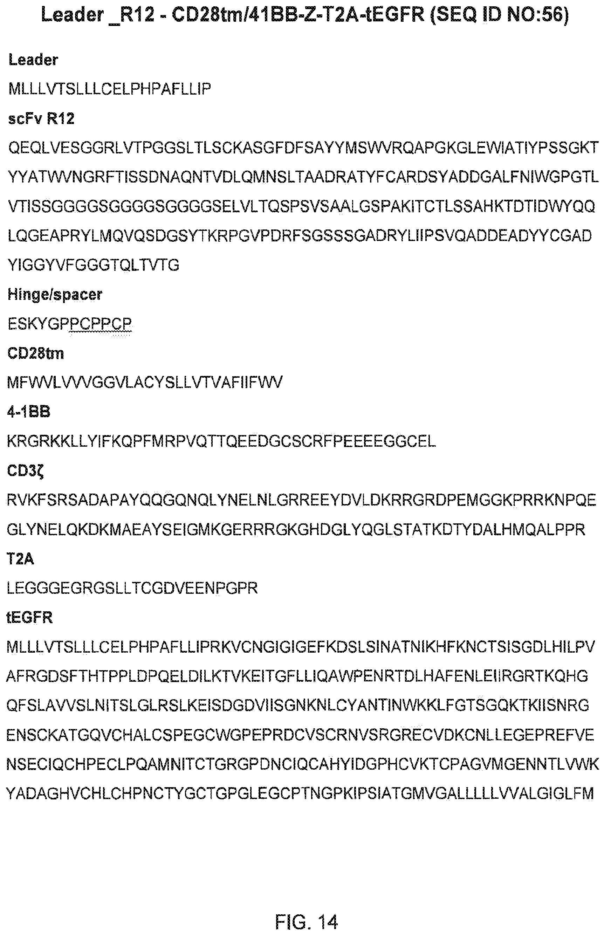

FIG. 14. Sequence of Leader_R12-CD28tm/41BB-Z-T2A-tEGFR. tEGFR can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

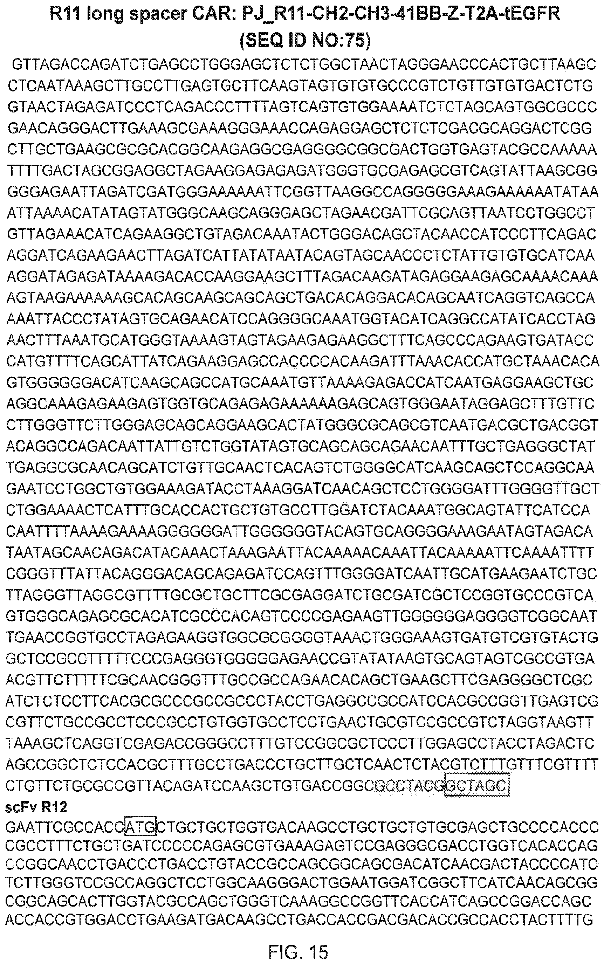

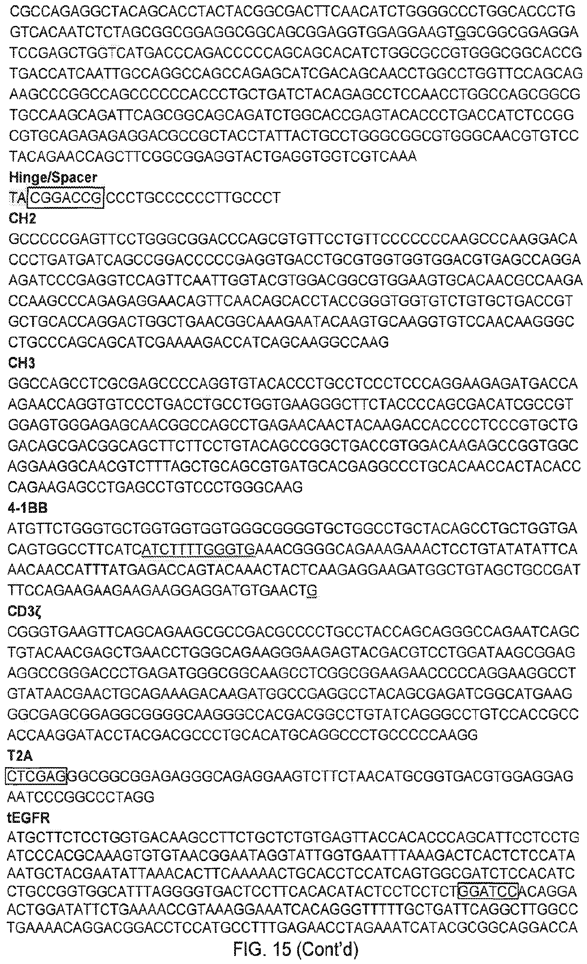

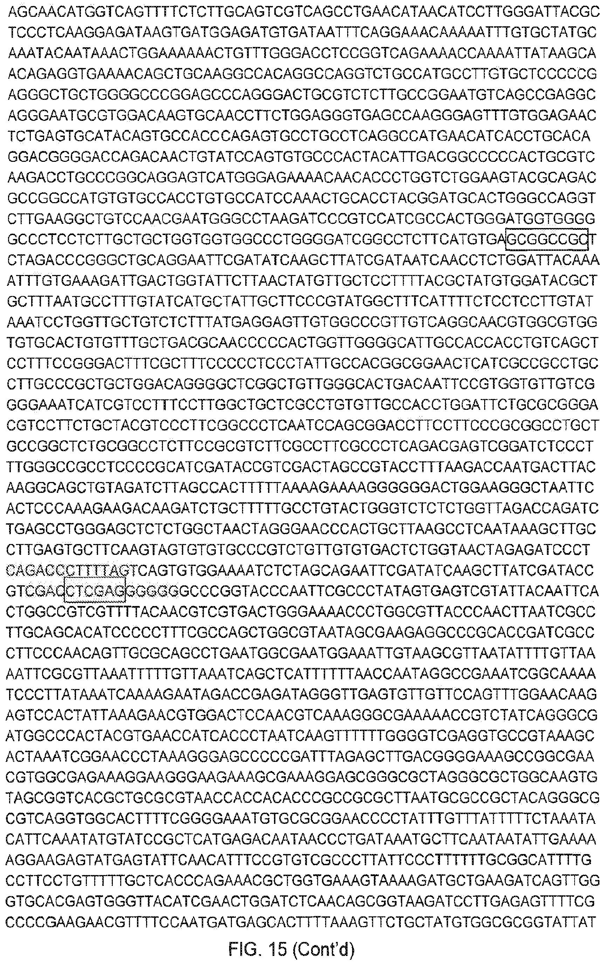

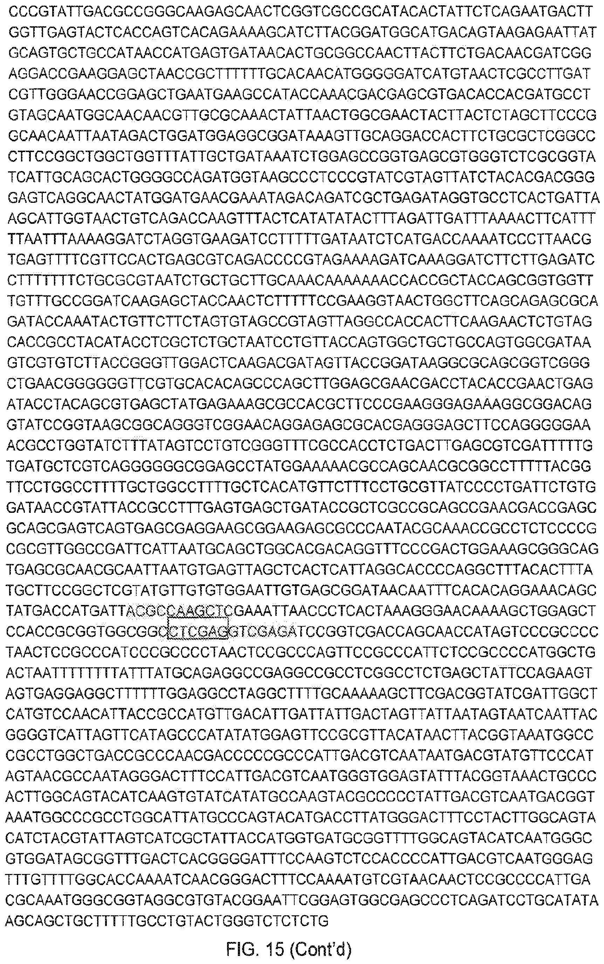

FIG. 15. Sequence of R11 long spacer CAR: PJ_R11-CH2-CH3-41BB-Z-T2A-tEGFR. tEGFR can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

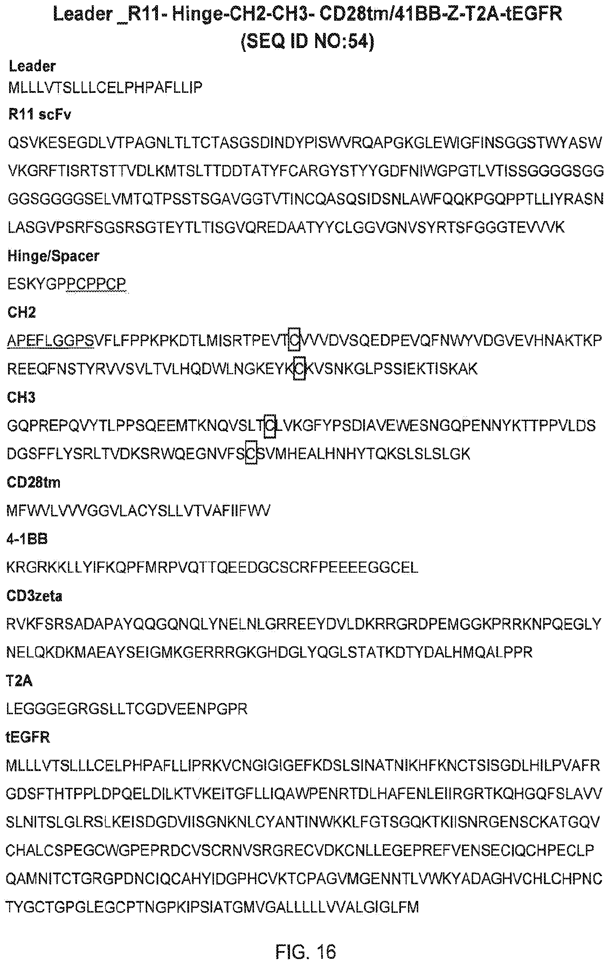

FIG. 16. Sequence of Leader_R11-Hinge-CH2-CH3-CD28tm/41BB-Z-T2A-tEGFR. tEGFR can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

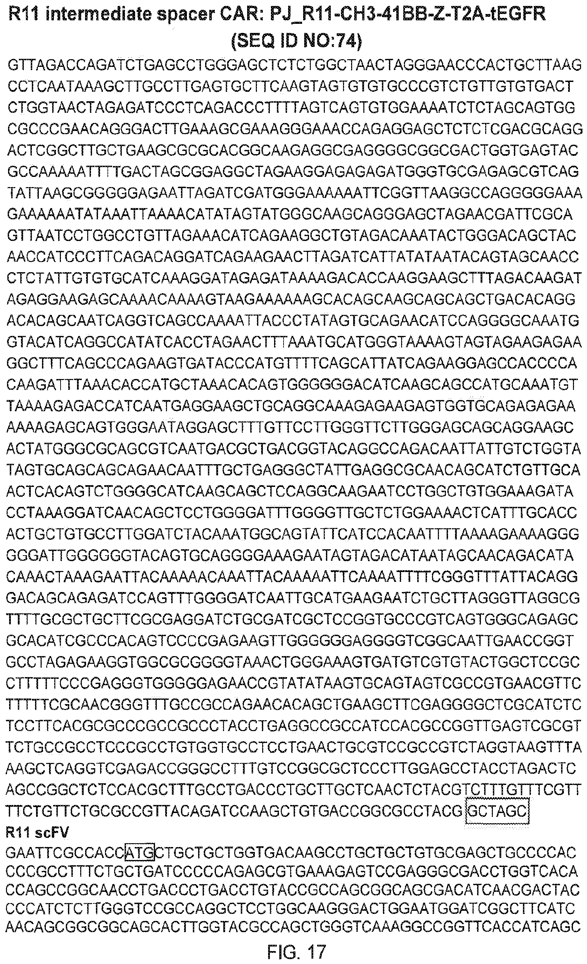

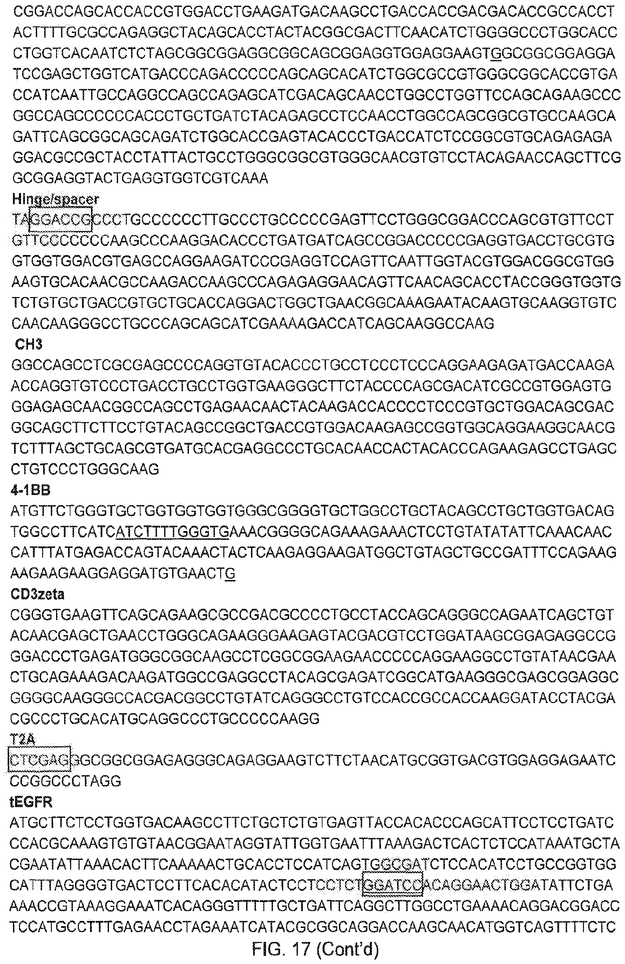

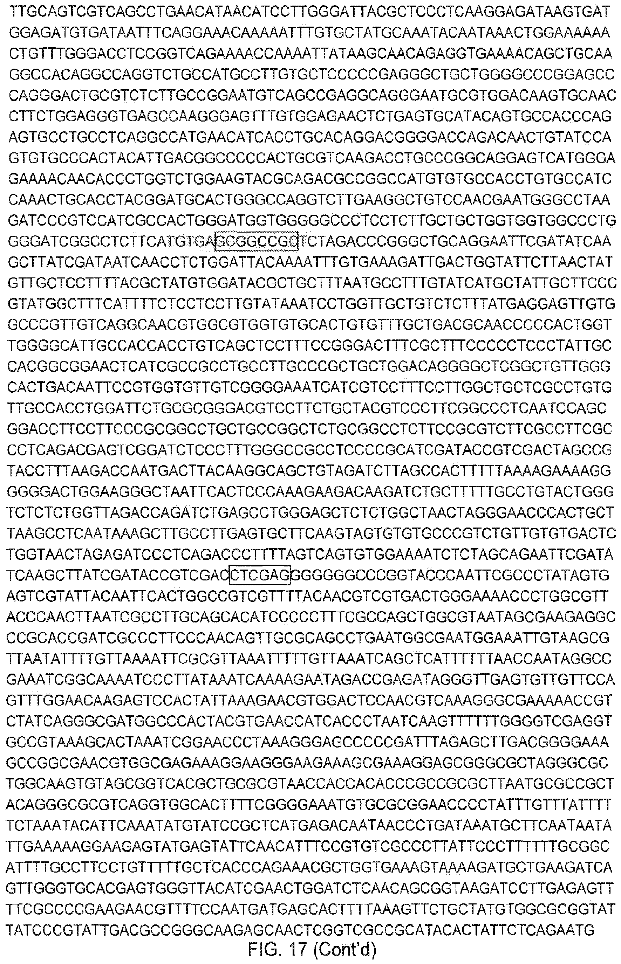

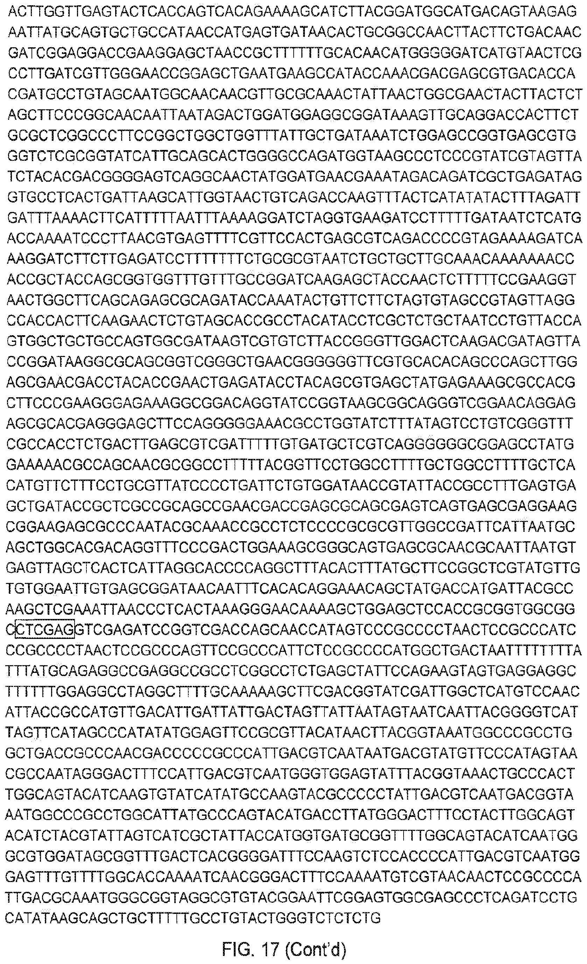

FIG. 17. Sequence of R11 intermediate spacer CAR: PJ_R11-CH3-41BB-Z-T2A-tEGFR. tEGFR can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

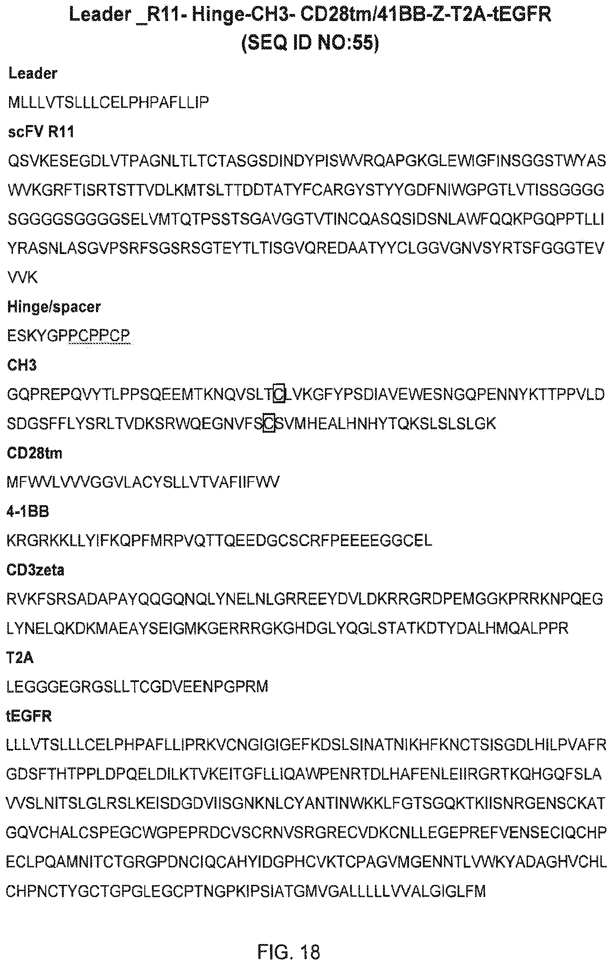

FIG. 18. Sequence of Leader_R11-Hinge-CH3-CD28tm/41 BB-Z-T2A-tEGFR. tEGFR can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

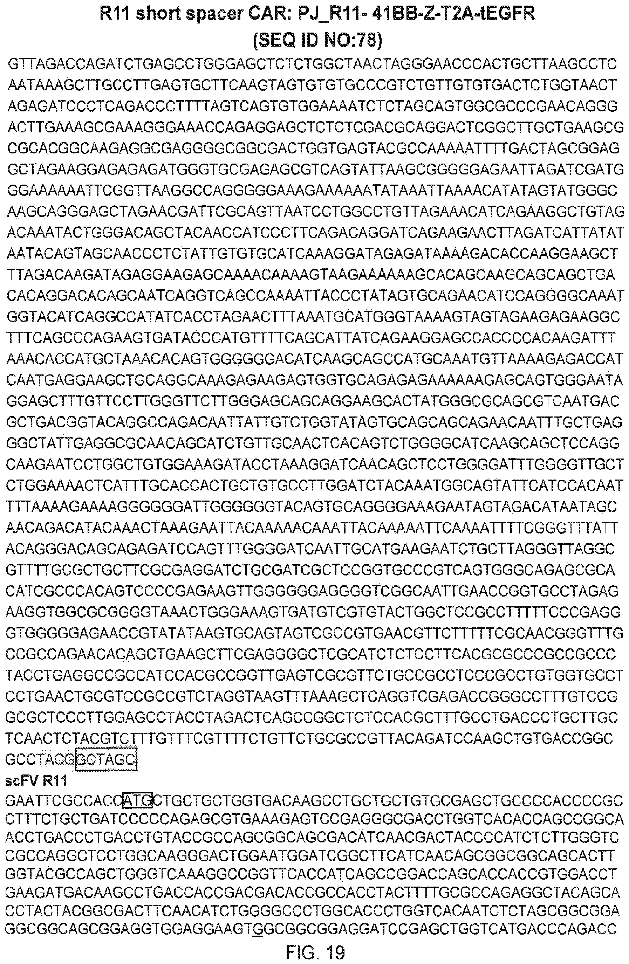

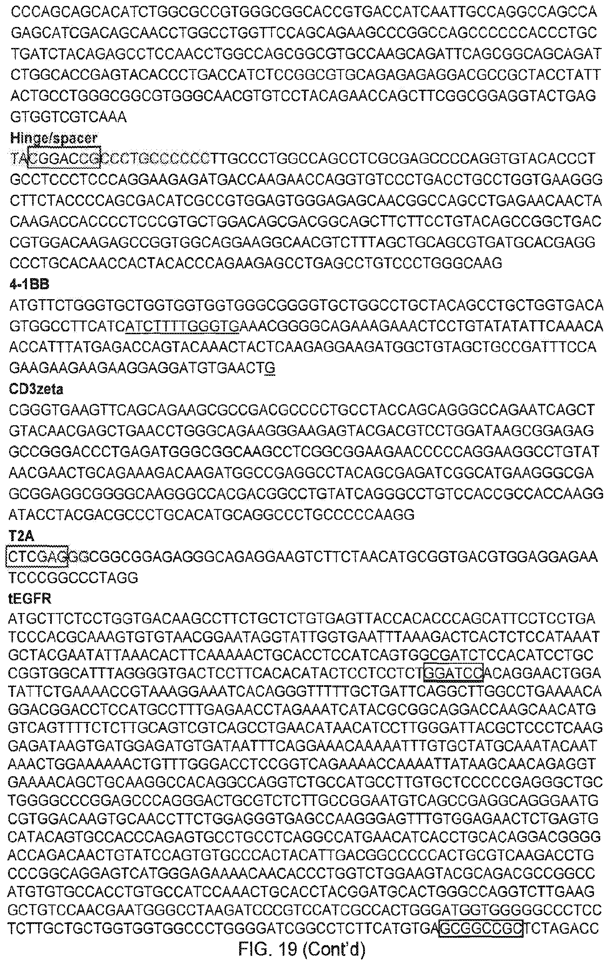

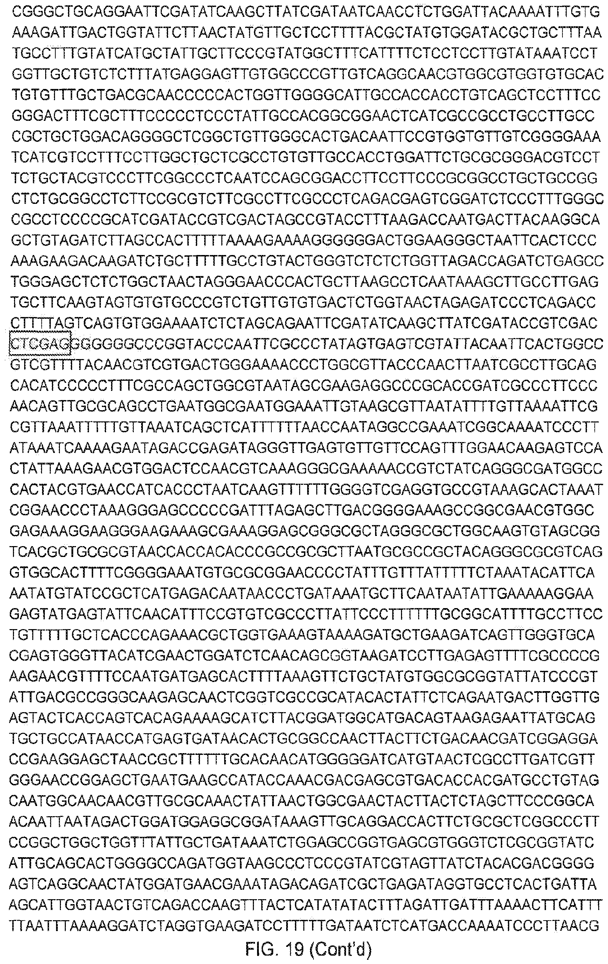

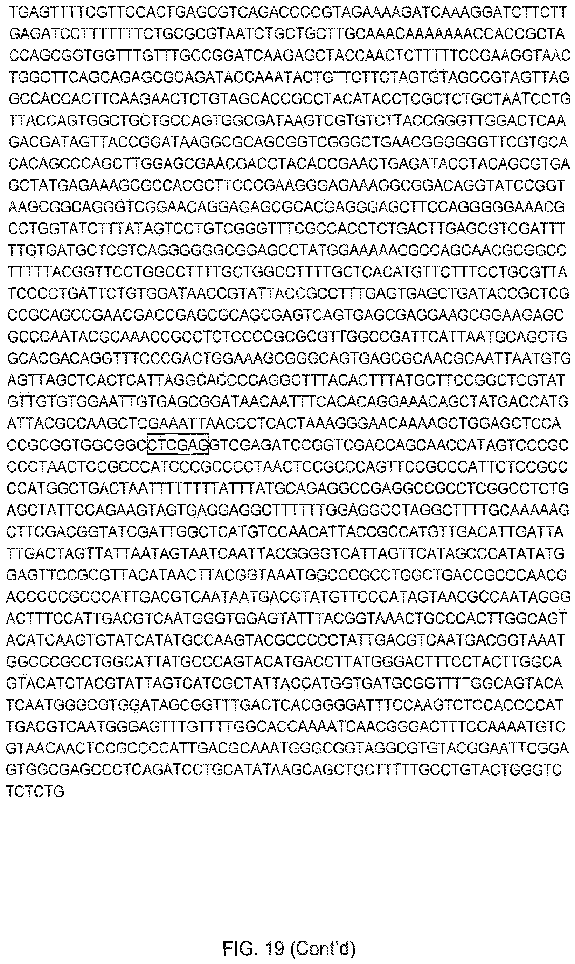

FIG. 19. Sequence of R11 short spacer CAR: PJ_R11-41 BB-Z-T2A-tEGFR. tEGFR can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

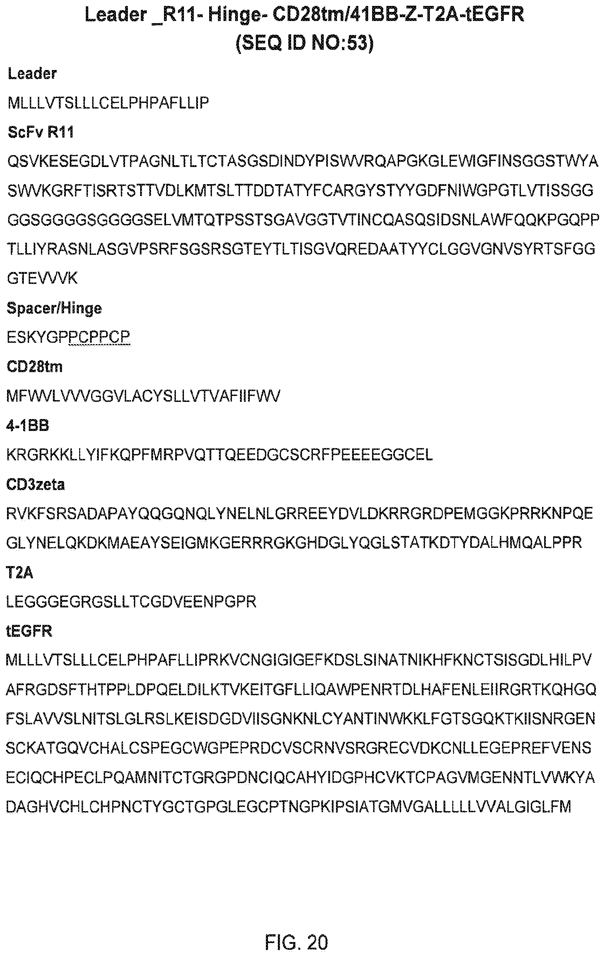

FIG. 20. Sequence of Leader_R11-Hinge-CD28tm/41BB-Z-T2A-tEGFR. tEGFR can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

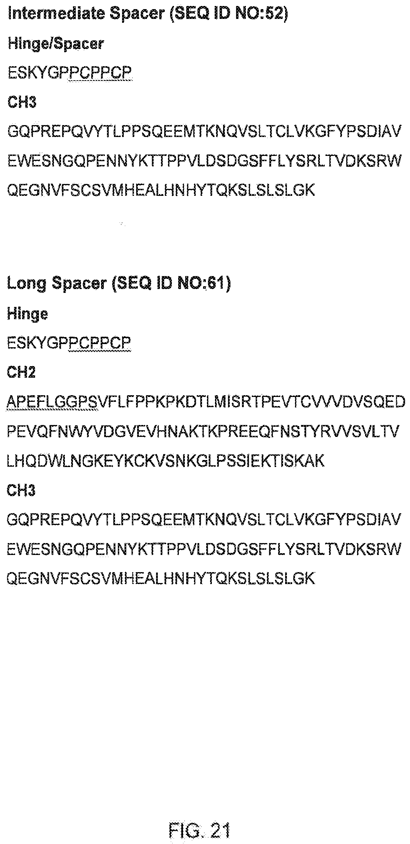

FIG. 21. Exemplary spacer sequences.

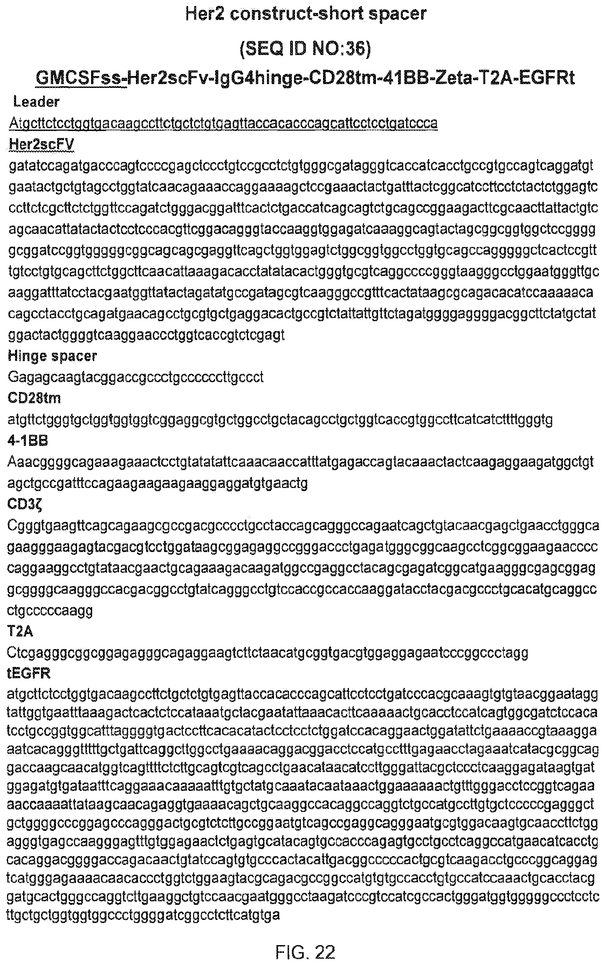

FIG. 22. Sequence of Her2 short-spacer construct, GMCSFss-Her2scFv-lgG4hinge-CD28tm-41BB-Zeta-T2A-EGFRt. EGFRt can be replaced or supplemented with a tag cassette binding an ExoCBM, such as STREP TAG^{®} II (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

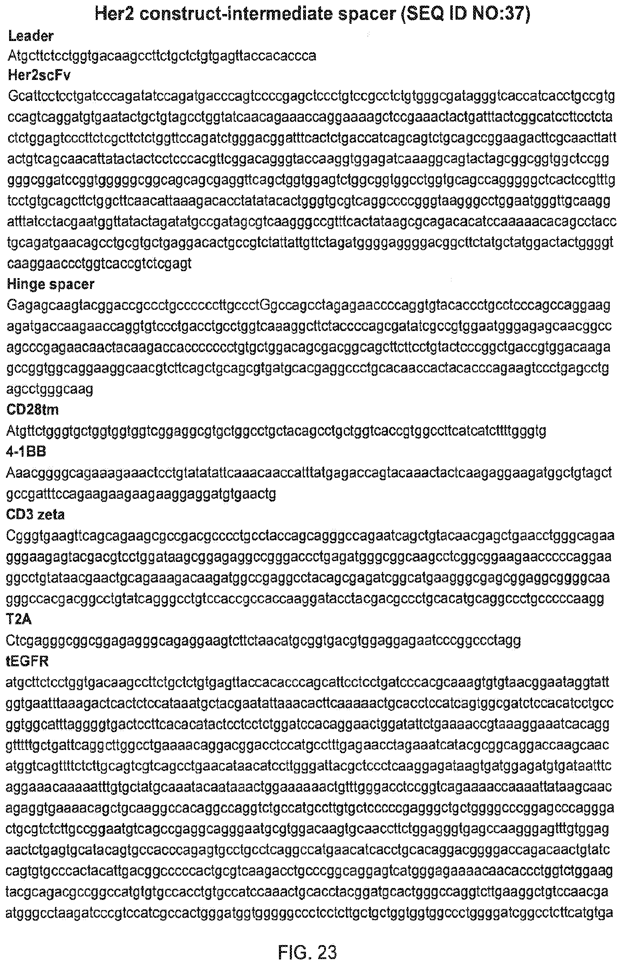

FIG. 23. Sequence of intermediate spacer Her2 construct.

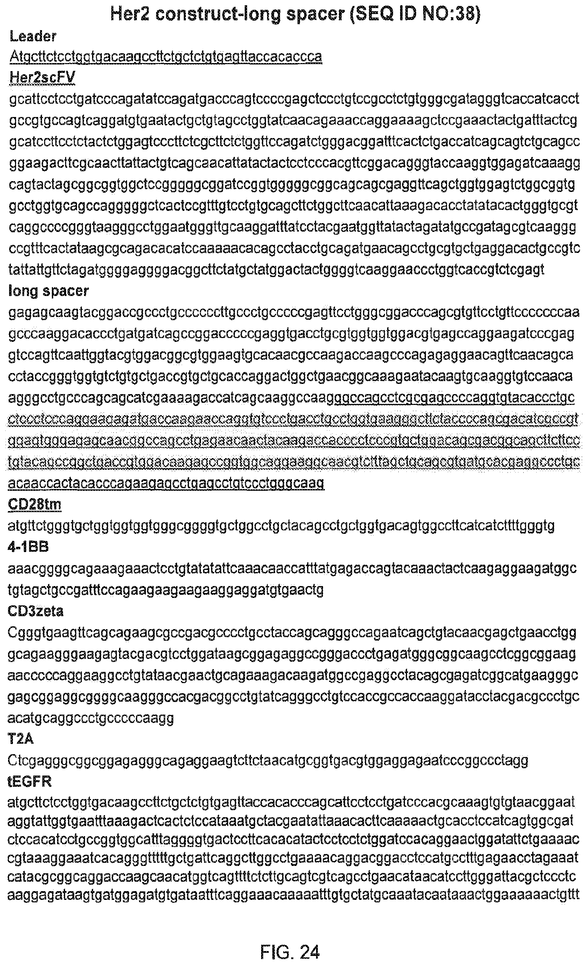

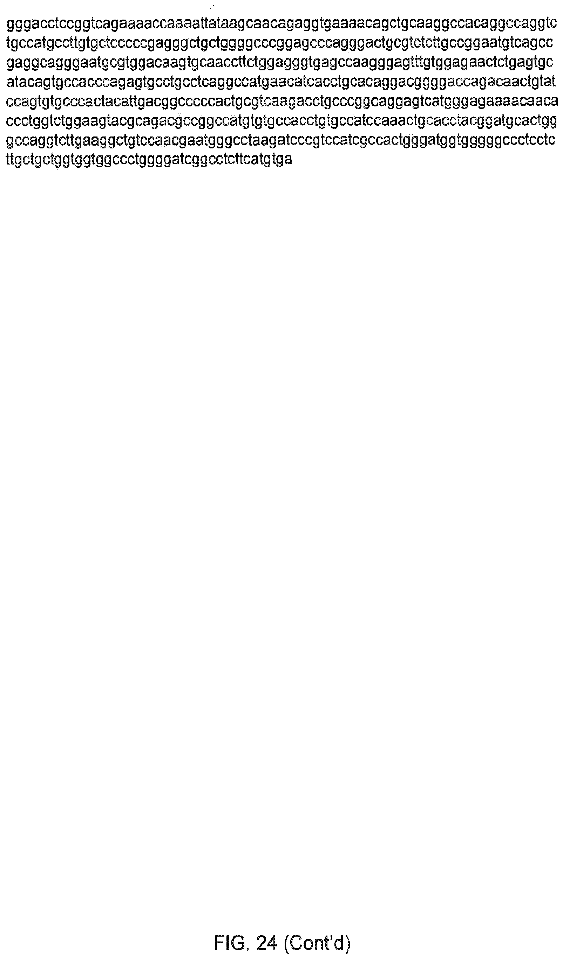

FIG. 24. Sequence of long spacer Her2 construct.

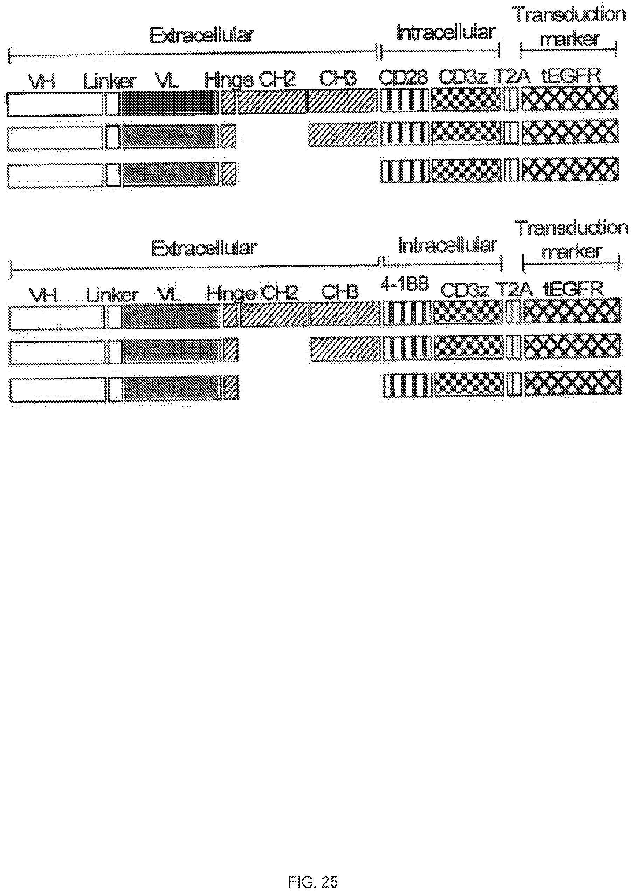

FIG. 25. Library of spacer sequences. A plasmid library was constructed which contains codon optimized DNA sequences that encode extracellular components including portions of the IgG4 hinge, the IgG4 hinge linked to CH2 and CH3 domains, or the IgG4 hinge linked to the CH3 domain. Any scFV sequence (VH and VL) can be cloned 5' to the sequences encoded in this library of variable spacer domains. The spacer domains are in turn linked to CD28 transmembrane and intracellular signaling domains and to CD3 ζ. A T2A sequence in the vector separates the chimeric receptor from a selectable marker encoding a truncated human epidermal growth factor receptor (EGFR). EGFR can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

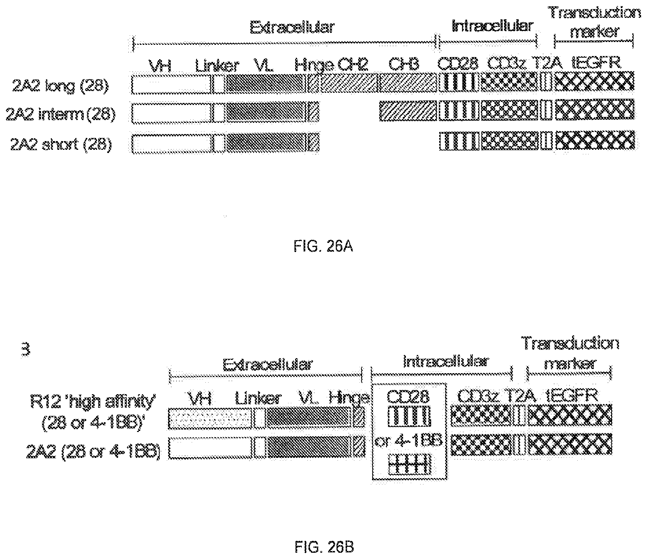

FIG. 26A and 26B. Design of ROR1 chimeric receptors with modified spacer length and derived from the 2A2 and R12 scFV with different affinity. (FIG. 26A) Design of lentiviral transgene inserts encoding a panel of ROR1 chimeric receptors containing the 2A2 scFV, an lgG4-Fc derived spacer of 'Hinge-CH2-CH3' (long spacer, 229 AA), 'Hinge-CH3' (intermediate, 119 AA), or 'Hinge' only (short, 12 AA), and a signaling module with CD3ζ and CD28. Each chimeric receptor cassette contains a truncated EGFR marker encoded downstream of a T2A element. EGFR can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein. (FIG. 26B) Lentiviral transgene inserts encoding ROR1-specific chimeric receptors derived from the R12 and 2A2 scFV with short lgG4-Fc 'Hinge' spacer (12 AA), and a signaling module containing CD28 or 4-1 BB and CD3ζ respectively (total: 4 constructs).

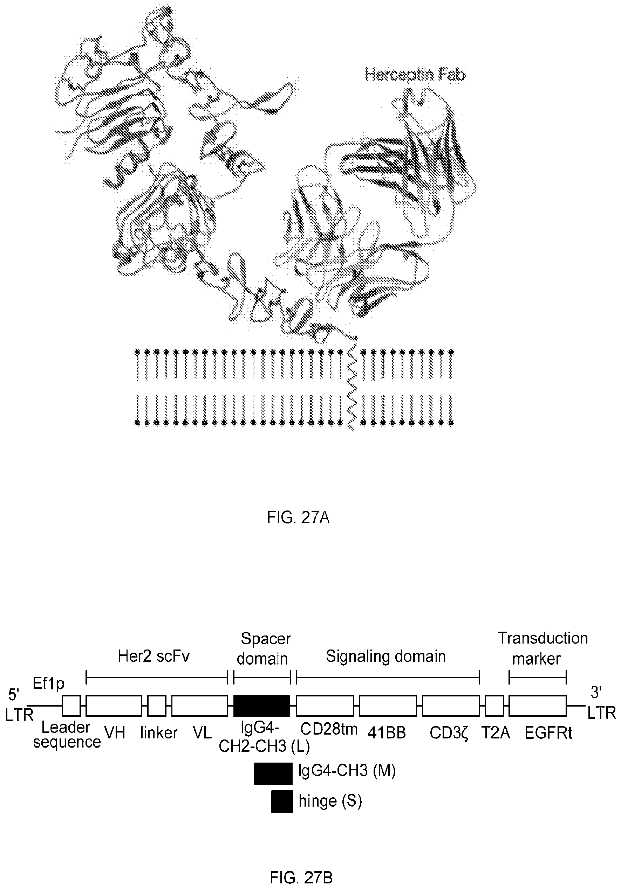

FIG. 27A and 27B. FIG. 27A) Depiction of Herceptin Fab epitope location on tumor cell membrane proximal epitope on human HER2, FIG. 27B) Structural formats of Herceptin scFv CAR spacer length variants as -T2A- linked proteins with the carboxyl EGFRt marker transmembrane protein. EGFRt can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

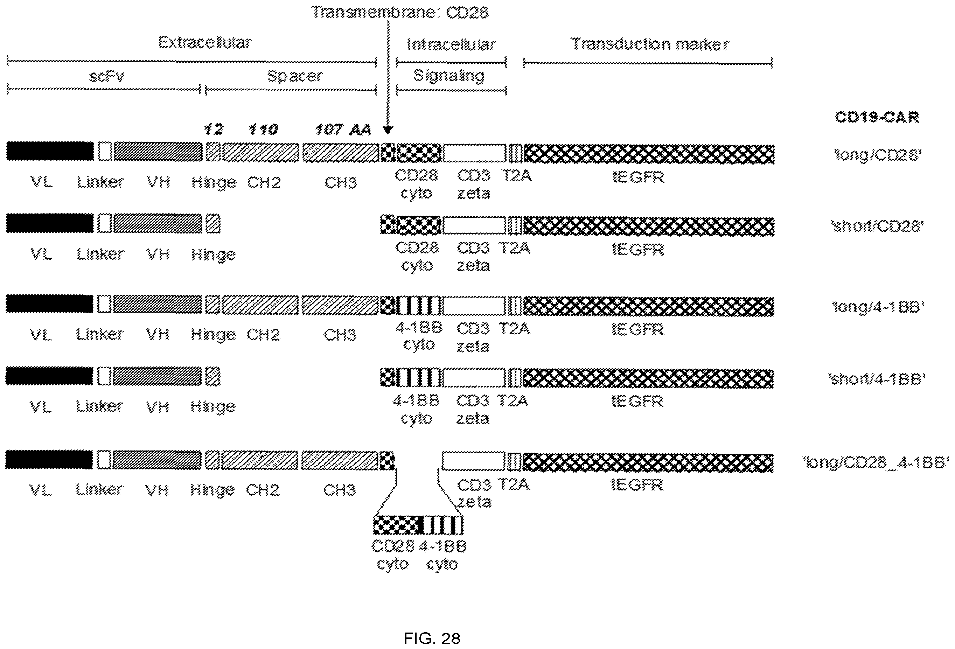

FIG. 28. CD19-chimeric receptor vectors. Design of lentiviral transgene inserts encoding a panel of CD19-specific chimeric receptors that differ in extracellular spacer length and intracellular co-stimulation. Each chimeric receptor encoded the CD19-specific single chain variable fragment derived from the FMC63 mAb in a VL-VH orientation, an lgG4-derived spacer domain of Hinge-CH2-CH3 (long spacer, 229 AA) or Hinge only (short spacer, 12 AA), and a signaling module containing CD3ζ with CD28 or 4-1BB alone or in tandem. Each chimeric receptor cassette contains a truncated EGFR marker encoded downstream of a cleavable 2A element. Truncated EGFR can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

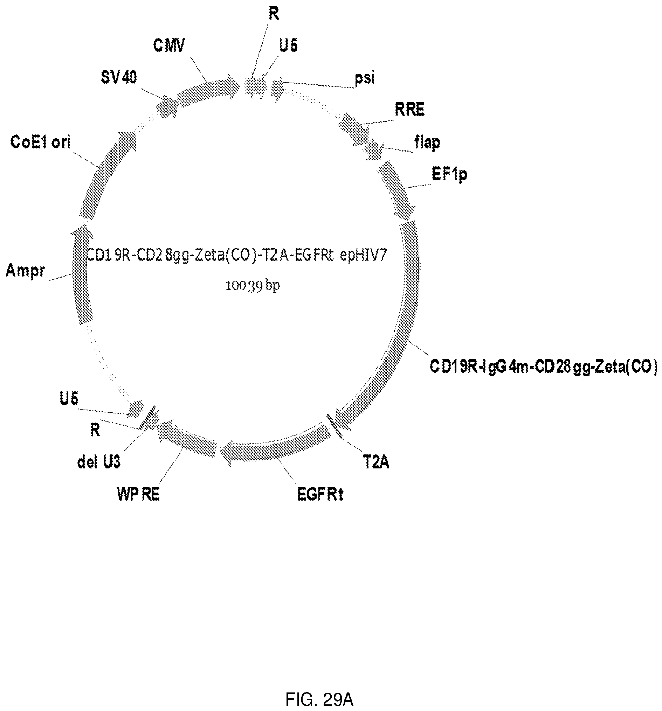

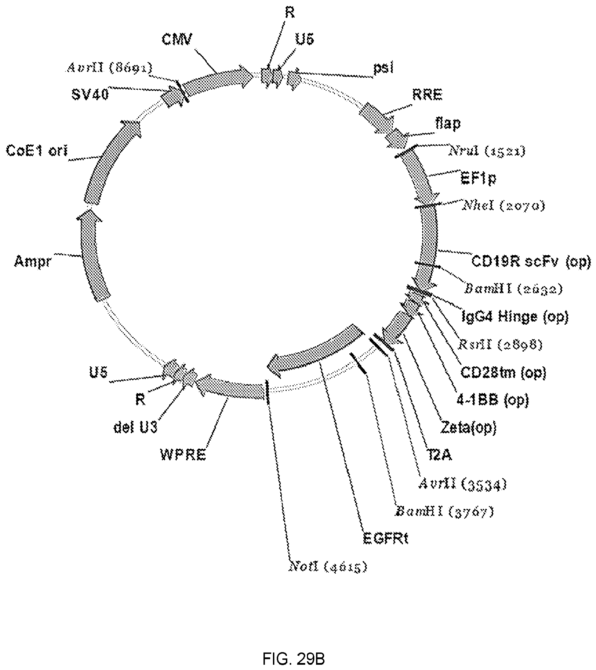

FIG. 29A and 29B. Exemplary SIN lentiviral plasmids. FIG. 29A shows a SIN CD19 specific scFvFc-CD3ζCD28 CAR and huEGFRt lentiviral plasmid. FIG. 29B shows SIN CD19-specific scFv-4-1BBCD3ζ CAR and huEGFRt lentiviral plasmid. huEGFRt can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

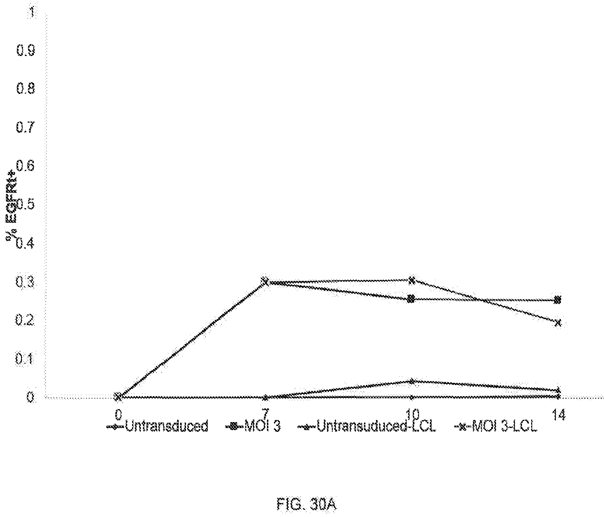

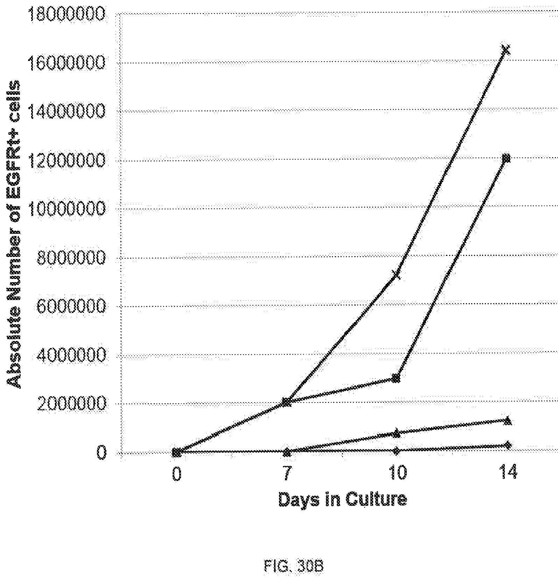

FIG. 30A and 30B. EGFR expression as a marker of transduction efficiency/gene expression stability by percent (FIG. 30A) and absolute number (FIG. 30B). HSPC were cultured on Delta as previously described. On day +3, the cells were transduced using scFvFc-CD3ζCD28 CAR and huEGFRt vector at an MOI of 3 in the presence of protamine sulfate and underwent spinfection. Transgene expression was measured over the course of the culture by flow using Erbitux, which binds to the EGFRt tag. Designated cultures had irradiated LCL added at a 1:1 ratio on day +7. EGFR can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

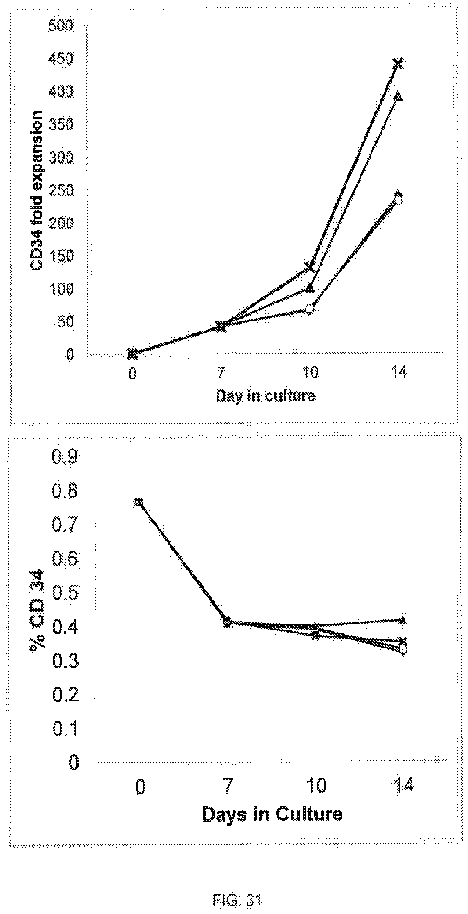

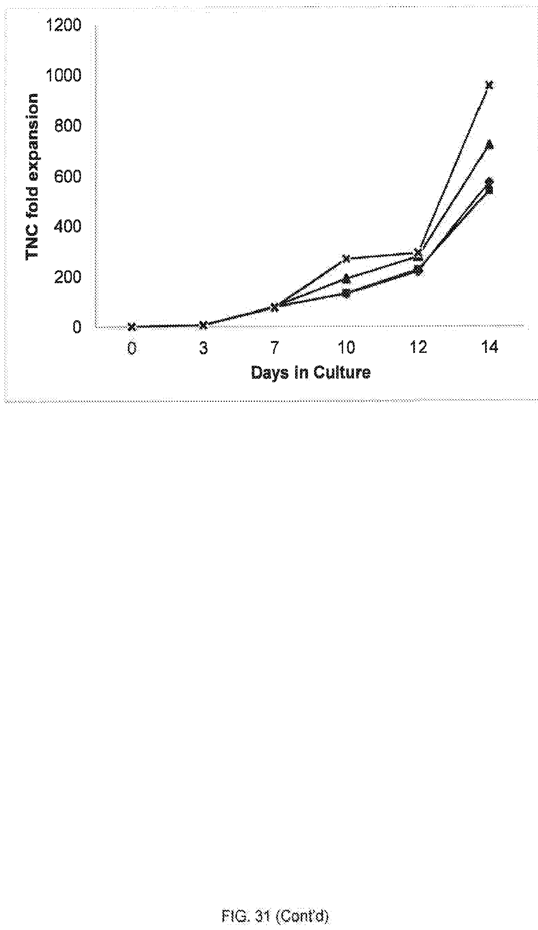

FIG. 31. CD34⁺ CB cells cultured on Notch ligand underwent transduction with lentivirus on day + 3 with a MOI of 3 using scFvFc-CD3ζCD28 CAR and huEGFRt vector. LCL was added to indicated cultures on day 7 at a 1:1 ratio (transduced (■), transduced with LCL (X), non-transduced (largely unseen, behind ■ line), non-transduced with LCL (▲)). CD34 fold expansion was enhanced with addition of LCL through an overall TNC fold expansion. huEGFRt can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

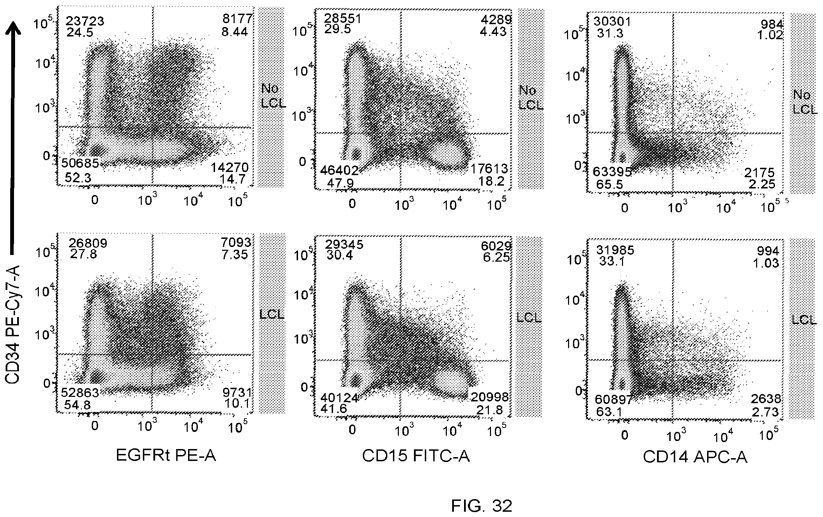

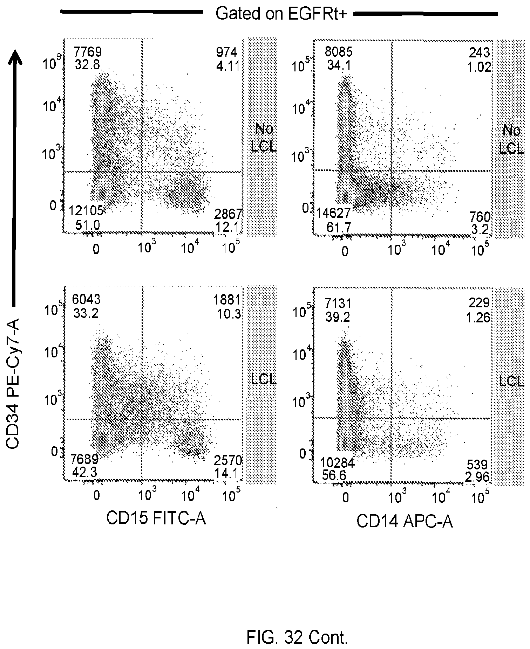

FIG. 32. Day 14 MOI 3 using scFv-4-1 BB/CD3ζ CAR and huEGFRt vector for transduction with and without LCL. The addition of LCL at day +7 did not appear to drive proliferation of CAR expressing HSPC or their progeny as noted by similar population distributions among the culture with and without LCL. huEGFRt can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

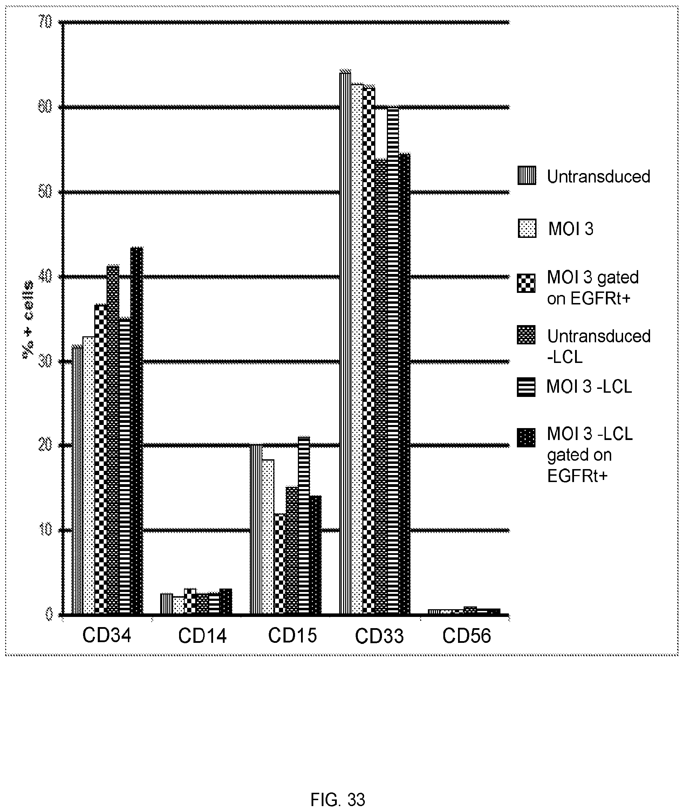

FIG. 33. End of culture phenotype. HSPC were cultured on Delta as previously described. Designated cultures were transduced on day + 3 at an MOI of 3 with lentivirus to express a scFv-4-1BB/CD3ζ CAR and huEGFRt. Additionally, designated cultures were given irradiated LCL at a 1:1 ratio on day +7. Cultures were analyzed by flow cytometry on day 14. There were no significant differences detected between the transduced and untransduced cultures. Likewise, there were no differences detected between the total population of cells and the EGFRt+ cells suggesting that the CAR construct is equally distributed among the subgroups. huEGFRt can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

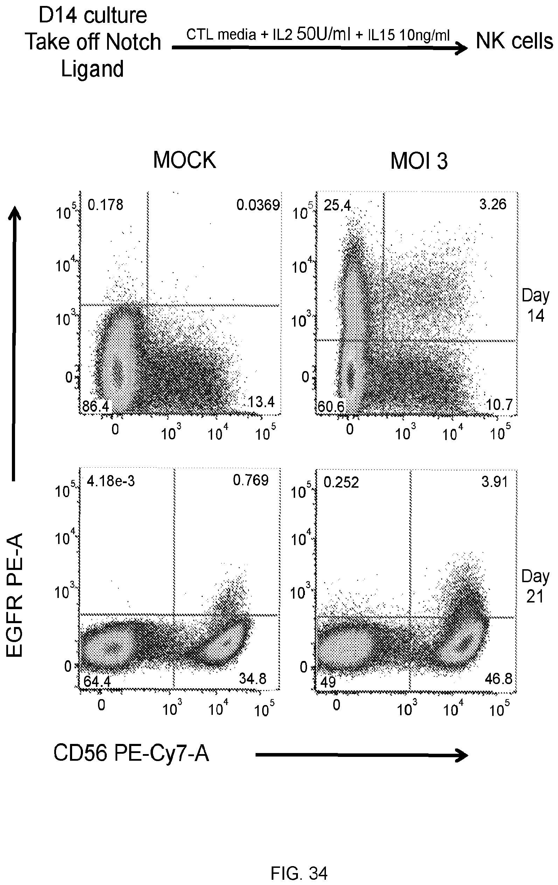

FIG. 34. Functional analysis of scFvFc-CD3ζCD28 CAR and huEGFRt vector. At the end of 14 days of culture on Delta, cells were taken off Delta, placed in RPMI media supplemented with IL-2 and IL-15 for an additional week to derive an NK population. huEGFRt can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

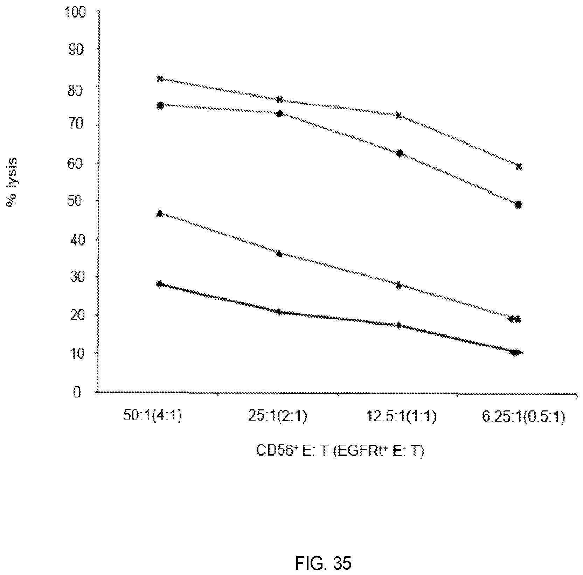

FIG. 35. A chromium release assay with target cell of K562 (x and • ) or LCL (▲ and ◆) using NK effector cells derived from CD34⁺ CB cells expanded on Notch ligand and transduced to express a CD19 specific scFvFc-CD3ζCD28 CAR and huEGFRt (• and ◆) or non-transduced (▲ and x). Mature NK cells were derived by an additional week in culture with RPMI, IL-2 and IL-15. huEGFRt can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

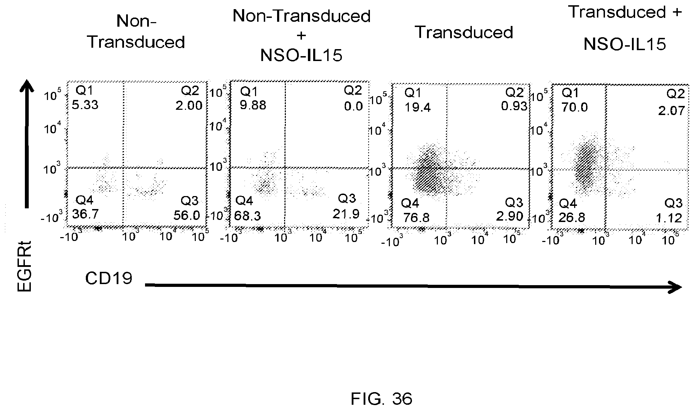

FIG. 36. Mice receiving transduced cells using scFv-4-1 BB/CD3ζ CAR and huEGFRt vector had impaired engraftment of CD19, thereby demonstrating anti-CD19 effects, which was dependent upon expression of the transgene. huEGFRt can be replaced or supplemented with a tag cassette binding an ExoCBM, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

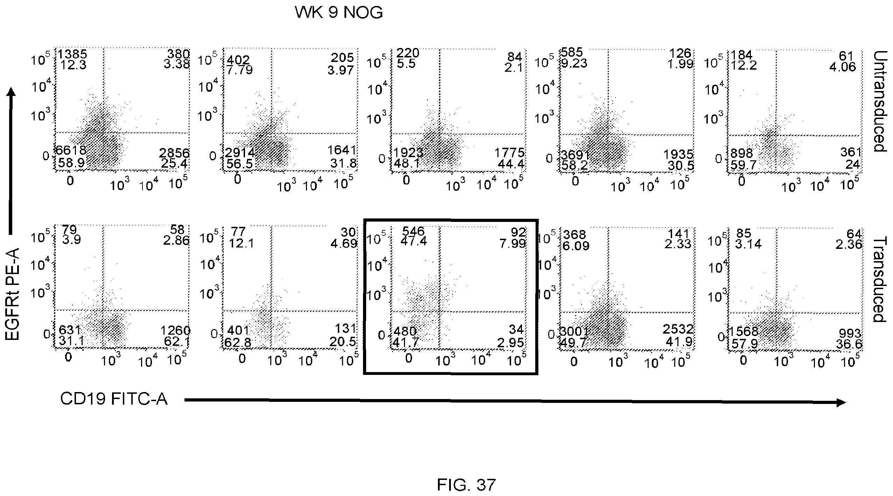

FIG. 37. NOG mice receiving cells from cultures that were transduced with lentivirus encoding for scFv-4-1BB/CD3ζ CAR and huEGFRt and show significant EGFRt expression and reduced CD19 engraftment. huEGFRt can be replaced or supplemented with a tag cassette binding an ExoCBM, such as STREP TAG^{®} II (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

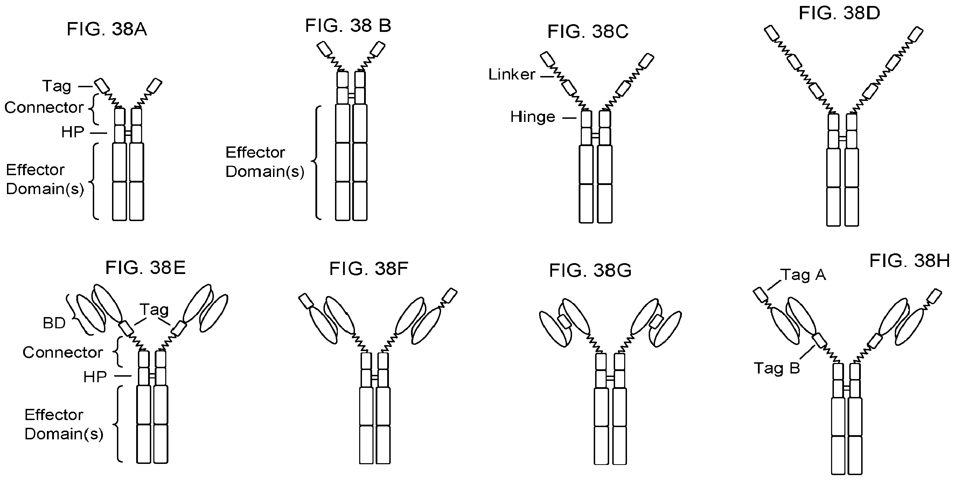

FIG. 38A - 38H show illustrations of various single chain chimeric molecules including one or more affinity tag cassettes (A-D), and optionally containing one or more ligand binding domains (E-G). In the depicted embodiments, the single chain chimeric molecules include an intracellular domain. The tag cassettes can include a tag sequence (e.g., tEGFR recognized by an endogenous cognate binding molecule (EndoCBM)) and/or any type of affinity tag, such as STREP TAG^{®} ∥ (SEQ ID NO:118), Myc tag (SEQ ID NO:119), V5 tag (SEQ ID NO:120), FLAG^{®} tag (SEQ ID NO:121), His tag, or other peptides or molecules, which are recognized by an ExoCBM (e.g., receptor, protein, antibody). As shown, chimeric molecules can include (A, B) one tag cassette, (C) two tag cassettes, (D) three tag cassettes, or more. In addition, the chimeric molecules may have multiple effector domains (e.g., the molecules of A and C-G have two, while the molecule shown in B has three effector domains), and the tag cassettes may be placed in various different areas of chimeric molecules. In these particular examples, E-G, one tag cassette is depicted between the ligand binding domain and the effector domain (E), at the distal end (e.g., amino-terminus) of the ligand binding domain (F), integrated within the ligand binding domain (G) (e.g., located within the flexible linker between the VH and VL chains of an scFv), and having two different tags - one C-terminal of the binding domain and one N-terminal of the binding domain (H). Chimeric molecules with ligand binding domains (e.g., E-H), may also have two, three or more tag cassettes as shown for A-D. As is evident in these illustrations, a tag cassette may be connected to other chimeric molecule components or another tag cassette via a linker sequence (e.g., a flexible (GlyₓSer)ₙ linker sequence). The linker sequence length may be tailored to be longer or shorter to achieve the best interaction of a tag cassette with its cognate binding molecule (e.g., an ExoCBM or EndoCBM) and/or to achieve the best interaction of a ligand binding domain with a target ligand or antigen, and/or to achieve the best interaction between the modified cell expressing the chimeric molecule and a target cell.

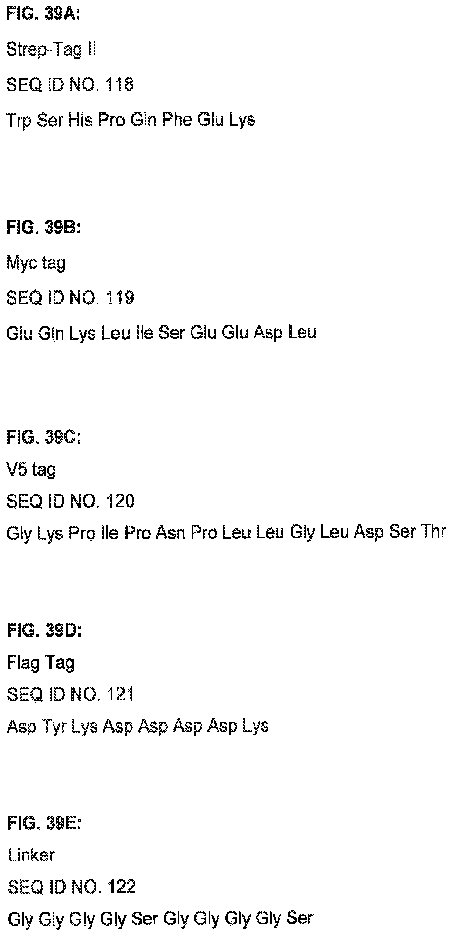

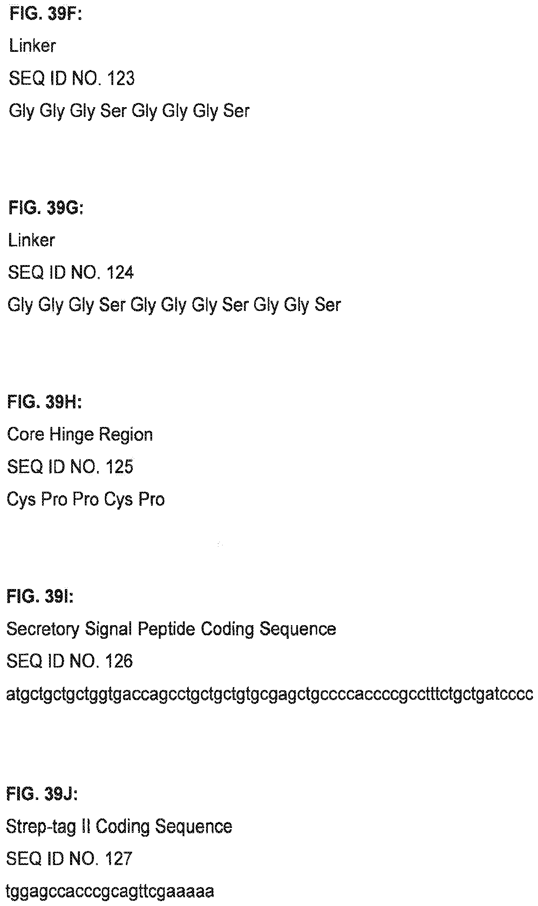

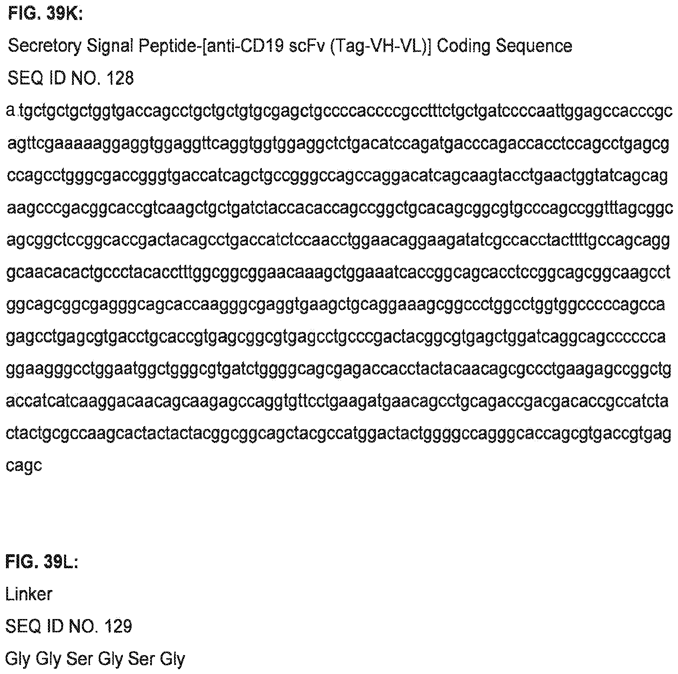

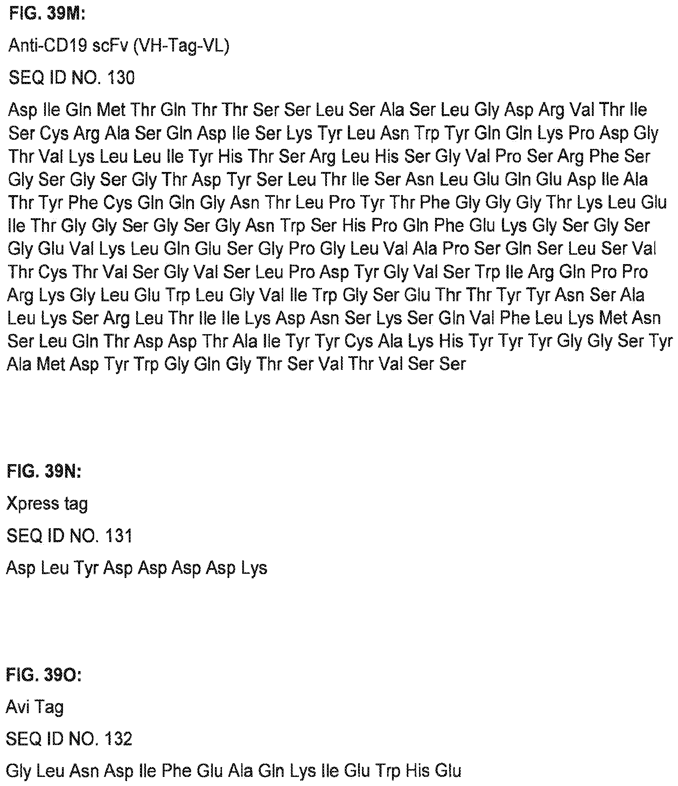

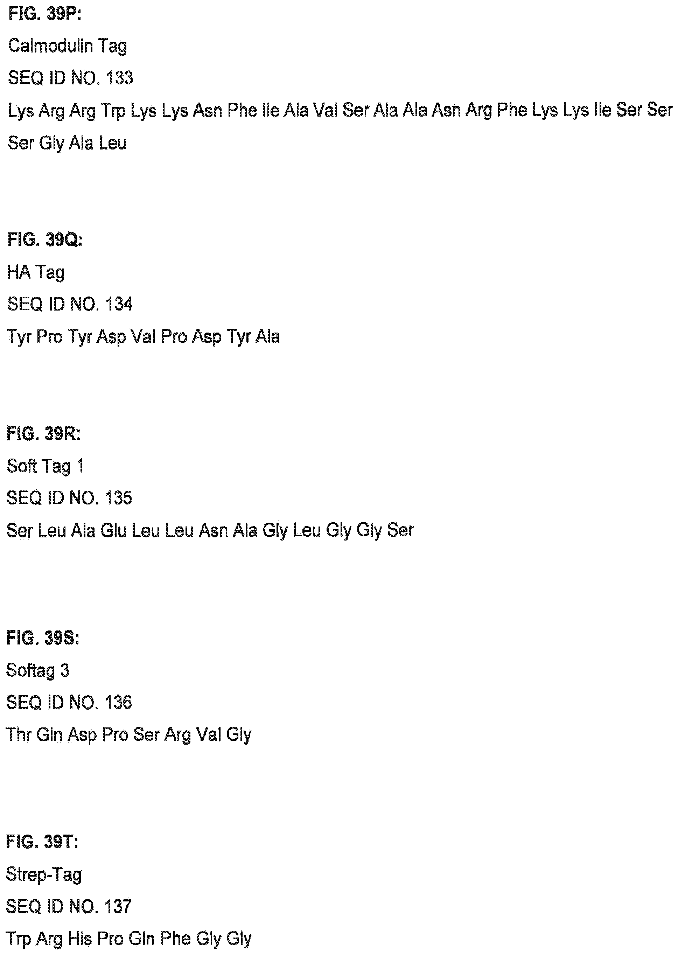

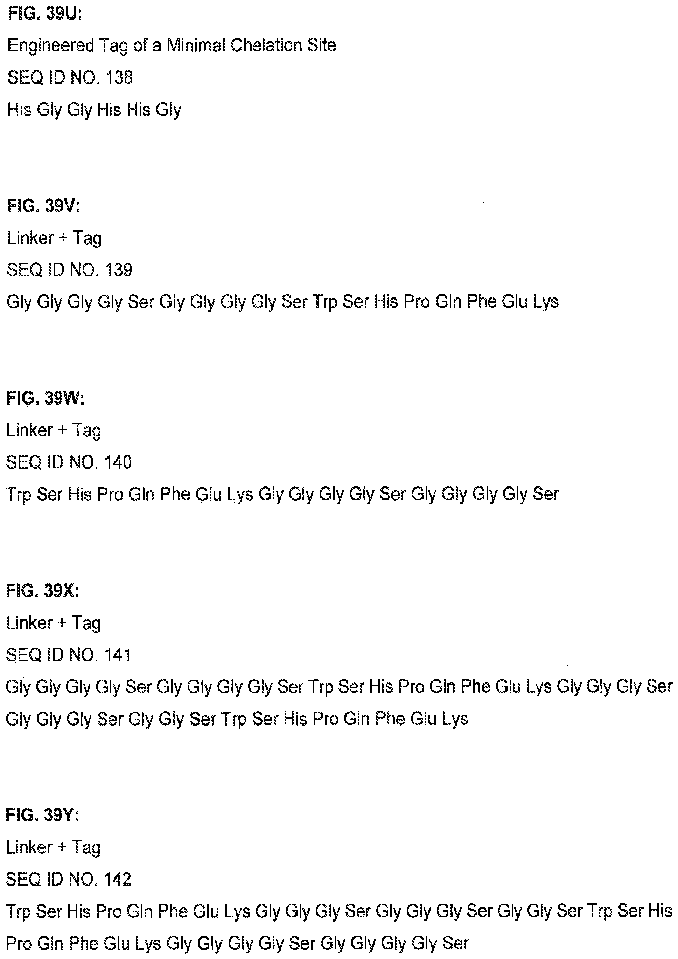

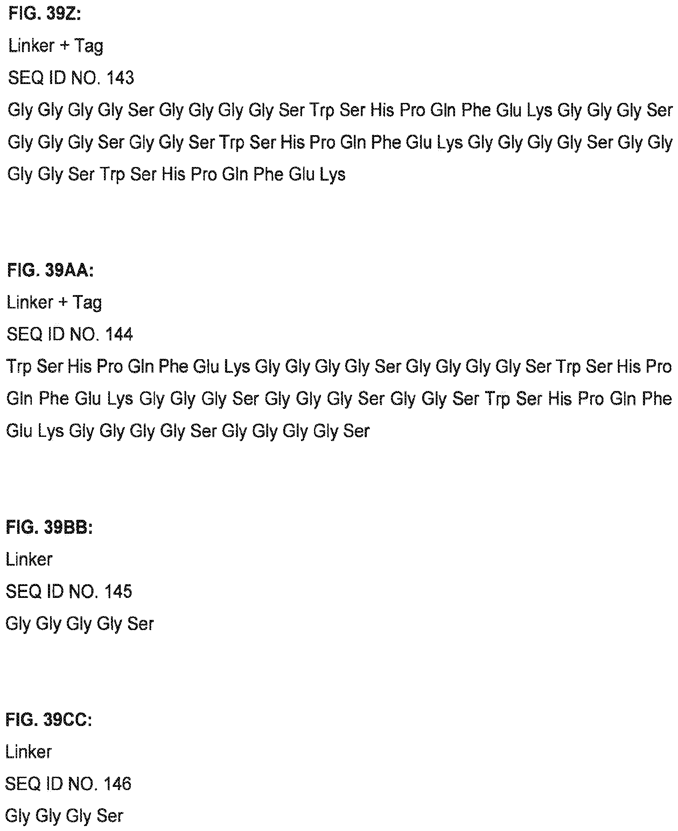

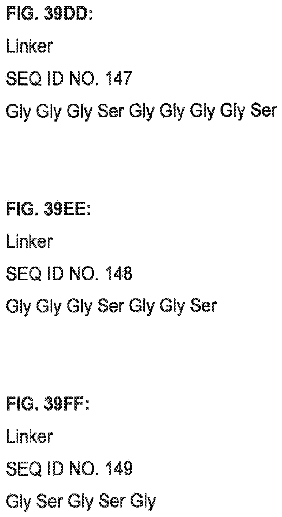

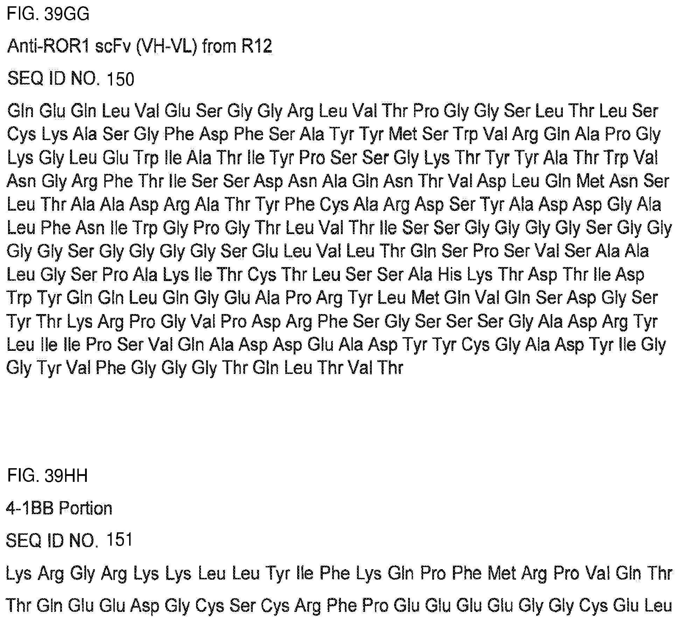

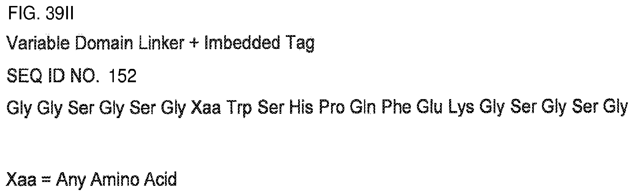

**FIG.** 39. Exemplary Sequences. FIG. 39A: Strep-Tag ∥ (SEQ ID NO: 118); FIG. 39B: Myc tag (SEQ ID NO: 119); FIG. 39C: V5 tag (SEQ ID NO: 120); FIG. 39D: Flag Tag (SEQ ID NO: 121); FIG. 39E: Linker (SEQ ID NO: 122); FIG. 39F: Linker (SEQ ID NO: 123); FIG. 39G: Linker (SEQ ID NO: 124); FIG. 39H: Core Hinge Region (SEQ ID NO: 125); FIG. 39I: Secretory Signal Peptide Coding Sequence (SEQ ID NO: 126); FIG. 39J: Strep-tag ∥ Coding Sequence (SEQ ID NO: 127); FIG. 39K: Secretory Signal Peptide-[anti-CD19 scFv (Tag-VH-VL)] Coding Sequence (SEQ ID NO: 128); FIG. 39L: Linker (SEQ ID NO: 129); FIG. 39M: Anti-CD19 scFv (VH-Tag-VL) (SEQ ID NO: 130); FIG. 39N: Xpress tag (SEQ ID NO: 131); FIG. 39O: Avi Tag (SEQ ID NO: 132); FIG. 39P: Calmodulin Tag (SEQ ID NO: 133); FIG. 39Q: HA Tag (SEQ ID NO: 134); FIG. 39R: Soft Tag 1 (SEQ ID NO: 135); FIG. 39S: Softag 3 (SEQ ID NO: 136); FIG. 39T: Strep-Tag (SEQ ID NO: 137); FIG. 39U: Engineered Tag of a Minimal Chelation Site (SEQ ID NO: 138); FIG. 39V: Linker + Tag (SEQ ID NO: 139); FIG. 39W: Linker + Tag (SEQ ID NO: 140); FIG. 39X: Linker + Tag (SEQ ID NO: 141); FIG. 39Y: Linker + Tag (SEQ ID NO: 142); FIG. 39Z: Linker + Tag (SEQ ID NO: 143); FIG. 39AA: Linker + Tag (SEQ ID NO: 144); FIG. 39BB: Linker (SEQ ID NO: 145); FIG. 39CC: Linker (SEQ ID NO: 146); FIG. 39DD: Linker (SEQ ID NO: 147); FIG. 39EE: Linker (SEQ ID NO: 148); FIG. 39FF: Linker (SEQ ID NO: 149); FIG. 39GG: Anti-ROR1 scFv (VH-VL) from R12 (SEQ ID NO: 150); FIG. 39HH: 4-1BB Portion (SEQ ID NO: 151); and FIG. 39II: Variable Domain Linker + Imbedded Tag (SEQ ID NO: 152).

### DETAILED DESCRIPTION

Significant progress has been made in genetically engineering T cells of the immune system to target and kill unwanted cell types, such as cancer cells. For example, T cells have been genetically engineered to express molecules having an extracellular component that binds particular target antigens and an intracellular component that directs actions of the T cell when the extracellular component has bound the target antigen. As an example, the extracellular component can be designed to bind target antigens preferentially found on cancer cells and, when bound, the intracellular component directs the T cell to destroy the bound cancer cell. Examples of such molecules include genetically engineered T cell receptors (TCR) and chimeric antigen receptors (CAR).

While genetically engineered T cells provide a significant advance in the ability to target and destroy unwanted cell types, they require immunological matching with each particular subject before they can be used in a treatment setting. Once a donor match is found (or T cells are obtained from a subject in need of treatment), the cells must be modified and expanded before they can be used in the subject. This time-intensive and expensive process can cause, in some instances, lethal delays in treatment.

**The** current disclosure provides genetically modified stem cells that can be administered to subjects without the need for immunological matching. Thus, these modified stem cells may be provided as "off-the-shelf' treatments eliminating delays and expenses in treatment associated with donor identification and subsequent cell modification and expansion. The modified stem cells can be administered alone or in combination with various other treatments to obtain numerous treatment objectives. In particular embodiments, the modified stem cells can be differentiated into non-T effector cells before administration.

More particularly, hematopoietic stem/progenitor cells (HSPC) are genetically modified to express molecules having an extracellular component having a tag cassette. Tag cassettes can be used to activate, promote proliferation of, detect, enrich for, isolate, track, deplete and/or eliminate genetically modified cells *in vitro*, *in vivo* and/or *ex vivo.* Such modified cells can be identified and isolated at higher yields as compared to HSPC that do not express a tag cassette. Thus, in their most basic form, modified cells disclosed herein express tag cassettes that remain associated with the expressing cell. "Tag cassette" refers to a unique peptide sequence affixed to, fused to, or that is part of a protein of interest, to which a cognate binding molecule (*e*.*g*., receptor, ligand, antibody, or other binding partner) is capable of specifically binding where the binding property can be used to activate, promote proliferation of, detect, enrich for, isolate, track, deplete and/or eliminate a tagged protein or cells expressing a tagged protein, particularly when a tagged protein is part of a heterogeneous population of proteins or other material, or when cells expressing a tagged protein are part of a heterogeneous population of cells (*e*.*g*., a biological sample like peripheral blood). In particular embodiments, the cognate binding molecule is an exogenous cognate binding molecule (ExoCBM). In certain embodiments, a cell expressing a tagged cassette can be contacted with an ExoCBM to induce a biological response, such as promote cell activation, cell proliferation or cell death.

"Exogenous" refers to any gene, protein, compound, molecule or activity that is not native to a host cell or a subject, or is any gene, protein, compound, molecule or activity native to a host or host cell but has been altered or mutated such that the structure, activity or both is different as between the native and mutated molecules. In certain embodiments, exogenous molecules are not endogenous to a host cell or subject, but instead nucleic acids encoding such molecules may have been added to a host cell by conjugation, transformation, transfection, electroporation, or the like, wherein the added nucleic acid molecule may integrate into a host cell genome or can exist as extra-chromosomal genetic material (e.g., as a plasmid or other self-replicating vector). Exogenous molecules can include heterologous and non-endogenous molecules. "Homologous" or "homolog" refers to a molecule or activity found in or derived from a host cell, species or strain. For example, a heterologous molecule or gene encoding the molecule may be homologous to a native host or host cell molecule or gene that encodes the molecule, respectively, but may have an altered structure, sequence, expression level or combinations thereof. A non-endogenous molecule may be from the same species, a different species or a combination thereof.

The term "endogenous" or "native" refers to a gene, protein, compound, molecule or activity that is normally present in a host or host cell. Exogenous molecules are not endogenous or native.

In particular embodiments, modified HSPC can be differentiated into non-T effector cells before administration.

In additional embodiments, modified cells (e.g., modified HSPC and/or modified non-T effector cells) express (i) a ligand binding domain as part of the extracellular component and (ii) an intracellular component. The ligand binding domain can bind particular cellular markers and the intracellular component can direct actions of the genetically modified cell when the ligand binding domain has bound the cellular marker. As an example, the ligand binding domain can be designed to bind cellular markers preferentially found on cancer cells and, when bound, the intracellular component directs the genetically modified cell to destroy the bound cancer cell. Examples of molecules with ligand binding domains and intracellular components include genetically engineered T cell receptors (TCR), chimeric antigen receptors (CAR), and other molecules disclosed herein. As indicated, modified HSPC can be differentiated into non-T effector cells before administration.

As an exemplary use of a particular embodiment with a tag cassette and a ligand binding domain, cord blood transplant (CBT) is a standard of care for relapsed pediatric acute lymphoblastic leukemia (ALL) when a suitably matched donor cannot be identified. This is particularly important for patients of minority or mixed ethnicity background (and 30% of Caucasians) who are very unlikely to find a suitable donor.

**The** ability of CBT to eradicate ALL and provide a durable remission is due in part to a graft-versus-leukemia (GVL) effect. Still, however, the rate of relapse for ALL post CBT is around 40% (Smith, et a/., 2009, Biol. Blood Marrow Transplant 15(9): p. 1086-93; Tomblyn, et al., 2009 J. Clin. Oncol. 27(22): p. 3634-41) with overall survival related to both relapse and treatment related mortality, including graft-versus-host disease (GVHD). Compositions and formulations disclosed herein can enhance the GVL effect, without increasing rates of GVHD. This strategy is clinically feasible using *ex vivo* expansion of cord blood (CB) HSPC through activation of the endogenous Notch signaling pathway using a Notch ligand, resulting in a greater than 100 fold increase of CD34⁺ cells. Clinically, the expanded HSPC can be infused along with an unmanipulated unit, leading to a transient engraftment of the expanded HSPC, with progeny derived from the expanded unit, while long-term engraftment is ultimately derived from the unmanipulated unit.

Notch ligand expanded CB HSPC are amenable to genetic modification using vectors that express a CD19-specific CAR. By taking advantage of the Notch ligand CB expansion system, GVL can be engineered into CBT by the genetic modification of expanded HSPC to express a CD19 CAR, whereby the engrafted myeloid and lymphoid effector cells recognize and lyse residual leukemia cells.

In the provided fusion proteins expressing tag cassettes, the ability of the tag cassette(s) to be specifically bound by the cognate binding molecule(s) is distinct from or in addition to the ability of the binding domain(s) to specifically bind to the cellular marker(s). Thus, the tag cassette generally is not an antigen-binding molecule, for example, is not an antibody or TCR or an antigen-binding portion thereof.

The claimed invention is now described more generally.

Hematopoietic Stem/Progenitor Cells or HSPC refer to hematopoietic stem cells and/or hematopoietic progenitor cells.

"Hematopoietic stem cells" refer to undifferentiated hematopoietic cells that are capable of self-renewal either *in vivo,* essentially unlimited propagation *in vitro,* and capable of differentiation to other cell types including non-T effector cells.

**A** "hematopoietic progenitor cell" is a cell derived from hematopoietic stem cells or fetal tissue that is capable of further differentiation into mature cells types. In certain embodiments, hematopoietic progenitor cells are CD24^{Io} Lin⁻ CD117⁺ hematopoietic progenitor cells. Hematopoietic progenitor cells include embryonic stem cells.

"Embryonic stem cells" or "ES cells" or "ESCs" refer to undifferentiated embryonic stem cells that have the ability to integrate into and become part of the germ line of a developing embryo. Embryonic stem cells are capable of differentiating into hematopoietic progenitor cells, and any tissue or organ. Embryonic stem cells that are suitable for use herein include cells from the J1 ES cell line, 129J ES cell line, murine stem cell line D3 (American Type Culture Collection), the R1 or E14K cell lines derived from 129/Sv mice, cell lines derived from Balb/c and C57BI/6 mice, and human embryonic stem cells (e.g. from WiCell Research Institute, Wl; or ES cell International, Melbourne, Australia).

Thus, HSPC can self-renew or can differentiate into (i) myeloid progenitor cells which ultimately give rise to monocytes and macrophages, neutrophils, basophils, eosinophils, erythrocytes, megakaryocytes/platelets, or dendritic cells; or (ii) lymphoid progenitor cells which ultimately give rise to T-cells, B-cells, and lymphocyte-like cells called natural killer cells (NK-cells). For a general discussion of hematopoiesis and HSPC differentiation, see Chapter 17, Differentiated Cells and the Maintenance of Tissues, Alberts et al., 1989, Molecular Biology of the Cell, 2nd Ed., Garland Publishing, New York, NY; Chapter 2 of Regenerative Medicine, Department of Health and Human Services, August 5, 2006, and Chapter 5 of Hematopoietic Stem Cells, 2009, Stem Cell Information, Department of Health and Human Services.

HSPC can be positive for a specific marker expressed in increased levels on HSPC relative to other types of hematopoietic cells. For example, such markers include CD34, CD43, CD45RO, CD45RA, CD59, CD90, CD109, CD117, CD133, CD166, HLA DR, or a combination thereof. Also, the HSPC can be negative for an expressed marker relative to other types of hematopoietic cells. For example, such markers include Lin, CD38, or a combination thereof. Preferably, the HSPC are CD34⁺ cells.

Sources of HSPC include umbilical cord blood, placental blood, and peripheral blood (see U.S. Patent Nos. 5,004,681; 7,399,633; and 7,147,626; Craddock, et al., 1997, Blood 90(12):4779-4788; Jin, et al., 2008, Journal of Translational Medicine 6:39; Pelus, 2008, Curr. Opin. Hematol. 15(4):285-292; Papayannopoulou, et al., 1998, Blood 91(7):2231-2239; Tricot, et al., 2008, Haematologica 93(11): 1739-1742; and Weaver et al., 2001, Bone Marrow Transplantation 27(2):S23-S29). Methods regarding collection, anti-coagulation and processing, etc. of blood samples can be found in, for example, Alsever, et al., 1941, N.Y. St. J. Med. 41:126; De Gowin, et al., 1940, J. Am. Med. Ass. 114:850; Smith, et al., 1959, J. Thorac. Cardiovasc. Surg. 38:573; Rous and Turner, 1916, J. Exp. Med. 23:219; and Hum, 1968, Storage of Blood, Academic Press, New York, pp. 26-160. Sources of HSPC also include bone marrow (see Kodo, et al., 1984, J. Clin. Invest. 73:1377-1384), embryonic cells, aortal-gonadal-mesonephros derived cells, lymph, liver, thymus, and spleen from age-appropriate donors. All collected samples of HSPC can be screened for undesirable components and discarded, treated, or used according to accepted current standards at the time.

HSPC initially can be collected and isolated from a sample using any appropriate technique. Appropriate collection and isolation procedures include magnetic separation; fluorescence activated cell sorting (FACS; Williams, et al., 1985, J. Immunol. 135:1004; Lu, et al., 1986, Blood 68(1):126-133); affinity chromatography; cytotoxic agents joined to a monoclonal antibody or used in conjunction with a monoclonal antibody, *e*.*g*., complement and cytotoxins; "panning" with antibody attached to a solid matrix (Broxmeyer, et al., 1984, J. Clin. Invest. 73:939-953); selective agglutination using a lectin such as soybean (Reisner, et al., 1980, Proc. Natl. Acad. Sci. U.S.A. 77:1164); etc.

In particular embodiments, a HSPC sample (for example, a fresh cord blood unit) initially can be processed to select/enrich for CD34+ cells using anti-CD34 antibodies directly or indirectly conjugated to magnetic particles in connection with a magnetic cell separator, for example, the CLINIMACS^{®} Cell Separation System (Miltenyi Biotec, Bergisch Gladbach, Germany). See also, sec. 5.4.1.1 of U.S. Patent No. 7,399,633 which describes enrichment of CD34⁺ HSPC from 1-2% of a normal bone marrow cell population to 50-80% of the population.

Similarly, HSPC expressing CD43, CD45RO, CD45RA, CD59, CD90, CD109, CD117, CD133, CD166, HLA DR, or a combination thereof, can be enriched for using antibodies against these antigens. U.S. Pat. No. 5,877,299 describes additional appropriate hematopoietic antigens that can be used to initially isolate, collect, and enrich HSPC cells from samples.

Following isolation and/or enrichment, HSPC can be expanded in order to increase the number of HSPC. Isolation and/or expansion methods are described in, for example, U.S. Patent Nos. 7,399,633 and 5,004,681; U.S. Patent Publication No. 2010/0183564; International Patent Publication Nos. (WO) WO2006/047569; WO2007/095594; WO 2011/127470; and WO 2011/127472; Vamum-Finney, et al., 1993, Blood 101:1784-1789; Delaney, et al., 2005, Blood 106:2693-2699; Ohishi, et al., 2002, J. Clin. Invest. 110:1165-1174; Delaney, et al., 2010, Nature Med. 16(2): 232-236; and Chapter 2 of Regenerative Medicine, Department of Health and Human Services, August 2006, and the references cited therein. Each of the referenced methods of collection, isolation, and expansion can be used in particular embodiments of the disclosure.

Preferred methods of expanding HSPC include expansion of HSPC with a Notch agonist. For information regarding expansion of HSPC using Notch agonists, *see* sec. 5.1 and 5.3 of U.S. Patent No. 7,399,633; U.S. Patent Nos. 5,780,300; 5,648,464; 5,849,869; and 5,856,441; WO 1992/119734; Schlondorfi and Blobel, 1999, J. Cell Sci. 112:3603-3617; Olkkonen and Stenmark, 1997, Int. Rev. Cytol. 176:1-85; Kopan, et al., 2009, Cell 137:216-233; Rebay, et al., 1991, Cell 67:687-699, and Jarriault, et al., 1998, Mol. Cell. Biol. 18:7423-7431. In particular embodiments, the Notch agonist is immobilized during expansion.

Notch agonists include any compound that binds to or otherwise interacts with Notch proteins or other proteins in the Notch pathway such that Notch pathway activity is promoted. Exemplary Notch agonists are the extracellular binding ligands Delta and Serrate (*e*.*g*., Jagged), RBP J 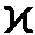 I Suppressor of Hairless, Deltex, Fringe, or fragments thereof which promote Notch pathway activation. Nucleic acid and amino acid sequences of Delta family members and Serrate family members have been isolated from several species and are described in, for example, WO 1993/12141; WO 1996/27610; WO 1997/01571; and Gray, et al., 1999, Am. J. Path. 154:785-794.

In particular embodiments, the Notch agonist is Delta1^{ext-IgG}. In particular embodiments, Delta1^{ext-IgG} is applied to a solid phase at a concentration between 0.2 and 20 µg/ml, between 1.25 and 10 µg/ml, or between 2 and 6 µg/ml.

In particular embodiments, during expansion, HSPC are cultured in the presence of a Notch agonist and an aryl hydrocarbon receptor antagonist. The Notch agonist can be immobilized and the aryl hydrocarbon receptor antagonist can be in a fluid contacting the cells.

Additional culture conditions can include expansion in the presence of one more growth factors, such as: angiopoietin-like proteins (Angptls, *e*.*g*., Angptl2, Angptl3, Angptl7, Angpt15, and Mfap4); erythropoietin; fibroblast growth factor-1 (FGF-1); Flt-3 ligand (Flt-3L); granulocyte colony stimulating factor (G-CSF); granulocyte-macrophage colony stimulating factor (GM-CSF); insulin growth factor-2 (IFG-2); interleukin-3 (IL-3); interleukin-6 (IL-6); interleukin-7 (IL-7); interleukin-11 (IL-11); stem cell factor (SCF; also known as the c-kit ligand or mast cell growth factor); thrombopoietin (TPO); and analogs thereof (wherein the analogs include any structural variants of the growth factors having the biological activity of the naturally occurring growth factor; see, e.g., WO 2007/1145227 and U.S. Patent Publication No. 2010/0183564).

In particular embodiments, the amount or concentration of growth factors suitable for expanding HSPC is the amount or concentration effective to promote proliferation of HSPC, but substantially no differentiation of the HSPC. Cell populations are also preferably expanded until a sufficient number of cells are obtained to provide for at least one infusion into a human subject, typically around 10⁴ cells/kg to 10⁹ cells/kg.

**The** amount or concentration of growth factors suitable for expanding HSPC depends on the activity of the growth factor preparation, and the species correspondence between the growth factors and HSPC, etc. Generally, when the growth factor(s) and HSPC are of the same species, the total amount of growth factor in the culture medium ranges from 1 ng/ml to 5 µg/ml, from 5 ng/ml to 1 µg/ml, or from 5 ng/ml to 250 ng/ml. In additional embodiments, the amount of growth factors can be in the range of 5-1000 or 50-100 ng/ml.

In particular embodiments, the foregoing growth factors are present in the culture condition for expanding HSPC at the following concentrations: 25-300 ng/ml SCF, 25-300 ng/ml Flt-3L, 25-100 ng/ml TPO, 25-100 ng/ml IL-6 and 10 ng/ml IL-3. In more specific embodiments, 50, 100, or 200 ng/ml SCF; 50, 100, or 200 ng/ml of Flt-3L; 50 or 100 ng/ml TPO; 50 or 100 ng/ml IL-6; and 10 ng/ml IL-3 can be used.

In particular embodiments, HSPC can be expanded by exposing the HSPC to an immobilized Notch agonist, and 50 ng/ml or 100 ng/ml SCF; to an immobilized Notch agonist, and 50 ng/ml or 100 ng/ml of each of Flt-3L, IL-6, TPO, and SCF; or an immobilized Notch agonist, and 50 ng/ml or 100 ng/ml of each of Flt-3L, IL-6, TPO, and SCF, and 10 ng/ml of IL-11 or IL-3.

HSPC can be expanded in a tissue culture dish onto which an extracellular matrix protein such as fibronectin (FN), or a fragment thereof (e.g., CH-296 (Dao, et a/., 1998, Blood 92(12):4612-21)) or RETRONECTIN^{®} (a recombinant human fibronectin fragment; (Clontech Laboratories, Inc., Madison, WI) is bound.

In a specific embodiment, methods of expanding HSPC include culturing isolated HSPC *ex vivo* on a solid phase coated with immobilized Delta1^{ext-IgG} and CH-296, and four or more growth factors selected from IL-6, TPO, Flt-3L, CSF, and IL-3; thereby producing an expanded HSPC sample.

In particular embodiments for expanding HSPC, the cells are cultured on a plastic tissue culture dish containing immobilized Delta ligand and fibronectin and 25 ng/ml or 100 ng/ml (or any range in between these values), and preferably 50 ng/ml, of each of SCF and TPO. In particular embodiments for expanding HSPC, the cells are cultured on a plastic tissue culture dish containing immobilized Delta ligand and fibronectin in the presence of and 25 ng/ml or 100 ng/ml (or any range in between these values), and preferably 50 ng/ml of each of SCF and Flt-3L. In particular embodiments for expanding HSPC, the cells are cultured on a plastic tissue culture dish containing immobilized Delta ligand and fibronectin and 25 ng/ml or 100 ng/ml (or any range in between these values), and preferably 50 ng/ml of each of SCF, Flt-3L and TPO. In particular embodiments for expanding HSPC, the cells are cultured on a plastic tissue culture dish containing immobilized Delta ligand and fibronectin and 25 ng/ml or 100 ng/ml (or any range in between these values), and preferably 50 ng/ml, of each of SCF, Flt-3L, TPO, and IL-6. In particular embodiments, the HSPC are cultured further in the presence of 5 to 15 ng/ml, and preferably 10 ng/ml of IL-3. In particular embodiments, the HSPC are cultured further in the presence of 5 to 15 ng/ml, and preferably 10 ng/ml, GM-CSF. In particular embodiments, the one or more growth factors used is not GM-SCF or IL-7. In particular alternative embodiments, fibronectin is excluded from the tissue culture dishes or is replaced by another extracellular matrix protein. Further methods and details regarding expansion of HSPC are found in WO 2013/086436.

In particular embodiments, the percentage of CD34⁺ cells in the expanded HSPC sample, obtained using the described methods is higher than the percentage of CD34⁺ cells in the isolated HSPC prior to expansion. For additional information regarding appropriate culturing conditions, see U.S. Patent No. 7,399,633; U.S. Patent Publication No. 2010/0183564; and Freshney Culture of Animal Cells, Wiley-Liss, Inc., New York, NY (1994)).

Modified HSPC. In particular embodiments, HSPC are modified to express a tag cassette. The tag cassette can bind an EndoCBM or an ExoCBM. HSPC can also be modified to express (i) an extracellular component including a tag cassette and a ligand binding domain; and (ii) an intracellular component. The extracellular and intracellular components can be linked directly or through, e.g., and in various embodiments, spacer region(s), linker sequence(s), junction amino acids and/or hydrophobic portions. As will be understood by one of ordinary skill in the art, classification as a spacer region(s), linker sequence(s), junction amino acid and/or hydrophobic portion is not mutually exclusive, and there can be overlap between these functions.

Extracellular Components. Extracellular components include at least one tag cassette, and optionally, a ligand binding domain (hereafter binding domain), among other potential components, as described herein.

Tag Cassettes. A tag cassette included within an expressed chimeric molecule (e.g., a single chain fusion protein) can be an extracellular component or part of an extracellular component that can specifically bind to a cognate binding molecule with high affinity or avidity, wherein, in particular embodiments, the cognate binding molecule is exogenous to a host or a cell expressing the chimeric molecule.

**Tag** cassettes that bind EndoCBMs include, for example, a truncated EGFR as shown in FIG. 2. An exemplary gene sequence encoding the truncated EGFR is shown in FIG. 1. (SEQ ID NO:9). Tag cassettes that bind ExoCBMs include, for example, Strep tag (which refers the original STREP^{®} tag, STREP^{®} tag II, or any variant thereof; see, e.g., U.S. Patent No. 7,981,632), His tag, Flag tag (SEQ ID NO:121), Xpress tag (SEQ ID NO:131), Avi tag (SEQ ID NO:132), Calmodulin tag (SEQ ID NO:133), Polyglutamate tag, HA tag (SEQ ID NO:134), Myc tag (SEQ ID NO:119), Nus tag, S tag, SBP tag, Softag 1 (SEQ ID NO:135), Softag 3 (SEQ ID NO:136), V5 tag (SEQ ID NO:120), CREB-binding protein (CBP), glutathione S-transferase (GST), maltose binding protein (MBP), green fluorescent protein (GFP), Thioredoxin tag, or any combination thereof. In certain embodiments, a tag cassette is a Strep tag having an amino acid sequence of Trp-Ser-His-Pro-Gln-Phe-Glu-Lys (SEQ ID NO: 118) or Trp-Arg-His-Pro-Gln-Phe-Gly-Gly (SEQ ID NO:137). In other embodiments, a tag cassette may be a genetically engineered affinity site, such as a minimal chelation site (*e*.*g*., HGGHHG, SEQ ID NO:138).

**Tag** cassettes may be present in multiple copies in fusion proteins. For example, a fusion protein can have one, two, three, four or five tag cassettes (*e*.*g*., Strep tag). In certain embodiments, an extracellular component of a chimeric molecule includes one tag cassette, two tag cassettes, three tag cassettes, four tag cassettes, or five tag cassettes. Each of the plurality of tag cassettes may be the same or different. Exemplary embodiments include a chimeric molecule having two Strep tag cassettes, or a His tag and a Strep tag cassette, or a HA tag and a Strep tag cassette, or a Myc tag and a Strep tag cassette. Alternatively, a chimeric molecule will have multiple tag cassettes of the same type or same amino acid sequence, such as two, three, four or five Strep tag cassettes (e.g., Strep tag II).

In some embodiments, a first tag cassette can provide a stimulation signal and a distinct second tag cassette might be used to associate with a detection reagent or associate with an antibody-toxin conjugate or with an antibody-imaging agent conjugate.

**A** chimeric molecule including one or more tag cassettes will be capable of associating with a cognate binding molecule, wherein the cognate binding molecule is exogenous to the host or cell expressing a fusion protein including a tag cassette as described herein. In certain embodiments, a tag cassette present in a chimeric molecule is a Strep tag, which has streptavidin, streptactin or both as a cognate binding molecule, or is recognized by antibodies specific for a Strep tag. In certain embodiments, the cognate binding molecule (e.g., receptor, protein, antibody) may be soluble, part of a matrix composition, or conjugated to a solid surface (e.g., plate, bead). Exemplary solid surfaces include beads and particles (e.g., micro and nano), such as magnetic beads and particles.

In particular embodiments chimeric molecule expressing modified cells can be identified by flow cytometry using a tag cassette specific binding agent. In particular examples, purified chimeric molecule expressing modified cells are using detected anti-strep tag II (STII) and/or with STREP-TACTIN^{®} APC (IBA Institut fur Bioanalytik, Germany).

In particular embodiments chimeric molecule expressing modified cells can be sorted by flow cytometry from low purity (e.g., 1% - 30%) to high purity (e.g., 75% - 99%) with a tag-specific binding agent linked to a fluorochrome. In particular embodiments, the tag can be StrepTag II and the tag-specific binding agent can be anti STII mAb linked to a fluorochrome.

In particular embodiments chimeric molecule expressing modified cells (e.g., with three Strep-tag tag cassettes) can be directly enriched by using STREP-TACTIN^{®} beads of various sizes. Thus, in certain embodiments, cells expressing a chimeric molecule can be identified, sorted, enriched or isolated by binding to antibodies having specificity to a tag cassette (e.g., anti-tag antibodies), or by other proteins that specifically bind a tag cassette (e.g., Streptactin binding to the Strep tag), which are conjugated to beads, a cell culture plate, agarose, or any other solid surface matrix. In certain embodiments, such cells are sorted, enriched or isolated by using an affinity column.

An advantage of the instant disclosure is that chimeric molecule expressing cells administered to a subject can be depleted using the ExoCBM to a tag cassette. In certain embodiments, the present disclosure provides a method for depleting a modified cell expressing a chimeric molecule by using an antibody specific for the tag cassette, using an ExoCBM specific for the tag cassette, or by using a second modified cell expressing a CAR and having specificity for the tag cassette. Elimination of modified cells may be accomplished using depletion agents specific for a tag cassette. For example, if a Strep tag is used, then an anti-Strep tag antibody, anti-Strep tag scFv, or Streptactin each fused to or conjugated to a cell-toxic reagent (such as a toxin, radiometal) may be used, or an anti-Strep tag /anti-CD3 bispecific scFv, or an anti-Strep tag CAR T cell may be used.

In certain further embodiments, modified cells expressing chimeric molecules as disclosed herein are activated *in vivo,* such as at the site of a tumor. For example, a composition (*e*.*g*., alginate, basement membrane matrix (MATRIGEL^{®}), biopolymer, or other matrix) or a carrier (*e*.*g*.*,* microbead, nanoparticle, or other solid surface) including a tag cassette cognate binding molecule can be used to locally activate at the site of a tumor a modified cell expressing a chimeric molecule as disclosed herein.

In certain embodiments, modified cells expressing a chimeric molecule may be detected or tracked *in vivo* by using antibodies that bind with specificity to a tag cassette (e.g., anti-Tag antibodies), or by other ExoCBMs that specifically bind the tag cassette (e.g., Streptactin binding to Strep tag), which binding partners for the tag cassette are conjugated to a fluorescent dye, radio-tracer, iron-oxide nanoparticle or other imaging agent known in the art for detection by X-ray, CT-scan, MRI-scan, PET-scan, ultrasound, flow-cytometry, near infrared imaging systems, or other imaging modalities (see, e.g., Yu, et al., Theranostics 2:3, 2012).

In further embodiments, cells expressing chimeric molecules of the instant disclosure may be used in diagnostic methods or imaging methods, including methods used in relation to the indications or conditions identified herein.

Thus, modified cells expressing tag cassettes can be, *e.g.,* more readily identified, isolated, sorted, induced to proliferate, tracked, and/or eliminated as compared to a modified cell without a tag cassette. That is, a tag cassette can essentially function as a handle or beacon to allow for, *e.g.,* the identification, enrichment, isolation, promotion of proliferation, activation, tracking, or elimination of cells expressing a chimeric molecule *in vitro, in vivo* and/or *ex vivo.*

In certain embodiments, a tag cassette includes from five to 500 amino acids, or from six to 100 amino acids, or from seven to 50 amino acids, or from eight to 20 amino acids. In some embodiments, a tag cassette has seven to ten amino acids. In particular embodiments, a tag cassette is non-immunogenic or minimally immunogenic. In particular embodiments, a tag cassette is immunogenic and provides adjuvant properties.

ExoCBMs that specifically bind tag cassette sequences disclosed herein are commercially available. As non-limiting examples, Strep tag antibodies are commercially available from suppliers including Abcam, Iba, and Qiagen. His tag antibodies are commercially available from suppliers including Life Technologies, Pierce Antibodies, and GenScript.Flag tag antibodies are commercially available from suppliers including Pierce Antibodies, GenScript, and Sigma-Aldrich. Xpress tag antibodies are commercially available from suppliers including Pierce Antibodies, Life Technologies and GenScript. Avi tag antibodies are commercially available from suppliers including Pierce Antibodies, IsBio, and Genecopoeia. Calmodulin tag antibodies are commercially available from suppliers including Santa Cruz Biotechnology, Abcam, and Pierce Antibodies. HA tag antibodies are commercially available from suppliers including Pierce Antibodies, Cell Signal and Abcam. Myc tag antibodies are commercially available from suppliers including Santa Cruz Biotechnology, Abcam, and Cell Signal.

When utilized, an extracellular binding domain is designed to target the modified cell to a particularly unwanted cell type by binding a cellular marker that is preferentially found on the unwanted cell type.

Cellular Markers. In particular embodiments, cellular markers are preferentially expressed by unwanted cells, such as unwanted cancer cells. "Preferentially expressed" means that a cellular marker is found at higher levels on an unwanted cell type as compared to other non-targeted cells. The difference in expression level is significant enough that, within sound medical judgment, administration of a cell that will target and kill the unwanted cell based on the presence of the marker outweighs the risk of collateral killing of other non-targeted cells that may also express the marker to a lesser degree. In some instances, a cellular marker is only expressed by the unwanted cell type. In other instances, the cellular marker is expressed on the unwanted cell type at least 25%, 35%, 45%, 55%, 65%, 75%, 85%, 95%, 96%, 97%, 98%, 99%, or 100% more than on non-targeted cells. Exemplary unwanted cancer cells include cancer cells from adrenal cancers, bladder cancers, blood cancers, bone cancers, brain cancers, breast cancers, carcinoma, cervical cancers, colon cancers, colorectal cancers, corpus uterine cancers, ear, nose and throat (ENT) cancers, endometrial cancers, esophageal cancers, gastrointestinal cancers, head and neck cancers, Hodgkin's disease, intestinal cancers, kidney cancers, larynx cancers, leukemias, liver cancers, lymph node cancers, lymphomas, lung cancers, melanomas, mesothelioma, myelomas, nasopharynx cancers, neuroblastomas, non-Hodgkin's lymphoma, oral cancers, ovarian cancers, pancreatic cancers, penile cancers, pharynx cancers, prostate cancers, rectal cancers, sarcoma, seminomas, skin cancers, stomach cancers, teratomas, testicular cancers, thyroid cancers, uterine cancers, vaginal cancers, vascular tumors, and metastases thereof.

The particular following cancers can be targeted by including within an extracellular component a binding domain that binds the associated cellular marker(s):

| Targeted Cancer | Cellular Marker(s) |
|---|---|
| Leukemia/Lymphoma | CD19, CD20, CD22, ROR1, CD33, WT-1 |
| Multiple Myeloma | B-cell maturation antiqen (BCMA) |
| Prostate Cancer | PSMA, WT1, Prostate Stem Cell antiqen (PSCA), SV40 T |
| Breast Cancer | HER2, ERBB2, ROR1 |
| Stem Cell Cancer | CD133 |
| Ovarian Cancer | L1-CAM, extracellular domain of MUC16 (MUC-CD), folate binding protein (folate receptor), Lewis Y, ROR1, mesothelin, WT-1 |
| Mesothelioma | mesothelin |
| Renal Cell Carcinoma | carboxy-anhydrase-IX (CAIX); |
| Melanoma | GD2 |
| Pancreatic Cancer | mesothelin, CEA, CD24, ROR1 |
| Lung Cancer | ROR1 |

Without limiting the foregoing, cellular markers also include A33; BAGE; Bcl-2; β-catenin; B7H4; BTLA; CA125; CA19-9; CD3, CD5; CD19; CD20; CD21; CD22; CD25; CD28; CD30; CD33; CD37; CD40; CD52; CD44v6; CD45; CD56; CD79b; CD80; CD81; CD86; CD123; CD134; CD137; CD151; CD171; CD276; CEA; CEACAM6; c-Met; CS-1; CTLA-4; cyclin B1; DAGE; EBNA; EGFR; EGFRvIII, ephrinB2; ErbB2; ErbB3; ErbB4; EphA2; estrogen receptor; FAP; ferritin; α-fetoprotein (AFP); FLT1; FLT4; folate-binding protein; Frizzled; GAGE; G250; GD-2; GHRHR; GHR; GITR; GM2; gp75; gp100 (Pmel 17); gp130; HLA; HER-2/neu; HPV E6; HPV E7; hTERT; HVEM; IGF1R; IL6R; KDR; Ki-67; Lewis A; Lewis Y; LIFRβ; LRP; LRP5; LTβR; MAGE; MART; mesothelin; MUC; MUC1; MUM-1-B; myc; NYESO-1; O-acetyl GD-2; O-acetyl GD3; OSMRβ; p53; PD1; PD-L1; PD-L2; PRAME; progesterone receptor; PSA; PSMA; PTCH1; RANK; ras; Robo1; RORI; survivin; TCRα; TCRβ; tenascin; TGFBR1; TGFBR2; TLR7; TLR9; TNFR1; TNFR2; TNFRSF4; TWEAK-R; TSTA tyrosinase; VEGF; and WT1.

Particular cancer cell cellular markers include:

| Cancer Antiqen | Sequence | SEQ ID NO: |
|---|---|---|
| PSMA | MWNLLHETDSAVATARRPRWLCAGALVLAGGFFLLGFLFGWFI KSSNEATNITPKHNMKAFLDELKAENIKKFLYNFTQIPHLAGTEQ NFQLAKQIQSQWKEFGLDSVELAHYDVLLSYPNKTHPNYISIINE DGNEIFNTSLFEPPPPGYENVSDIVPPFSAFSPQGMPEGDLVY VNYARTEDFFKLERDMKINCSGKIVIARYGKVFRGNKVKNAQLA GAKGVILYSDPADYFAPGVKSYPDGWNLPGGGVQRGNILNLN GAGDPLTPGYPANEYAYRRGIAEAVGLPSIPVHPIGYYDAQKLL EKMGGSAPPDSSWRGSLKVPYNVGPGFTGNFSTQKVKMHIHS TNEVTRIYNVIGTLRGAVEPDRYVILGGHRDSWVFGGIDPQSGA AVVHEIVRSFGTLKKEGWRPRRTILFASWDAEEFGLLGSTEWA EENSRLLQERGVAYINADSSIEGNYTLRVDCTPLMYSLVHNLTK ELKSPDEGFEGKSLYESWTKKSPSPEFSGMPRISKLGSGNDFE | 69 |
| | VFFQRLGIASGRARYTKNWETNKFSGYPLYHSVYETYELVEKF YDPMFKYHLTVAQVRGGMVFELANSIVLPFDCRDYAVVLRKYA DKIYSISMKHPQEMKTYSVSFDSLFSAVKNFTEIASKFSERLQD FDKSNPIVLRMMNDQLMFLERAFIDPLGLPDRPFYRHVIYAPSS HNKYAGESFPGIYDALFDIESKVDPSKAWGEVKRQIYVAAFTVQ AAAETLSEVA | |
| PSCA | MKAVLLALLMAGLALQPGTALLCYSCKAQVSNEDCLQVENCTQ LGEQCWTARIRAVGLLTVISKGCSLNCVDDSQDYYVGKKNITC CDTDLCNASGAHALQPAAAILALLPALGLLLWGPGQL | 72 |
| Mesothelin | | 63 |
| CD19 | | 7 |
| CD20 | MTTPRNSVNGTFPAEPMKGPIAMQSGPKPLFRRMSSLVGPTQ SFFMRESKTLGAVQIMNGLFHIALGGLLMIPAGIYAPICVTVWYP LWGGIMYIISGSLLAATEKNSRKCLVKGKMIMNSLSLFAAISGMI LSIMDILNIKISHFLKMESLNFIRAHTPYINIYNCEPANPSEKNSPS TQYCYSIQSLFLGILSVMLIFAFFQELVIAGIVENEWKRTCSRPK SNIVLLSAEEKKEQTIEIKEEVVGLTETSSQPKNEEDIEIIPIQEEE EEETETNFPEPPQDQESSPIENDSSP | 11 |
| ROR1 | MHRPRRRGTRPPLLALLAALLLAARGAAAQETELSVSAELVPTS SWNISSELNKDSYLTLDEPMNNITTSLGQTAELHCKVSGNPPPT | 84 |
| | IRWFKNDAPVVQEPRRLSFRSTIYGSRLRIRNLDTTDTGYFQCV ATNGKEVVSSTGVLFVKFGPPPTASPGYSDEYEEDGFCQPYR GIACARFIGNRTVYMESLHMQGEIENQITAAFTMIGTSSHLSDK CSQFAIPSLCHYAFPYCDETSSVPKPRDLCRDECEILENVLCQT EYIFARSNPMILMRLKLPNCEDLPQPESPEAANCIRIGIPMADPI NKNHKCYNSTGVDYRGTVSVTKSGRQCQPWNSQYPHTHTFT ALRFPELNGGHSYCRNPGNQKEAPWCFTLDENFKSDLCDIPA CDSKDSKEKNKMEILYILVPSVAIPLAIALLFFFICVCRNNQKSSS APVQRQPKHVRGQNVEMSMLNAYKPKSKAKELPLSAVRFMEE LGECAFGKIYKGHLYLPGMDHAQLVAIKTLKDYNNPQQWTEFQ QEASLMAELHHPNIVCLLGAVTQEQPVCMLFEYINQGDLHEFLI MRSPHSDVGCSSDEDGTVKSSLDHGDFLHIAIQIAAGMEYLSS HFFVHKDLAARNILIGEQLHVKISDLGLSREIYSADYYRVQSKSL LPIRWMPPEAIMYGKFSSDSDIWSFGVVLWEIFSFGLQPYYGFS NQEVIEMVRKRQLLPCSEDCPPRMYSLMTECWNEIPSRRPRF KDIHVRLRSWEGLSSHTSSTTPSGGNATTQTTSLSASPVSNLS NPRYPNYMFPSQGITPQGQIAGFIGPPIPQNQRFIPINGYPIPPG YAAFPAAHYQPTGPPRVIQHCPPPKSRSPSSASGSTSTGHVTS LPSSGSNQEANIPLLPHMSIPNHPGGMGITVFGNKSQKPYKIDS KQASLLGDANIHGHTESMISAEL | |
| WT1 | MGHHHHHHHHHHSSGHIEGRHMRRVPGVAPTLVRSASETSEK RPFMCAYPGCNKRYFKLSHLQMHSRKHTGEKPYQCDFKDCE RRFFRSDQLKRHQRRHTGVKPFQCKTCQRKFSRSDHLKTHTR THTGEKPFSCRWPSCQKKFARSDELVRHHNMHQRNMTKLQL AL | 97 |

Unwanted cells and cellular markers are not restricted to cancer cells and cancer cellular markers but can also include for example, virally-infected cells, such as those expressing hepatitis B surface antigen.

Binding Domains. Binding domains include any substance that binds to a cellular marker to form a complex. Examples of binding domains include cellular marker ligands, receptor ligands, antibodies, peptides, peptide aptamers, receptors (e.g., T cell receptors), or combinations thereof.

In particular embodiments, a "binding domain" (refers to a molecule, such as a peptide, oligopeptide, polypeptide, or protein that possesses the ability to specifically and non-covalently associate, unite, or combine with a cellular marker (e.g., CD19, CD20, CD22, ROR1, mesothelin, PD-L1, PD-L2, PSMA). A binding domain includes any naturally occurring, synthetic, semi-synthetic, or recombinantly produced binding partner for a cellular marker. In some embodiments, the binding domain is an antigen-binding domain, such as an antibody or T cell receptor (TCR) or functional binding domain or antigen-binding fragment thereof. Exemplary binding domains include single chain antibody variable regions (*e*.*g*., domain antibodies, sFv, scFv, Fab), receptor ectodomains (*e*.*g*., TNF-α), ligands (*e*.*g*., cytokines, chemokines), antigen-binding regions of T cell receptors (TCRs), such as single chain TCRs (scTCRs), or synthetic polypeptides selected for the specific ability to bind to a biological molecule.

As stated, antibodies are one example of binding domains and include whole antibodies or binding fragments of an antibody, *e*.*g*., Fv, Fab, Fab', F(ab')₂, Fc, and single chain (sc) forms and fragments thereof that bind specifically to a cellular marker. Additional examples include scFv-based grababodies and soluble VH domain antibodies. These antibodies form binding regions using only heavy chain variable regions. See, for example, Jespers, et a/., 2004, Nat. Biotechnol. 22:1161; Cortez-Retamozo, et a/., 2004, Cancer Res. 64:2853; Baral, et a/., 2006, Nature Med. 12:580; and Barthelemy, et al., 2008, J. Biol. Chem. 283:3639).

Antibodies or antigen binding fragments can include all or a portion of polyclonal antibodies, monoclonal antibodies, human antibodies, humanized antibodies, synthetic antibodies, chimeric antibodies, bispecific antibodies, mini bodies, and linear antibodies.

Antibodies from human origin or humanized antibodies have lowered or no immunogenicity in humans and have a lower number of non-immunogenic epitopes compared to non-human antibodies. Antibodies and their fragments will generally be selected to have a reduced level or no antigenicity in human subjects.

Antibodies that specifically bind a particular cellular marker can be prepared using methods of obtaining monoclonal antibodies, methods of phage display, methods to generate human or humanized antibodies, or methods using a transgenic animal or plant engineered to produce antibodies as is known to those of ordinary skill in the art (*see*, for example, U.S. Patent Nos. 6,291,161 and 6,291,158). Phage display libraries of partially or fully synthetic antibodies are available and can be screened for an antibody or fragment thereof that can bind to a cellular marker. For example, binding domains may be identified by screening a Fab phage library for Fab fragments that specifically bind to a cellular marker of interest (*see* Hoet, et al., 2005, Nat. Biotechnol. 23:344). Phage display libraries of human antibodies are also available. Additionally, traditional strategies for hybridoma development using a cellular marker of interest as an immunogen in convenient systems (*e*.*g*., mice, HUMAB MOUSE^{®} (GenPharm Int'l. Inc., Mountain View, CA), TC MOUSE^{®} (Kirin Pharma Co. Ltd., Tokyo, JP), KM-MOUSE^{®} (Medarex, Inc., Princeton, NJ), llamas, chicken, rats, hamsters, rabbits, etc.) can be used to develop binding domains. In particular embodiments, antibodies specifically bind to a cellular marker preferentially expressed by a particular unwanted cell type and do not cross react with nonspecific components or unrelated targets. Once identified, the amino acid sequence of the antibody and gene sequence encoding the antibody can be isolated and/or determined.

An alternative source of binding domains includes sequences that encode random peptide libraries or sequences that encode an engineered diversity of amino acids in loop regions of alternative non-antibody scaffolds, such as scTCR (*see*, *e*.*g*., Lake, et al., 1999, Int. Immunol.11:745; Maynard, et a/., 2005, J. Immunol. Methods 306:51; U.S. Patent No. 8,361,794), fibrinogen domains (*see*, *e.g.,* Weisel, et al., 1985, Science 230:1388), Kunitz domains (*see*, *e.g.,* U.S. Patent No. 6,423,498), designed ankyrin repeat proteins (DARPins; Binz, et a/., 2003, J. Mol. Biol. 332:489 and Binz, et al., 2004, Nat. Biotechnol. 22:575), fibronectin binding domains (adnectins or monobodies; Richards, et a/., 2003, J. Mol. Biol. 326:1475; Parker, et al., 2005, Protein Eng. Des. Selec. 18:435 and Hackel, et al., 2008, J. Mol. Biol. 381:1238-1252), cysteine-knot miniproteins (Vita, et al., 1995, Proc. Nat'l. Acad. Sci. (USA) 92:6404-6408; Martin, et al., 2002, Nat. Biotechnol. 21:71 and Huang, et a/., 2005, Structure 13:755), tetratricopeptide repeat domains (Main, et al., 2003, Structure 11:497 and Cortajarena, et al., 2008, ACS Chem. Biol. 3:161), leucine-rich repeat domains (Stumpp, et al., 2003, J. Mol. Biol. 332:471), lipocalin domains (*see*, *e.g.,* WO 2006/095164; Beste, et a/., 1999, Proc. Nat'l. Acad. Sci. (USA) 96:1898; and Schönfeld, et al., 2009, Proc. Nat'l. Acad. Sci. (USA) 106:8198), V-like domains (*see*, *e.g.,* U.S. Patent Application Publication No. 2007/0065431), C-type lectin domains (Zelensky and Gready, 2005, FEBS J. 272:6179; Beavil, et al., 1992, Proc. Nat'l. Acad. Sci. (USA) 89:753; and Sato, et a/., 2003, Proc. Nat'l. Acad. Sci. (USA) 100:7779), mAb2 or Fcab^{™} (*see*, *e.g.,* WO 2007/098934 and WO 2006/072620), armadillo repeat proteins (*see*, *e.g.,* Madhurantakam, et al., 2012, Protein Sci. 21: 1015; WO 2009/040338), affilin (Ebersbach, et al., 2007, J. Mol. Biol. 372:172), affibody, avimers, knottins, fynomers, atrimers, cytotoxic T-lymphocyte associated protein-4 (Weidle, et al., 2013, Cancer Gen. Proteo. 10:155), or the like (Nord, et al., 1995, Protein Eng. 8:601; Nord, et al., 1997, Nat. Biotechnol. 15:772; Nord, et al., 2001, Euro. J. Biochem. 268:4269; Binz, et a/., 2005, Nat. Biotechnol. 23:1257; Boersma and Pluckthun, 2011, Curr. Opin. Biotechnol. 22:849).

In particular embodiments, a binding domain is a single chain T cell receptor (scTCR) including Vα/β and Cα/β chains (*e*.*g*., Vα-Cα, Vβ-Cβ, Vα-Vβ) or including a Vα-Cα, Vβ-Cβ, Vα-Vβ pair specific for a cellular marker of interest (e.g., peptide-MHC complex).

Peptide aptamers include a peptide loop (which is specific for a cellular marker) attached at both ends to a protein scaffold. This double structural constraint increases the binding affinity of peptide aptamers to levels comparable to antibodies. The variable loop length is typically 8 to 20 amino acids and the scaffold can be any protein that is stable, soluble, small, and non-toxic. Peptide aptamer selection can be made using different systems, such as the yeast two-hybrid system (*e*.*g*., Gal4 yeast-two-hybrid system), or the LexA interaction trap system.

In particular embodiments, the binding domain can be an antibody that binds the cellular marker CD19. In particular embodiments, a binding domain is a single chain Fv fragment (scFv) that includes VH and VL regions specific for CD19. In particular embodiments, the VH and VL regions are human. Exemplary VH and VL regions include the segments of the anti-CD19 specific monoclonal antibody FMC63. In particular embodiments, the scFV is human or humanized and includes a variable light chain including a CDRL1 sequence of RASQDISKYLN (SEQ ID NO: 108), a CDRL2 sequence of SRLHSGV (SEQ ID NO: 111), and a CDRL3 sequence of GNTLPYTFG (SEQ ID NO: 104). In other embodiments, the scFV is a human or humanized ScFv including a variable heavy chain including a CDRH1 sequence of DYGVS (SEQ ID NO: 103), a CDRH2 sequence of VTWGSETTYYNSALKS (SEQ ID NO: 114), and a CDRH3 sequence of YAMDYWG (SEQ ID NO: 115).

A gene sequence encoding a binding domain is shown in FIG. 1 as the scFv from an antibody that specifically binds CD19, such as FMC63. A gene sequence encoding a flexible linker including the amino acids GSTSGSGKPGSGEGSTKG (SEQ ID NO:30) separates the VH and VL chains in the scFV. The amino acid sequence of the scFv including the linker is shown in FIG. 2 (SEQ ID NO:34). Other CD19-targeting antibodies such as SJ25C1 (Bejcek, *et al.,* 2005, *Cancer Res.*, 1;55(11):2346-51, PMID 7538901) and HD37 (Pezutto, *et al., J. Immun.* 1987, 1;138(9):2793-9, PMID 2437199) are known. SEQ ID NO: 10 provides the anti-CD19 scFv (VH-VL) DNA sequence and SEQ ID NO: 9 provides the anti-CD19 scFv (VH-VL) amino acid sequence.

In particular embodiments, the binding domain binds the cellular marker ROR1. In particular embodiments, the scFV is a human or humanized scFv including a variable light chain including a CDRL1 sequence of ASGFDFSAYYM (SEQ ID NO: 101), a CDRL2 sequence of TIYPSSG (SEQ ID NO: 112), and a CDRL3 sequence of ADRATYFCA (SEQ ID NO: 100). In particular embodiments, the scFV is a human or humanized scFv including a variable heavy chain including a CDRH1 sequence of DTIDWY (SEQ ID NO: 102), a CDRH2 sequence of VQSDGSYTKRPGVPDR (SEQ ID NO: 113), and a CDRH3 sequence of YIGGYVFG (SEQ ID NO: 117).

In particular embodiments, the binding domain binds the cellular marker ROR1. In particular embodiments, the scFV is a human or humanized scFv including a variable light chain including a CDRL1 sequence of SGSDINDYPIS (SEQ ID NO: 109), a CDRL2 sequence of INSGGST (SEQ ID NO: 105), and a CDRL3 sequence of YFCARGYS (SEQ ID NO: 116). In particular embodiments, the scFV is a human or humanized ScFv including a variable heavy chain including a CDRH1 sequence of SNLAW (SEQ ID NO: 110), a CDRH2 sequence of RASNLASGVPSRFSGS (SEQ ID NO: 107), and a CDRH3 sequence of NVSYRTSF (SEQ ID NO: 106). A number of additional antibodies specific for ROR1 are known to those of skill in the art.

In particular embodiments, the binding domain binds the cellular marker Her2. A number of antibodies specific for Her2 are known to those of skill in the art and can be readily characterized for sequence, epitope binding, and affinity. In particular embodiments, the binding domain includes a scFV sequence from the Herceptin antibody. In particular embodiments, the binding domain includes a human or humanized ScFv including a variable light chain including a CDRL1 sequence, a CDRL2 sequence and a CDRL3 sequence of the Herceptin antibody. In particular embodiments, the scFV is a human or humanized ScFv including a variable heavy chain including a CDRH1 sequence, a CDRH2 sequence, and a CDRH3 sequence of the Herceptin antibody. The CDR sequences can readily be determined from the amino acid sequence of Herceptin. An exemplary gene sequence encoding a Her2 binding domain is found in SEQ ID NOs: 39 and 40.

In particular embodiments, CDR regions are found within antibody regions as numbered by Kabat as follows: for the light chain: CDRL1 are amino acids 24-34;CDRL2 are amino acids 50-56; CDRL3 are amino acids 89-97 and for the heavy chain: CDRH1 are amino acids 31-35; CDRH2 are amino acids 50-65; and CDRH3 are amino acids 95-102.

Other antibodies are well-known and commercially available. For example, anti-PSMA and anti-PSCA antibodies are available from Abcam plc (ab66912 and ab15168, respectively). Mesothelin and WT1 antibodies are available from Santa Cruz Biotechnology, Inc. Anti-CD20 antibodies, such as rituximab (trade names Rituxan, MabThera and Zytux), have been developed by IDEC Pharmaceuticals.

**As** indicated, binding domains can also include T cell receptors (TCRs). TCRs refers to a molecule found on the surface of T cells (or T lymphocytes) that, in association with CD3, is generally responsible for recognizing antigens bound to major histocompatibility complex (MHC) molecules. The TCR has a disulfide-linked heterodimer of the highly variable α and β chains (also known as TCRα and TCRβ, respectively) in most T cells. In a small subset of T cells, the TCR is made up of a heterodimer of variable γ and δ chains (also known as TCRγ and TCRδ, respectively). Each chain of the TCR is a member of the immunoglobulin superfamily and possesses one N-terminal immunoglobulin variable domain, one immunoglobulin constant domain, a transmembrane region, and a short cytoplasmic tail at the C-terminal end (*see* Janeway, et al., 1997, Immunobiology: The Immune System in Health and Disease, 3rd Ed., Current Biology Publications, p. 4:33). TCR may be from various animal species, including human, mouse, rat, cat, dog, goat, horse, or other mammals. TCRs may be cell-bound (*i*.*e*., have a transmembrane region or domain) or in soluble form.

Major histocompatibility complex molecules (MHC molecules) refer to glycoproteins that deliver peptide antigens to a cell surface. MHC class I molecules are heterodimers consisting of a membrane spanning α chain (with three α domains) and a non-covalently associated β2 microglobulin. MHC class II molecules are composed of two transmembrane glycoproteins, α and β, both of which span the membrane. Each chain has two domains. MHC class I molecules deliver peptides originating in the cytosol to the cell surface, where peptide:MHC complex is recognized by CD8⁺ T cells. MHC class II molecules deliver peptides originating in the vesicular system to the cell surface, where they are recognized by CD4⁺ T cells. An MHC molecule may be from various animal species, including human, mouse, rat, or other mammals.

Spacer regions facilitate the interaction of chimeric molecule binding domains, so that the resulting polypeptide structure maintains a specific binding affinity to a cellular marker or maintains signaling activity (*e*.*g*., effector domain activity) or both.

Thus, in particular embodiments, a spacer region is found between the binding domain and intracellular component of an expressed chimeric molecule. In particular embodiments, the spacer region is part of the extracellular component of an expressed chimeric molecule.

The length of a spacer region can be customized for individual cellular markers on unwanted cells to optimize unwanted cell recognition and destruction. In particular embodiments, a spacer region length can be selected based upon the location of a cellular marker epitope, affinity of a binding domain for the epitope, and/or the ability of the modified cells expressing the molecule to proliferate *in vitro, in vivo* and/or *ex vivo* in response to cellular marker recognition.

Typically a spacer region is found between the binding domain and a hydrophobic portion of an expressed chimeric molecule. Spacer regions can provide for flexibility of the binding domain and allow for high expression levels in modified cells. In particular embodiments, a spacer region can have at least 10 to 250 amino acids, at least 10 to 200 amino acids, at least 10 to 150 amino acids, at least 10 to 100 amino acids, at least 10 to 50 amino acids, or at least 10 to 25 amino acids. In further embodiments, a spacer region has 250 amino acids or less; 200 amino acids or less, 150 amino acids or less; 100 amino acids or less; 50 amino acids or less; 40 amino acids or less; 30 amino acids or less; 20 amino acids or less; or 10 amino acids or less.

In particular embodiments, spacer regions can include or be derived from a hinge region of an immunoglobulin like molecule, for example all or a portion of the hinge region from a human IgG1, IgG2, IgG3, or IgG4. Hinge regions can be modified to avoid undesirable structural interactions such as dimerization. In particular embodiments, all or a portion of a hinge region can be combined with one or more domains of a constant region of an immunoglobulin. For example, a portion of a hinge region can be combined with all or a portion of a CH2 or CH3 domain. In particular embodiments, the spacer region does not include the 47-48 amino acid hinge region sequence from CD8α.

In particular embodiments, the spacer region is selected from the group including a hinge region sequence from IgG1, IgG2, IgG3, or IgG4 in combination with all or a portion of a CH2 region; all or a portion of a CH3 region; or all or a portion of a CH2 region and all or a portion of a CH3 region.

In particular embodiments, a short spacer region has 12 amino acids or less and includes all or a portion of a lgG4 hinge region sequence (*e*.*g*., the protein encoded by SEQ ID NO:50), an intermediate spacer region has 119 amino acids or less and includes all or a portion of a lgG4 hinge region sequence and a CH3 region (*e*.*g*., SEQ ID NO:52), and a long spacer has 229 amino acids or less and includes all or a portion of a lgG4 hinge region sequence, a CH2 region, and a CH3 region (*e*.*g*., SEQ ID NO:50).

In particular embodiments, when a binding domain binds to a portion of a cellular marker that is very proximal to the unwanted cell's membrane, a long spacer (e.g. 229 amino acids or less and greater than 119 amino acids) is selected. Very proximal to the unwanted cell's membrane means within the first 100 extracellular amino acids of a cellular marker.

In particular embodiments, when a binding domain binds to a portion of a cellular marker that is distal to the unwanted cell's membrane, an intermediate or short spacer is selected (e.g. 119 amino acids or less or 12 amino acids or less).

Whether a binding portion of a cellular marker is proximal or distal to a membrane can also be determined by modeling three dimensional structures or based on analysis of crystal structure.

In a particular embodiment, an expressed chimeric molecule includes a binding domain including a scFV that binds to a ROR1 epitope located in the membrane distal to the Ig/Frizzled domain and a spacer that is 15 amino acids or less. In particular embodiments, an expressed chimeric molecule includes a binding domain including an scFV that binds a ROR1 epitope located in the membrane proximal to the Kringle domain and a spacer that is longer than 15 amino acids. In particular embodiments an expressed chimeric molecule includes a binding domain including a scFV that binds CD19 and a spacer that is 15 amino acids or less.

In particular embodiments, when the binding domain includes (i) a variable light chain including a CDRL1 sequence of RASQDISKYLN (SEQ ID NO: 108), a CDRL2 sequence of SRLHSGV (SEQ ID NO: 111), and a CDRL3 sequence of GNTLPYTFG (SEQ ID NO: 104) and a variable heavy chain including a CDRH1 sequence of DYGVS (SEQ ID NO: 103), a CDRH2 sequence of VTWGSETTYYNSALKS (SEQ ID NO: 114), and a CDRH3 sequence of YAMDYWG (SEQ ID NO: 115), or (ii) a variable light chain including a CDRL1 sequence of ASGFDFSAYYM (SEQ ID NO: 101), a CDRL2 sequence of TIYPSSG (SEQ ID NO: 112), and a CDRL3 sequence of ADRATYFCA (SEQ ID NO: 100), and a variable heavy chain including a CDRH1 sequence of DTIDWY (SEQ ID NO: 102), a CDRH2 sequence of VQSDGSYTKRPGVPDR (SEQ ID NO: 113), and a CDRH3 sequence of YIGGYVFG (SEQ ID NO: 117), the spacer can be 12 amino acid or less and, in a more particular embodiment can include SEQ ID NO:47.

In particular embodiments, when the binding domain includes (i) a variable light chain including a CDRL1 sequence of SGSDINDYPIS (SEQ ID NO: 109), a CDRL2 sequence of INSGGST (SEQ ID NO: 105), and a CDRL3 sequence of YFCARGYS (SEQ ID NO: 116), and a variable heavy chain including a CDRH1 sequence of SNLAW (SEQ ID NO: 110), a CDRH2 sequence of RASNLASGVPSRFSGS (SEQ ID NO: 107), and a CDRH3 sequence of NVSYRTSF (SEQ ID NO: 106), or (ii) a variable light chain including a CDRL1 sequence, a CDRL2 sequence and a CDRL3 sequence of the Herceptin antibody and a variable heavy chain including a CDRH1 sequence, a CDRH2, and a CDRH3 sequence of the Herceptin antibody, the spacer can be 229 amino acid or less and, in a more particular embodiment can include SEQ ID NO:61.

In particular embodiments, a "hinge region" or a "hinge" refers to (a) an immunoglobulin hinge sequence (made up of, for example, upper and core regions) or a functional fragment or variant thereof, (b) a type II C-lectin interdomain (stalk) region or a functional fragment or variant thereof, or (c) a cluster of differentiation (CD) molecule stalk region or a functional variant thereof. A "wild type immunoglobulin hinge region" refers to a naturally occurring upper and middle hinge amino acid sequences interposed between and connecting the CH1 and CH2 domains (*e*.*g*., for IgG, IgA, and IgD) or interposed between and connecting the CH1 and CH3 domains (*e*.*g*., for IgE and IgM) found in the heavy chain of an antibody. In certain embodiments, a hinge region is human, and in particular embodiments, includes a human IgG hinge region.

A "stalk region" of a type II C-lectin or CD molecule refers to the portion of the extracellular domain of the type II C-lectin or CD molecule that is located between the C-type lectin-like domain (CTLD; *e*.*g*., similar to CTLD of natural killer cell receptors) and the hydrophobic portion (*e*.*g*., a transmembrane domain). For example, the extracellular domain of human CD94 (GenBank Accession No. AAC50291.1) corresponds to amino acid residues 34-179, but the CTLD corresponds to amino acid residues 61-176, so the stalk region of the human CD94 molecule includes amino acid residues 34-60, which are located between the hydrophobic portion (e.g., transmembrane domain) and CTLD (see Boyington, et a/., 1999, Immunity 10:75; for descriptions of other stalk regions, see also Beavil, et al., 1992, Proc. Nat'l. Acad. Sci. USA 89:753; and Figdor, et a/., 2002, Nat. Rev. Immunol. 2:77). These type II C-lectin or CD molecules may also have junction amino acids between the stalk region and the transmembrane region or the CTLD. In another example, the 233 amino acid human NKG2A protein (GenBank Accession No. P26715.1) has a hydrophobic portion (e.g., a transmembrane domain) ranging from amino acids 71-93 and an extracellular domain ranging from amino acids 94-233. The CTLD includes amino acids 119-231, and the stalk region includes amino acids 99-116, which may be flanked by additional junction amino acids. Other type II C-lectin or CD molecules, as well as their extracellular binding domains, stalk regions, and CTLDs are known in the art (see, e.g., GenBank Accession Nos. NP_001993.2; AAH07037.1; NP_001773.1; AAL65234.1; CAA04925.1; for the sequences of human CD23, CD69, CD72, NKG2A and NKG2D and their descriptions, respectively).

A "derivative" of a stalk region hinge, or fragment thereof, of a type II C-lectin or CD molecule includes an eight to 150 amino acid sequence in which one, two, or three amino acids of the stalk region of a wild type type II C-lectin or CD molecule have a deletion, insertion, substitution, or any combination thereof. For instance, a derivative can include one or more amino acid substitutions and/or an amino acid deletion. In certain embodiments, a derivative of a stalk region is more resistant to proteolytic cleavage as compared to the wild-type stalk region sequence, such as those derived from eight to 20 amino acids of NKG2A, NKG2D, CD23, CD64, CD72, or CD94.

In certain embodiments, stalk region hinges may include from seven to 18 amino acids and can form an α-helical coiled coil structure. In certain embodiments, stalk region hinges contain 0, 1, 2, 3, or 4 cysteines. Exemplary stalk region hinges include fragments of the stalk regions, such as those portions including from ten to 150 amino acids from the stalk regions of CD69, CD72, CD94, NKG2A and NKG2D.

Alternative hinges that can be used in chimeric molecules are from portions of cell surface receptors (interdomain regions) that connect immunoglobulin V-like or immunoglobulin C-like domains. Regions between Ig V-like domains where the cell surface receptor contains multiple Ig V-like domains in tandem and between Ig C-like domains where the cell surface receptor contains multiple tandem Ig C-like regions are also contemplated as hinges useful in chimeric molecules. In certain embodiments, hinge sequences including cell surface receptor interdomain regions may further contain a naturally occurring or added motif, such as an IgG core hinge sequence to provide one or more disulfide bonds to stabilize the chimeric molecule dimer formation. Additional examples of hinges include interdomain regions between the Ig V-like and Ig C-like regions of CD2, CD4, CD22, CD33, CD48, CD58, CD66, CD80, CD86, CD150, CD166, and CD244.

ln certain embodiments, hinge sequences include 5 to 150 amino acids, 5 to 10 amino acids, 10 to 20 amino acids, 20 to 30 amino acids, 30 to 40 amino acids, 40 to 50 amino acids, 50 to 60 amino acids, 5 to 60 amino acids, 5 to 40 amino acids, for instance, 8 to 20 amino acids or 10 to 15 amino acids. The hinges may be primarily flexible, but may also provide more rigid characteristics or may contain primarily α-helical structure with minimal β-sheet structure.

In certain embodiments, a hinge sequence is stable in plasma and serum, and is resistant to proteolytic cleavage. For example, the first lysine in an IgG1 upper hinge region may be mutated or deleted to minimize proteolytic cleavage, and hinges may include junction amino acids. In some embodiments, a hinge sequence may contain a naturally occurring or added motif, such as an immunoglobulin hinge core structure CPPCP (SEQ. ID. NO:125) that confers the capacity to form a disulfide bond or multiple disulfide bonds to stabilize dimer formation.

A "linker sequence" can be an amino acid sequence having from two up to 500 amino acids, which can provide flexibility and room for conformational movement between two regions, domains, motifs, cassettes or modules connected by a linker. Exemplary linker sequences include those having from one to ten repeats of GlyₓSer_{y}, wherein x and y are independently an integer from 0 to 10 provided that x and y are not both 0 (e.g., (Gly₄Ser)₂ (SEQ ID NO: 122), (Gly₃Ser)₂ (SEQ ID NO: 123), Gly₂Ser, or a combination thereof such as (Gly₃Ser)₂Gly₂Ser)(SEQ ID NO: 124). In certain other embodiments, a linker sequence can include one or more immunoglobulin heavy chain constant regions, such as a CH3 alone or a CH2CH3 sequence.

Linker sequences often provide junction amino acids. Junction amino acids refer to one or more (e.g., 2-20) amino acid residues between two adjacent motifs, regions or domains of a polypeptide, such as between a binding domain and a hydrophobic portion and an adjacent effector domain or on one or both ends of a linker region that links two motifs, regions or domains (*e.g.,* between a linker and an adjacent binding domain and/or between a linker and an adjacent hinge). Junction amino acids may result from the construct design of a fusion protein (*e.g.,* amino acid residues resulting from the use of a restriction enzyme site during the construction of a nucleic acid molecule encoding a fusion protein). For example, a single junction amino acid, asparagine, is encoded by the AAT codon found between the nucleic acid sequence encoding the secretory signal sequence (SEQ ID NO:126) and the sequence encoding the tag cassette (SEQ ID NO:127) in the chimeric molecule encoded by the nucleic acid sequence set forth in SEQ ID NO:58. Similarly, an asparagine (N) junction amino acid is found between the flexible linker amino acid sequence of GGSGSG (SEQ ID NO:129) and the amino acid tag sequence WSHPQFEK (SEQ ID NO:118) found in the chimeric molecule having the amino acid sequence set forth in SEQ ID NO: 130.

In particular embodiments, an extracellular component can include a hinge and one or more linker sequences, or an extracellular component can include a hinge, one or more linker sequences, and one or more tag cassettes.

Within a chimeric molecule structure, a tag cassette may be located (a) immediately amino-terminal to a spacer region, (b) interposed between and connecting linker sequences, (c) immediately carboxy-terminal to a binding domain, (d) interposed between and connecting a binding domain (*e*.*g*., scFv) to an effector domain, (e) interposed between and connecting subunits of a binding domain, or (f) at the amino-terminus of a chimeric molecule. In certain embodiments, one or more junction amino acids may be disposed between and connecting a tag cassette with a hydrophobic portion, or disposed between and connecting a tag cassette with a spacer region, or disposed between and connecting a tag cassette with a linker sequence, or disposed between and connecting a tag cassette with a binding domain.

In further embodiments, the two or more first tag cassettes may be located in different areas of a chimeric molecule. In certain embodiments, a first tag cassette is located in a connector region including one or more spacer regions and a second tag cassette is located at the amino-terminus or carboxy terminus or both of a chimeric molecule (see, e.g., FIG. 38H).

In certain embodiments, a tag cassette is located within a connector region including one or more spacer regions of a fusion protein of this disclosure. In particular embodiments, an extracellular component can include a linker sequence adjacent to a tag cassette, wherein the linker sequence with the tag cassette has an amino acid sequence of (Gly-Gly-Gly-Gly-Ser)₂-Trp-Ser-His-Pro-Gln-Phe-Glu-Lys (SEQ ID NO: 139), Trp-Ser-His-Pro-Gln-Phe-Glu-Lys-(Gly-Gly-Gly-Gly-Ser)₂ (SEQ ID NO: 140), (Gly-Gly-Gly-Gly-Ser)₂-Trp-Ser-His-Pro-Gln-Phe-Glu-Lys-(Gly-Gly-Gly-Ser)₂-Gly-Gly-Ser-Trp-Ser-His-Pro-Gln-Phe-Glu-Lys (SEQ ID NO: 141), Trp-Ser-His-Pro-Gln-Phe-Glu-Lys-(Gly-Gly-Gly-Ser)₂-Gly-Gly-Ser-Trp-Ser-His-Pro-Gln-Phe-Glu-Lys-(Gly-Gly-Gly-Gly-Ser)₂ (SEQ ID NO: 142), (Gly-Gly-Gly-Gly-Ser)₂-Trp-Ser-His-Pro-Gln-Phe-Glu-Lys-(Gly-Gly-Gly-Ser)₂-Gly-Gly-Ser-Trp-Ser-His-Pro-Gln-Phe-Glu-Lys-(Gly-Gly-Gly-Gly-Ser)₂-Trp-Ser-His-Pro-Gln-Phe-Glu-Lys (SEQ ID NO:143), or Trp-Ser-His-Pro-Gln-Phe-Glu-Lys-(Gly-Gly-Gly-Gly-Ser)₂-Trp-Ser-His-Pro-Gln-Phe-Glu-Lys-(Gly-Gly-Gly-Ser)₂-Gly-Gly-Ser-Trp-Ser-His-Pro-Gln-Phe-Glu-Lys-(Gly-Gly-Gly-Gly-Ser)₂ (SEQ ID NO:144).

In chimeric molecule fusion protein embodiments, a protein complex can form between a fusion protein and a cognate binding molecule, which is a result of binding between the tag cassette and cognate binding molecule. In certain embodiments, a chimeric molecule includes a scFv or scTCR binding domain where the tag cassette is located within the variable region linker (between binding domain subunits). In particular embodiments, a chimeric molecule has a tag cassette located at the amino-terminus of the binding domain. In such protein complexes or fusion protein structures, a chimeric molecule binding domain will retain its cellular marker specificity or its specific cellular marker binding affinity.

A "variable region linker" specifically refers to a five to 35 amino acid sequence that connects a heavy chain immunoglobulin variable region to a light chain immunoglobulin variable region or connects T cell receptor V_{α/β} and C_{α/β} chains (*e*.*g*., V_{α}-C_{α}, V_{β}-C_{β}, V_{α}-V_{β}) or connects each V_{α}-C_{α}, V_{β}-C_{β}, V_{α}-V_{β} pair to a hinge or hydrophobic portion, which provides a spacer function and flexibility sufficient for interaction of the two sub-binding domains so that the resulting single chain polypeptide retains a specific binding affinity to the same cellular marker as an antibody or T cell receptor. In certain embodiments, a variable region linker includes from ten to 30 amino acids or from 15 to 25 amino acids. In particular embodiments, a variable region linker peptide includes from one to ten repeats of GlyₓSer_{y}, wherein x and y are independently an integer from 0 to 10 provided that x and y are not both 0 (*e*.*g*., Gly₄Ser (SEQ ID NO: 145), Gly₃Ser (SEQ ID NO: 146), Gly₂Ser, or (Gly₃Ser)ₙ(Gly₄Ser)₁ (SEQ ID NO: 147), (Gly₃Ser)ₙ(Gly₂Ser)ₙ, (SEQ ID NO: 148) (Gly₃Ser)ₙ(Gly₄Ser)ₙ (SEQ ID NO: 147), or (Gly₄Ser)ₙ (SEQ ID NO: 145), wherein n is an integer of 1, 2, 3, 4, 5, 6, 7, 8, 9 or 10) and wherein linked variable regions form a functional immunoglobulin-like binding domain (*e*.*g*., scFv, scTCR). Exemplary variable region linkers include those amino acid sequences set forth in SEQ IDNOS:30, 129, 149-152, and 146-148, and (Gly₄Ser)ₙ (SEQ ID NO: 145), wherein n is 3, as found in chimeric molecule having the amino acid sequence set forth in SEQ ID NO: 150.

Hydrophobic Portions (*e*.*g*., Transmembrane Domains). A "hydrophobic portion" means any amino acid sequence having a three-dimensional structure that is thermodynamically stable in a cell membrane, and generally ranges in length from 15 amino acids to 30 amino acids. The structure of a hydrophobic portion may include an alpha helix, a beta barrel, a beta sheet, a beta helix, or any combination thereof. A hydrophobic portion can be a transmembrane domain and vice versa.

A hydrophobic portion contained in a chimeric molecule will allow a fusion protein to associate with a cellular membrane such that a portion of the fusion protein will be located extracellularly (*e*.*g*.*,* tag cassette, connector domain, binding domain) and a portion will be located intracellularly (*e.g.,* effector domain). A hydrophobic portion will generally be disposed within the cellular membrane phospholipid bilayer. In certain embodiments, one or more junction amino acids may be disposed between and connecting a hydrophobic portion with an effector domain, or disposed between and connecting a hydrophobic portion with a portion of an extracellular component, or disposed between and connecting a hydrophobic portion with a tag cassette.

In certain embodiments, a hydrophobic portion is a transmembrane domain.

Accordingly, expressed chimeric molecules disclosed herein can also include a transmembrane domain, at least a portion of which is located between the extracellular component and the intracellular component. The transmembrane domain can anchor the expressed chimeric molecule in the modified cell's membrane. The transmembrane domain can be derived either from a natural and/or a synthetic source. When the source is natural, the transmembrane domain can be derived from any membrane-bound or transmembrane protein. Particular examples can be derived from an integral membrane protein (e.g., receptor, cluster of differentiation (CD) molecule, enzyme, transporter, cell adhesion molecule, or the like). Transmembrane domains can include at least the transmembrane region(s) of the alpha, beta or zeta chain of a T-cell receptor, CD8, CD27, CD28, CD3, CD45, CD4, CD5, CD9, CD16, CD22; CD33, CD37, CD64, CD80, CD86, CD134, CD137 and CD154. Transmembrane domains can include those shown in FIG. 2 or FIG. 6.

In particular embodiments, the transmembrane domain includes the amino acid sequence of the CD28 transmembrane domain as shown in FIG. 2 or the amino acid sequence of the CD4 transmembrane domain. A representative gene sequence encoding the CD28 transmembrane domain is shown in FIG. 1 (SEQ ID NO:12). SEQ ID NO: 118 is a representative gene sequence encoding the CD4 transmembrane domain.

Intracellular Components. Intracellular components of expressed chimeric molecules can include effector domains. Effector domains are capable of transmitting functional signals to a cell. In particular embodiments, an effector domain will directly or indirectly promote a cellular response by associating with one or more other proteins that directly promote a cellular response. Effector domains can provide for activation of at least one function of a modified cell upon binding to the cellular marker expressed on an unwanted cell. Activation of the modified cell can include one or more of differentiation, proliferation and/or activation or other effector functions.

**An** effector domain can include one, two, three or more receptor signaling domains, intracellular signaling domains (e.g., cytoplasmic signaling sequences), costimulatory domains, or combinations thereof. Exemplary effector domains include signaling and stimulatory domains selected from: 4-1 BB, CARD11, CD3 gamma, CD3 delta, CD3 epsilon, CD3ζ, CD27, CD28, CD79A, CD79B, DAP10, FcRα, FcRβ, FcRγ, Fyn, HVEM, ICOS, LAG3, LAT, Lck, LRP, NKG2D, NOTCH1, pTα, PTCH2, OX40, ROR2, Ryk, SLAMF1, Slp76, TCRα, TCRβ, TRIM, Wnt, Zap70, or any combination thereof.

Primary cytoplasmic signaling sequences that act in a stimulatory manner may contain signaling motifs which are known as receptor tyrosine-based activation motifs or iTAMs. Examples of iTAM containing primary cytoplasmic signaling sequences include those derived from CD3γ, CD3δ, CD3ε, CD3ζ, CD5, CD22, CD66d, CD79a, CD79b, and FeR gamma. In particular embodiments, variants of CD3ζ retain at least one, two, three, or all ITAM regions as shown in FIG. 7.

In particular embodiments, an effector domain includes a cytoplasmic portion that associates with a cytoplasmic signaling protein, wherein the cytoplasmic signaling protein is a lymphocyte receptor or signaling domain thereof, a protein including a plurality of ITAMs, a costimulatory domain, or any combination thereof.

Examples of intracellular signaling domains include the cytoplasmic sequences of the CD3ζ chain, and/or co- receptors that act in concert to initiate signal transduction following binding domain engagement.

In particular embodiments, an intracellular signaling domain of a molecule expressed by a modified cell can be designed to include an intracellular signaling domain combined with any other desired cytoplasmic domain(s). For example, the intracellular signaling domain of a molecule can include an intracellular signaling domain and a costimulatory domain, such as a costimulatory signaling region.

**The** costimulatory signaling region refers to a portion of the molecule including the intracellular domain of a costimulatory domain. A costimulatory domain is a cell surface molecule other than the expressed cellular marker binding domain that can be required for a lymphocyte response to cellular marker binding. Examples of such molecules include CD27, CD28, 4-1BB (CD 137), OX40, CD30, CD40, lymphocyte function-associated antigen-1 (LFA-1), CD2, CD7, LIGHT, NKG2C, B7-H3, and a ligand that specifically binds with CD83.

In particular embodiments, the amino acid sequence of the intracellular signaling domain including a variant of CD3ζ and a portion of the 4-1 BB intracellular signaling domain as provided in FIG. 2. A representative gene sequence is provided in FIG. 1 (SEQ ID NO:16; SEQ ID NO:1).

In particular embodiments, the intracellular signaling domain includes (i) all or a portion of the signaling domain of CD3ζ, (ii) all or a portion of the signaling domain of CD28, (iii) all or a portion of the signaling domain of 4-1 BB, or (iv) all or a portion of the signaling domain of CD3ζ, CD28 and/or 4-1 BB.

Additional exemplary effector domains useful in the chimeric molecules of this disclosure may be from a protein of a Wnt signaling pathway (*e*.*g*.*,* LRP, Ryk, ROR2), NOTCH signaling pathway (*e.g.,* NOTCH1, NOTCH2, NOTCH3, NOTCH4), Hedgehog signaling pathway (*e.g.,* PTCH, SMO), receptor tyrosine kinases (RTKs) (*e*.*g*.*,* epidermal growth factor (EGF) receptor family, fibroblast growth factor (FGF) receptor family, hepatocyte growth factor (HGF) receptor family, Insulin receptor (IR) family, platelet-derived growth factor (PDGF) receptor family, vascular endothelial growth factor (VEGF) receptor family, tropomycin receptor kinase (Trk) receptor family, ephrin (Eph) receptor family, AXL receptor family, leukocyte tyrosine kinase (LTK) receptor family, tyrosine kinase with immunoglobulin-like and EGF-like domains 1 (TIE) receptor family, receptor tyrosine kinase-like orphan (ROR) receptor family, discoidin domain (DDR) receptor family, rearranged during transfection (RET) receptor family, tyrosine-protein kinase-like (PTK7) receptor family, related to receptor tyrosine kinase (RYK) receptor family, muscle specific kinase (MuSK) receptor family); G-protein-coupled receptors, GPCRs (Frizzled, Smoothened); serine/threonine kinase receptors (BMPR, TGFR); or cytokine receptors (IL1R, IL2R, IL7R, IL15R).

The intracellular signaling domain sequences of the expressed chimeric molecule can be linked to each other in a random or specified order. Optionally, a short oligo- or protein linker, preferably between 2 and 10 amino acids in length may form the linkage.

Thus, an effector domain contained in a chimeric molecule will be an intracellular component and capable of transmitting functional signals to a cell. In certain embodiments, a single chain chimeric molecule will dimerize with a second single chain chimeric molecule, respectively, wherein the dimerization allows the intracellular component including an effector domains to be in close proximity and promote signal transduction when exposed to the proper signal. As indicated, in addition to forming such dimer protein complexes, the effector domains may further associate with other signaling factors, such as costimulatory factors, to form multiprotein complexes that produce an intracellular signal. In certain embodiments, an effector domain will indirectly promote a cellular response by associating with one or more other proteins that directly promote a cellular response. An effector domain may include one, two, three or more receptor signaling domains, costimulatory domains, or combinations thereof. Any intracellular component including an effector domain, costimulatory domain or both from any of a variety of signaling molecules (e.g., signal transduction receptors) may be used in the fusion proteins of this disclosure.

The design of particular molecules to be expressed by the modified cells can be customized depending on the type of tag cassette, a targeted cellular marker, the affinity of the binding domain for the cellular marker, the flexibility needed for the cellular marker binding domain, and/or the intracellular signaling domain. In particular embodiments, a number of constructs are tested *in vitro* and in *in vivo* models to determine the ability of modified cells to expand in culture and/or kill unwanted cells. In particular embodiments, a molecule is selected that provides for capability of at least 30% of modified-effectors (*e*.*g*., differentiated modified HSPC) to proliferate through at least two generations *in vitro* and/or within 72 hours after introduction *in vivo.* In particular embodiments, a molecule is not selected that results in greater than 50% of the cells undergoing activation induced cell death (AICD) within 72 hours *in vivo* in immunodeficient mice, and fails to reduce presence of tumor cells.

The following disclosure provides more particular examples of expressed chimeric molecules and associated vectors.

"Chimeric antigen receptor" or "CAR" refer to a synthetically designed receptor including a binding domain that binds to a cellular marker preferentially associated with an unwanted cell that is linked to an effector domain. The binding domain and effector domain can be linked via a spacer domain, transmembrane domain, tag cassette, and/or linker sequence.

ln particular embodiments, ROR1-specific and CD19-specific CARs can be constructed using VL and VH chain segments of the 2A2, R12, and R11 mAhs (ROR1) and FMC63 mAb (CD19). Variable region sequences for R11 and R12 are provided in Yang et al, Plos One 6(6):e21018, June 15, 2011. Each scFV can be linked by a (Gly₄Ser)₃ (SEQ ID NO:60) protein to a spacer domain derived from IgG4-Fc (Uniprot Database: P0186I, SEQ ID NO:92) including either 'Hinge-CH2-CH3' (229 AA, SEQ ID NO:61), 'Hinge-CH3' (119 AA, SEQ ID NO: 52) or 'Hinge' only (12 AA, SEQ. ID NO:47) sequences (FIG. 1). All spacers can contain a S→P substitution within the 'Hinge' domain located at position 108 of the native lgG4-Fc protein, and can be linked to the 27 AA transmembrane domain of human CD28 (Uniprot: P10747, SEQ ID NO:93) and to an effector domain signaling module including either (i) the 41 AA cytoplasmic domain of human CD28 with an LL→GG substitution located at positions 186-187 of the native CD28 protein (SEQ ID NO:93) or (ii) the 42 AA cytoplasmic domain of human 4-1 BB (Uniprot: Q07011, SEQ ID NO: 95), each of which can be linked to the 112 AA cytoplasmic domain of isoform 3 of human CD3ζ (Uniprot: P20963, SEQ ID NO:94). The construct encodes a T2A ribosomal skip element (SEQ ID NO:88)) and a tEGFR sequence (SEQ ID NO:27) downstream of the chimeric receptor. tEGFR can be replaced or supplemented with a tag cassette binding a ExoCBM, such as STREP TAG^{®} II (SEQ ID NO:118), Myc tag (SEQ ID NO:119), V5 tag (SEQ ID NO:120), FLAG^{®} tag (SEQ ID NO:121), His tag, or other peptides or molecules as disclosed herein. Codon-optimized gene sequences encoding each transgene can be synthesized (Life Technologies) and cloned into the epHIV7 lentiviral vector using Nhel and Not1 restriction sites. The epHIV7 lentiviral vector can be derived from the pHIV7 vector by replacing the cytomegalovirus promoter of pHIV7 with an EF-1 promoter. ROR1-chimeric receptor, CD19-chimeric receptor, tEGFR, or tag cassette-encoding lentiviruses can be produced in 293T cells using the packaging vectors pCHGP-2, pCMV-Rev2 and pCMV-G, and CALPHOS^{®} transfection reagent (Clontech).

HER2-specific chimeric receptors can be constructed using VL and VH chain segments of a HER2-specific mAb that recognizes a membrane proximal epitope on HER2 (FIG. 12A), and the scFVs can be linked to lgG4 hinge/CH2/CH3, IgG4 hinge/CH3, and lgG4 hinge only extracellular spacer domains and to the CD28 transmembrane domain, 4-1 BB and CD3ζ signaling domains (FIG. 12B).

As indicated, each CD19 chimeric receptor can include a single chain variable fragment corresponding to the sequence of the CD19-specific mAb FMC63 (scFv: VL-VH), a spacer derived from lgG4-Fc including either the 'Hinge-CH2-CH3' domain (229 AA, long spacer) or the 'Hinge' domain only (12 AA, short spacer), and a signaling module of CD3ζ with membrane proximal CD28 or 4-1 BB costimulatory domains, either alone or in tandem (FIG. 13A). The transgene cassette can include a truncated EGFR (tEGFR) downstream from the chimeric receptor gene and be separated by a cleavable T2A element, to serve as a tag sequence for transduction, selection and *in vivo* tracking for chimeric receptor-modified cells. tEGFR can be replaced or supplemented with a tag cassette binding a ExoCBM, such as STREP TAG^{®} II (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

Particular embodiments include modified cells (e.g., modified HSPC or modified non-T effector cells) expressing a chimeric molecule including an extracellular component and an intracellular component connected by a hydrophobic portion, wherein the extracellular component includes a binding domain that specifically binds a cellular marker, a tag cassette, and a spacer region including a hinge, and wherein the intracellular component includes an effector domain.

Particular embodiments include modified cell expressing a chimeric antigen receptor molecule, including a fusion protein having one or more extracellular tag cassettes (a) located at the amino-terminus of an extracellular binding domain, (b) imbedded within an extracellular binding domain, or (c) disposed between and connecting an extracellular binding domain and an intracellular component including an effector domain.

Particular embodiments include modified HSPC expressing a chimeric molecule including a hydrophobic portion disposed between and connecting an extracellular component and an intracellular component, wherein the extracellular component includes a tag cassette and a spacer region including a hinge, and wherein the intracellular component includes an effector domain.

Particular embodiments include a method for targeting (e.g., for identification, isolation, expansion) a modified cell, such as a modified HSPC cell, including contacting the cell with a ExoCBM molecule specific for a tag cassette expressed by the cell, wherein the cell includes a nucleic acid molecule encoding a fusion protein to express the tag cassette and wherein the ExoCBM specific for the tag cassette is attached to a solid surface.

Particular embodiments include a method for promoting modified cell proliferation, such as modified HSPC cell proliferation, including contacting the cell with (i) an ExoCBM specific for a tag cassette expressed by the cell and (ii) a growth factor cytokine for a time sufficient to allow cell growth, wherein the cell includes a nucleic acid molecule including the tag cassette and the ExoCBM specific for the tag cassette is attached to a solid surface.

Particular embodiments include a method for detecting a modified cell, such as a modified HSPC, including contacting a sample including a modified cell with an ExoCBM specific for a tag cassette expressed by the modified cell wherein the ExoCBM specific for the tag cassette includes a detectable moiety, and detecting the presence of the modified cell.

Particular embodiments include a method for sorting a modified cell, including contacting a sample including a modified cell with an ExoCBM specific for a tag cassette expressed by the modified cell wherein the ExoCBM specific for the tag cassette includes a detectable moiety, and sorting modified cells from other cells not expressing the tag cassette in the sample.

Particular embodiments include a method for enriching or isolating a modified cell, including contacting a sample including a modified cell with an ExoCBM specific for a tag cassette expressed by the modified cell, wherein the ExoCBM specific for the tag cassette includes a detectable moiety, and enriching for or isolating the modified cell expressing the tag cassette away from other cells not expressing the tag cassette in the sample.

In certain aspects, the present disclosure provides a single chain fusion protein, referred to as a chimeric molecule, which includes an extracellular component and an intracellular component connected by a hydrophobic portion, wherein the extracellular component includes a tag cassette and a spacer region including a hinge, and wherein the intracellular component includes an effector domain. In certain embodiments, a connector region further includes a linker sequence, or one or more tag cassettes are located within the connector region. In certain other embodiments, one or more tag cassettes are linked to the connector region by a linker sequence. Connector sequences generally include more than one portion of an extracellular component (*e.g.,* a spacer region and a linker sequence; a linker sequence and a junction amino acid).

In further chimeric molecule embodiments, the fusion protein includes from amino-terminus to carboxy-terminus: a tag cassette, a connector region including a hinge, a hydrophobic portion, and an intracellular component including an effector domain (*see*, *e.g.,* FIGs. 38A and 38B). In still further chimeric molecule embodiments, the fusion protein includes from amino-terminus to carboxy-terminus: a first connector region, a tag cassette, a second connector region including a hinge, a hydrophobic portion, and an intracellular component including an effector domain. In yet further chimeric molecule embodiments, the fusion protein includes from amino-terminus to carboxy-terminus: a first tag cassette, a first connector region, a second tag cassette, a second connector region including a hinge, a hydrophobic portion, and an intracellular component including an effector domain (*see*, *e.g.,* FIG. 38C). In even further chimeric molecule embodiments, the fusion protein includes from amino-terminus to carboxy-terminus: a first tag cassette, a first connector region, a second tag cassette, a second connector region, a third tag cassette, a third connector region including a hinge, a hydrophobic portion, and an intracellular component including an effector domain (*see*, *e.g.,* FIG. 38D).

In certain other chimeric molecule embodiments, the fusion protein further includes a non-covalently associated binding domain, such as a binding domain associated with the tag cassette (*i.e.,* a multichain chimeric molecule). In still other chimeric molecule embodiments, the non-covalently associated binding domain is bi-specific, wherein the first binding end is specific for the tag cassette and the second binding end is specific for a cellular marker other than the tag cassette, or the first and second binding ends are both specific for the tag cassette. In yet other chimeric molecule embodiments, the non-covalently associated binding domain is multispecific, wherein a first end binds to a tag cassette and a second end is specific for one or more cellular markers other than the tag cassette. In such embodiments, a chimeric molecule includes a multimer protein. In some embodiments, such chimeric molecules including one or more non-covalently associated binding domains comprise heteromultimers.

In other aspects, the present disclosure provides a single chain fusion chimeric molecule which includes an extracellular component and an intracellular component connected by a hydrophobic portion, wherein the extracellular component includes a binding domain that specifically binds a cellular marker, a tag cassette, and a connector region including a hinge, and wherein the intracellular component includes an effector domain. In certain embodiments, a chimeric molecule binding domain is a scFv, scTCR, receptor ectodomain, or ligand.

In further chimeric molecule embodiments, the fusion protein includes from amino-terminus to carboxy-terminus: an extracellular binding domain, a tag cassette, a connector region including a hinge, a hydrophobic portion, and an intracellular component including an effector domain (*see*, *e.g.,* FIG. 38E). In still further chimeric molecule embodiments, the fusion protein includes from amino-terminus to carboxy-terminus: an extracellular binding domain, a first connector region, a tag cassette, a second connector region including a hinge, a hydrophobic portion, and an intracellular component including an effector domain. In yet further chimeric molecule embodiments, the fusion protein includes from amino-terminus to carboxy-terminus: an extracellular binding domain, a first tag cassette, a first connector region, a second tag cassette, a second connector region including a hinge, a hydrophobic portion, and an intracellular component including an effector domain. In even further chimeric molecule embodiments, the fusion protein includes from amino-terminus to carboxy-terminus: an extracellular binding domain, a first tag cassette, a first connector region, a second tag cassette, a second connector region, a third tag cassette, a third connector region including a hinge, a hydrophobic portion, and an intracellular component including an effector domain.

In certain other chimeric molecule embodiments, the fusion protein includes from amino-terminus to carboxy-terminus: a tag cassette, an extracellular binding domain, a connector region including a hinge, a hydrophobic portion, and an intracellular component including an effector domain (*see*, *e.g.,* FIG. 38F). In still other chimeric molecule embodiments, the fusion protein includes from amino-terminus to carboxy-terminus: an extracellular scFv or scTCR binding domain including a variable region linker containing a tag cassette disposed between the variable regions (*e*.*g*., at or closer to the N-terminal end of the variable region linker, at or closer to the C-terminal end of the variable region linker, or imbedded closer to the middle of the variable region linker), a connector region including a hinge, a hydrophobic portion, and an intracellular component including an effector domain. An exemplary tag cassette imbedded in a variable region linker includes GGSGSG(X)nWSHPQFEKGSGSG (SEQ ID NO:152), wherein X is optional, may be any amino acid and n is 0, 1, 2, 3, 4 or 5. In SEQ ID NO:130, such a variable region linker having an imbedded tag is present, wherein n is 1 and X is asparagine (N).

A chimeric molecule may be cell-bound (*e*.*g*.*,* expressed on a cell surface) or in soluble form. In certain embodiments, nucleic acid molecules encoding chimeric molecule fusion proteins may be codon optimized to enhance or maximize expression in certain types of cells, such as T cells (Scholten, et al., 2006, Clin. Immunol. 119:135).

In other embodiments, chimeric molecules may further comprise a cytotoxic component (*e.g.,* chemotherapeutic drugs such as anti-mitotics (*e.g.,* vindesine), antifolates, alkylating agents (*e.g.,* temozolomide), bacterial toxins, ricin, anti-virals, radioisotopes, radiometals), which is useful for specific killing or disabling a cancer cell, infected cell or other diseased cell. In further embodiments, chimeric molecules may further comprise a detectable component (*e.g.,* biotin, fluorescent moiety, radionuclide), which is useful for tracking or imaging cancer cells, infected cells, or other tissues (*e.g.,* tissue under autoimmune attack). In still further embodiments, chimeric molecules may further comprise a functional component (*e.g.,* an immunostimulatory moiety, cytokine, immune modulator, immunoglobulin protein, or the like).

Modified HSPC can additionally utilize positive and/or negative selection markers. For example, positive selectable markers may be encoded by a gene, which upon being introduced into the modified cell, expresses a dominant phenotype permitting positive selection of cells carrying the gene. Genes of this type include, hygromycin-B phosphotransferase gene (hph) which confers resistance to hygromycin B, the amino glycoside phosphotransferase gene (neo or aph) from Tn5 which codes for resistance to the antibiotic 0418, the dihydrofolate reductase (DHFR) gene, the adenosine deaminase gene (ADA), and the multi-drug resistance (MDR) gene.

In particular embodiments, functional genes can be introduced into the modified HSPC to allow for negative selection *in vivo.* "Negative selection" means that an administered cell can be eliminated as a result of a change in the *in vivo* condition of a subject. The negative selectable phenotype can result from the insertion of a gene that confers sensitivity to an administered agent. Negative selectable genes include: the Herpes simplex virus type I thymidine kinase (HSV-I TK) gene which confers ganciclovir sensitivity; the cellular hypoxanthine phosphribosyltransferase (HPRT) gene, the cellular adenine phosphoribosyltransferase (APRT) gene, and bacterial cytosine deaminase. For additional supporting disclosure regarding negative selection, see Lupton S.D., et al., 1991, Mol. and Cell Biol. 11:6; Riddell, et al., 1992, Human Gene Therapy 3:319-338; WO 1992/008796 and WO 1994/028143 and U.S. Patent No. 6,040,177 at columns 14-17).

Modified HSPC can be made recombinant by the introduction of a recombinant gene sequence into the HSPC. A description of genetically engineered HSPC can be found in sec. 5.1 of U.S. Patent No. 7,399,633. A gene whose expression is desired in the modified cell is introduced into the HSPC such that it is expressible by the cells and/or their progeny.

Desired genes can be introduced into HSPC by any method known in the art, including transfection, electroporation, microinjection, lipofection, calcium phosphate mediated transfection, infection with a viral or bacteriophage vector containing the gene sequences, cell fusion, chromosome-mediated gene transfer, microcell-mediated gene transfer, sheroplast fusion, etc. Numerous techniques are known in the art for the introduction of foreign genes into cells (*see e.g.,* Loeffler and Behr, 1993, Meth. Enzymol. 217:599-618; Cohen, et al., 1993, Meth. Enzymol. 217:618-644; Cline, 1985, Pharmac. Ther. 29:69-92) and may be used, provided that the necessary developmental and physiological functions of the recipient cells are not disrupted. The technique should provide for the stable transfer of the gene to the cell, so that the gene is expressible by the cell and preferably heritable and expressible by its cell progeny. As indicated, in particular embodiments, the method of transfer includes the transfer of a selectable tag cassette to the cells. The cells are then placed under selection to isolate those cells that have taken up and are expressing the transferred gene.

The term "gene" refers to a nucleic acid sequence (used interchangeably with polynucleotide or nucleotide sequence) that encodes a chimeric molecule as described herein. This definition includes various sequence polymorphisms, mutations, and/or sequence variants wherein such alterations do not substantially affect the function of the encoded chimeric molecule. The term "gene" may include not only coding sequences but also regulatory regions such as promoters, enhancers, and termination regions. The term further can include all introns and other DNA sequences spliced from the mRNA transcript, along with variants resulting from alternative splice sites. Gene sequences encoding the molecule can be DNA or RNA that directs the expression of the chimeric molecule. These nucleic acid sequences may be a DNA strand sequence that is transcribed into RNA or an RNA sequence that is translated into protein. The nucleic acid sequences include both the full-length nucleic acid sequences as well as non-full-length sequences derived from the full-length protein. The sequences can also include degenerate codons of the native sequence or sequences that may be introduced to provide codon preference in a specific cell type. Portions of complete gene sequences are referenced throughout the disclosure as is understood by one of ordinary skill in the art.

A gene sequence encoding a tag cassette, binding domain, effector domain, spacer region, linker sequence, hydrophobic portion, or any other protein or peptide sequence described herein can be readily prepared by synthetic or recombinant methods from the relevant amino acid sequence. In embodiments, the gene sequence encoding any of these sequences can also have one or more restriction enzyme sites at the 5' and/or 3' ends of the coding sequence in order to provide for easy excision and replacement of the gene sequence encoding the sequence with another gene sequence encoding a different sequence. In embodiments, the gene sequence encoding the sequences can be codon optimized for expression in mammalian cells.

"Encoding" refers to the property of specific sequences of nucleotides in a gene, such as a cDNA, or an mRNA, to serve as templates for synthesis of other macromolecules such as a defined sequences of amino acids. Thus, a gene codes for a protein if transcription and translation of mRNA corresponding to that gene produces the protein in a cell or other biological system. A "gene sequence encoding a protein" includes all nucleotide sequences that are degenerate versions of each other and that code for the same amino acid sequence or amino acid sequences of substantially similar form and function.

Polynucleotide gene sequences encoding more than one portion of an expressed chimeric molecule can be operably linked to each other and relevant regulatory sequences. For example, there can be a functional linkage between a regulatory sequence and an exogenous nucleic acid sequence resulting in expression of the latter. For another example, a first nucleic acid sequence can be operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary or helpful, join coding regions, into the same reading frame.

A "vector" is a nucleic acid molecule that is capable of transporting another nucleic acid. Vectors may be, for example, plasmids, cosmids, viruses, or phage. An "expression vector" is a vector that is capable of directing the expression of a protein encoded by one or more genes carried by the vector when it is present in the appropriate environment.

"Retroviruses" are viruses having an RNA genome. "Gammaretrovirus" refers to a genus of the retroviridae family. Exemplary gammaretroviruses include mouse stem cell virus, murine leukemia virus, feline leukemia virus, feline sarcoma virus, and avian reticuloendotheliosis viruses.

Retroviral vectors (*see* Miller, et al., 1993, Meth. Enzymol. 217:581-599) can be used. In such embodiments, the gene to be expressed is cloned into the retroviral vector for its delivery into HSPC. In particular embodiments, a retroviral vector contains all of the cis-acting sequences necessary for the packaging and integration of the viral genome, *i.e.,* (a) a long terminal repeat (LTR), or portions thereof, at each end of the vector; (b) primer binding sites for negative and positive strand DNA synthesis; and (c) a packaging signal, necessary for the incorporation of genomic RNA into virions. More detail about retroviral vectors can be found in Boesen, et al., 1994, Biotherapy 6:291-302; Clowes, et al., 1994, J. Clin. Invest. 93:644-651; Kiem, et al., 1994, Blood 83:1467-1473; Salmons and Gunzberg, 1993, Human Gene Therapy 4:129-141; and Grossman and Wilson, 1993, Curr. Opin. in Genetics and Devel. 3:110-114. Adenoviruses, adena-associated viruses (AAV) and alphaviruses can also be used. See Kozarsky and Wilson, 1993, Current Opinion in Genetics and Development 3:499-503, Rosenfeld, et a/., 1991, Science 252:431-434; Rosenfeld, et a/., 1992, Cell 68:143-155; Mastrangeli, et a/., 1993, J. Clin. Invest. 91:225-234; Walsh, et a/., 1993, Proc. Soc. Exp. Bioi. Med. 204:289-300; and Lundstrom, 1999, J. Recept. Signal Transduct. Res. 19: 673-686. Other methods of gene delivery include the use of mammalian artificial chromosomes (Vos, 1998, Curr. Op. Genet. Dev. 8:351-359); liposomes (Tarahovsky and Ivanitsky, 1998, Biochemistry (Mosc) 63:607-618); ribozymes (Branch and Klotman, 1998, Exp. Nephrol. 6:78-83); and triplex DNA (Chan and Glazer, 1997, J. Mol. Med. 75:267-282).

"Lentivirus" refers to a genus of retroviruses that are capable of infecting dividing and non-dividing cells. Several examples of lentiviruses include HIV (human immunodeficiency virus: including HIV type 1, and HIV type 2); equine infectious anemia virus; feline immunodeficiency virus (FIV); bovine immune deficiency virus (BIV); and simian immunodeficiency virus (SIV).

There are a large number of available viral vectors suitable within the current disclosure, including those identified for human gene therapy applications (see Pfeifer and Verma, 2001, Ann. Rev. Genomics Hum. Genet. 2:177). Suitable viral vectors include vectors based on RNA viruses, such as retrovirus-derived vectors, *e.g.,* Moloney murine leukemia virus (MLV)-derived vectors, and include more complex retrovirus-derived vectors, *e.g.,* lentivirus-derived vectors. HIV-1-derived vectors belong to this category. Other examples include lentivirus vectors derived from HIV-2, FIV, equine infectious anemia virus, SIV, and Maedi-Visna virus (ovine lentivirus). Methods of using retroviral and lentiviral viral vectors and packaging cells for transducing mammalian host cells with viral particles containing chimeric antigen receptor transgenes are described in, for example, U.S. Patent 8,119,772; Walchli, et a/., 2011, PLoS One 6:327930; Zhao, et a/., 2005, J. Immunol. 174:4415; Engels, et al., 2003, Hum. Gene Ther. 14:1155; Frecha, et a/., 2010, Mol. Ther. 18:1748; and Verhoeyen, et al., 2009, Methods Mol. Biol. 506:97. Retroviral and lentiviral vector constructs and expression systems are also commercially available.

"Nucleic acid molecules", or polynucleotides, may be in the form of RNA or DNA, which includes cDNA, genomic DNA, and synthetic DNA. A nucleic acid molecule may be double stranded or single stranded, and if single stranded, may be the coding strand or non-coding (anti-sense strand). A coding molecule may have a coding sequence identical to a coding sequence known in the art or may have a different coding sequence, which, as the result of the redundancy or degeneracy of the genetic code, or by splicing, can encode the same polypeptide.

Additional embodiments include sequences having 70% sequence identity; 80% sequence identity; 81 % sequence identity; 82% sequence identity; 83% sequence identity; 84% sequence identity; 85% sequence identity; 86% sequence identity; 87% sequence identity; 88% sequence identity; 89% sequence identity; 90% sequence identity; 91 % sequence identity; 92% sequence identity; 93% sequence identity; 94% sequence identity; 95% sequence identity; 96% sequence identity; 97% sequence identity; 98% sequence identity; or 99% sequence identity to any gene, protein or peptide sequence disclosed herein.

"% sequence identity" refers to a relationship between two or more sequences, as determined by comparing the sequences. In the art, "identity" also means the degree of sequence relatedness between protein sequences as determined by the match between strings of such sequences. "Identity" (often referred to as "similarity") can be readily calculated by known methods, including those described in: Computational Molecular Biology (Lesk, A. M., ed.) Oxford University Press, NY (1988); Biocomputing: Informatics and Genome Projects (Smith, D. W., ed.) Academic Press, NY (1994); Computer Analysis of Sequence Data, Part I (Griffin, A. M., and Griffin, H. G., eds.) Humana Press, NJ (1994); Sequence Analysis in Molecular Biology (Von Heijne, G., ed.) Academic Press (1987); and Sequence Analysis Primer (Gribskov, M. and Devereux, J., eds.) Oxford University Press, NY (1992). Preferred methods to determine sequence identity are designed to give the best match between the sequences tested. Methods to determine sequence identity and similarity are codified in publicly available computer programs. Sequence alignments and percent identity calculations may be performed using the Megalign program of the LASERGENE bioinformatics computing suite (DNASTAR, Inc., Madison, Wisconsin). Multiple alignment of the sequences can also be performed using the Clustal method of alignment (Higgins and Sharp CABIOS, 5, 151-153 (1989) with default parameters (GAP PENALTY=10, GAP LENGTH PENALTY=10). Relevant programs also include the GCG suite of programs (Wisconsin Package Version 9.0, Genetics Computer Group (GCG), Madison, Wisconsin); BLASTP, BLASTN, BLASTX (Altschul, et al., 1990, J. Mol. Biol. 215:403-410; DNASTAR^{®} (DNASTAR, Inc., Madison, Wisconsin); and the FASTA program incorporating the Smith-Waterman algorithm (Pearson, Comput. Methods Genome Res., [Proc. Int. Symp.] (1994), Meeting Date 1992, 111-20. Editor(s): Suhai, Sandor. Publisher: Plenum, New York, N.Y.. Within the context of this disclosure it will be understood that where sequence analysis software is used for analysis, the results of the analysis are based on the "default values" of the program referenced. "Default values" mean any set of values or parameters which originally load with the software when first initialized.

Without limiting the foregoing, proteins or peptides having a sequence identity to a sequence disclosed herein include variants and D-substituted analogs thereof.

"Variants" of sequences disclosed herein include sequences having one or more additions, deletions, stop positions, or substitutions, as compared to a sequence disclosed herein.

An amino acid substitution can be a conservative or a non-conservative substitution. Variants of protein or peptide sequences disclosed herein can include those having one or more conservative amino acid substitutions. A "conservative substitution" involves a substitution found in one of the following conservative substitutions groups: Group 1: alanine (Ala or A), glycine (Gly or G), Ser, Thr; Group 2: aspartic acid (Asp or D), Glu; Group 3: asparagine (Asn or N), glutamine (Gin or Q); Group 4: Arg, lysine (Lys or K), histidine (His or H); Group 5: lie, leucine (Leu or L), methionine (Met or M), valine (Val or V); and Group 6: Phe, Tyr, Trp.

Additionally, amino acids can be grouped into conservative substitution groups by similar function, chemical structure, or composition (*e.g.,* acidic, basic, aliphatic, aromatic, sulfur-containing). For example, an aliphatic grouping may include, for purposes of substitution, Gly, Ala, Val, Leu, and Ile. Other groups containing amino acids that are considered conservative substitutions for one another include: sulfur-containing: Met and Cys; acidic: Asp, Glu, Asn, and Gln; small aliphatic, nonpolar or slightly polar residues: Ala, Ser, Thr, Pro, and Gly; polar, negatively charged residues and their amides: Asp, Asn, Glu, and Gln; polar, positively charged residues: His, Arg, and Lys; large aliphatic, nonpolar residues: Met, Leu, Ile, Val, and Cys; and large aromatic residues: Phe, Tyr, and Trp. Additional information is found in Creighton (1984) Proteins, W.H. Freeman and Company.

"D-substituted analogs" include proteins or peptides disclosed herein having one more L-amino acids substituted with one or more D-amino acids. The D-amino acid can be the same amino acid type as that found in the reference sequence or can be a different amino acid. Accordingly, D-analogs can also be variants.

Without limiting the foregoing, and for exemplary purposes only:

In particular embodiments, a tag cassette includes a sequence that has at least 80%; 81%; 82%; 83%; 84%; 85%; 86%; 87%; 88%; 89%; 90%; 91%; 92%; 93%; 94%; 95%; 96%; 97%; 98%; or 99% sequence identity to the sequence of Strep tag, His tag, Flag tag, Xpress tag, Avi tag, Calmodulin tag, Polyglutamate tag, HA tag, Myc tag, Nus tag, S tag, X tag, SBP tag, Softag, V5 tag, CBP, GST, MBP, GFP, Thioredoxin tag

In particular embodiments, a binding domain includes a sequence that has at least 80%; 81%; 82%; 83%; 84%; 85%; 86%; 87%; 88%; 89%; 90%; 91%; 92%; 93%; 94%; 95%; 96%; 97%; 98%; or 99% sequence identity to an amino acid sequence of a light chain variable region (VL) or to a heavy chain variable region (VH) disclosed herein, or both, wherein each CDR includes zero changes or at most one, two, or three changes, from a monoclonal antibody or fragment thereof that specifically binds a cellular marker of interest.

"Specifically binds" refers to an association or union of a tag cassette or binding domain, or a fusion protein thereof, to a cognate binding molecule or cellular marker respectively, with an affinity or Kₐ (*i.e.,* an equilibrium association constant of a particular binding interaction with units of 1/M) equal to or greater than 10⁵ M⁻¹, while not significantly associating or uniting with any other molecules or components in a sample. Tag cassettes or binding domains (or fusion proteins thereof) may be classified as "high affinity" or "low affinity". "High affinity" tag cassette or binding domains refer to those tag cassette or binding domains with a Kₐ of at least 10⁷ M⁻¹, at least 10⁸ M⁻¹, at least 10⁹ M⁻¹, at least 10¹⁰ M⁻¹, at least 10¹¹ M⁻¹, at least 10¹² M⁻¹, or at least 10¹³ M⁻¹. "Low affinity" tag cassette or binding domains refer to those tag cassette or binding domains with a Kₐ of up to 10⁷ M⁻¹, up to 10⁶ M⁻¹, up to 10⁵ M⁻¹. Alternatively, affinity may be defined as an equilibrium dissociation constant (K_{d}) of a particular binding interaction with units of M (*e.g.,* 10⁻⁵ M to 10⁻¹³ M). In certain embodiments, a tag cassette or binding domain may have "enhanced affinity," which refers to a selected or engineered tag cassette or binding domain with stronger binding to a cognate binding molecule or cellular marker respectively, than a wild type (or parent) tag cassette or binding domain. For example, enhanced affinity may be due to a Ka (equilibrium association constant) for the cognate binding molecule or cellular marker respectively, cellular marker that is higher than the wild type tag cassette or binding domain, or due to a K_{d} (dissociation constant) for the cognate binding molecule or cellular marker respectively, that is less than that of the wild type tag cassette or binding domain, or due to an off-rate (K_{off}) for the cognate binding molecule or cellular marker respectively, that is less than that of the wild type tag cassette or binding domain. A variety of assays are known for identifying tag cassettes or binding domains that specifically bind a particular cognate binding molecule or cellular marker respectively, as well as determining tag cassette or binding domain or fusion protein affinities, such as Western blot, ELISA, and BIACORE^{®} analysis (*see* also, *e.g.,* Scatchard et al., 1949, Ann. N.Y. Acad. Sci. 51:660; and U.S. Patent Nos. 5,283,173, 5,468,614, or the equivalent).

In particular embodiments, binding domains include a sequence that has at least 80%; 81%; 82%; 83%; 84%; 85%; 86%; 87%; 88%; 89%; 90%; 91%; 92%; 93%; 94%; 95%; 96%; 97%; 98%; or 99% sequence identity to an amino acid sequence of a TCR Vα, Vβ, Cα, or Cβ, wherein each CDR includes zero changes or at most one, two, or three changes, from a TCR or fragment or thereof that specifically binds to a cellular marker of interest.

In particular embodiments, the binding domain Vα, Vβ, Cα, or Cβ region can be derived from or based on a Vα, Vβ, Cα, or Cβ of a known TCR (*e.g.,* a high-affinity TCR) and contain one or more (*e.g.,* 2, 3, 4, 5, 6, 7, 8, 9, 10) insertions, one or more (*e.g.,* 2, 3, 4, 5, 6, 7, 8, 9, 10) deletions, one or more (e.g., 2, 3, 4, 5, 6, 7, 8, 9, 10) amino acid substitutions (e.g., conservative amino acid substitutions or non-conservative amino acid substitutions), or a combination of the above-noted changes, when compared with the Vα, Vβ, Cα, or Cβ of a known TCR. An insertion, deletion or substitution may be anywhere in a Vα, Vβ, Cα, or Cβ region, including at the amino- or carboxy-terminus or both ends of these regions, provided that each CDR includes zero changes or at most one, two, or three changes and provided a binding domain containing a modified Vα, Vβ, Cα, or Cβ region can still specifically bind its target cellular marker with an affinity similar to the wild type.

In particular embodiments, a binding domain VH or VL region can be derived from or based on a VH or VL of a known monoclonal antibody and can individually or collectively contain one or more (*e.g.,* 2, 3, 4, 5, 6, 7, 8, 9, 10) insertions, one or more (*e.g.,* 2, 3, 4, 5, 6, 7, 8, 9, 10) deletions, one or more (e.g., 2, 3, 4, 5, 6, 7, 8, 9, 10) amino acid substitutions (*e.g.,* conservative amino acid substitutions or non-conservative amino acid substitutions), or a combination of the above-noted changes, when compared with the VH or VL of a known monoclonal antibody. An insertion, deletion or substitution may be anywhere in the VH or VL region, including at the amino- or carboxy-terminus or both ends of these regions, provided that each CDR includes zero changes or at most one, two, or three changes and provided a binding domain containing the modified VH or VL region can still specifically bind its target cellular marker with an affinity similar to the wild type binding domain.

In particular embodiments, a binding domain includes a sequence that has at least 80%; 81%; 82%; 83%; 84%; 85%; 86%; 87%; 88%; 89%; 90%; 91%; 92%; 93%; 94%; 95%; 96%; 97%; 98%; or 99% sequence identity to that of the (i) scFv for FMC63 (ii) scFv for R12; (iii) scFv for R11; or (iv) scFv for Herceptin.

In particular embodiments, an intracellular signaling domain can have at least 80%; 81%; 82%; 83%; 84%; 85%; 86%; 87%; 88%; 89%; 90%; 91%; 92%; 93%; 94%; 95%; 96%; 97%; 98%; or 99% sequence identity a to CD3ζ having a sequence provided in FIG. 2.

In particular embodiments, a costimulatory signaling domain can have at least 80%; 81%; 82%; 83%; 84%; 85%; 86%; 87%; 88%; 89%; 90%; 91%; 92%; 93%; 94%; 95%; 96%; 97%; 98%; or 99% sequence identity to the intracellular domain of CD28 as shown in FIG. 5 or to 4-1 BB having a sequence provided in FIG. 2. In particular embodiments, a variant of the CD28 intracellular domain includes an amino acid substitution at positions 186-187, wherein LL is substituted with GG.

In particular embodiments, a transmembrane domain can be selected or modified by an amino acid substitution(s) to avoid binding of such domains to the transmembrane domains of the same or different surface membrane proteins to minimize interactions with other members of the receptor complex. In further particular embodiments, synthetic or variant transmembrane domains include predominantly hydrophobic residues such as leucine and valine. Variant transmembrane domains preferably have a hydrophobic score of at least 50 as calculated by Kyte Doolittle. In particular embodiments, a transmembrane domain can have at least 80%; 81%; 82%; 83%; 84%; 85%; 86%; 87%; 88%; 89%; 90%; 91%; 92%; 93%; 94%; 95%; 96%; 97%; 98%; or 99% sequence identity with a sequence of FIG. 2 or 6.

Proteins and peptides having the same functional capability as those expressly disclosed herein are also included.

When not expressly provided here, sequence information provided by public databases and the knowledge of those of ordinary skill in the art can be used to identify related and relevant protein and peptide sequences and gene sequences encoding such proteins and peptides.

Differentiation. In particular embodiments, modified HSPC are differentiated into modified non-T effector cells before administration to a subject. Where differentiation of modified HSPC is desired, HSPC can be exposed to one or more growth factors that promote differentiation into non-T effector cells. The growth factors and cell culture conditions that promote differentiation are known in the art (*see*, *e.g.,* U.S. Patent No. 7,399,633 at Section 5.2 and Section 5.5). For example, SCF can be used in combination with GM-SCF or IL-7 to differentiate HSPC into myeloid stem/progenitor cells or lymphoid stem/progenitor cells, respectively. In particular embodiments, HSPC can be differentiated into a lymphoid stem/progenitor cell by exposing HSPC to 100 ng/ml of each of SCF and GM-SCF or IL-7. In particular embodiments, a retinoic acid receptor (RAR) agonist, or preferably all trans retinoic acid (ATRA) is used to promote the differentiation of HSPC. Differentiation into natural killer cells, for example, can be achieved by exposing cultured HSPC to RPMI media supplemented with human serum, IL-2 at 50 U/mL and IL-15 at 500ng/mL. In additional embodiments, RPMI media can also be supplemented L-glutamine.

In particular embodiments, modified HSPC can be differentiated into non-T effector cells including natural killer (NK) cells or neutrophils. NK cells perform two major functions: (i) recognizing and killing tumor cells and other virally infected cells; and (ii) regulating innate and adaptive immune responses by secreting CCL3, CCL4, CCL5, and/or XCL1 chemokines or cytokines such as granulocyte-macrophage colony-stimulating factor, tumor necrosis factor-a, or IFN-γ. Neutrophils generally circulate in the blood stream until they travel to sites of inflammation where they target and destroy aberrant cell types.

Compositions and Formulations. Cells and modified cells can be prepared as compositions and/or formulations for administration to a subject. A composition refers to a cell or modified cell prepared with a pharmaceutically acceptable carrier for administration to a subject. A formulation refers to at least two cell types within a pharmaceutically acceptable carrier (hereafter carrier) for administration to a subject.

At various points during preparation of a composition or formulation, it can be necessary or beneficial to cryopreserve a cell. The terms "frozen/freezing" and "cryopreserved/cryopreserving" can be used interchangeably. Freezing includes freeze drying.

As is understood by one of ordinary skill in the art, the freezing of cells can be destructive (see Mazur, P., 1977, Cryobiology 14:251-272) but there are numerous procedures available to prevent such damage. For example, damage can be avoided by (a) use of a cryoprotective agent, (b) control of the freezing rate, and/or (c) storage at a temperature sufficiently low to minimize degradative reactions. Exemplary cryoprotective agents include dimethyl sulfoxide (DMSO) (Lovelock and Bishop, 1959, Nature 183:1394-1395; Ashwood-Smith, 1961, Nature 190:1204-1205), glycerol, polyvinylpyrrolidine (Rinfret, 1960, Ann. N.Y. Acad. Sci. 85:576), polyethylene glycol (Sloviter and Ravdin, 1962, Nature 196:548), albumin, dextran, sucrose, ethylene glycol, i-erythritol, D-ribitol, D-mannitol (Rowe, et a/., 1962, Fed. Proc. 21:157), D-sorbitol, i-inositol, D-lactose, choline chloride (Bender, et a/., 1960, J. Appl. Physiol. 15:520), amino acids (Phan The Tran and Bender, 1960, Exp. Cell Res. 20:651), methanol, acetamide, glycerol monoacetate (Lovelock, 1954, Biochem. J. 56:265), and inorganic salts (Phan The Tran and Bender, 1960, Proc. Soc. Exp. Biol. Med. 104:388; Phan The Tran and Bender, 1961, in Radiobiology, Proceedings of the Third Australian Conference on Radiobiology, IIbery ed., Butterworth, London, p. 59). In particular embodiments, DMSO can be used. Addition of plasma (*e.g.,* to a concentration of 20-25%) can augment the protective effects of DMSO. After addition of DMSO, cells can be kept at 0° C until freezing, because DMSO concentrations of 1% can be toxic at temperatures above 4° C.

In the cryopreservation of cells, slow controlled cooling rates can be critical and different cryoprotective agents (Rapatz, et a/., 1968, Cryobiology 5(1): 18-25) and different cell types have different optimal cooling rates (*see e.g.,* Rowe and Rinfret, 1962, Blood 20:636; Rowe, 1966, Cryobiology 3(1):12-18; Lewis, et a/., 1967, Transfusion 7(1):17-32; and Mazur, 1970, Science 168:939- 949 for effects of cooling velocity on survival of stem cells and on their transplantation potential). The heat of fusion phase where water turns to ice should be minimal. The cooling procedure can be carried out by use of, *e.g.,* a programmable freezing device or a methanol bath procedure. Programmable freezing apparatuses allow determination of optimal cooling rates and facilitate standard reproducible cooling.

In particular embodiments, DMSO-treated cells can be pre-cooled on ice and transferred to a tray containing chilled methanol which is placed, in turn, in a mechanical refrigerator (*e.g.,* Harris or Revco) at -80° C. Thermocouple measurements of the methanol bath and the samples indicate a cooling rate of 1° to 3° C/minute can be preferred. After at least two hours, the specimens can have reached a temperature of -80° C and can be placed directly into liquid nitrogen (-196° C).

After thorough freezing, the cells can be rapidly transferred to a long-term cryogenic storage vessel. In a preferred embodiment, samples can be cryogenically stored in liquid nitrogen (-196° C) or vapor (-1° C). Such storage is facilitated by the availability of highly efficient liquid nitrogen refrigerators.

Further considerations and procedures for the manipulation, cryopreservation, and long-term storage of cells, can be found in the following exemplary references: U.S. Patent Nos. 4,199,022; 3,753,357; and 4,559,298; Gorin, 1986, Clinics In Haematology 15(1): 19-48; Bone-Marrow Conservation, Culture and Transplantation, Proceedings of a Panel, Moscow, July 22-26, 1968, International Atomic Energy Agency, Vienna, pp. 107-186; Livesey and Linner, 1987, Nature 327:255; Linner, et al., 1986, J. Histochem. Cytochem. 34(9):1123-1135; Simione, 1992, J. Parenter. Sci. Technol. 46(6):226-32).

Following cryopreservation, frozen cells can be thawed for use in accordance with methods known to those of ordinary skill in the art. Frozen cells are preferably thawed quickly and chilled immediately upon thawing. In particular embodiments, the vial containing the frozen cells can be immersed up to its neck in a warm water bath; gentle rotation will ensure mixing of the cell suspension as it thaws and increase heat transfer from the warm water to the internal ice mass. As soon as the ice has completely melted, the vial can be immediately placed on ice.

In particular embodiments, methods can be used to prevent cellular clumping during thawing. Exemplary methods include: the addition before and/or after freezing of DNase (Spitzer, et a/., 1980, Cancer 45:3075-3085), low molecular weight dextran and citrate, hydroxyethyl starch (Stiff, et al., 1983, Cryobiology 20:17-24), etc.

As is understood by one of ordinary skill in the art, if a cryoprotective agent that is toxic to humans is used, it should be removed prior to therapeutic use. DMSO has no serious toxicity.

Exemplary carriers and modes of administration of cells are described at pages 14-15 of U.S. Patent Publication No. 2010/0183564. Additional pharmaceutical carriers are described in Remington: The Science and Practice of Pharmacy, 21st Edition, David B. Troy, ed., Lippicott Williams & Wilkins (2005).

In particular embodiments, cells can be harvested from a culture medium, and washed and concentrated into a carrier in a therapeutically-effective amount. Exemplary carriers include saline, buffered saline, physiological saline, water, Hanks' solution, Ringer's solution, Nonnosol-R (Abbott Labs), PLASMA-LYTE A^{®} (Baxter Laboratories, Inc., Morton Grove, IL), glycerol, ethanol, and combinations thereof.

In particular embodiments, carriers can be supplemented with human serum albumin (HSA) or other human serum components or fetal bovine serum. In particular embodiments, a carrier for infusion includes buffered saline with 5% HAS or dextrose. Additional isotonic agents include polyhydric sugar alcohols including trihydric or higher sugar alcohols, such as glycerin, erythritol, arabitol, xylitol, sorbitol, or mannitol.

Carriers can include buffering agents, such as citrate buffers, succinate buffers, tartrate buffers, fumarate buffers, gluconate buffers, oxalate buffers, lactate buffers, acetate buffers, phosphate buffers, histidine buffers, and/or trimethylamine salts.

Stabilizers refer to a broad category of excipients which can range in function from a bulking agent to an additive which helps to prevent cell adherence to container walls. Typical stabilizers can include polyhydric sugar alcohols; amino acids, such as arginine, lysine, glycine, glutamine, asparagine, histidine, alanine, ornithine, L-leucine, 2-phenylalanine, glutamic acid, and threonine; organic sugars or sugar alcohols, such as lactose, trehalose, stachyose, mannitol, sorbitol, xylitol, ribitol, myoinisitol, galactitol, glycerol, and cyclitols, such as inositol; PEG; amino acid polymers; sulfur-containing reducing agents, such as urea, glutathione, thioctic acid, sodium thioglycolate, thioglycerol, alpha-monothioglycerol, and sodium thiosulfate; low molecular weight polypeptides (*i.e.,* <10 residues); proteins such as HSA, bovine serum albumin, gelatin or immunoglobulins; hydrophilic polymers such as polyvinylpyrrolidone; monosaccharides such as xylose, mannose, fructose and glucose; disaccharides such as lactose, maltose and sucrose; trisaccharides such as raffinose, and polysaccharides such as dextran.

Where necessary or beneficial, compositions or formulations can include a local anesthetic such as lidocaine to ease pain at a site of injection.

Exemplary preservatives include phenol, benzyl alcohol, meta-cresol, methyl paraben, propyl paraben, octadecyldimethylbenzyl ammonium chloride, benzalkonium halides, hexamethonium chloride, alkyl parabens such as methyl or propyl paraben, catechol, resorcinol, cyclohexanol, and 3-pentanol.

Therapeutically effective amounts of cells within compositions or formulations can be greater than 10² cells, greater than 10³ cells, greater than 10⁴ cells, greater than 10⁵ cells, greater than 10⁶ cells, greater than 10⁷ cells, greater than 10⁸ cells, greater than 10⁹ cells, greater than 10¹⁰ cells, or greater than 10¹¹.

In compositions and formulations disclosed herein, cells are generally in a volume of a liter or less, 500 mls or less, 250 mls or less or 100 mls or less. Hence the density of administered cells is typically greater than 10⁴ cells/ml, 10⁷ cells/ml or 10⁸ cells/ml.

As indicated, compositions include one cell type (e.g., modified HSPC or modified effectors). Formulations can include HSPC, modified-HSPC and/or modified-effectors (such as modified-NK cells) in combination. In particular embodiments, combinations of modified-HSPC and modified-effectors with the same binding domain are combined. In other embodiments, modified-HSPC and modified-effectors of different binding domains are combined. Similarly, all other aspects of an expressed chimeric molecule (e.g., tag cassettes, effector domain components, spacer regions, etc.) can be the same or different in various combinations between modified HSPC and modified effectors within a formulation. Additionally, modified HSPC expressing different chimeric molecules or components thereof can be included together within a formulation and modified effectors expressing different chimeric molecules or components thereof can be included together within a formulation. In particular embodiments, a formulation can include at least two modified HSPC expressing different chimeric molecules and at least two modified effector cells expressing different chimeric molecules.

HSPC, modified-HSPC and modified-effectors can be combined in different ratios for example, a 1:1:1 ratio, 2:1:1 ratio, 1:2:1 ratio, 1:1:2 ratio, 5:1:1 ratio, 1:5:1 ratio, 1:1:5 ratio, 10:1:1 ratio, 1:10:1 ratio, 1:1:10 ratio, 2:2:1 ratio, 1:2:2 ratio, 2:1:2 ratio, 5:5:1 ratio, 1:5:5 ratio, 5:1:5 ratio, 10:10:1 ratio, 1:10:10 ratio, 10:1:10 ratio, etc. These ratios can also apply to numbers of cells expressing the same or different chimeric molecule components. If only two of the cell types are combined or only 2 combinations of expressed chimeric molecule components are included within a formulation, the ratio can include any 2 number combination that can be created from the 3 number combinations provided above. In embodiments, the combined cell populations are tested for efficacy and/or cell proliferation *in vitro, in vivo* and/or ex *vivo,* and the ratio of cells that provides for efficacy and/or proliferation of cells is selected.

The compositions and formulations disclosed herein can be prepared for administration by, for example, injection, infusion, perfusion, or lavage. The compositions and formulations can further be formulated for bone marrow, intravenous, intradermal, intraarterial, intranodal, intralymphatic, intraperitoneal, intralesional, intraprostatic, intravaginal, intrarectal, topical, intrathecal, intratumoral, intramuscular, intravesicular, and/or subcutaneous injection.

Kits. Kits can include one or more containers including one or more of the cells, compositions or formulations described herein. In particular embodiments, the kits can include one or more containers containing one or more cells, compositions or formulations and/or compositions to be used in combination with other cells, compositions or formulations. Associated with such container(s) can be a notice in the form prescribed by a governmental agency regulating the manufacture, use, or sale of pharmaceuticals or biological products, which notice reflects approval by the agency of manufacture, use, or sale for human administration. The notice may state that the provided cells, compositions or formulations can be administered to a subject without immunological matching. The kits can include further instructions for using the kit, for example, instructions regarding preparation of cells, compositions and/or formulations for administration; proper disposal of related waste; and the like. The instructions can be in the form of printed instructions provided within the kit or the instructions can be printed on a portion of the kit itself. Instructions may be in the form of a sheet, pamphlet, brochure, CD-Rom, or computer-readable device, or can provide directions to instructions at a remote location, such as a website. In particular embodiments, kits can also include some or all of the necessary medical supplies needed to use the kit effectively, such as syringes, ampules, tubing, facemask, a needleless fluid transfer device, an injection cap, sponges, sterile adhesive strips, Chloraprep, gloves, and the like. Variations in contents of any of the kits described herein can be made.

Methods of Use. Methods disclosed herein include treating subjects (humans, veterinary animals (dogs, cats, reptiles, birds, etc.), livestock (horses, cattle, goats, pigs, chickens, etc.), and research animals (monkeys, rats, mice, fish, etc.) with cells disclosed herein. Treating subjects includes delivering therapeutically effective amounts. Therapeutically effective amounts include those that provide effective amounts, prophylactic treatments, and/or therapeutic treatments.

An "effective amount" is the number of cells necessary to result in a desired physiological change in a subject. Effective amounts are often administered for research purposes. Effective amounts disclosed herein do one or more of: (i) provide blood support by reducing immunodeficiency, pancytopenia, neutropenia and/or leukopenia (*e.g*., repopulating cells of the immune system and (ii) have an anti-cancer effect.

A "prophylactic treatment" includes a treatment administered to a subject who does not display signs or symptoms of a condition to be treated or displays only early signs or symptoms of the condition to be treated such that treatment is administered for the purpose of diminishing, preventing, or decreasing the risk of developing the condition. Thus, a prophylactic treatment functions as a preventative treatment against a condition.

A "therapeutic treatment" includes a treatment administered to a subject who displays symptoms or signs of a condition and is administered to the subject for the purpose of reducing the severity or progression of the condition.

**The** actual dose amount administered to a particular subject can be determined by a physician, veterinarian, or researcher taking into account parameters such as physical and physiological factors including cellular marker; body weight; type of condition; severity of condition; upcoming relevant events, when known; previous or concurrent therapeutic interventions; idiopathy of the subject; and route of administration, for example. In addition, *in vitro, in vivo* and/or *ex vivo* assays can optionally be employed to help identify optimal dosage ranges.

Therapeutically effective amounts to administer can include greater than 10² cells, greater than 10³ cells, greater than 10⁴ cells, greater than 10⁵ cells, greater than 10⁶ cells, greater than 10⁷ cells, greater than 10⁸ cells, greater than 10⁹ cells, greater than 10¹⁰ cells, or greater than 10¹¹.

As indicated, the compositions and formulations disclosed herein can be administered by, for example, injection, infusion, perfusion, or lavage and can more particularly include administration through one or more bone marrow, intravenous, intradermal, intraarterial, intranodal, intralymphatic, intraperitoneal, intralesional, intraprostatic, intravaginal, intrarectal, topical, intrathecal, intratumoral, intramuscular, intravesicular, and/or subcutaneous infusions and/or bolus injections.

Uses of non-modified HSPC are described in sec. 5.6.1 of U.S. Patent No. 7,399,633 and WO 2013/086436. HSPC and modified HSPC can be administered for the same purposes or different purposes. Common purposes include to provide hematopoietic function to a subject in need thereof; and/or to treat one or more of immunodeficiency, pancytopenia, neutropenia and/or leukopenia (including cyclic neutropenia and idiopathic neutropenia) (collectively, "the purposes"). HSPC and modified HSPC can be administered to subjects who have a decreased blood cell level, or are at risk of developing a decreased blood cell level as compared to a control blood cell level. In particular embodiments, the subject has anemia or is at risk for developing anemia.

Treatment for the purposes can be needed based on exposure to an intensive chemotherapy regimen including exposure to one or more of alkylating agents, Ara-C, azathioprine, carboplatin, cisplatin, chlorambucil, clofarabine, cyclophosphamide, ifosfamide, mechlorethamine, mercaptopurine, oxaliplatin, taxanes, and vinca alkaloids (*e.g.,* vincristine, vinblastine, vinorelbine, and vindesine).

Treatment for the purposes can also be needed based on exposure to a myeloablative regimen for hematopoietic cell transplantation (HCT). In particular embodiments, HSPC and/or modified-HSPC are administered to a bone marrow donor, at risk of depleted bone marrow, or at risk for depleted or limited blood cell levels. Administration can occur prior to and/or after harvesting of the bone marrow. HSPC and/or modified-HSPC can also be administered to a recipient of a bone marrow transplant.

Treatment for the purposes can also be needed based on exposure to acute ionizing radiation and/or exposure to other drugs that can cause bone marrow suppression or hematopoietic deficiencies including antibiotics, penicillin, gancyclovir, daunomycin, sulfa drugs, phenothiazones, tranquilizers, meprobamate, analgesics, aminopyrine, dipyrone, anticonvulsants, phenytoin, carbamazepine, antithyroids, propylthiouracil, methimazole, and diuretics.

Treatment for the purposes can also be needed based on viral (e.g., HIVI, HIVII, HTLVI, HTLVII, HTLVIII), microbial or parasitic infections and/or as a result of treatment for renal disease or renal failure, *e.g.,* dialysis. Various immunodeficiencies, *e.g.,* in T and/or B lymphocytes, or immune disorders, *e.g.,* rheumatoid arthritis, may also be beneficially affected by treatment with HSPC and/or modified-HSPC. Immunodeficiencies may also be the result of other medical treatments.

HSPC and modified-HSPC can also be used to treat aplastic anemia, Chediak-Higashi syndrome, systemic lupus erythematosus (SLE), leukemia, myelodysplastic syndrome, myelofibrosis or thrombocytopenia. Severe thrombocytopenia may result from genetic defects such as Fanconi's Anemia, Wiscott- Aldrich, or May-Hegglin syndromes. Acquired thrombocytopenia may result from auto- or allo-antibodies as in Immune Thrombocytopenia Purpura, Systemic Lupus Erythromatosis, hemolytic anemia, or fetal maternal incompatibility. In addition, splenomegaly, disseminated intravascular coagulation, thrombotic thrombocytopenic purpura, infection, and/or prosthetic heart valves may result in thrombocytopenia. Thrombocytopenia may also result from marrow invasion by carcinoma, lymphoma, leukemia or fibrosis.

In particular embodiments, the subject has blood loss due to, *e.g.,* trauma, or is at risk for blood loss. In particular embodiments, the subject has depleted bone marrow related to, *e.g.,* congenital, genetic or acquired syndrome characterized by bone marrow loss or depleted bone marrow. In particular embodiments, the subject is in need of hematopoiesis.

As indicated in relation to bone marrow donors, administration of HSPC or modified-HSPC to a subject can occur at any time within a treatment regimen deemed helpful by an administering professional. As non-limiting examples, HSPC and/or modified-HSPC can be administered to a subject, e.g., before, at the same time, or after chemotherapy, radiation therapy or a bone marrow transplant. HSPC and/or modified -HSPC can be effective to provide engraftment when assayed at 1, 2, 3, 4, 5, 6, 7, 8, 9, 10 days (or more or less than 1, 2, 3, 4, 5, 6, 7, 8, 9, 10 days); 1, 2, 3, 4, 5, 6, 7, 8, 9, 10 weeks (or more or less than 1, 2, 3, 4, 5, 6, 7, 8, 9, 10 weeks); 1; 2, 3, 4, 5, 6, 7, 8, 9, 10, 11, 12 months (or more or less than 1, 2, 3, 4, 5, 6, 7, 8, 9, 10, 11, 12 months); or 1, 2, 3, 4, 5 years (or more or less than 1, 2, 3, 4, 5 years) after administration of the HSPC and/or modified-HSPC to a subject. In particular embodiments, the HSPC and/or modified-HSPC are effective to provide engraftment when assayed within 10 days, 2 weeks, 3 weeks, 4 weeks, 6 weeks, or 13 weeks after administration of the HSPC and/or CAR-HSPC to a subject.

HSPC, Modified-HSPC and Modified Effectors. HSPC, modified-HSPC and modified-effectors can be administered for different purposes within a treatment regimen. The use of HSPC and modified HSPC to provide blood support, and modified HSPC and modified effectors to provide a graft vs. leukemia effect in the treatment of ALL is described above. Similar approaches can be used to provide blood support and/or to target unwanted cancer cells and as an adjunct treatment to chemotherapy or radiation.

Exemplary cancers that can be treated with modified HSPC and modified effectors include adrenal cancers, bladder cancers, blood cancers, bone cancers, brain cancers, breast cancers, carcinoma, cervical cancers, colon cancers, colorectal cancers, corpus uterine cancers, ear, nose and throat (ENT) cancers, endometrial cancers, esophageal cancers, gastrointestinal cancers, head and neck cancers, Hodgkin's disease, intestinal cancers, kidney cancers, larynx cancers, leukemias, liver cancers, lymph node cancers, lymphomas, lung cancers, melanomas, mesothelioma, myelomas, nasopharynx cancers, neuroblastomas, non-Hodgkin's lymphoma, oral cancers, ovarian cancers, pancreatic cancers, penile cancers, pharynx cancers, prostate cancers, rectal cancers, sarcoma, seminomas, skin cancers, stomach cancers, teratomas, testicular cancers, thyroid cancers, uterine cancers, vaginal cancers, vascular tumors, and metastases thereof.

In the context of cancers, therapeutically effective amounts have an anti-cancer effect. An anti-cancer effect can be quantified by observing a decrease in the number of tumor cells, a decrease in the number of metastases, a decrease in tumor volume, an increase in life expectancy, induction of apoptosis of cancer cells, induction of cancer cell death, inhibition of cancer cell proliferation, inhibition of tumor growth, prevention of metastasis, prolongation of a subject's life, and/or reduction of relapse or re-occurrence of the cancer following treatment.

In the context of blood support, therapeutically effective amounts treat immunodeficiency, pancytopenia, neutropenia and/or leukopenia by increasing the number of desired cells in a subject's circulation. Increasing the desired number of cells in a subject's circulation can re-populate the subject's immune system by increasing the number of immune system cells and/or immune system cell progenitors.

In particular embodiments utilizing modified-HSPC and modified-effectors, a subject's cancer cells can be characterized for presence of cellular markers. The binding domain expressed by a modified-HSPC or modified-effector can be selected based on the characterization of the cellular marker. In particular embodiments, modified-HSPC and modified-effectors previously generated are selected for a subject's treatment based on their ability to bind a cellular marker preferentially expressed on a particular subject's cancer cells.

When formulated to treat cancer, the disclosed compositions and formulations can also include plasmid DNA carrying one or more anticancer genes selected from p53, RB, BRCA1, E1A, bcl-2, MDR-1, p21, p16, bax, bcl-xs, E2F, IGF-I VEGF, angiostatin, oncostatin, endostatin, GM-CSF, IL-12, IL-2, IL-4, IL-7, IFN-γ, TNF-α and/or HSV-tk. Compositions and formulations can also include or be administered in combination with one or more antineoplastic drugs including adriamycin, angiostatin, azathioprine, bleomycin, busulfane, camptothecin, carboplatin, carmustine, chlorambucile, chlormethamine, chloroquinoxaline sulfonamide, cisplatin, cyclophosphamide, cycloplatam, cytarabine, dacarbazine, dactinomycin, daunorubicin, didox, doxorubicin, endostatin, enloplatin, estramustine, etoposide, extramustinephosphat, flucytosine, fluorodeoxyuridine, fluorouracil, gallium nitrate, hydroxyurea, idoxuridine, interferons, interleukins, leuprolide, lobaplatin, lomustine, mannomustine, mechlorethamine, mechlorethaminoxide, melphalan, mercaptopurine, methotrexate, mithramycin, mitobronitole, mitomycin, mycophenolic acid, nocodazole, oncostatin, oxaliplatin, paclitaxel, pentamustine, platinum-triamine complex, plicamycin, prednisolone, prednisone, procarbazine, protein kinase C inhibitors, puromycine, semustine, signal transduction inhibitors, spiroplatin, streptozotocine, stromelysin inhibitors, taxol, tegafur, telomerase inhibitors, teniposide, thalidomide, thiamiprine, thioguanine, thiotepa, tiamiprine, tretamine, triaziquone, trifosfamide, tyrosine kinase inhibitors, uramustine, vidarabine, vinblastine, vinca alcaloids, vincristine, vindesine, vorozole, zeniplatin, zeniplatin or zinostatin.

Modified-HSPC and Modified Effectors. Modified-HSPC and/or modified-effectors can be used without HSPC when a treatment to provide hematopoietic function or to treat immunodeficiency; pancytopenia; neutropenia and/or leukopenia is not desired or needed.

As is understood by one of ordinary skill in the art, animal models of different blood disorders and cancers are well known and can be used to assess effectiveness of particular treatment paradigms, as necessary or beneficial.

In certain embodiments, the present disclosure provides a method for selectively activating a modified cell (*e.g.,* a modified stem cell or non-T effector cell) by contacting a modified cell expressing a chimeric molecule with a cognate binding molecule specific for a tag cassette and attached to a solid surface or as part of a biocompatible matrix (*e.g.,* alginate, basement membrane matrix (MATRIGEL^{®}), biopolymer). For example, a modified cell expressing a chimeric molecule may be activated with beads coated or conjugated with a cognate binding molecule (*e.g.,* antibody) specific for the tag cassette. For example, if the tag cassette is a Strep tag, then StrepTactin coated beads or anti-Strep tag antibody conjugated beads can be used to induce modified cell activation. In certain embodiments, the method includes activating *in vitro* or ex *vivo* modified cells expressing a chimeric molecule of this disclosure and is optionally further expressing a chimeric antigen receptor (CAR). Such activated modified cells are useful in the disease treatment methods described herein.

In another aspect, the present disclosure provides a method for selectively promoting proliferation of a modified stem cell expressing a chimeric molecule of this disclosure. In certain embodiments, the method includes selective *in vitro* or ex *vivo* proliferation of modified cells expressing a chimeric molecule using a tag binding partner, such as an antibody. In further embodiments, the method includes expanding modified cells with a tag binding partner. In certain embodiments, anti-tag binding partners may be used to activate a chimeric molecule (*e.g.,* a Wnt or Notch chimeric molecule) transduced hematopoietic stem cell, embryonic stem cell, or tissue stem cell (*e.g.,* neural stem cell) to self-renew, proliferate or differentiate into one or more desired phenotype for therapeutic use.

In still further embodiments, a chimeric molecule allows for selective promotion of modified cell proliferation *in vivo* when expressing a chimeric molecule of this disclosure. In certain embodiments, a modified cell expressing a CAR including a tag cassette allows for expansion of the CAR cells *in vivo* when contacting cells expressing a ligand. Such expanded modified cells are useful in the disease treatment methods described herein. In certain embodiments, proliferation or expansion of cells expressing a chimeric molecule as disclosed herein is induced *in vivo,* which may be induced with a tag cassette binding partner (such as an anti-tag antibody).

As indicated, the modified cells disclosed herein also have important uses in manufacturing and/or as research tools. With regard to uses as research tools, the modified cells can be administered and tracked. In particular embodiments, the modified cells can be tracked following in vivo activation. The effect of depleting or eliminating the cells at various time points following administration can also be assessed. These examples are just a small subset of potential research uses of the modified cells disclosed herein.

Exemplary Embodiments.
1. A hematopoietic stem progenitor cell (HSPC) or non-T effector cell genetically modified to express a chimeric molecule including an extracellular component including a tag cassette that specifically binds an exogenous cognate binding molecule (ExoCBM).
2. A HSPC or non-T effector cell of embodiment 1 wherein the extracellular component has one, two, three, four or five tag cassettes.
3. A HSPC or non-T effector cell of embodiments 1 or 2 wherein at least one tag cassette is or includes a Strep tag, His tag, Flag tag, Xpress tag, Avi tag, Calmodulin tag, Polyglutamate tag, HA tag, Myc tag, Nus tag, S tag, X tag, SBP tag, Softag, V5 tag, CBP, GST, MBP, GFP, Thioredoxin tag, or any combination thereof.
4. A HSPC or non-T effector cell of embodiments 1-3 wherein at least one tag cassette is or includes a Strep tag including the amino acid sequence Trp-Ser-His-Pro-Gln-Phe-Glu-Lys (SEQ ID NO:118) or Trp-Arg-His-Pro-Gln-Phe-Gly-Gly (SEQ ID NO:137).
5. A HSPC or non-T effector cell of embodiment of embodiments 1-4 wherein the extracellular component is linked to an intracellular component through a hydrophobic portion.
6. A HSPC or non-T effector cell of embodiment 5 wherein the extracellular component includes (i) a binding domain that specifically binds a cellular marker, and (ii) a hinge; and wherein the intracellular component includes an effector domain.
7. A HSPC or non-T effector cell of embodiments 5 or 6 wherein at least one tag cassette is located amino-terminal to the binding domain or carboxy-terminal to the binding domain.
8. A HSPC or non-T effector cell of embodiments 5-7 including 2 or more extracellular tag cassettes wherein the tag cassettes are located amino-terminal to the binding domain, carboxy-terminal to the binding domain, or at least one tag cassette is located amino-terminal to the binding domain and at least one tag cassette is located carboxy-terminal to the binding domain.
9. A HSPC or non-T effector cell of embodiments 5-8 wherein the binding domain includes one or more tag cassettes.
10. A HSPC or non-T effector cell of embodiments 5-9 wherein the binding domain is a scFv, scTCR, receptor ectodomain, or ligand.
11. A HSPC or non-T effector cell of embodiment 10 wherein the scFv or scTCR includes a variable region linker including one or more tag cassettes.
12. A HSPC or non-T effector cell of embodiments 6-11 wherein the cellular marker includes CD3, CEACAM6, c-Met, EGFR, EGFRvIII, ErbB2, ErbB3, ErbB4, EphA2, IGF1R, GD2, O-acetyl GD2, O-acetyl GD3, GHRHR, GHR, FLT1, KDR, FLT4, CD44v6, CD151, CA125, CEA, CTLA-4, GITR, BTLA, TGFBR2, TGFBR1, IL6R, gp130, Lewis A, Lewis Y, TNFR1, TNFR2, PD1, PD-L1, PD-L2, HVEM, MAGE-A, mesothelin, NY-ESO-1, PSMA, RANK, ROR1, TNFRSF4, CD40, CD137, TWEAK-R, HLA, tumor or pathogen associated peptide bound to HLA, hTERT peptide bound to HLA, tyrosinase peptide bound to HLA, WT-1 peptide bound to HLA, LTβR, LIFRβ, LRP5, MUC1, OSMRβ, TCRα, TCRβ, CD19, CD20, CD22, CD25, CD28, CD30, CD33, CD52, CD56, CD80, CD81, CD86, CD123, CD171, CD276, B7H4, TLR7, TLR9, PTCH1, WT-1, Robo1, α-fetoprotein (AFP), Frizzled, OX40, or CD79b, B7H4, TLR7, TLR9, PTCH1, WT-1, Robo1, α-fetoprotein (AFP), Frizzled, OX40, or CD79b.
13. A HSPC or non-T effector cell of embodiments 6-11 wherein the cellular marker includes CD19, ROR1, PSMA, PSCA, mesothelin, CD20, WT1, or Her2.
14. A HSPC or non-T effector cell of embodiments 6-11 wherein the ligand binding domain binds CD19; wherein the extracellular component includes a spacer region including a hinge region of human IgG4; wherein the intracellular component includes an effector domain including a cytoplasmic domain of CD28 or 4-1 BB; and wherein the hydrophobic portion includes a human transmembrane domain.
15. A HSPC or non-T effector cell of embodiments 6-11 wherein the ligand binding domain is a single chain Fv fragment (scFv) including a CDRL1 sequence of RASQDISKYLN (SEQ ID NO: 108), a CDRL2 sequence of SRLHSGV (SEQ ID NO: 111), a CDRL3 sequence of GNTLPYTFG (SEQ ID NO: 104), a CDRH1 sequence of DYGVS (SEQ ID NO: 103), a CDRH2 sequence of VTWGSETTYYNSALKS (SEQ ID NO: 114), and a CDRH3 sequence of YAMDYWG (SEQ ID NO: 115).
16. A HSPC or non-T effector cell of embodiment 15 wherein the extracellular component comprises a spacer region of 12 amino acids or less.
17. A HSPC or non-T effector cell of embodiment 16 wherein the spacer region includes SEQ ID NO: 47.
18. A HSPC or non-T effector cell of embodiments 1-11 genetically modified to express a chimeric antigen receptor (CAR) including SEQ ID NO: 34, 53, 54, 55, 56, 57, or 58.
19. A HSPC or non-T effector cell of embodiments 6-11 wherein the ligand binding domain binds ROR1.
20. A HSPC or non-T effector cell of embodiments 6-11 wherein the ligand binding domain is a scFv including a CDRL1 sequence of ASGFDFSAYYM (SEQ ID NO: 101), a CDRL2 sequence of TIYPSSG (SEQ ID NO: 112), a CDRL3 sequence of ADRATYFCA (SEQ ID NO: 100), a CDRH1 sequence of DTIDWY (SEQ ID NO: 102), a CDRH2 sequence of VQSDGSYTKRPGVPDR (SEQ ID NO: 113), and a CDRH3 sequence of YIGGYVFG (SEQ ID NO: 117).
21. A HSPC or non-T effector cell of embodiments 6-11 wherein the ligand binding domain is a scFv including a CDRL1 sequence of SGSDINDYPIS (SEQ ID NO: 109), a CDRL2 sequence of INSGGST (SEQ ID NO: 105), a CDRL3 sequence of YFCARGYS (SEQ ID NO: 116), a CDRH1 sequence of SNLAW (SEQ ID NO: 110, a CDRH2 sequence of RASNLASGVPSRFSGS (SEQ ID NO: 107), and a CDRH3 sequence of NVSYRTSF (SEQ ID NO: 106).
22. A HSPC or non-T effector cell of embodiment 21 wherein the extracellular component comprises a spacer region of 229 amino acids or less.
23. A HSPC or non-T effector cell of embodiment 22 wherein the spacer region includes SEQ ID NO: 61.
24. A HSPC or non-T effector cell of embodiments 5-17 or 19-23 wherein the intracellular component includes an effector domain including one or more signaling, stimulatory or co-stimulatory domains selected from: 4-1 BB, B7-H3, CARD11, CD2, CD3γ, CD3δ, CD3ε, CD3ζ, CD7, CD25, CD27, CD28, CD30, CD40, CD79A, CD79B, DAP10, FcRα, FcRβ, FcRγ, Fyn, HVEM, ICOS, LAG3, LAT, Lck, LFA-1, LIGHT, LRP, NKG2C, NKG2D, NOTCH1, NOTCH2, NOTCH3, NOTCH4, pTα, PTCH2, OX40, ROR2, Ryk, SLAMF1, Slp76, TCRα, TCRβ, TRIM, Wnt, and Zap70.
25. A HSPC or non-T effector cell of embodiments 5-17 or 19-23 wherein the intracellular component includes an effector domain including an intracellular signaling domain including (i) all or a portion of the signaling domain of CD3ζ, (ii) all or a portion of the signaling domain of CD28, (iii) all or a portion of the signaling domain of 4-1 BB, or (iv) all or a portion of the signaling domain of CD3ζ, CD28, and/or 4-1BB.
26. A HSPC or non-T effector cell of embodiments 5-17 or 19-23 wherein the intracellular component includes an effector domain including a variant of CD3ζ and/or a portion of the 4-1BB intracellular signaling domain.
27. A HSPC or non-T effector cell of embodiments 1-26 wherein the extracellular component includes a spacer region.
28. A HSPC or non-T effector cell of embodiment 27 wherein the spacer region includes a portion of a hinge region of a human antibody.
29. A HSPC or non-T effector cell of embodiments 27 or 28 wherein the spacer region includes a hinge region and at least one other portion of an Fc domain of a human antibody selected from CH1, CH2, CH3, or combinations thereof.
30. A HSPC or non-T effector cell of embodiment 27 or 28 wherein the spacer region includes a Fc domain and a human IgG4 heavy chain hinge.
31. A HSPC or non-T effector cell of embodiments 27-30 wherein the spacer region is of a length selected from 12 amino acids or less, 119 amino acids or less, or 229 amino acids or less.
32. A HSPC or non-T effector cell of embodiment 27 wherein the spacer region is SEQ ID NO:47, SEQ ID NO:52, or SEQ ID NO:61.
33. A HSPC or non-T effector cell of embodiment 5-32 wherein the hydrophobic portion includes a human transmembrane domain.
34. A HSPC or non-T effector cell of embodiment 33 wherein the transmembrane domain is a CD28 transmembrane domain, a CD4 transmembrane domain, a CD8 transmembrane domain or a CD27 transmembrane domain.
35. A HSPC or non-T effector cell of embodiment 1-34 wherein the extracellular component further includes a tag sequence that binds an endogenous cognate binding molecule (EndoCBM).
36. A HSPC or non-T effector cell of embodiment 35 wherein the tag sequence is EGFR lacking an intracellular signaling domain.
37. A HSPC or non-T effector cell of embodiment 1-36 wherein the chimeric molecule includes a linker sequence.
38. A HSPC or non-T effector cell of embodiment 37 wherein the linker sequence includes a (GlyxSery)n sequence, wherein n is an integer from 1 to 10, and x and y are independently an integer from 0 to 10 provided that x and y are not both 0.
39. A HSPC or non-T effector cell of embodiment 37 wherein the linker sequence is a CH2CH3 or a CH3.
40. A HSPC or non-T effector cell of embodiment 37 wherein the linker sequence has an amino acid sequence of Gly-Gly-Gly-Gly-Ser (SEQ ID NO:145), (Gly-Gly-Gly-Gly-Ser)₂ (SEQ ID NO: 122), or (Gly-Gly-Gly-Ser)₂-Gly-Gly-Ser (SEQ ID NO:156).
41. A HSPC or non-T effector cell of embodiment 1-37 wherein the chimeric molecule includes a linker sequence adjacent to one or more tag cassettes, wherein the linker sequence and adjacent tag cassette collectively have an amino acid sequence of (Gly-Gly-Gly-Gly-Ser)₂-Trp-Ser-His-Pro-Gln-Phe-Glu-Lys (SEQ ID NO:139), Trp-Ser-His-Pro-Gln-Phe-Glu-Lys-(Gly-Gly-Gly-Gly-Ser)₂ (SEQ ID NO:140), (Gly-Gly-Gly-Gly-Ser)₂-Trp-Ser-His-Pro-Gln-Phe-Glu-Lys-(Gly-Gly-Gly-Ser)₂-Gly-Gly-Ser-Trp-Ser-His-Pro-Gln-Phe-Glu-Lys (SEQ ID NO:141), Trp-Ser-His-Pro-Gln-Phe-Glu-Lys-(Gly-Gly-Gly-Ser)₂-Gly-Gly-Ser-Trp-Ser-His-Pro-Gln-Phe-Glu-Lys-(Gly-Gly-Gly-Gly-Ser)₂ (SEQ ID NO:142), (Gly-Gly-Gly-Gly-Ser)₂-Trp-Ser-His-Pro-Gln-Phe-Glu-Lys-(Gly-Gly-Gly-Ser)₂-Gly-Gly-Ser-Trp-Ser-His-Pro-Gln-Phe-Glu-Lys-(Gly-Gly-Gly-Gly-Ser)₂-Trp-Ser-His-Pro-Gln-Phe-Glu-Lys (SEQ ID NO:143), or Trp-Ser-His-Pro-Gln-Phe-Glu-Lys-(Gly-Gly-Gly-Gly-Ser)₂-Trp-Ser-His-Pro-Gln-Phe-Glu-Lys-(Gly-Gly-Gly-Ser)₂-Gly-Gly-Ser-Trp-Ser-His-Pro-Gln-Phe-Glu-Lys-(Gly-Gly-Gly-Gly-Ser)₂ (SEQ ID NO:144).
42. A HSPC or non-T effector cell of embodiments 1-6 or 9-41 wherein the chimeric molecule includes from amino-terminus to carboxy-terminus: an extracellular binding domain, a tag cassette, a hinge, a hydrophobic portion, and an intracellular component including an effector domain.
43. A HSPC or non-T effector cell of embodiments 1-6 or 9-41 wherein the chimeric molecule includes from amino-terminus to carboxy-terminus: an extracellular binding domain, a first tag cassette, a second tag cassette, a hinge, a hydrophobic portion, and an intracellular component including an effector domain.
44. A HSPC or non-T effector cell of embodiments 1-6 or 9-41 wherein the chimeric molecule includes from amino-terminus to carboxy-terminus: an extracellular binding domain, a first tag cassette, a second tag cassette, a third tag cassette, a hinge, a hydrophobic portion, and an intracellular component including an effector domain.
45. A HSPC or non-T effector cell of embodiments 1-6 or 9-41 wherein the chimeric molecule includes from amino-terminus to carboxy-terminus: a tag cassette, an extracellular binding domain, a hinge, a hydrophobic portion, and an intracellular component including an effector domain.
46. A HSPC or non-T effector cell of embodiments 1-6 or 9-41 wherein the chimeric molecule includes from amino-terminus to carboxy-terminus: an extracellular binding domain, two to five tag cassettes, a hinge, a hydrophobic portion, and an intracellular component including an effector domain.
47. A HSPC or non-T effector cell embodiments 1-6 or 9-41 wherein the chimeric molecule includes from amino-terminus to carboxy-terminus: an extracellular scFv or scTCR binding domain including a variable region linker disposed between the variable regions and containing a tag cassette, a hinge, a hydrophobic portion, and an intracellular component including an effector domain.
48. A HSPC or non-T effector cell of embodiment 1 wherein the chimeric molecule includes from amino-terminus to carboxy-terminus: an extracellular scFv or scTCR binding domain, a tag cassette, an IgG hinge, a transmembrane domain, and an intracellular component including an effector domain, wherein the effector domain includes 4-1BB and CD3ζ, CD27 and CD3ζ, CD28 and CD3ζ, OX40 and CD3ζ, CD28, 4-1 BB and CD3ζ, OX40, 4-1 BB and CD3ζ, or CD28, OX40 and CD3ζ.
49. A HSPC or non-T effector cell of embodiment 1 wherein the chimeric molecule includes from amino-terminus to carboxy-terminus: an extracellular binding domain including a receptor ectodomain, a tag cassette, a hinge, a hydrophobic portion, and an intracellular component including an effector domain, wherein the effector domain includes 4-1BB, CD27, CD28, or OX40.
50. A HSPC or non-T effector cell of embodiments 1-49 wherein the chimeric molecule further includes a cytotoxic, radioisotope, radiometal, or detectable agent.
51. A HSPC or non-T effector cell of embodiments 1-49 wherein the extracellular component further includes a cytotoxic, radioisotope, radiometal, or detectable agent.
52. A HSPC or non-T effector cell of embodiments 1-51 wherein the HSPC is CD34⁺ HSPC and/or the non-T effector cell is a natural killer cell.
53. A composition including a pharmaceutically acceptable carrier and a genetically modified HSPC or non-T effector cell of any one of embodiments 1-52.
54. A composition of embodiment 53 further including an ExoCBM that specifically binds a tag cassette expressed by the HSPC or non-T effector cell within the composition.
55. A composition of embodiment 53 further including an EndoCBM that specifically binds a stimulatory molecule expressed by the HSPC or non-T effector cell within the composition.
56. A composition of embodiment 53 further including an ExoCBM that specifically binds a tag cassette expressed by the HSPC or non-T effector cell within the composition and an EndoCBM that specifically binds a stimulatory molecule expressed by the HSPC or non-T effector cell within the composition.
57. A composition of embodiments 53-56 formulated for infusion or injection.
58. A formulation including a pharmaceutically acceptable carrier and a genetically modified HSPC and non-T effector cell of any one of embodiments 1-52.
59. A formulation of embodiment 58 further including an ExoCBM that specifically binds a tag cassette expressed by the HSPC and/or non-T effector cell within the composition.
60. A formulation of embodiment 58 further including an EndoCBM that specifically binds a stimulatory molecule expressed by the HSPC and/or non-T effector cell within the composition.
61. A formulation of embodiment 58 further including an ExoCBM that specifically binds a tag cassette expressed by the HSPC and/or non-T effector cell within the composition and an EndoCBM that specifically binds a stimulatory molecule expressed by the HSPC and/or non-T effector cell within the composition.
62. A formulation of embodiments 58-61 formulated for infusion or injection.
63. A composition including an ExoCBM that specifically binds a tag cassette expressed by a HSPC or non-T effector cell of any one of embodiments 1-52.
64. A composition of embodiment 63 further including an EndoCBM that specifically binds a stimulatory molecule expressed by the HSPC or non-T effector cell.
65. A method for activating a HSPC or non-T effector cell of any one of embodiments 1-52 including contacting the HSPC or non-T effector cell with an ExoCBM that specifically binds a tag cassette expressed by the HSPC or non-T effector cell thereby activating the HSPC or non-T effector cell.
66. A method of embodiment 65 further including contacting the HSPC or non-T effector cell with an EndoCBM that specifically binds a stimulatory molecule expressed by the HSPC or non-T effector cell.
67. A method of embodiment 65 wherein the EndoCBM is selected from a Notch agonist, an angiopoietin-like protein, erythropoietin, fibroblast growth factor-1 (FGF-1); Flt-3 ligand (Flt-3L); granulocyte colony stimulating factor (G-CSF); granulocyte-macrophage colony stimulating factor (GM-CSF); insulin growth factor-2 (IFG-2); interleukin-3 (IL-3); interleukin-6 (IL-6); interleukin-7 (IL-7); interleukin-11 (IL-11); stem cell factor (SCF); and thrombopoietin (TPO).
68. A method of embodiment 67 wherein the EndoCBM is SCF, Flt-3L, TPO, IL-6 and IL-3.
69. A method of embodiments 65-68 wherein the ExoCBM is a cognate receptor, an anti-tag antibody, and/or an anti-tag scFv.
70. A method of embodiments 65-69 wherein the tag cassette is a Strep tag having amino acid sequence Trp-Ser-His-Pro-Gln-Phe-Glu-Lys (SEQ ID NO:118) or Trp-Arg-His-Pro-Gln-Phe-Gly-Gly (SEQ ID NO:137).
71. A method of embodiments 65-70 wherein the ExoCBM that specifically binds the tag cassette is a biotin binding protein or an anti-Strep tag antibody.
72. A method of embodiments 65-71 wherein the ExoCBM is attached to a solid surface.
73. A method of embodiments 65-72 wherein the ExoCBM is attached to a planar surface, agarose, resin, 3D fabric matrix, or a bead.
74. A method of embodiments 65-73 wherein the ExoCBM is attached to a microbead or a nanobead.
75. A method of embodiments 65-74 wherein the activating is performed *in vitro, in vivo* or ex *vivo.*
76. A method for promoting proliferation of a HSPC or non-T effector cell of any one of embodiments 1-52 including contacting the HSPC or non-T effector cell with (i) an ExoCBM that specifically binds a tag cassette expressed by the HSPC or non-T effector cell and (ii) an EndoCBM that specifically binds a stimulatory molecule expressed by the HSPC or non-T effector cell for a time sufficient to promote HSPC or non-T effector cell growth.
77. A method of embodiment 76 wherein the EndoCBM is selected from a Notch agonist, an angiopoietin-like protein, erythropoietin, fibroblast growth factor-1 (FGF-1); Flt-3 ligand (Flt-3L); granulocyte colony stimulating factor (G-CSF); granulocyte-macrophage colony stimulating factor (GM-CSF); insulin growth factor-2 (IFG-2); interleukin-3 (IL-3); interleukin-6 (IL-6); interleukin-7 (IL-7); interleukin-11 (IL-11); stem cell factor (SCF); and thrombopoietin (TPO).
78. A method of embodiment 77 wherein the EndoCBM is SCF, Flt-3L, TPO, IL-6 and IL-3.
79. A method of embodiments 76-78 wherein the ExoCBM is a cognate receptor, an anti-tag antibody, and/or an anti-tag scFv.
80. A method of embodiments 76-79 wherein the tag cassette is a Strep tag having amino acid sequence Trp-Ser-His-Pro-Gln-Phe-Glu-Lys (SEQ ID NO:118) or Trp-Arg-His-Pro-Gln-Phe-Gly-Gly (SEQ ID NO:137).
81. A method of embodiments 76-80 wherein the ExoCBM that specifically binds the tag cassette is a biotin binding protein or an anti-Strep tag antibody.
82. A method of embodiments 76-81 wherein the ExoCBM is attached to a solid surface.
83. A method of embodiments 76-82 wherein the ExoCBM is attached to a planar surface, agarose, resin, 3D fabric matrix, or a bead.
84. A method of embodiments 76-83 wherein the ExoCBM is attached to a microbead or a nanobead.
85. A method of embodiments 76-84 wherein the activating is performed *in vitro, in vivo* or ex *vivo.*
86. A method for detecting a HSPC or non-T effector cell including:
   contacting a sample including a HSPC or non-T effector cell of any one of embodiments 1-52 with an ExoCBM that specifically binds a tag cassette expressed by the HSPC or non-T effector cell, wherein the ExoCBM includes a detectable moiety, and
   detecting the presence of the HSPC or non-T effector cell in the sample based on the specific binding of the ExoCBM including the detectable moiety.
87. A method of embodiment 86 wherein the ExoCBM is a cognate receptor, an anti-tag antibody, and/or an anti-tag scFv.
88. A method of embodiment 86 or 87 wherein the tag cassette is a Strep tag having amino acid sequence Trp-Ser-His-Pro-Gln-Phe-Glu-Lys (SEQ ID NO:118) or Trp-Arg-His-Pro-Gln-Phe-Gly-Gly (SEQ ID NO:137).
89. A method of embodiments 86-88 wherein the ExoCBM that specifically binds the tag cassette is a biotin binding protein or an anti-Strep tag antibody.
90. A method of embodiments 86-89 wherein the ExoCBM is attached to a solid surface.
91. A method of embodiments 86-90 wherein the ExoCBM is attached to a planar surface, agarose, resin, 3D fabric matrix, or a bead.
92. A method of embodiments 86-91 wherein the ExoCBM is attached to a microbead or a nanobead.
93. A method of embodiments 86-92 wherein the detecting is performed *in vitro, in vivo* or ex *vivo.*
94. A method of embodiments 86-93 wherein the detectable moiety is fluorescent marker.
95. A method of embodiments 86-94 wherein the detectable moiety is APC, PE, Pacific blue, Alex fluor, or FITC.
96. A method of embodiments 86-95 wherein detection occurs using flow cytometry.
97. A method for enriching for or isolating a HSPC or non-T effector cell of any of embodiments 1-52 including contacting a sample including a HSPC or non-T effector cell with an ExoCBM that specifically binds a tag cassette expressed by the HSPC or non-T effector cell and enriching for or isolating the HSPC or non-T effector cell away from other cells not expressing the tag cassette in the sample.
98. A method of embodiment 97 wherein the ExoCBM is a cognate receptor, an anti-tag antibody, and/or an anti-tag scFv.
99. A method of embodiment 97 or 98 wherein the tag cassette is a Strep tag having amino acid sequence Trp-Ser-His-Pro-Gln-Phe-Glu-Lys (SEQ ID NO:118) or Trp-Arg-His-Pro-Gln-Phe-Gly-Gly (SEQ ID NO:137).
100. A method of embodiments 97-99 wherein the ExoCBM that specifically binds the tag cassette is a biotin binding protein or an anti-Strep tag antibody.
101. A method of embodiments 9-100 wherein the ExoCBM is attached to a solid surface.
102. A method of embodiments 97-101 wherein the ExoCBM is attached to a planar surface, agarose, resin, 3D fabric matrix, or a bead.
103. A method of embodiments 97-102 wherein the ExoCBM is attached to a microbead or a nanobead.
104. A method of embodiments 97-103 wherein the HSPC or non-T effector cell is enriched for or isolated by magnetic column chromatography.
105. A method of embodiments 97-104 including detecting the enriched for or isolated HSPC or non-T effector cells by contacting the HSPC or non-T effector cells with an ExoCBM that specifically binds the tag cassette expressed by the enriched or isolated HSPC or non-T effector cells wherein the ExoCBM includes a detectable moiety and detecting the presence of the HSPC or non-T effector cell in the sample based on the specific binding of the ExoCBM including the detectable moiety.
106. A method of embodiment 105 wherein the detectable moiety is fluorescent marker.
107. A method of embodiment 105 or 106 wherein the detectable moiety is APC, PE, Pacific blue, Alex fluor, or FITC.
108. A method of embodiments 105-107 wherein the detection occurs using flow cytometry.
109. A method for depleting or eliminating a HSPC or non-T effector cell of any of embodiments 1-52 including contacting a sample including the HSPC or non-T effector cell with an ExoCBM that specifically binds a tag cassette expressed by the HSPC or non-T effector cells, wherein binding of the ExoCBM to the tag cassette leads to cell death of the HSPC or non-T effector cells expressing the tag cassette.
110. A method of embodiment 109 wherein the ExoCBM includes a bispecific binding domain, wherein a first binding domain is specific for the tag cassette and the second binding domain is specific for CD3.
111. A method of embodiment 109 or 110 wherein the ExoCBM includes a cytotoxic, radioisotope, or radiometal agent.
112. A method of embodiments 109-111 wherein the ExoCBM includes a cognate receptor, an anti-tag antibody, an anti-tag scFv, or a cell with an anti-tag binding domain on its cell surface.
113. A method of embodiments 109-112 wherein the tag cassette is a Strep tag having amino acid sequence Trp-Ser-His-Pro-Gln-Phe-Glu-Lys (SEQ ID NO:118) or Trp-Arg-His-Pro-Gln-Phe-Gly-Gly (SEQ ID NO:137).
114. A method of embodiments 109-113 wherein the ExoCBM that specifically binds the tag cassette is a biotin binding protein or an anti-Strep tag antibody.
115. A method of embodiments 109-114 wherein the ExoCBM is attached to a solid surface.
116. A method of embodiments 109-115 wherein the ExoCBM is attached to a planar surface, agarose, resin, 3D fabric matrix, or a bead.
117. A method of embodiments 109-116 wherein the ExoCBM is attached to a microbead or a nanobead.
118. A method of tracking administered HSPC or non-T effector cells of any of embodiments 1-52 including administering to a subject an ExoCBM that specifically binds a tag cassette expressed by the HSPC or non-T effector cells wherein the ExoCBM includes a detectable moiety, and detecting the presence of the HSPC or non-T effector cell within the subject based on the specific binding of the ExoCBM including the detectable moiety.
119. A method of embodiment 118 wherein the HSPC or non-T effector cells and the ExoCBM are administered simultaneously.
120. A method of embodiment 118 or 119 wherein HSPC or non-T effector cells and the ExoCBM are administered as a composition or formulation.
121. A method of embodiments 118-120 wherein the ExoCBM is a cognate receptor, an anti-tag antibody, and/or an anti-tag scFv.
122. A method of embodiments 118-121 wherein the tag cassette is a Strep tag having amino acid sequence Trp-Ser-His-Pro-Gln-Phe-Glu-Lys (SEQ ID NO:118) or Trp-Arg-His-Pro-Gln-Phe-Gly-Gly (SEQ ID NO:137).
123. A method of embodiments 118-122 wherein the ExoCBM that specifically binds the tag cassette is a biotin binding protein or an anti-Strep tag antibody.
124. A method of embodiments 118-123 wherein the ExoCBM is attached to a solid surface.
125. A method of embodiments 118-124 wherein the ExoCBM is attached to a planar surface, an agarose bead, a resin, a 3D fabric matrix, or a bead.
126. A method of embodiment 118-125 wherein the ExoCBM is attached to a microbead or a nanobead.
127. A method of embodiments 118-126 wherein the detectable moiety includes a fluorescent marker.
128. A method of embodiments 118-127 wherein the detectable moiety includes a APC, PE, Pacific blue, Alex fluor, or FITC.
129. A method of embodiments 118-128 wherein the detectable moiety includes a magnetic particle, superparamagnetic iron oxide (SPIO), fluorodeoxyglucose (18F), fluorescent compounds, or any combination thereof.
130. A method of embodiments 118-129 wherein the tracking includes use of MRI, PET, or near infrared imaging.
131. A method for activating administered HSPC or non-T effector cells of any of embodiments 1-52 including administering to a subject (i) an ExoCBM that specifically binds a tag cassette expressed by the HSPC or non-T effector cell; (ii) an EndoCBM that specifically binds a stimulatory molecule expressed by the HSPC or non-T effector cell; wherein specific binding of the ExoCBM and the EndoCBM activates the HSPC or non-T effector cell *in vivo.*
132. A method of embodiment 131 wherein the EndoCBM is selected from a Notch agonist, an angiopoietin-like protein, erythropoietin, fibroblast growth factor-1 (FGF-1); Flt-3 ligand (Flt-3L); granulocyte colony stimulating factor (G-CSF); granulocyte-macrophage colony stimulating factor (GM-CSF); insulin growth factor-2 (IFG-2); interleukin-3 (IL-3); interleukin-6 (IL-6); interleukin-7 (IL-7); interleukin-11 (IL-11); stem cell factor (SCF); and thrombopoietin (TPO).
133. A method of embodiment 132 wherein the EndoCBM is SCF, Flt-3L, TPO, IL-6 and IL-3.
134. A method of embodiments 131-133 wherein the HSPC or non-T effector cells, the ExoCBM, and the EndoCBM are administered simultaneously.
135. A method of embodiments 131-134 wherein HSPC or non-T effector cells, the ExoCBM, and the EndoCBM are administered as a composition or formulation.
136. A method of embodiments 131-135 wherein the ExoCBM is a cognate receptor, an anti-tag antibody, and/or an anti-tag scFv.
137. A method of embodiments 131-136 wherein the tag cassette is a Strep tag having amino acid sequence Trp-Ser-His-Pro-Gln-Phe-Glu-Lys (SEQ ID NO:118) or Trp-Arg-His-Pro-Gln-Phe-Gly-Gly (SEQ ID NO:137).
138. A method of embodiments 131-137 wherein the ExoCBM that specifically binds the tag cassette is a biotin binding protein or an anti-Strep tag antibody.
139. A method of depleting administered HSPC or non-T effector cells of any of embodiments 1-52 including administering an ExoCBM that specifically binds a tag cassette expressed by the administered HSPC or non-T effector cells, wherein binding of the ExoCBM to the tag cassette leads to cell death of the HSPC or non-T effector cells expressing the tag cassette
140. A method of embodiment 139 wherein the ExoCBM includes a bispecific binding domain, wherein a first binding domain is specific for the tag cassette and the second binding domain is specific for CD3.
141. A method of embodiment 139 or 140 wherein the ExoCBM includes a cytotoxic, radioisotope, or radiometal agent.
142. A method of embodiments 139-141 wherein the ExoCBM includes a cognate receptor, an anti-tag antibody, an anti-tag scFv, or a cell with an anti-tag binding domain on its cell surface.
143. A method of embodiments 139-142 wherein the tag cassette is a Strep tag having amino acid sequence Trp-Ser-His-Pro-Gln-Phe-Glu-Lys (SEQ ID NO:118) or Trp-Arg-His-Pro-Gln-Phe-Gly-Gly (SEQ ID NO:137).
144. A method of embodiments 139-143 wherein the ExoCBM that specifically binds the tag cassette is a biotin binding protein or an anti-Strep tag antibody.
145. A method of embodiments 139-144 wherein the ExoCBM is attached to a solid surface.
146. A method of embodiments 139-145 wherein the ExoCBM is attached to a planar surface, agarose, resin, 3D fabric matrix, or a bead.
147. A method of embodiments 139-146 wherein the ExoCBM is attached to a microbead or a nanobead.
148. A method of treating a condition in a subject, including administering a therapeutically-effective amount of an HSPC or non-T effector cell of any one of embodiments 1-52, a therapeutically effective amount of a composition of any one of embodiments 53-57, 63 or 64 or a therapeutically effective amount of a formulation of any one of embodiments 58-62 to the subject, thereby treating the condition in the subject.
149. A method of embodiment 148 wherein immunological matching to the subject is not required before the administering.
150. A method of embodiment 148 or 149 wherein the subject is a relapsed pediatric acute lymphoblastic leukemia patient.
151. A method of embodiments 148-150 wherein the method further includes monitoring cytokine levels in the subject after administering the ExoCBM that specifically binds the tag cassette.
152. A method of embodiments 148-151 wherein the condition is immunodeficiency, pancytopenia, neutropenia, and/or leukopenia.
153. A method of embodiment 152 wherein the immunodeficiency, pancytopenia, neutropenia, and/or leukopenia is due to chemotherapy, radiation therapy, and/or a myeloablative regimen for HCT and/or acute ionizing radiation.
154. A method of embodiments 148-151 wherein the condition is a depleted immune system.
155. A method of embodiment 154 wherein the depleted immune system arose due to a viral infection, microbial infection, parasitic infection, renal disease, and/or renal failure.
156. A method of embodiment 154 or 155 wherein the depleted immune system arose due to exposure to drugs that cause bone marrow suppression or hematopoietic deficiencies.
157. A method of embodiments 154-156 wherein the depleted immune system arose due to exposure to penicillin, gancyclovir, daunomycin, meprobamate, aminopyrine, dipyrone, phenytoin, carbamazepine, propylthiouracil, and/or methimazole.
158. A method of embodiments 154-157 wherein the depleted immune system arose due to exposure to dialysis.
159. A method of embodiments 148-158 further including administering non-genetically-modified HSPC to the subject.
160. A method of embodiments 148-159 further including activating, tracking or depleting the administered HSPC and/or non-T effector cells according to any of the methods of embodiments 118-147.
161. A method of repopulating an immune system in a subject in need thereof including administering a therapeutically-effective amount of an HSPC or non-T effector cell of any one of embodiments 1-52, a therapeutically effective amount of a composition of any one of embodiments 53-57, 63 or 64 or a therapeutically effective amount of a formulation of any one of embodiments 58-62 to the subject, thereby repopulating the immune system of the subject.
162. A method of embodiment 161 wherein immunological matching to the subject is not required before the administering.
163. A method of embodiment 161 or 162 further including targeting cancer cells expressing a cellular marker in the subject by administering a therapeutically effective amount of genetically modified HSPC and/or genetically modified non-T effector cells of any one of embodiments 1-52 to the subject thereby targeting the cancer cells.
164. A method of embodiment 163 wherein the cancer cells are from an adrenal cancer, a bladder cancer, a blood cancer, a bone cancer, a brain cancer, a breast cancer, a carcinoma, a cervical cancer, a colon cancer, a colorectal cancer, a corpus uterine cancer, an ear, nose and throat (ENT) cancer, an endometrial cancer, an esophageal cancer, a gastrointestinal cancer, a head and neck cancer, a Hodgkin's disease, an intestinal cancer, a kidney cancer, a larynx cancer, a leukemia, a liver cancer, a lymph node cancer, a lymphoma, a lung cancer, a melanoma, a mesothelioma, a myeloma, a nasopharynx cancer, a neuroblastoma, a non-Hodgkin's lymphoma, an oral cancer, an ovarian cancer, a pancreatic cancer, a penile cancer, a pharynx cancer, a prostate cancer, a rectal cancer, a sarcoma, a seminoma, a skin cancer, a stomach cancer, a teratoma, a testicular cancer, a thyroid cancer, a uterine cancer, a vaginal cancer, a vascular tumor, and/or a metastasis thereof.
165. A method of embodiment 163 wherein the cellular marker(s) of the cancer cells are selected from A33; BAGE; Bcl-2; β-catenin; B7H4; BTLA; CA125; CA19-9; CD5; CD19; CD20; CD21; CD22; CD33; CD37; CD44v6; CD45; CD123; CEA; CEACAM6; c-Met; CS-1; cyclin B1; DAGE; EBNA; EGFR; ephrinB2; ErbB2; ErbB3; ErbB4; EphA2; estrogen receptor; FAP; ferritin; α-fetoprotein (AFP); FLT1; FLT4; folate-binding protein; Frizzled; GAGE; G250; GD-2; GHRHR; GHR; GM2; gp75; gp100 (Pmel 17); gp130; HLA; HER-2/neu; HPV E6; HPV E7; hTERT; HVEM; IGF1R; IL6R; KDR; Ki-67; LIFRβ; LRP; LRP5; LTβR; mesothelin; OSMRβ; p53; PD1; PD-L1; PD-L2; PRAME; progesterone receptor; PSA; PSMA; PTCH1; MAGE; MART; mesothelin; MUC; MUC1; MUM-1-B; myc; NYESO-1; RANK; ras; Robo1; RORI; survivin; TCRα; TCRβ; tenascin; TGFBR1; TGFBR2;TLR7; TLR9; TNFR1; TNFR2; TNFRSF4; TWEAK-R; TSTA tyrosinase; VEGF; and WT1.
166. A method of embodiment 163 wherein the cancer is leukemia/lymphoma and the cellular marker(s) are one or more of CD19, CD20, CD22, ROR1, CD33, and WT-1; wherein the cancer is multiple myeloma and the cellular marker is BCMA; wherein the cancer is prostate cancer and the cellular marker(s) are one or more of PSMA, WT1, PSCA, and SV40 T; wherein the cancer is breast cancer and the cellular marker(s) are one or more of HER2, ERBB2, and ROR1; wherein the cancer is stem cell cancer and the cellular marker is CD133; wherein the cancer is ovarian cancer and the cellular marker(s) are one or more of L1-CAM, MUC-CD, folate receptor, Lewis Y, ROR1, mesothelin, and WT-1; wherein the cancer is mesothelioma and the cellular marker is mesothelin; wherein the cancer is renal cell carcinoma and the cellular marker is CAIX; wherein the cancer is melanoma and the cellular marker is GD2; wherein the cancer is pancreatic cancer and the cellular marker(s) are one or more of mesothelin, CEA, CD24, and ROR1; or wherein the cancer is lung cancer and the cellular marker is ROR1.
167. A method of embodiment 163 wherein the cancer cells are acute lymphoblastic leukemia cells expressing CD19.
168. A method of embodiment 163 wherein the cancer is acute lymphoblastic leukemia and the subject is a pediatric patient.
169. A method of embodiments 163-168 further including activating, tracking or depleting the administered HSPC and/or non-T effector cells according to any of the methods of embodiments 118-147.
170. A method of targeting cells preferentially expressing CD19 for destruction including administering to a subject in need thereof a therapeutically effective amount of genetically modified HSPC and/or genetically modified non-T effector cells wherein the genetically modified cells express (i) an extracellular component including at least one tag cassette and a CD19 ligand binding domain, and (ii) an intracellular component including an effector domain thereby targeting and destroying cells preferentially expressing CD19.
171. A method of embodiment 170 wherein immunological matching to the subject is not required before the administering.
172. A method of embodiment 170 or 171 wherein the cells preferentially expressing CD19 are acute lymphoblastic leukemia cells.
173. A method of embodiments 170-172 wherein the subject is a relapsed pediatric acute lymphoblastic leukemia patient.
174. A method of embodiments 170-173 wherein the at least one tag cassette is or includes a Strep tag, His tag, Flag tag, Xpress tag, Avi tag, Calmodulin tag, Polyglutamate tag, HA tag, Myc tag, Nus tag, S tag, X tag, SBP tag, Softag, V5 tag, CBP, GST, MBP, GFP, Thioredoxin tag, or any combination thereof.
175. A method of embodiments 170-174 wherein at least one tag cassette is or includes a Strep tag including the amino acid sequence Trp-Ser-His-Pro-Gln-Phe-Glu-Lys (SEQ ID NO:118) or Trp-Arg-His-Pro-Gln-Phe-Gly-Gly (SEQ ID NO:137).
176. A method of embodiments 170-175 further including treating immunodeficiency, pancytopenia, neutropenia, and/or leukopenia in the subject by administering a therapeutically effective amount of HSPC to the subject.
177. A method of embodiment 176 wherein the immunodeficiency, pancytopenia, neutropenia, and/or leukopenia is due to chemotherapy, radiation therapy, and/or a myeloablative regimen for HCT.
178. A method of embodiments 170-177 further including activating, tracking or depleting the administered HSPC and/or non-T effector cells according to any of the methods of embodiments 118-147.
179. A method of targeting cancer cells in a subject including identifying at least one cellular marker preferentially expressed on a cancer cell from the subject; administering a therapeutically-effective amount of an HSPC or non-T effector cell of any one of embodiments 1-52, a therapeutically effective amount of a composition of any one of embodiments 53-57, 63 or 64 or a therapeutically effective amount of a formulation of any one of embodiments 58-62to the subject based on the identified at least one cellular marker.
180. A kit including the compositions of any one of embodiments 53-57, 63 or 64 wherein the kit includes instructions advising that the compositions can be administered to a subject without immunological matching.
181. A kit including the formulations of any one of embodiments 58-62 wherein the kit includes instructions advising that the formulations can be administered to a subject without immunological matching.
182. A kit including the compositions of any one of embodiments 53-57, 63 or 64 and the formulations of embodiment any one of embodiments 58-62 wherein the kit includes instructions advising that the compositions or formulations can be administered to a subject without immunological matching.

The Examples and Exemplary Embodiments below are included to demonstrate particular embodiments of the disclosure. Those of ordinary skill in the art should recognize in light of the present disclosure that many changes can be made to the specific embodiments disclosed herein and still obtain a like or similar result without departing from the spirit and scope of the disclosure.

### Exemplary Embodiments.

Example 1. Design and cGMP production of two third generation lentiviral vectors for the coordinate expression of the CD19-CAR and a huEGFRt selection/suicide construct have been created. For both a SIN vesicular stomatitis virus G (VSV-G) pseudotyped lentiviral vector under cGMP conditions that encodes for a CD19 specific CAR and huEGFRt, which is a truncated human EGFR protein that does not contain an intracellular signaling domain was developed. The CD19 specific scFvFc-CD3ζCD28 CAR and huEGFRt vector contains a hybrid 5'LTR in which the U3 region is replaced with the CMV promoter, and a 3' LTR in which the cis-acting regulatory sequences are completely removed from the U3 region. As a result, both the 5' and 3' LTRs are inactivated when the provirus is produced and integrated into the chromosome. The CD19 CAR includes the human GMCSFRα chain leader sequence, the VL and VH sequences derived from the CD19 specific murine IgG1mAb (FMC63), the Fc and hinge regions of human IgG4 heavy chain, the human CD28 transmembrane region, and the cytoplasmic domain of CD3ζ and CD28. This construct has been cloned into a modified pHIV7 in which the CMV promoter was swapped for the human EF-1 alpha promoter (FIG. 29A). The vector allows approximately 1:1 expression of the CD19 CAR and huEGFRt through the use of a T2A element. The second, is the CD19-specific scFv-4-1BB/CD3ζ CAR fragment encodes an N-terminal leader peptide of the human GMCSF receptor alpha chain signal sequence to direct surface expression, CD19-specific scFv derived from the IgG1 murine monoclonal antibody (FMC63), human IgG4 hinge and human CD28 transmembrane region and 4-1BB costimulatory element with the cytoplasmic tail of human CD3ζ (FIG. 29B). Again the vector allows approximately 1:1 expression of the CD19 CAR and huEGFRt through the use of a T2A element.

The expression of huEGFRt provides for a second cell surface marker that allows easy examination of transduction efficiency. Biotinylated Erbitux binds to the huEGFRt expressed on the cell surface and can be labeled with flurochrome for analysis with flow cytometry. Additionally it can be used as a suicide gene in the clinical setting with the treatment of Erbitux. A similar vector with eGFP in place of the CAR has also been generated. huEGFRt can be replaced or supplemented with a tag cassette binding an an ExoCBM, such as STREP TAG^{®} II (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

Example 2. Notch-mediated *ex vivo* expansion of CB HSPC is a clinically validated cell therapy product that is well tolerated, can be given off the shelf without HLA matching, and provides transient myeloid engraftment in both the HCT and intensive chemotherapy setting. Off the shelf expanded units have been infused into >85 subjects and no serious adverse events have been noted except for one allergic reaction attributed to DMSO. Additionally, there has been no persistent engraftment beyond day 180 in the HCT setting and 14 days post infusion in the chemotherapy setting.

Methods. Umbilical cord blood/placental blood unit(s) were collected from human(s) at birth. The collected blood was mixed with an anti-coagulant to prevent clotting and stored. Prior to planned initiation of expansion cultures, tissue culture vessels were first coated overnight at 4°C or a minimum of 2 hours at 37°C with Delta1^{ext-IgG} at 2.5 µg/ml and RETRONECTIN^{®} (a recombinant human fibronectin fragment) (Clontech Laboratories, Inc., Madison, WI) at 5 µg/ml in phosphate buffered saline (PBS). The flasks were then washed with PBS and then blocked with PBS-2% Human Serum Albumin (HSA). The fresh cord blood unit is red cell lysed and processed to select for CD34⁺ cells using the AUTOMACS^{®} Cell Separation System (Miltenyi Biotec GmbH, Gladbach, Germany). After enrichment, the percentage of CD34⁺ cells in the sample is increased relative to the percentage of CD34⁺ cells in the sample prior to enrichment. The enriched CD34⁺ cell fraction was resuspended in final culture media, which consists of STEMSPAN^{™} Serum Free Expansion Medium (StemCell Technologies, Vancouver, British Columbia) supplemented with rhIL-3 (10 ng/ml), rhlL-6 (50 ng/ml), rhTPO (50 ng/ml), rhFIt-3L (50 ng/ml), rhSCF (50 ng/ml).

A SIN lentiviral vector that directs the co-expression of a CD19-specific scFvFc:CD28:ζ chimeric antigen receptor and a huEGFRt selection suicide construct was transduced into the Notch expanded CB stem cells on day 3 or 4 via centrifugation at 800 × g for 45 minutes at 32°C with lentiviral supernatant (MOI 3) and 4 µg/ml of protamine sulfate. Alternatively, the SIN lentiviral vector encoded for 4-1BB costimulation (see Brief Description of the Figures). Due to concerns of expression of the CAR on HSPC with potential signaling capacity, irradiated LCL was added on day 7 of culture at a 1:1 ratio to provide antigen stimulation. huEGFRt can be replaced or supplemented with a tag cassette binding an ExoCBM, such as STREP TAG^{®} II (SEQ ID NO:118), Myc tag (SEQ ID NO:119), V5 tag (SEQ ID NO:120), FLAG^{®} tag (SEQ ID NO:121), His tag, or other peptides or molecules as disclosed herein.

At the end of the expansion culture, NK cells and neutrophils are still immature. In order to fully assess lytic capabilities, culture methods were devised to increase maturity. For the NK cells, the culture was replated in RPMI media supplemented with human serum, IL-2 at 50 U/mL and IL-15 at 500 ng/mL or RPMI media supplemented with human serum, L-glutamine, IL-2 at 50 U/mL and IL-15 at 500 ng/mL for an additional week of culture.

A NOD/SCID IL2R null (NOG) mouse model was used to assess engraftment of expanded CB cells. After undergoing sub-lethal irradiation, mice are able to reliably engraft expanded CB cells. In order to look at engraftment with transduced expanded CB cells, NOG mice were irradiated at a dose of 325cGy by linear accelerator and infused via tail vein injection with the progeny generated from 10,000 - 30,000 CD34⁺ CB cells cultured on Delta-1ext-IgG.

Results. Transduction efficiency ranged from 10 to > 50% and there was generally equal transduction between CD34⁺ and CD34- cells. Copy number analysis demonstrated between 1-4 copies/cell as determined by validated real time, quantitative PCR analysis, which is in line with the FDA requirements for clinical gene therapy cell products.

CD34⁺ CB cells cultured on Notch ligand contain a variety of cell types, which can be identified based on immunophenotyping. Cultures transduced with the CD19 CAR lentivirus have been compared with an untransduced culture from the same cord blood unit and no significant differences have been detected in regards to the final immunophenotyping at the time of harvest, or the overall growth of the cells in culture including the CD34 fold expansion and the TNC fold expansion.

Expression of the transgene did not affect the final culture phenotype at 14 days and transgene expression is seen in all cell subsets and appears relatively stable over the culture period.

Additional experiments were carried out exposing the cell cultures to CD19⁺ LCL to determine if exposure to antigen causes untoward effects on the culture. Adding irradiated LCL to the culture on day 7 at a 1:1 ratio did not have untoward outcomes, and in fact enhanced the growth and viability in both the transduced and untransduced cultures. The LCL did not appear to increase the CAR+ population, suggesting that antigen does not enhance the proliferation of CAR expressing immature cells. Additionally, the transgene has been detected equivalently in all phenotypic cell subsets of the final product. For a graphical depiction of these results, see FIGs. 30A, 30B, 31, 32 and 33.

The transfer of effector function upon encountering CD19 through the expression of the CD19 CAR is important for the ultimate anti-cancer (e.g., anti-leukemic) activity of the modified CB HSPC cells. Differentiating culture conditions resulted in an increase of NK cells (FIG. 34). The CD56⁺ cell fraction was sorted and used in a CRA with target cells of K562 and LCL. As expected, both untransduced and transduced cells were able to kill K562, and although the LCL was also killed by both, the lysis of the LCL was significantly enhanced through the expression of the CAR. More particularly, the CD19-CAR expressing NK cells had enhanced cytotoxic activity compared with non-transduced NK cells (50 v 30%) whereas both killed K562 targets equally (75 v 80%). See FIG. 35.

The NOG model when transplanted with expanded CB cells led to the development of a large population of CD19⁺ cells, beginning around week 4-5 post transplant. There was no effect on early engraftment of transduced cells, however there was a substantial reduction in CD19 engraftment in the mice transplanted with CD19 CAR expressing cells compared with untransduced cells, in which the CD19 population was >20% of the engrafted cells, indicating anti-CD19 activity. NK cell populations were increased using NS0-IL15 secreting cells, irradiated and injected subcutaneously three times per week starting at week 3 to provide enhanced effector function. This effect enhances the amount of CD56⁺ cells *in vivo.* See FIGs. 36 and 37.

The data show that transduction of expanded CB cells during culture in the presence of immobilized Delta1^{ext-IgG} to express a CD19 specific CAR does not have detectable effects of the quality or quantity of the expansion, nor on its repopulating abilities in the mouse model. These results are promising as a way to engineer a graft versus cancer (*e.g.,* leukemia) effect into cord blood transplant. Furthermore, transduction of a CD19 CAR into universal donor expanded CB HSPC allows for infusion of an anti-CD19 cell product to be given immediately (*e.g.,* immunological matching not required before administration) following identification of a subject with clinical need for therapy, for example one in relapse or with persistent MRD. Reliable transduction of CD34⁺ cord blood cells expanded on Notch ligand without affecting the overall culture nor *in vivo* engraftment capacity while at the same time engineering anti-CD19 activity has been demonstrated. Because expanded cord blood cells are already being used clinically as an off the shelf, non-HLA matched cellular therapy, the described Examples show additional use as an off the shelf cellular therapy, enabling patients to receive immunotherapy even if unable to obtain and engineer an autologous T cell product.

As indicated, the practice of the present disclosure can employ, unless otherwise indicated, conventional methods of virology, microbiology, molecular biology and recombinant DNA techniques within the ordinary skill of the art. Such techniques are explained fully in the literature; see, *e.g.,* Sambrook, et al., "Molecular Cloning: A Laboratory Manual (Current Edition)"; DNA Cloning: A Practical Approach, vol. I & II (D. Glover, ed.); Oligonucleotide Synthesis (N. Gait, ed., Current Edition); Nucleic Acid Hybridization (B. Hames & S. Higgins, eds., Current Edition); Transcription and Translation (B. Hames & S. Higgins, eds., Current Edition); CRC Handbook of Parvoviruses, vol. I & II (P. Tijessen, ed.); Fundamental Virology, 2nd Edition, vol. I & II (B. N. Fields and D. M. Knipe, eds.) each of which is incorporated by reference herein for its teachings regarding the same.

As will be understood by one of ordinary skill in the art, each embodiment disclosed herein can comprise, consist essentially of or consist of its particular stated element, step, ingredient or component. "Includes" or "including" means "comprises, consists essentially of or consists of." The transition term "comprise" or "comprises" means includes, but is not limited to, and allows for the inclusion of unspecified elements, steps, ingredients, or components, even in major amounts. The transitional phrase "consisting of' excludes any element, step, ingredient or component not specified. The transition phrase "consisting essentially of" limits the scope of the embodiment to the specified elements, steps, ingredients or components and to those that do not materially affect the embodiment. A material effect would result in (i) a statistically significant reduction in the effectiveness of a cell administration to create an anti-cancer effect in a subject and/or (ii) a statistically significant reduction in the effectiveness of a cell administration to re-populate a subject's immune system.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (*e.g*., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Particular embodiments of this invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Furthermore, numerous references have been made to books, journal articles, treatises, patents, printed publications, etc. (collectively "references") throughout this specification. Each of the above-cited references are individually incorporated by reference herein for their cited teachings.

In closing, it is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein. Accordingly, the present invention is not limited to that precisely as shown and described.

The particulars shown herein are by way of example and for purposes of illustrative discussion of the preferred embodiments of the present invention only and are presented in the cause of providing what is believed to be the most useful and readily understood description of the principles and conceptual aspects of various embodiments of the invention. In this regard, no attempt is made to show structural details of the invention in more detail than is necessary for the fundamental understanding of the invention, the description taken with the drawings and/or examples making apparent to those skilled in the art how the several forms of the invention may be embodied in practice.

Definitions and explanations used in the present disclosure are meant and intended to be controlling in any future construction unless clearly and unambiguously modified in the following examples or when application of the meaning renders any construction meaningless or essentially meaningless. In cases where the construction of the term would render it meaningless or essentially meaningless, the definition should be taken from Webster's Dictionary, 3rd Edition or a dictionary known to those of ordinary skill in the art, such as the Oxford Dictionary of Biochemistry and Molecular Biology (Ed. Anthony Smith, Oxford University Press, Oxford, 2004).

The present invention will now be described by way of reference to the following clauses:
1. A hematopoietic stem progenitor cell (HSPC) or non-T effector cell genetically modified to express a chimeric molecule comprising an extracellular component comprising a tag cassette that specifically binds an exogenous cognate binding molecule (ExoCBM).
2. A HSPC or non-T effector cell of clause 1 wherein the extracellular component has one, two, three, four or five tag cassettes.
3. A HSPC or non-T effector cell of clause 1 wherein at least one tag cassette is or comprises a Strep tag, His tag, Flag tag, Xpress tag, Avi tag, Calmodulin tag, Polyglutamate tag, HA tag, Myc tag, Nus tag, S tag, X tag, SBP tag, Softag, V5 tag, CBP, GST, MBP, GFP, Thioredoxin tag, or any combination thereof.
4. A HSPC or non-T effector cell of clause 3 wherein at least one tag cassette is or comprises a Strep tag comprising the amino acid sequence Trp-Ser-His-Pro-Gln-Phe-Glu-Lys (SEQ ID NO:118) or Trp-Arg-His-Pro-Gln-Phe-Gly-Gly (SEQ ID NO:137).
5. A HSPC or non-T effector cell of clause 1 wherein the extracellular component is linked to an intracellular component through a hydrophobic portion.
6. A HSPC or non-T effector cell of clause 5 wherein the extracellular component comprises (i) a binding domain that specifically binds a cellular marker, and (ii) a hinge; and wherein the intracellular component comprises an effector domain.
7. A HSPC or non-T effector cell of clause 6 wherein at least one tag cassette is located amino-terminal to the binding domain or carboxy-terminal to the binding domain.
8. A HSPC or non-T effector cell of clause 6 comprising 2 or more extracellular tag cassettes wherein the tag cassettes are located amino-terminal to the binding domain, carboxy-terminal to the binding domain, or at least one tag cassette is located amino-terminal to the binding domain and at least one tag cassette is located carboxy-terminal to the binding domain.
9. A HSPC or non-T effector cell of clause 6 wherein the binding domain comprises one or more tag cassettes.
10. A HSPC or non-T effector cell of clause 6 wherein the binding domain is a scFv, scTCR, receptor ectodomain, or ligand.
11. A HSPC or non-T effector cell of clause 10 wherein the scFv or scTCR comprises a variable region linker comprising one or more tag cassettes.
12. A HSPC or non-T effector cell of clause 6 wherein the cellular marker comprises CD3, CEACAM6, c-Met, EGFR, EGFRvIII, ErbB2, ErbB3, ErbB4, EphA2, IGF1R, GD2, O-acetyl GD2, O-acetyl GD3, GHRHR, GHR, FLT1, KDR, FLT4, CD44v6, CD151, CA125, CEA, CTLA-4, GITR, BTLA, TGFBR2, TGFBR1, IL6R, gp130, Lewis A, Lewis Y, TNFR1, TNFR2, PD1, PD-L1, PD-L2, HVEM, MAGE-A, mesothelin, NY-ESO-1, PSMA, RANK, ROR1, TNFRSF4, CD40, CD137, TWEAK-R, HLA, tumor or pathogen associated peptide bound to HLA, hTERT peptide bound to HLA, tyrosinase peptide bound to HLA, WT-1 peptide bound to HLA, LTβR, LIFRβ, LRP5, MUC1, OSMRβ, TCRα, TCRβ, CD19, CD20, CD22, CD25, CD28, CD30, CD33, CD52, CD56, CD80, CD81, CD86, CD123, CD171, CD276, B7H4, TLR7, TLR9, PTCH1, WT-1, Robo1, α-fetoprotein (AFP), Frizzled, OX40, or CD79b, B7H4, TLR7, TLR9, PTCH1, WT-1, Robo1, α-fetoprotein (AFP), Frizzled, OX40, or CD79b.
13. A HSPC or non-T effector cell of clause 6 wherein the cellular marker comprises CD19, ROR1, PSMA, PSCA, mesothelin, CD20, WT1, or Her2.
14. A HSPC or non-T effector cell of clause 6 wherein the ligand binding domain binds CD19; wherein the extracellular component comprises a spacer region comprising a hinge region of human IgG4; wherein the intracellular component comprises an effector domain comprising a cytoplasmic domain of CD28 or 4-1BB; and wherein the hydrophobic portion comprises a human transmembrane domain.
15. A HSPC or non-T effector cell of clause 6 wherein the ligand binding domain is a single chain Fv fragment (scFv) comprising a CDRL1 sequence of RASQDISKYLN (SEQ ID NO: 108), a CDRL2 sequence of SRLHSGV (SEQ ID NO: 111), a CDRL3 sequence of GNTLPYTFG (SEQ ID NO: 104), a CDRH1 sequence of DYGVS (SEQ ID NO: 103), a CDRH2 sequence of VTWGSETTYYNSALKS (SEQ ID NO: 114), and a CDRH3 sequence of YAMDYWG (SEQ ID NO: 115).
16. A HSPC or non-T effector cell of clause 15 wherein the extracellular component comprises a spacer region of 12 amino acids or less.
17. A HSPC or non-T effector cell of clause 16 wherein the spacer region comprises SEQ ID NO: 47.
18. A HSPC or non-T effector cell of clause 6 genetically modified to express a chimeric antigen receptor (CAR) comprising SEQ ID NO: 34, 53, 54, 55, 56, 57, or 58.
19. A HSPC or non-T effector cell of clause 6 wherein the ligand binding domain binds ROR1.
20. A HSPC or non-T effector cell of clause 6 wherein the ligand binding domain is a scFv comprising a CDRL1 sequence of ASGFDFSAYYM (SEQ ID NO: 101), a CDRL2 sequence of TIYPSSG (SEQ ID NO: 112), a CDRL3 sequence of ADRATYFCA (SEQ ID NO: 100), a CDRH1 sequence of DTIDWY (SEQ ID NO: 102), a CDRH2 sequence of VQSDGSYTKRPGVPDR (SEQ ID NO: 113), and a CDRH3 sequence of YIGGYVFG (SEQ ID NO: 117).
21. A HSPC or non-T effector cell of clause 6 wherein the ligand binding domain is a scFv comprising a CDRL1 sequence of SGSDINDYPIS (SEQ ID NO: 109), a CDRL2 sequence of INSGGST (SEQ ID NO: 105), a CDRL3 sequence of YFCARGYS (SEQ ID NO: 116), a CDRH1 sequence of SNLAW (SEQ ID NO: 110, a CDRH2 sequence of RASNLASGVPSRFSGS (SEQ ID NO: 107), and a CDRH3 sequence of NVSYRTSF (SEQ ID NO: 106).
22. A HSPC or non-T effector cell of clause 21 wherein the extracellular component comprises a spacer region of 229 amino acids or less.
23. A HSPC or non-T effector cell of clause 22 wherein the spacer region comprises SEQ ID NO: 61.
24. A HSPC or non-T effector cell of clause 5 wherein the intracellular component comprises an effector domain comprising one or more signaling, stimulatory or co-stimulatory domains selected from: 4-1 BB, B7-H3, CARD11, CD2, CD3γ, CD3δ, CD3ε, CD3ζ, CD7, CD25, CD27, CD28, CD30, CD40, CD79A, CD79B, DAP10, FcRα, FcRβ, FcRγ, Fyn, HVEM, ICOS, LAG3, LAT, Lck, LFA-1, LIGHT, LRP, NKG2C, NKG2D, NOTCH1, NOTCH2, NOTCH3, NOTCH4, pTα, PTCH2, OX40, ROR2, Ryk, SLAMF1, Slp76, TCRα, TCRβ, TRIM, Wnt, and Zap70.
25. A HSPC or non-T effector cell of clause 5 wherein the intracellular component comprises an effector domain comprising an intracellular signaling domain comprising (i) all or a portion of the signaling domain of CD3ζ, (ii) all or a portion of the signaling domain of CD28, (iii) all or a portion of the signaling domain of 4-1BB, or (iv) all or a portion of the signaling domain of CD3ζ, CD28, and/or 4-1BB.
26. A HSPC or non-T effector cell of clause 5 wherein the intracellular component comprises an effector domain comprising a variant of CD3ζ and/or a portion of the 4-1BB intracellular signaling domain.
27. A HSPC or non-T effector cell of clause 1 wherein the extracellular component comprises a spacer region.
28. A HSPC or non-T effector cell of clause 27 wherein the spacer region comprises a portion of a hinge region of a human antibody.
29. A HSPC or non-T effector cell of clause 27 wherein the spacer region comprises a hinge region and at least one other portion of an Fc domain of a human antibody selected from CH1, CH2, CH3, or combinations thereof.
30. A HSPC or non-T effector cell of clause 27 wherein the spacer region comprises a Fc domain and a human IgG4 heavy chain hinge.
31. A HSPC or non-T effector cell of clause 27 wherein the spacer region is of a length selected from 12 amino acids or less, 119 amino acids or less, or 229 amino acids or less.
32. A HSPC or non-T effector cell of clause 27 wherein the spacer region is SEQ ID NO:47, SEQ ID NO:52, or SEQ ID NO:61.
33. A HSPC or non-T effector cell of clause 5 wherein the hydrophobic portion comprises a human transmembrane domain.
34. A HSPC or non-T effector cell of clause 33 wherein the transmembrane domain is a CD28 transmembrane domain, a CD4 transmembrane domain, a CD8 transmembrane domain or a CD27 transmembrane domain.
35. A HSPC or non-T effector cell of clause 1 wherein the extracellular component further includes a tag sequence that binds an endogenous cognate binding molecule (EndoCBM).
36. A HSPC or non-T effector cell of clause 35 wherein the tag sequence is EGFR lacking an intracellular signaling domain.
37. A HSPC or non-T effector cell of clause 1 wherein the chimeric molecule comprises a linker sequence.
38. A HSPC or non-T effector cell of clause 37 wherein the linker sequence comprises a (GlyxSery)n sequence, wherein n is an integer from 1 to 10, and x and y are independently an integer from 0 to 10 provided that x and y are not both 0.
39. A HSPC or non-T effector cell of clause 37 wherein the linker sequence is a CH2CH3 or a CH3.
40. A HSPC or non-T effector cell of clause 37 wherein the linker sequence has an amino acid sequence of Gly-Gly-Gly-Gly-Ser (SEQ ID NO:145), (Gly-Gly-Gly-Gly-Ser)₂ (SEQ ID NO: 122), or (Gly-Gly-Gly-Ser)₂-Gly-Gly-Ser (SEQ ID NO:156).
41. A HSPC or non-T effector cell of clause 1 wherein the chimeric molecule comprises a linker sequence adjacent to one or more tag cassettes, wherein the linker sequence and adjacent tag cassette collectively have an amino acid sequence of (Gly-Gly-Gly-Gly-Ser)₂-Trp-Ser-His-Pro-Gln-Phe-Glu-Lys (SEQ ID NO:139), Trp-Ser-His-Pro-Gln-Phe-Glu-Lys-(Gly-Gly-Gly-Gly-Ser)₂ (SEQ ID NO:140), (Gly-Gly-Gly-Gly-Ser)₂-Trp-Ser-His-Pro-Gln-Phe-Glu-Lys-(Gly-Gly-Gly-Ser)₂-Gly-Gly-Ser-Trp-Ser-His-Pro-Gln-Phe-Glu-Lys (SEQ ID NO:141), Trp-Ser-His-Pro-Gln-Phe-Glu-Lys-(Gly-Gly-Gly-Ser)₂-Gly-Gly-Ser-Trp-Ser-His-Pro-Gln-Phe-Glu-Lys-(Gly-Gly-Gly-Gly-Ser)₂ (SEQ ID NO:142), (Gly-Gly-Gly-Gly-Ser)₂-Trp-Ser-His-Pro-Gln-Phe-Glu-Lys-(Gly-Gly-Gly-Ser)₂-Gly-Gly-Ser-Trp-Ser-His-Pro-Gln-Phe-Glu-Lys-(Gly-Gly-Gly-Gly-Ser)₂-Trp-Ser-His-Pro-Gln-Phe-Glu-Lys (SEQ ID NO:143), or Trp-Ser-His-Pro-Gln-Phe-Glu-Lys-(Gly-Gly-Gly-Gly-Ser)₂-Trp-Ser-His-Pro-Gln-Phe-Glu-Lys-(Gly-Gly-Gly-Ser)₂-Gly-Gly-Ser-Trp-Ser-His-Pro-Gln-Phe-Glu-Lys-(Gly-Gly-Gly-Gly-Ser)₂ (SEQ ID NO:144).
42. A HSPC or non-T effector cell of clause 1 wherein the chimeric molecule comprises from amino-terminus to carboxy-terminus: an extracellular binding domain, a tag cassette, a hinge, a hydrophobic portion, and an intracellular component comprising an effector domain.
43. A HSPC or non-T effector cell of clause 1 wherein the chimeric molecule comprises from amino-terminus to carboxy-terminus: an extracellular binding domain, a first tag cassette, a second tag cassette, a hinge, a hydrophobic portion, and an intracellular component comprising an effector domain.
44. A HSPC or non-T effector cell of clause 1 wherein the chimeric molecule comprises from amino-terminus to carboxy-terminus: an extracellular binding domain, a first tag cassette, a second tag cassette, a third tag cassette, a hinge, a hydrophobic portion, and an intracellular component comprising an effector domain.
45. A HSPC or non-T effector cell of clause 1 wherein the chimeric molecule comprises from amino-terminus to carboxy-terminus: a tag cassette, an extracellular binding domain, a hinge, a hydrophobic portion, and an intracellular component comprising an effector domain.
46. A HSPC or non-T effector cell of clause 1 wherein the chimeric molecule comprises from amino-terminus to carboxy-terminus: an extracellular binding domain, two to five tag cassettes, a hinge, a hydrophobic portion, and an intracellular component comprising an effector domain.
47. A HSPC or non-T effector cell of clause 1 wherein the chimeric molecule comprises from amino-terminus to carboxy-terminus: an extracellular scFv or scTCR binding domain comprising a variable region linker disposed between the variable regions and containing a tag cassette, a hinge, a hydrophobic portion, and an intracellular component comprising an effector domain.
48. A HSPC or non-T effector cell of clause 1 wherein the chimeric molecule comprises from amino-terminus to carboxy-terminus: an extracellular scFv or scTCR binding domain, a tag cassette, an IgG hinge, a transmembrane domain, and an intracellular component comprising an effector domain, wherein the effector domain comprises 4-1BB and CD3ζ, CD27 and CD3ζ, CD28 and CD3ζ, OX40 and CD3ζ, CD28, 4-1BB and CD3ζ, OX40, 4-1BB and CD3ζ, or CD28, OX40 and CD3ζ.
49. A HSPC or non-T effector cell of clause 1 wherein the chimeric molecule comprises from amino-terminus to carboxy-terminus: an extracellular binding domain comprising a receptor ectodomain, a tag cassette, a hinge, a hydrophobic portion, and an intracellular component comprising an effector domain, wherein the effector domain comprises 4-1BB, CD27, CD28, or OX40.
50. A HSPC or non-T effector cell of clause 1 wherein the chimeric molecule further comprises a cytotoxic, radioisotope, radiometal, or detectable agent.
51. A HSPC or non-T effector cell of clause 1 wherein the extracellular component further comprises a cytotoxic, radioisotope, radiometal, or detectable agent.
52. A HSPC or non-T effector cell of clause 1 wherein the HSPC is CD34⁺ HSPC and/or the non-T effector cell is a natural killer cell.
53. A composition comprising a pharmaceutically acceptable carrier and a genetically modified HSPC or non-T effector cell of any one of clauses 1-52.
54. A composition of clause 53 further comprising an ExoCBM that specifically binds a tag cassette expressed by the HSPC or non-T effector cell within the composition.
55. A composition of clause 53 further comprising an EndoCBM that specifically binds a stimulatory molecule expressed by the HSPC or non-T effector cell within the composition.
56. A composition of clause 53 further comprising an ExoCBM that specifically binds a tag cassette expressed by the HSPC or non-T effector cell within the composition and an EndoCBM that specifically binds a stimulatory molecule expressed by the HSPC or non-T effector cell within the composition.
57. A composition of clause 53 formulated for infusion or injection.
58. A formulation comprising a pharmaceutically acceptable carrier and a genetically modified HSPC and non-T effector cell of any one of clauses 1-52.
59. A formulation of clause 58 further comprising an ExoCBM that specifically binds a tag cassette expressed by the HSPC and/or non-T effector cell within the composition.
60. A formulation of clause 58 further comprising an EndoCBM that specifically binds a stimulatory molecule expressed by the HSPC and/or non-T effector cell within the composition.
61. A formulation of clause 58 further comprising an ExoCBM that specifically binds a tag cassette expressed by the HSPC and/or non-T effector cell within the composition and an EndoCBM that specifically binds a stimulatory molecule expressed by the HSPC and/or non-T effector cell within the composition.
62. A formulation of clause 58 formulated for infusion or injection.
63. A composition comprising an ExoCBM that specifically binds a tag cassette expressed by a HSPC or non-T effector cell of any one of clauses 1-52.
64. A composition of clause 63 further comprising an EndoCBM that specifically binds a stimulatory molecule expressed by the HSPC or non-T effector cell.
65. A method for activating a HSPC or non-T effector cell of any one of clauses 1-52 comprising contacting the HSPC or non-T effector cell with an ExoCBM that specifically binds a tag cassette expressed by the HSPC or non-T effector cell thereby activating the HSPC or non-T effector cell.
66. A method of clause 65 further comprising contacting the HSPC or non-T effector cell with an EndoCBM that specifically binds a stimulatory molecule expressed by the HSPC or non-T effector cell.
67. A method of clause 65 wherein the EndoCBM is selected from a Notch agonist, an angiopoietin-like protein, erythropoietin, fibroblast growth factor-1 (FGF-1); Flt-3 ligand (Flt-3L); granulocyte colony stimulating factor (G-CSF); granulocyte-macrophage colony stimulating factor (GM-CSF); insulin growth factor-2 (IFG-2); interleukin-3 (IL-3); interleukin-6 (IL-6); interleukin-7 (IL-7); interleukin-11 (IL-11); stem cell factor (SCF); and thrombopoietin (TPO).
68. A method of clause 67 wherein the EndoCBM is SCF, Flt-3L, TPO, IL-6 and IL-3.
69. A method of clause 65 wherein the ExoCBM is a cognate receptor, an anti-tag antibody, and/or an anti-tag scFv.
70. A method of clause 65 wherein the tag cassette is a Strep tag having amino acid sequence Trp-Ser-His-Pro-Gln-Phe-Glu-Lys (SEQ ID NO:118) or Trp-Arg-His-Pro-Gln-Phe-Gly-Gly (SEQ ID NO:137).
71. A method of clause 65 wherein the ExoCBM that specifically binds the tag cassette is a biotin binding protein or an anti-Strep tag antibody.
72. A method of clause 65 wherein the ExoCBM is attached to a solid surface.
73. A method of clause 65 wherein the ExoCBM is attached to a planar surface, agarose, resin, 3D fabric matrix, or a bead.
74. A method of clause 65 wherein the ExoCBM is attached to a microbead or a nanobead.
75. A method of clause 65 wherein the activating is performed *in vitro, in vivo* or ex *vivo.*
76. A method for promoting proliferation of a HSPC or non-T effector cell of any one of clauses 1-52 comprising contacting the HSPC or non-T effector cell with (i) an ExoCBM that specifically binds a tag cassette expressed by the HSPC or non-T effector cell and (ii) an EndoCBM that specifically binds a stimulatory molecule expressed by the HSPC or non-T effector cell for a time sufficient to promote HSPC or non-T effector cell growth.
77. A method of clause 76 wherein the EndoCBM is selected from a Notch agonist, an angiopoietin-like protein, erythropoietin, fibroblast growth factor-1 (FGF-1); Flt-3 ligand (Flt-3L); granulocyte colony stimulating factor (G-CSF); granulocyte-macrophage colony stimulating factor (GM-CSF); insulin growth factor-2 (IFG-2); interleukin-3 (IL-3); interleukin-6 (IL-6); interleukin-7 (IL-7); interleukin-11 (IL-11); stem cell factor (SCF); and thrombopoietin (TPO).
78. A method of clause 77 wherein the EndoCBM is SCF, Flt-3L, TPO, IL-6 and IL-3.
79. A method of clause 76 wherein the ExoCBM is a cognate receptor, an anti-tag antibody, and/or an anti-tag scFv.
80. A method of clause 76 wherein the tag cassette is a Strep tag having amino acid sequence Trp-Ser-His-Pro-Gln-Phe-Glu-Lys (SEQ ID NO:118) or Trp-Arg-His-Pro-Gln-Phe-Gly-Gly (SEQ ID NO:137).
81. A method of clause 76 wherein the ExoCBM that specifically binds the tag cassette is a biotin binding protein or an anti-Strep tag antibody.
82. A method of clause 76 wherein the ExoCBM is attached to a solid surface.
83. A method of clause 76 wherein the ExoCBM is attached to a planar surface, agarose, resin, 3D fabric matrix, or a bead.
84. A method of clause 76 wherein the ExoCBM is attached to a microbead or a nanobead.
85. A method of clause 76 wherein the activating is performed *in vitro, in vivo* or ex *vivo.*
86. A method for detecting a HSPC or non-T effector cell comprising:
   contacting a sample comprising a HSPC or non-T effector cell of any one of clauses 1-52 with an ExoCBM that specifically binds a tag cassette expressed by the HSPC or non-T effector cell,
   wherein the ExoCBM comprises a detectable moiety, and
   detecting the presence of the HSPC or non-T effector cell in the sample based on the specific binding of the ExoCBM comprising the detectable moiety.
87. A method of clause 86 wherein the ExoCBM is a cognate receptor, an anti-tag antibody, and/or an anti-tag scFv.
88. A method of clause 86 wherein the tag cassette is a Strep tag having amino acid sequence Trp-Ser-His-Pro-Gln-Phe-Glu-Lys (SEQ ID NO:118) or Trp-Arg-His-Pro-Gln-Phe-Gly-Gly (SEQ ID NO:137).
89. A method of clause 86 wherein the ExoCBM that specifically binds the tag cassette is a biotin binding protein or an anti-Strep tag antibody.
90. A method of clause 86 wherein the ExoCBM is attached to a solid surface.
91. A method of clause 86 wherein the ExoCBM is attached to a planar surface, agarose, resin, 3D fabric matrix, or a bead.
92. A method of clause 86 wherein the ExoCBM is attached to a microbead or a nanobead.
93. A method of clause 86 wherein the detecting is performed *in vitro, in vivo* or ex *vivo.*
94. A method of clause 86 wherein the detectable moiety is fluorescent marker.
95. A method of clause 86 wherein the detectable moiety is APC, PE, Pacific blue, Alex fluor, or FITC.
96. A method of clause 86 wherein detection occurs using flow cytometry.
97. A method for enriching for or isolating a HSPC or non-T effector cell of any of clauses 1-52 comprising contacting a sample comprising a HSPC or non-T effector cell with an ExoCBM that specifically binds a tag cassette expressed by the HSPC or non-T effector cell and enriching for or isolating the HSPC or non-T effector cell away from other cells not expressing the tag cassette in the sample.
98. A method of clause 97 wherein the ExoCBM is a cognate receptor, an anti-tag antibody, and/or an anti-tag scFv.
99. A method of clause 97 wherein the tag cassette is a Strep tag having amino acid sequence Trp-Ser-His-Pro-Gln-Phe-Glu-Lys (SEQ ID NO:118) or Trp-Arg-His-Pro-Gln-Phe-Gly-Gly (SEQ ID NO:137).
100. A method of clause 97 wherein the ExoCBM that specifically binds the tag cassette is a biotin binding protein or an anti-Strep tag antibody.
101. A method of clause 97 wherein the ExoCBM is attached to a solid surface.
102. A method of clause 97 wherein the ExoCBM is attached to a planar surface, agarose, resin, 3D fabric matrix, or a bead.
103. A method of clause 97 wherein the ExoCBM is attached to a microbead or a nanobead.
104. A method of clause 97 wherein the HSPC or non-T effector cell is enriched for or isolated by magnetic column chromatography.
105. A method of clause 97 comprising detecting the enriched for or isolated HSPC or non-T effector cells by contacting the HSPC or non-T effector cells with an ExoCBM that specifically binds the tag cassette expressed by the enriched or isolated HSPC or non-T effector cells wherein the ExoCBM comprises a detectable moiety and detecting the presence of the HSPC or non-T effector cell in the sample based on the specific binding of the ExoCBM comprising the detectable moiety.
106. A method of clause 105 wherein the detectable moiety is fluorescent marker.
107. A method of clause 105 wherein the detectable moiety is APC, PE, Pacific blue, Alex fluor, or FITC.
108. A method of clause 105 wherein the detection occurs using flow cytometry.
109. A method for depleting or eliminating a HSPC or non-T effector cell of any of clauses 1-52 comprising contacting a sample comprising the HSPC or non-T effector cell with an ExoCBM that specifically binds a tag cassette expressed by the HSPC or non-T effector cells, wherein binding of the ExoCBM to the tag cassette leads to cell death of the HSPC or non-T effector cells expressing the tag cassette.
110. A method of clause 109 wherein the ExoCBM comprises a bispecific binding domain, wherein a first binding domain is specific for the tag cassette and the second binding domain is specific for CD3.
111. A method of clause 109 wherein the ExoCBM comprises a cytotoxic, radioisotope, or radiometal agent.
112. A method of clause 109 wherein the ExoCBM comprises a cognate receptor, an anti-tag antibody, an anti-tag scFv, or a cell with an anti-tag binding domain on its cell surface.
113. A method of clause 109 wherein the tag cassette is a Strep tag having amino acid sequence Trp-Ser-His-Pro-Gln-Phe-Glu-Lys (SEQ ID NO:118) or Trp-Arg-His-Pro-Gln-Phe-Gly-Gly (SEQ ID NO:137).
114. A method of clause 109 wherein the ExoCBM that specifically binds the tag cassette is a biotin binding protein or an anti-Strep tag antibody.
115. A method of clause 109 wherein the ExoCBM is attached to a solid surface.
116. A method of clause 109 wherein the ExoCBM is attached to a planar surface, agarose, resin, 3D fabric matrix, or a bead.
117. A method of clause 109 wherein the ExoCBM is attached to a microbead or a nanobead.
118. A method of tracking administered HSPC or non-T effector cells of any of clauses 1-52 comprising administering to a subject an ExoCBM that specifically binds a tag cassette expressed by the HSPC or non-T effector cells wherein
   the ExoCBM comprises a detectable moiety, and
   detecting the presence of the HSPC or non-T effector cell within the subject based on the specific binding of the ExoCBM comprising the detectable moiety.
119. A method of clause 118 wherein the HSPC or non-T effector cells and the ExoCBM are administered simultaneously.
120. A method of clause 118 wherein HSPC or non-T effector cells and the ExoCBM are administered as a composition or formulation.
121. A method of clause 118 wherein the ExoCBM is a cognate receptor, an anti-tag antibody, and/or an anti-tag scFv.
122. A method of clause 118 wherein the tag cassette is a Strep tag having amino acid sequence Trp-Ser-His-Pro-Gln-Phe-Glu-Lys (SEQ ID NO:118) or Trp-Arg-His-Pro-Gln-Phe-Gly-Gly (SEQ ID NO:137).
123. A method of clause 118 wherein the ExoCBM that specifically binds the tag cassette is a biotin binding protein or an anti-Strep tag antibody.
124. A method of clause 118 wherein the ExoCBM is attached to a solid surface.
125. A method of clause 118 wherein the ExoCBM is attached to a planar surface, an agarose bead, a resin, a 3D fabric matrix, or a bead.
126. A method of clause 118 wherein the ExoCBM is attached to a microbead or a nanobead.
127. A method of clause 118 wherein the detectable moiety comprises a fluorescent marker.
128. A method of clause 118 wherein the detectable moiety comprises a APC, PE, Pacific blue, Alex fluor, or FITC.
129. A method of clause 118 wherein the detectable moiety comprises a magnetic particle, superparamagnetic iron oxide (SPIO), fluorodeoxyglucose (18F), fluorescent compounds, or any combination thereof.
130. A method of clause 118 wherein the tracking comprises use of MRI, PET, or near infrared imaging.
131. A method for activating administered HSPC or non-T effector cells of any of clauses 1-52 comprising administering to a subject (i) an ExoCBM that specifically binds a tag cassette expressed by the HSPC or non-T effector cell; (ii) an EndoCBM that specifically binds a stimulatory molecule expressed by the HSPC or non-T effector cell; wherein specific binding of the ExoCBM and the EndoCBM activates the HSPC or non-T effector cell *in vivo.*
132. A method of clause 131 wherein the EndoCBM is selected from a Notch agonist, an angiopoietin-like protein, erythropoietin, fibroblast growth factor-1 (FGF-1); Flt-3 ligand (Flt-3L); granulocyte colony stimulating factor (G-CSF); granulocyte-macrophage colony stimulating factor (GM-CSF); insulin growth factor-2 (IFG-2); interleukin-3 (IL-3); interleukin-6 (IL-6); interleukin-7 (IL-7); interleukin-11 (IL-11); stem cell factor (SCF); and thrombopoietin (TPO).
133. A method of clause 132 wherein the EndoCBM is SCF, Flt-3L, TPO, IL-6 and IL-3.
134. A method of clause 131 wherein the HSPC or non-T effector cells, the ExoCBM, and the EndoCBM are administered simultaneously.
135. A method of clause 131 wherein HSPC or non-T effector cells, the ExoCBM, and the EndoCBM are administered as a composition or formulation.
136. A method of clause 131 wherein the ExoCBM is a cognate receptor, an anti-tag antibody, and/or an anti-tag scFv.
137. A method of clause 131 wherein the tag cassette is a Strep tag having amino acid sequence Trp-Ser-His-Pro-Gln-Phe-Glu-Lys (SEQ ID NO:118) or Trp-Arg-His-Pro-Gln-Phe-Gly-Gly (SEQ ID NO:137).
138. A method of clause 131 wherein the ExoCBM that specifically binds the tag cassette is a biotin binding protein or an anti-Strep tag antibody.
139. A method of depleting administered HSPC or non-T effector cells of any of clauses 1-52 comprising administering an ExoCBM that specifically binds a tag cassette expressed by the administered HSPC or non-T effector cells, wherein binding of the ExoCBM to the tag cassette leads to cell death of the HSPC or non-T effector cells expressing the tag cassette
140. A method of clause 139 wherein the ExoCBM comprises a bispecific binding domain, wherein a first binding domain is specific for the tag cassette and the second binding domain is specific for CD3.
141. A method of clause 139 wherein the ExoCBM comprises a cytotoxic, radioisotope, or radiometal agent.
142. A method of clause 139 wherein the ExoCBM comprises a cognate receptor, an anti-tag antibody, an anti-tag scFv, or a cell with an anti-tag binding domain on its cell surface.
143. A method of clause 139 wherein the tag cassette is a Strep tag having amino acid sequence Trp-Ser-His-Pro-Gln-Phe-Glu-Lys (SEQ ID NO:118) or Trp-Arg-His-Pro-Gln-Phe-Gly-Gly (SEQ ID NO:137).
144. A method of clause 139 wherein the ExoCBM that specifically binds the tag cassette is a biotin binding protein or an anti-Strep tag antibody.
145. A method of clause 139 wherein the ExoCBM is attached to a solid surface.
146. A method of clause 139 wherein the ExoCBM is attached to a planar surface, agarose, resin, 3D fabric matrix, or a bead.
147. A method of clause 139 wherein the ExoCBM is attached to a microbead or a nanobead.
148. A method of treating a condition in a subject, comprising administering a therapeutically-effective amount of an HSPC or non-T effector cell of any one of clauses 1-52, a therapeutically effective amount of a composition of any one of clauses 53-57, 63 or 64 or a therapeutically effective amount of a formulation of any one of clauses 58-62 to the subject, thereby treating the condition in the subject.
149. A method of clause 148 wherein immunological matching to the subject is not required before the administering.
150. A method of clause 148 wherein the subject is a relapsed pediatric acute lymphoblastic leukemia patient.
151. A method of clause 148 wherein the method further comprises monitoring cytokine levels in the subject after administering the ExoCBM that specifically binds the tag cassette.
152. A method of clause 148 wherein the condition is immunodeficiency, pancytopenia, neutropenia, and/or leukopenia.
153. A method of clause 152 wherein the immunodeficiency, pancytopenia, neutropenia, and/or leukopenia is due to chemotherapy, radiation therapy, and/or a myeloablative regimen for HCT and/or acute ionizing radiation.
154. A method of clause 148 wherein the condition is a depleted immune system.
155. A method of clause 154 wherein the depleted immune system arose due to a viral infection, microbial infection, parasitic infection, renal disease, and/or renal failure.
156. A method of clause 154 wherein the depleted immune system arose due to exposure to drugs that cause bone marrow suppression or hematopoietic deficiencies.
157. A method of clause 154 wherein the depleted immune system arose due to exposure to penicillin, gancyclovir, daunomycin, meprobamate, aminopyrine, dipyrone, phenytoin, carbamazepine, propylthiouracil, and/or methimazole.
158. A method of clause 154 wherein the depleted immune system arose due to exposure to dialysis.
159. A method of clause 148 further comprising administering non-genetically-modified HSPC to the subject.
160. A method of clause 148 further comprising activating, tracking or depleting the administered HSPC and/or non-T effector cells according to any of the methods of clauses 118-147.
161. A method of repopulating an immune system in a subject in need thereof comprising administering a therapeutically-effective amount of an HSPC or non-T effector cell of any one of clauses 1-52, a therapeutically effective amount of a composition of any one of clauses 53-57, 63 or 64 or a therapeutically effective amount of a formulation of any one of clauses 58-62 to the subject, thereby repopulating the immune system of the subject.
162. A method of clause 161 wherein immunological matching to the subject is not required before the administering.
163. A method of clause 161 further comprising targeting cancer cells expressing a cellular marker in the subject by administering a therapeutically effective amount of genetically modified HSPC and/or genetically modified non-T effector cells of any one of clauses 1-52 to the subject thereby targeting the cancer cells.
164. A method of clause 163 wherein the cancer cells are from an adrenal cancer, a bladder cancer, a blood cancer, a bone cancer, a brain cancer, a breast cancer, a carcinoma, a cervical cancer, a colon cancer, a colorectal cancer, a corpus uterine cancer, an ear, nose and throat (ENT) cancer, an endometrial cancer, an esophageal cancer, a gastrointestinal cancer, a head and neck cancer, a Hodgkin's disease, an intestinal cancer, a kidney cancer, a larynx cancer, a leukemia, a liver cancer, a lymph node cancer, a lymphoma, a lung cancer, a melanoma, a mesothelioma, a myeloma, a nasopharynx cancer, a neuroblastoma, a non-Hodgkin's lymphoma, an oral cancer, an ovarian cancer, a pancreatic cancer, a penile cancer, a pharynx cancer, a prostate cancer, a rectal cancer, a sarcoma, a seminoma, a skin cancer, a stomach cancer, a teratoma, a testicular cancer, a thyroid cancer, a uterine cancer, a vaginal cancer, a vascular tumor, and/or a metastasis thereof.
165. A method of clause 163 wherein the cellular marker(s) of the cancer cells are selected from A33; BAGE; Bcl-2; β-catenin; B7H4; BTLA; CA125; CA19-9; CD5; CD19; CD20; CD21; CD22; CD33; CD37; CD44v6; CD45; CD123; CEA; CEACAM6; c-Met; CS-1; cyclin B1; DAGE; EBNA; EGFR; ephrinB2; ErbB2; ErbB3; ErbB4; EphA2; estrogen receptor; FAP; ferritin; α-fetoprotein (AFP); FLT1; FLT4; folate-binding protein; Frizzled; GAGE; G250; GD-2; GHRHR; GHR; GM2; gp75; gp100 (Pmel 17); gp130; HLA; HER-2/neu; HPV E6; HPV E7; hTERT; HVEM; IGF1R; IL6R; KDR; Ki-67; LIFRβ; LRP; LRP5; LTβR; mesothelin; OSMRβ; p53; PD1; PD-L1; PD-L2; PRAME; progesterone receptor; PSA; PSMA; PTCH1; MAGE; MART; mesothelin; MUC; MUC1; MUM-1-B; myc; NYESO-1; RANK; ras; Robo1; RORI; survivin; TCRα; TCRβ; tenascin; TGFBR1; TGFBR2;TLR7; TLR9; TNFR1; TNFR2; TNFRSF4; TWEAK-R; TSTA tyrosinase; VEGF; and WT1.
166. A method of clause 163 wherein the cancer is leukemia/lymphoma and the cellular marker(s) are one or more of CD19, CD20, CD22, ROR1, CD33, and WT-1; wherein the cancer is multiple myeloma and the cellular marker is BCMA; wherein the cancer is prostate cancer and the cellular marker(s) are one or more of PSMA, WT1, PSCA, and SV40 T; wherein the cancer is breast cancer and the cellular marker(s) are one or more of HER2, ERBB2, and ROR1; wherein the cancer is stem cell cancer and the cellular marker is CD133; wherein the cancer is ovarian cancer and the cellular marker(s) are one or more of L1-CAM, MUC-CD, folate receptor, Lewis Y, ROR1, mesothelin, and WT-1; wherein the cancer is mesothelioma and the cellular marker is mesothelin; wherein the cancer is renal cell carcinoma and the cellular marker is CAIX; wherein the cancer is melanoma and the cellular marker is GD2; wherein the cancer is pancreatic cancer and the cellular marker(s) are one or more of mesothelin, CEA, CD24, and ROR1; or wherein the cancer is lung cancer and the cellular marker is ROR1.
167. A method of clause 163 wherein the cancer cells are acute lymphoblastic leukemia cells expressing CD19.
168. A method of clause 163 wherein the cancer is acute lymphoblastic leukemia and the subject is a pediatric patient.
169. A method of clause 163 further comprising activating, tracking or depleting the administered HSPC and/or non-T effector cells according to any of the methods of clauses 118-147.
170. A method of targeting cells preferentially expressing CD19 for destruction comprising administering to a subject in need thereof a therapeutically effective amount of genetically modified HSPC and/or genetically modified non-T effector cells wherein the genetically modified cells express (i) an extracellular component comprising at least one tag cassette and a CD19 ligand binding domain, and (ii) an intracellular component including an effector domain thereby targeting and destroying cells preferentially expressing CD19.
171. A method of clause 170 wherein immunological matching to the subject is not required before the administering.
172. A method of clause 170 wherein the cells preferentially expressing CD19 are acute lymphoblastic leukemia cells.
173. A method of clause 170 wherein the subject is a relapsed pediatric acute lymphoblastic leukemia patient.
174. A method of clause 170 wherein the at least one tag cassette is or comprises a Strep tag, His tag, Flag tag, Xpress tag, Avi tag, Calmodulin tag, Polyglutamate tag, HA tag, Myc tag, Nus tag, S tag, X tag, SBP tag, Softag, V5 tag, CBP, GST, MBP, GFP, Thioredoxin tag, or any combination thereof.
175. A method of clause 170 wherein at least one tag cassette is or comprises a Strep tag comprising the amino acid sequence Trp-Ser-His-Pro-Gln-Phe-Glu-Lys (SEQ ID NO:118) or Trp-Arg-His-Pro-Gln-Phe-Gly-Gly (SEQ ID NO:137).
176. A method of clause 170 further including treating immunodeficiency, pancytopenia, neutropenia, and/or leukopenia in the subject by administering a therapeutically effective amount of HSPC to the subject.
177. A method of clause 176 wherein the immunodeficiency, pancytopenia, neutropenia, and/or leukopenia is due to chemotherapy, radiation therapy, and/or a myeloablative regimen for HCT.
178. A method of clause 170 further comprising activating, tracking or depleting the administered HSPC and/or non-T effector cells according to any of the methods of clauses 118-147.
179. A method of targeting cancer cells in a subject comprising identifying at least one cellular marker preferentially expressed on a cancer cell from the subject; administering a therapeutically-effective amount of an HSPC or non-T effector cell of any one of clauses 1-52, a therapeutically effective amount of a composition of any one of clauses 53-57, 63 or 64 or a therapeutically effective amount of a formulation of any one of clauses 58-62to the subject based on the identified at least one cellular marker.
180. A kit comprising the compositions of any one of clauses 53-57, 63 or 64 wherein the kit comprises instructions advising that the compositions can be administered to a subject without immunological matching.
181. A kit comprising the formulations of any one of clauses 58-62 wherein the kit comprises instructions advising that the formulations can be administered to a subject without immunological matching.
182. A kit comprising the compositions of any one of clauses 53-57, 63 or 64 and the formulations of clause any one of clauses 58-62 wherein the kit comprises instructions advising that the compositions or formulations can be administered to a subject without immunological matching.

## Claims

1. A composition comprising a hematopoietic stem progenitor cell (HSPC) or non-T effector cell genetically modified to express a chimeric molecule comprising an extracellular component comprising a tag cassette that specifically binds an exogenous cognate binding molecule (ExoCBM), wherein the composition further comprises one or both of:
(i) an ExoCBM that specifically binds the tag cassette; and
(ii) an endogenous cognate binding molecule (EndoCBM) that specifically binds a stimulatory molecule expressed by the HSPC or non-T effector cell,
and wherein, optionally, the HSPC is CD34⁺ and/or the non-T effector cell is a natural killer cell.

2. The composition of claim 1, wherein the EndoCBM is selected from a Notch agonist, an angiopoietin-like protein, erythropoietin, fibroblast growth factor-1 (FGF-1); Flt-3 ligand (Flt-3L); granulocyte colony stimulating factor (G-CSF); granulocyte-macrophage colony stimulating factor (GM-CSF); insulin growth factor-2 (IFG-2); interleukin-3 (IL-3); interleukin-6 (IL-6); interleukin-7 (IL-7); interleukin-11 (IL-11); stem cell factor (SCF); and thrombopoietin (TPO).

3. The composition of claim 1 or 2, comprising: (a) SCF and: GM-CSF or IL-7; (b) a retinoic acid receptor (RAR) agonist, preferably all trans retinoic acid (ATRA); or (c) RPMI media supplemented with human serum, IL-2 at 50 U/mL, IL-15 at 500ng/mL, and, optionally, L-glutamine

4. The composition of any one of claims 1-3, wherein:
(i) the extracellular component of the chimeric molecule has one, two, three, four, or five tag cassettes; and/or
(ii) at least one tag cassette is or comprises a Strep tag, His tag, Flag tag, Xpress tag, Avi tag, Calmodulin tag, Polyglutamate tag, HA tag, Myc tag, Nus tag, S tag, X tag, SBP tag, Softag, V5 tag, CBP, GST, MBP, GFP, Thioredoxin tag, or any combination thereof, wherein, optionally, at least one tag cassette is or comprises a Strep tag comprising the amino acid sequence Trp-Ser-His-Pro-Gln-Phe-Glu-Lys (SEQ ID NO:118) or Trp-Arg-His-Pro-Gln-Phe-Gly-Gly (SEQ ID NO:137).

5. The composition of any one of claims 1-4, wherein the extracellular component is linked to an intracellular component through a hydrophobic portion, wherein the extracellular component comprises (a) a binding domain that specifically binds a cellular marker, and (b) a hinge; and wherein the intracellular component comprises an effector domain, wherein, optionally:
(i) at least one tag cassette is located amino-terminal to the binding domain or carboxy-terminal to the binding domain;
(ii) the HSPC or non-T effector cell comprises 2 or more extracellular tag cassettes wherein the tag cassettes are located amino-terminal to the binding domain, carboxy-terminal to the binding domain, or at least one tag cassette is located amino-terminal to the binding domain and at least one tag cassette is located carboxy-terminal to the binding domain;
(iii) the binding domain comprises one or more tag cassettes; or
(iv) the binding domain is a scFv, scTCR, receptor ectodomain, or ligand, wherein, optionally, the scFv or scTCR comprises a variable region linker comprising one or more tag cassettes.

6. The composition of claim 5, wherein the cellular marker comprises CD3, CEACAM6, c-Met, EGFR, EGFRvIII, ErbB2, ErbB3, ErbB4, EphA2, IGF1R, GD2, O-acetyl GD2, O-acetyl GD3, GHRHR, GHR, FLT1, KDR, FLT4, CD44v6, CD151, CA125, CEA, CTLA-4, GITR, BTLA, TGFBR2, TGFBR1, IL6R, gp130, Lewis A, Lewis Y, TNFR1, TNFR2, PD1, PD-L1, PD-L2, HVEM, MAGE-A, mesothelin, NY-ESO-1, PSMA, RANK, ROR1, TNFRSF4, CD40, CD137, TWEAK-R, HLA, tumor or pathogen associated peptide bound to HLA, hTERT peptide bound to HLA, tyrosinase peptide bound to HLA, WT-1 peptide bound to HLA, LTR, LIFR, LRP5, MUC1, OSMR, TCRα, TCRβ, CD19, CD20, CD22, CD25, CD28, CD30, CD33, CD52, CD56, CD80, CD81, CD86, CD123, CD171, CD276, B7H4, TLR7, TLR9, PTCH1, WT-1, Robol, α-fetoprotein (AFP), Frizzled, OX40, or CD79b.

7. The composition of claim 5 or 6, wherein:
(i) the intracellular component comprises an effector domain comprising one or more signaling, stimulatory or co-stimulatory domains selected from: 4-1BB, B7-H3, CARD11, CD2, CD3γ, CD3δ, CD3ε, CD3ζ, CD7, CD25, CD27, CD28, CD30, CD40, CD79A, CD79B, DAP10, FcRα, FcRβ, FcRγ, Fyn, HVEM, ICOS, LAG3, LAT, Lck, LFA-1, LIGHT, LRP, NKG2C, NKG2D, NOTCH1, NOTCH2, NOTCH3, NOTCH4, pTα, PTCH2, OX40, ROR2, Ryk, SLAMF1, Slp76, TCRα, TCRβ, TRIM, Wnt, and Zap70;
(ii) the intracellular component comprises an effector domain comprising an intracellular signaling domain comprising (a) all or a portion of the signaling domain of CD3ζ, (b) all or a portion of the signaling domain of CD28, (c) all or a portion of the signaling domain of 4-1BB, or (d) all or a portion of the signaling domain of CD3ζ, CD28, and/or 4-1BB; or
(iii) the intracellular component comprises an effector domain comprising a variant of CD3ζ and/or a portion of the 4-1BB intracellular signaling domain.

8. The composition of any one of claims 1-7, wherein the extracellular component further includes a tag sequence that binds an EndoCBM, wherein, optionally, the tag sequence is EGFR lacking an intracellular signaling domain.

9. A hematopoietic stem progenitor cell (HSPC) or non-T effector cell genetically modified to express a chimeric molecule comprising an extracellular component comprising (i) a tag cassette that specifically binds an exogenous cognate binding molecule (ExoCBM) and (ii) a tag sequence that specifically binds an endogenous cognate binding molecule (EndoCBM), wherein, optionally, the HSPC is CD34+ and/or the non-T effector cell is a natural killer cell.

10. The HSPC or non-T effector cell of claim 9, wherein the tag sequence is EGFR lacking an intracellular signaling domain.

11. The HSPC or non-T effector cell of claim 9 or 10, wherein the EndoCBM is elected from a Notch agonist, an angiopoietin-like protein, erythropoietin, fibroblast growth factor-1 (FGF-1 ); Flt-3 ligand (Flt-3L); granulocyte colony stimulating factor (G-CSF); granulocyte-macrophage colony stimulating factor (GM-CSF); insulin growth factor-2 (IFG-2); interleukin-3 (IL-3); interleukin-6 (IL-6); interleukin-7 (IL-7); interleukin-1 1 (IL-11 ); stem cell factor (SCF); and thrombopoietin (TPO).

12. The HSPC or non-T effector cell of any one of claims 9-11, wherein:
(i) the extracellular component of the chimeric molecule of has one, two, three, four, or five tag cassettes; and/or
(ii) the at least one tag cassette is or comprises a Strep tag, His tag, Flag tag, Xpress tag, Avi tag, Calmodulin tag, Polyglutamate tag, HA tag, Myc tag, Nus tag, S tag, X tag, SBP tag, Softag, V5 tag, CBP, GST, MBP, GFP, Thioredoxin tag, or any combination thereof, wherein, optionally, at least one tag cassette is or comprises a Strep tag comprising the amino acid sequence Trp-Ser-His-Pro-Gln-Phe-Glu-Lys (SEQ ID NO: 118) or Trp-Arg-His-Pro-Gln-Phe-Gly-Gly (SEQ ID NO: 137).

13. The HSPC or non-T effector cell of any one of claims 9-12, wherein the extracellular component is linked to an intracellular component through a hydrophobic portion, wherein the extracellular component comprises (a) a binding domain that specifically binds a cellular marker, and (b) a hinge; and wherein the intracellular component comprises an effector domain, wherein, optionally:
(i) at least one tag cassette is located amino-terminal to the binding domain or carboxy-terminal to the binding domain;
(ii) the HSPC or non-T effector cell comprises 2 or more extracellular tag cassettes wherein the tag cassettes are located amino-terminal to the binding domain, carboxy-terminal to the binding domain, or at least one tag cassette is located amino-terminal to the binding domain and at least one tag cassette is located carboxy-terminal to the binding domain;
(iii) the binding domain comprises one or more tag cassettes; or
(iv) the binding domain is a scFv, scTCR, receptor ectodomain, or ligand, wherein, optionally, the scFv or scTCR comprises a variable region linker comprising one or more tag cassettes.

14. The HSPC or non-T effector cell of claim 13, wherein:
(i) the intracellular component comprises an effector domain comprising one or more signaling, stimulatory or co-stimulatory domains selected from: 4-1BB, B7-H3, CARD11, CD2, CD3γ, CD3δ, CD3ε, CD3ζ, CD7, CD25, CD27, CD28, CD30, CD40, CD79A, CD79B, DAP10, FcRα, FcR, FcRγ, Fyn, HVEM, ICOS, LAG3, LAT, Lck, LFA-1, LIGHT, LRP, NKG2C, NKG2D, NOTCH 1, NOTCH2, NOTCH3, NOTCH4, pTα, PTCH2, OX40, ROR2, Ryk, SLAMF1, Slp76, TCRα, TCRβ, TRIM, Wnt, and Zap70;
(ii) the intracellular component comprises an effector domain comprising an intracellular signaling domain comprising (a) all or a portion of the signaling domain of CD3ζ, (b) all or a portion of the signaling domain of CD28, (c) all or a portion of the signaling domain of 4-1BB, or (d) all or a portion of the signaling domain of CD3ζ, CD28, and/or 4-1BB; or
(iii) the intracellular component comprises an effector domain comprising a variant of CD3ζ and/or a portion of the 4-1BB intracellular signaling domain.

15. A method for activating the HSPC or non-T effector cell of any one of claims 9-14, comprising contacting the HSPC or non-T effector cell with an ExoCBM that specifically binds the tag cassette expressed by the HSPC or non-T effector cell, and optionally further comprising contacting the HSPC or non-T effector cell with an EndoCBM that specifically binds a stimulatory molecule expressed by the HSPC or non-T effector cell,
thereby activating the HSPC or non-T effector cell.

16. A method of treating a condition in a subject, including administering a therapeutically effective amount of an HSPC or non-T effector cell of any one of claims 9-15 to the subject and optionally further administering non-genetically-modified HPSC to the subject, thereby treating the subject.
